# NEW EUROPEAN PATENT SPECIFICATION

(11) **EP 3 366 775 B2**
(45) Date of publication and mention of the opposition decision: **06.08.2025**
(45) Mention of the grant of the patent: 27.04.2022
(21) Application number: 17209680.2
(22) Date of filing: 16.11.2012
(51) Int. Cl.: C12N 15/113, A61K 31/7125

(54) **MODIFIED RNAI AGENTS**
MODIFIZIERTE RNAI-MITTEL
AGENTS D'ARNI MODIFIÉS

(30) Priority: 18.11.2011 US 201161561710 P
(43) Date of publication of application: 29.08.2018
(62) Divisional of application: 12795962.5
(73) Proprietor: Alnylam Pharmaceuticals, Inc., Cambridge, MA 02142 (US)
(72) Inventor: RAJEEV, Kallanthottathil, G., Cambridge, MA 02142 (US); ZIMMERMANN, Tracy, Cambridge, MA 02142 (US); MANOHARAN, Muthiah, Cambridge, MA 02142 (US); MAIER, Martin, Cambridge, MA 02142 (US); KUCHIMANCHI, Satyanarayana, Cambridge, MA 02142 (US); CHARISSE, Klaus, Cambridge, MA 02142 (US)
(74) Representative: Vossius & Partner Patentanwälte Rechtsanwälte mbB

(56) References cited:
- WO-A1-2009/002944
- WO-A1-2010/078536
- WO-A1-2011/123468
- WO-A2-2007/092059
- WO-A2-2009/073809
- WO-A2-2009/134487
- WO-A2-2011/072082
- WO-A2-2013/163430
- US-A1- 2006 217 331
- THAZHA P PRAKASH ET AL: "Positional effect of chemical modifications on short interference RNA activity in mammalian cells", JOURNAL OF MEDICINAL CHEMI, AMERICAN CHEMICAL SOCIETY, vol. 48, no. 13, 27 May 2005 (2005-05-27), pages 4247 - 4253, XP008155072, ISSN: 0022-2623, [retrieved on 20070527], DOI: 10.1021/JM050044O
- CZAUDERNA F ET AL: "Structural variations and stabilising modifications of synthetic siRNAs in mammalian cells", NUCLEIC ACIDS RESEARCH, INFORMATION RETRIEVAL LTD, GB, vol. 31, no. 11, 1 June 2003 (2003-06-01), pages 2705 - 2716, XP002270732, ISSN: 0305-1048, DOI: 10.1093/NAR/GKG393
- BEHLKE M A: "Chemical modification of siRNAs for in vivo use", OLIGONUCLEOTIDES, MARY ANN LIEBERT, NEW YORK, NY, US, vol. 18, no. 4, 22 November 2008 (2008-11-22), pages 305 - 320, XP002546697, ISSN: 1545-4576, [retrieved on 20081129], DOI: 10.1089/OLI.2008.0164
- MICHAEL A COLLINGWOOD ET AL: "Chemical Modification Patterns Compatible with High Potency Dicer-Substrate Small Interfering RNAs", OLIGONUCLEOTIDES, MARY ANN LIEBERT, NEW YORK, NY, US, vol. 18, no. 2, 18 July 2008 (2008-07-18), pages 187 - 200, XP002623649, ISSN: 1545-4576, DOI: 10.1089/OLI.2008.0123
- J. B. BRAMSEN ET AL: "A large-scale chemical modification screen identifies design rules to generate siRNAs with high activity, high stability and low toxicity", NUCLEIC ACIDS RESEARCH, vol. 37, no. 9, 12 March 2009 (2009-03-12), pages 2867 - 2881, XP055000747, ISSN: 0305-1048, DOI: 10.1093/nar/gkp106
- DAVID V MORRISSEY ET AL: "Potent and persistent in vivo anti-HBV activity of chemically modified siRNAs", NATURE BIOTECHNOLOGY, GALE GROUP INC, NEW YORK, vol. 23, no. 8, 1 August 2005 (2005-08-01), pages 1002 - 1007, XP008157520, ISSN: 1087-0156, [retrieved on 20050724], DOI: 10.1038/NBT1122

## Description

### FIELD OF THE INVENTION

The invention is defined by the claims and relates to RNAi duplex agents as defined by the claims having particular motifs that are advantageous for inhibition of target gene expression, as well as RNAi compositions suitable for therapeutic use. Also described herein are methods of inhibiting the expression of a target gene by administering these RNAi duplex agents, *e.g.,* for the treatment of various diseases.

### BACKGROUND

RNA interference or "RNAi" is a term initially coined by Fire and co-workers to describe the observation that double-stranded RNAi (dsRNA) can block gene expression (Fire et al. (1998) Nature 391, 806-811; Elbashir et al. (2001) Genes Dev. 15, 188-200). Short dsRNA directs gene-specific, post-transcriptional silencing in many organisms, including vertebrates, and has provided a new tool for studying gene function. RNAi is mediated by RNA-induced silencing complex (RISC), a sequence-specific, multicomponent nuclease that destroys messenger RNAs homologous to the silencing trigger. RISC is known to contain short RNAs (approximately 22 nucleotides) derived from the double-stranded RNA trigger, but the protein components of this activity remained unknown.

Double-stranded RNA (dsRNA) molecules with good gene-silencing properties are needed for drug development based on RNA interference (RNAi). An initial step in RNAi is the activation of the RNA induced silencing complex (RISC), which requires degradation of the sense strand of the dsRNA duplex. Sense strand was known to act as the first RISC substrate that is cleaved by Argonaute 2 in the middle of the duplex region. Immediately after the cleaved 5'-end and 3'-end fragments of the sense strand are removed from the endonuclease Ago2, the RISC becomes activated by the antisense strand (Rand et al. (2005) Cell 123, 621).

It was believed that when the cleavage of the sense strand is inhibited, the endonucleolytic cleavage of target mRNA is impaired (Leuschner et al. (2006) EMBO Rep., 7, 314; Rand et al. (2005) Cell 123, 621; Schwarz et al. (2004) Curr. Biol. 14, 787). Leuschner et al. showed that incorporation of a 2'-O-Me ribose to the Ago2 cleavage site in the sense strand inhibits RNAi in HeLa cells (Leuschner et al. (2006) EMBO Rep., 7, 314). A similar effect was observed with phosphorothioate modifications, showing that cleavage of the sense strand was required for efficient RNAi also in mammals.

Morrissey et al. used a siRNA duplex containing 2'-F modified residues, among other sites and modifications, also at the Ago2 cleavage site, and obtained compatible silencing compared to the unmodified siRNAs (Morrissey et al. (2005) Hepatology 41, 1349). However, Morrissey's modification is not motif specific, e.g., one modification includes 2'-F modifications on all pyrimidines on both sense and antisense strands as long as pyrimidine residue is present, without any selectivity; and hence it is uncertain, based on these teachings, if specific motif modification at the cleavage site of sense strand can have any actual effect on gene silencing acitivity.

Muhonen et al. used a siRNA duplex containing two 2'-F modified residues at the Ago2 cleavage site on the sense or antisense strand and found it was tolerated (Muhonen et al. (2007) Chemistry & Biodiversity 4, 858-873). However, Muhonen's modification is also sequence specific, e.g., for each particular strand, Muhonen only modifies either all pyrimidines or all purines, without any selectivity.

Choung et al. used a siRNA duplex containing alternative modifications by 2'-OMe or various combinations of 2'-F, 2'-OMe and phosphorothioate modifications to stabilize siRNA in serum to Sur10058 (Choung et al. (2006) Biochemical and Biophysical Research Communications 342, 919-927). Choung suggested that the residues at the cleavage site of the antisense strand should not be modified with 2'-OMe in order to increase the stability of the siRNA.

There is thus an ongoing need for iRNA duplex agents to improve the gene silencing efficacy of siRNA gene therapeutics. This invention is directed to that need.

### SUMMARY

This invention is defined by the claims and accordingly provides a double-stranded RNAi agent capable of inhibiting the expression of a target gene, comprising a sense strand and an antisense strand, each strand having 14 to 30 nucleotides, wherein the duplex is represented by formula (III):
sense:

   5' nₚ-Nₐ-(XXX)ᵢ-N_{b}-YYY-N_{b}-(ZZZ)ⱼ-Nₐ-n_{q}3'
antisense:

   3' nₚ'-Nₐ'-(X'X'X')ₖ-N_{b}'-Y'Y'Y'-N_{b}'-(Z'Z'Z')ₗ-Nₐ'-n_{q}' 5' (III)

   wherein:
   i, j, k, and l are each independently 0 or 1;
   p and q are each independently 0-6;
   each Nₐ and Nₐ' independently represents an oligonucleotide sequence comprising 0-25 nucleotides which are either modified or unmodified or combinations thereof, each sequence comprising at least two differently modified nucleotides, each N_{b} and N_{b}' independently represents an oligonucleotide sequence comprising 0-10 nucleotides which are either modified or unmodified or combinations thereof;
   each nₚ, nₚ', n_{q} and n_{q}' independently represents an overhang nucleotide sequence comprising 0-6 nucleotides; and
   XXX, YYY, ZZZ, X'X'X', Y'Y'Y', and Z'Z'Z' each independently represent one motif of three identical modifications on three consecutive nucleotides;
   the Y'Y'Y' motif occurs at the 11, 12 and 13 positions of the antisense strand from the 5'-end;
   the Y' is 2'-OMe;
   said agent further comprises at least one phosphorothioate internucleotide linkage, wherein the antisense strand comprises two blocks of two phosphorothioate internucleotide linkages separated by 1, 2, 3, 4, 5, 6, 7, 8, 9, 10, 11, 12, 13, 14, 15, 16, 17 or 18 phosphate internucleotide linkages, and said antisense strand is paired with a sense strand comprising any combination of phosphorothioate, methylphosphonate and phosphate internucleotide linkages;
      and
   wherein the modification on Nb is different than the modification on Y and the modification on Nb' is different than the modification on Y',
   provided that the double-stranded RNAi is not a double-stranded RNAi consisting of
   Sense strand: GfsgsUfuAfaCfaCfCfAfuUfuAfcUfuCfaAfL96
   Antisense strand: usUfsgAfaGfuAfaAfuggUfgUfuAfaCfcsasg,
   Sense strand: GfsgsUfuAfaCfaCfCfAfuUf(Uhd)AfcUfuCfaAfL96
   Antisense strand: usUfsgAfaGfuAfaAfuggUfgUfuAfaCfcsasg, and
   Sense strand: GfgUfuAfaCfaCfCfAfuUfuAfcUfuCfaAfL96
   Antisense strand: usUfsgAfaGfuAfaAfuggUfgUfuAfaCfcsasg, wherein
   Gf is 2'-fluoroguanosine-3'-phosphate,
   g is 2'-O-methylguanosine-3'-phosphate,
   Uf is 2'-fluorouridine-3'-phosphate,
   u is 2'-O-methyluridine-3'-phosphate,
   Af is 2'-fluoroadenosine-3'-phosphate,
   a is 2'-O-methyladenosine-3'-phosphate,
   Cf is 2'-fluorocytidine-3'-phosphate,
   c is 2'-O-methylcytidine-3'-phosphate,
   (Uhd) is 2'-O-hexadecyl-uridine-3'-phosphate,
   s is phosphorothioate linkage, and
   L96 is N-[tris(GalNAc-alkyl)-amidodecanoyl)]-4-hydroxyprolinol
   (Hyp-(GalNAc-alkyl)₃).

Generally described herein are effective nucleotide or chemical motifs for dsRNA agents optionally conjugated to at least one ligand, which are advantageous for inhibition of target gene expression, as well as RNAi compositions suitable for therapeutic use.

The inventors surprisingly discovered that introducing one or more motifs of three identical modifications on three consecutive nucleotides at or near the cleavage site of a dsRNA agent that is comprised of modified sense and antisense strands enhances the gene silencing acitivity of the dsRNA agent.

In one implementation, the disclosure relates to a double-stranded RNAi (dsRNA) agent capable of inhibiting the expression of a target gene. The dsRNA agent comprises a sense strand and an antisense strand, each strand having 14 to 30 nucleotides. The dsRNA duplex is represented by formula (III):
sense: 5' nₚ-Nₐ-(X X X)ᵢ-N_{b}-Y Y Y -N_{b}-(Z Z Z)ⱼ-Nₐ- n_{q} 3'
antisense: 3' nₚ'-Nₐ'-(X' X' X')ₖ-N_{b}'-Y' Y' Y' -N_{b}'-(Z' Z' Z')ᵢ-Nₐ'-n_{q}' 5' (III),

In formula (III), i, j, k, and 1 are each independently 0 or 1; p and q are each independently 0-6; n represents a nucleotide; each Na and Na' independently represents an oligonucleotide sequence comprising 0-25 nucleotides which are either modified or unmodified or combinations thereof, each sequence comprising at least two differently modified nucleotides; each Nb and Nb' independently represents an oligonucleotide sequence comprising 0-10 nucleotides which are either modified or unmodified or combinations thereof; each nₚ and n_{q} independently represents an overhang nucleotide sequence comprising 0-6 nucleotides; and XXX, YYY, ZZZ, X'X'X', Y'Y'Y', and Z'Z'Z' each independently represent one motif of three identical modifications on three consecutive nucleotides; wherein the modifications on Nb is different than the modification on Y and the modifications on Nb' is different than the modification on Y'. At least one of the Y nucleotides forms a base pair with its complementary Y' nucleotides, and wherein the modification on the Y nucleotide is different than the modification on the Y' nucleotide.

Each nₚ and n_{q} independently represents an overhang nucleotide sequence comprising 0-6 nucleotides; each n and n' represents an overhang nucleotide; and p and q are each independently 0-6.

In another implementation, the disclosure relates to a dsRNA agent capable of inhibiting the expression of a target gene. The dsRNA agent comprises a sense strand and an antisense strand, each strand having 14 to 30 nucleotides. The sense strand contains at least two motifs of three identical modifications on three consecutive nucleotides, where at least one of the motifs occurs at or near the cleavage site within the strand and at least one of the motifs occurs at another portion of the strand that is separated from the motif at the cleavage site by at least one nucleotide. The antisense strand contains at least one motif of three identical modifications on three consecutive nucleotides, where at least one of the motifs occurs at or near the cleavage site within the strand and at least one of the motifs occurs at another portion of the strand that is separated from the motif at or near cleavage site by at least one nucleotide. The modification in the motif occurring at or near the cleavage site in the sense strand is different than the modification in the motif occurring at or near the cleavage site in the antisense strand.

In another implementation, the disclosure relates to a dsRNA agent capable of inhibiting the expression of a target gene. The dsRNA agent comprises a sense strand and an antisense strand, each strand having 14 to 30 nucleotides. The sense strand contains at least one motif of three 2'-F modifications on three consecutive nucleotides, where at least one of the motifs occurs at or near the cleavage site in the strand. The antisense strand contains at least one motif of three 2'-O-methyl modifications on three consecutive nucleotides at or near the cleavage site.

In another implementationt, the disclosure relates to a dsRNA agent capable of inhibiting the expression of a target gene. The dsRNA agent comprises a sense strand and an antisense strand, each strand having 14 to 30 nucleotides. The sense strand contains at least one motif of three 2'-F modifications on three consecutive nucleotides at positions 9,10,11 from the 5'end. The antisense strand contains at least one motif of three 2'-O-methyl modifications on three consecutive nucleotides at positions 11,12,13 from the 5'end.

In another implementation, the disclosure further provides a method for delivering the dsRNA to a specific target in a subject by subcutaneous or intravenenuous administration.

### DETAILED DESCRIPTION

A superior result may be obtained by introducing one or more motifs of three identical modifications on three consecutive nucleotides into a sense strand and/or antisense strand of a dsRNA agent, particularly at or near the cleavage site. The sense strand and antisense strand of the dsRNA agent may otherwise be completely modified. The introduction of these motifs interrupts the modification pattern, if present, of the sense and/or antisense strand. The dsRNA agent optionally conjugates with a GalNAc derivative ligand, for instance on the sense strand. The resulting dsRNA agents present superior gene silencing activity.

The inventors surprisingly discovered that having one or more motifs of three identical modifications on three consecutive nucleotides at or near the cleavage site of at least one strand of a dsRNA agent superiorly enhanced the gene silencing acitivity of the dsRNA agent.

Accordingly, the disclosure provides a double-stranded RNAi (dsRNA) agent capable of inhibiting the expression of a target gene. The dsRNA agent comprises a sense strand and an antisense strand. Each strand of the dsRNA agent can range from 12-30 nucleotides in length. For example, each strand can be between 14-30 nucleotides in length, 17-30 nucleotides in length, 25-30 nucleotides in length, 27-30 nucleotides in length, 17-23 nucleotides in length, 17-21 nucleotides in length, 17-19 nucleotides in length, 19-25 nucleotides in length, 19-23 nucleotides in length, 19-21 nucleotides in length, 21-25 nucleotides in length, or 21-23 nucleotides in length.

The sense strand and antisense strand typically form a duplex dsRNA. The duplex region of a dsRNA agent may be 12-30 nucleotide pairs in length. For example, the duplex region can be between 14-30 nucleotide pairs in length, 17-30 nucleotide pairs in length, 25-30 nucleotides in length, 27-30 nucleotide pairs in length, 17 - 23 nucleotide pairs in length, 17-21 nucleotide pairs in length, 17-19 nucleotide pairs in length, 19-25 nucleotide pairs in length, 19-23 nucleotide pairs in length, 19- 21 nucleotide pairs in length, 21-25 nucleotide pairs in length, or 21-23 nucleotide pairs in length. In another example, the duplex region is selected from 15, 16, 17, 18, 19, 20, 21, 22, 23, 24, 25, 26, and 27.

In one implementation, the dsRNA agent of the disclosure comprises may contain one or more overhang regions and/or capping groups of dsRNA agent at the 3'-end, or 5'-end or both ends of a strand. The overhang can be 1-6 nucleotides in length, for instance 2-6 nucleotides in length, 1-5 nucleotides in length, 2-5 nucleotides in length, 1-4 nucleotides in length, 2-4 nucleotides in length, 1-3 nucleotides in length, 2-3 nucleotides in length, or 1-2 nucleotides in length. The overhangs can be the result of one strand being longer than the other, or the result of two strands of the same length being staggered. The overhang can form a mismatch with the target mRNA or it can be complementary to the gene sequences being targeted or can be other sequence. The first and second strands can also be joined, e.g., by additional bases to form a hairpin, or by other non-base linkers.

In one implementation, the nucleotides in the overhang region of the dsRNA agent of the diclosure can each independently be a modified or unmodified nucleotide including, but no limited to 2'-sugar modified, such as, 2-F 2'-Omethyl, thymidine (T), 2'-O-methoxyethyl-5-methyluridine (Teo), 2'-O-methoxyethyladenosine (Aeo), 2'-O-methoxyethyl-5-methylcytidine (m5Ceo), and any combinations thereof. For example, TT can be an overhang sequence for either end on either strand. The overhang can form a mismatch with the target mRNA or it can be complementary to the gene sequences being targeted or can be other sequence.

The 5'- or 3'- overhangs at the sense strand, antisense strand or both strands of the dsRNA agent of the disclosure may be phosphorylated. In some implementation, the overhang region contains two nucleotides having a phosphorothioate between the two nucleotides, where the two nucleotides can be the same or different. In one implementation, the overhang is present at the 3'-end of the sense strand, antisense strand or both strands. In one implementation, this 3'-overhang is present in the antisense strand. In one implementation, this 3'-overhang is present in the sense strand.

The dsRNA agent of the disclosure comprises only single overhang, which can strengthen the interference activity of the dsRNA, without affecting its overall stability. For example, the single-stranded overhang is located at the 3'-terminal end of the sense strand or, alternatively, at the 3'-terminal end of the antisense strand. The dsRNA may also have a blunt end, located at the 5'-end of the antisense strand (or the 3'-end of the sense strand) or vice versa. Generally, the antisense strand of the dsRNA has a nucleotide overhang at the 3'-end, and the 5'-end is blunt. While not bound by theory, the asymmetric blunt end at the 5'-end of the antisense strand and 3'-end overhang of the antisense strand favor the guide strand loading into RISC process.

In one implementation, the dsRNA agent of the disclosure may also have two blunt ends, at both ends of the dsRNA duplex.

In one implementation, the dsRNA agent of the disclosure is a double ended bluntmer of 19 nt in length, wherein the sense strand contains at least one motif of three 2'-F modifications on three consecutive nucleotides at positions 7,8,9 from the 5'end. The antisense strand contains at least one motif of three 2'-O-methyl modifications on three consecutive nucleotides at positions 11,12,13 from the 5'end.

In one implementation, the dsRNA agent of the disclosure is a double ended bluntmer of 20 nt in length, wherein the sense strand contains at least one motif of three 2'-F modifications on three consecutive nucleotides at positions 8,9,10 from the 5'end. The antisense strand contains at least one motif of three 2'-O-methyl modifications on three consecutive nucleotides at positions 11,12,13 from the 5'end.

In one implementation, the dsRNA agent of the disclosure is a double ended bluntmer of 21 nt in length, wherein the sense strand contains at least one motif of three 2'-F modifications on three consecutive nucleotides at positions 9,10,11 from the 5'end. The antisense strand contains at least one motif of three 2'-O-methyl modifications on three consecutive nucleotides at positions 11,12,13 from the 5'end.

In one implementation, the dsRNA agent of the disclosure comprises a 21 nucleotides (nt) sense strand and a 23 nucleotides (nt) antisense, wherein the sense strand contains at least one motif of three 2'-F modifications on three consecutive nucleotides at positions 9,10,11 from the 5'end; the antisense strand contains at least one motif of three 2'-O-methyl modifications on three consecutive nucleotides at positions 11,12,13 from the 5'end, wherein one end of the dsRNA is blunt, while the other end is comprises a 2 nt overhang. Preferably, the 2 nt overhang is at the 3'-end of the antisense. Optionally, the dsRNA further comprises a ligand (preferably GalNAc₃).

In one implementation, the dsRNA agent of the disclosure comprising a sense and antisense strands, wherein: the sense strand is 25-30 nucleotide residues in length, wherein starting from the 5' terminal nucleotide (position 1) positions 1 to 23 of said first strand comprise at least 8 ribonucleotides; antisense strand is 36-66 nucleotide residues in length and, starting from the 3' terminal nucleotide, comprises at least 8 ribonucleotides in the positions paired with positions 1- 23 of sense strand to form a duplex; wherein at least the 3 ' terminal nucleotide of antisense strand is unpaired with sense strand, and up to 6 consecutive 3' terminal nucleotides are unpaired with sense strand, thereby forming a 3' single stranded overhang of 1-6 nucleotides; wherein the 5' terminus of antisense strand comprises from 10-30 consecutive nucleotides which are unpaired with sense strand, thereby forming a 10-30 nucleotide single stranded 5' overhang; wherein at least the sense strand 5' terminal and 3' terminal nucleotides are base paired with nucleotides of antisense strand when sense and antisense strands are aligned for maximum complementarity, thereby forming a substantially duplexed region between sense and antisense strands; and antisense strand is sufficiently complementary to a target RNA along at least 19 ribonucleotides of antisense strand length to reduce target gene expression when said double stranded nucleic acid is introduced into a mammalian cell; and wherein the sense strand contains at least one motif of three 2'-F modifications on three consecutive nucleotides, where at least one of the motifs occurs at or near the cleavage site. The antisense strand contains at least one motif of three 2'-O-methyl modifications on three consecutive nucleotides at or near the cleavage site.

In one implementation, the dsRNA agent of the disclosure comprising a sense and antisense strands, wherein said dsRNA agent comprises a first strand having a length which is at least 25 and at most 29 nucleotides and a second strand having a length which is at most 30 nucleotides with at least one motif of three 2'-O-methyl modifications on three consecutive nucleotides at position 11,12,13 from the 5' end; wherein said 3' end of said first strand and said 5' end of said second strand form a blunt end and said second strand is 1-4 nucleotides longer at its 3' end than the first strand, wherein the duplex region region which is at least 25 nucleotides in length, and said second strand is sufficiently complemenatary to a target mRNA along at least 19 nt of said second strand length to reduce target gene expression when said dsRNA agent is introduced into a mammalian cell, and wherein dicer cleavage of said dsRNA preferentially results in an siRNA comprising said 3' end of said second strand, thereby reducing expression of the target gene in the mammal. Optionally, the dsRNA agent further comprises a ligand.

In one implementation, the sense strand of the dsRNA agent contains at least one motif of three identical modifications on three consecutive nucleotides, where one of the motifs occurs at the cleavage site in the sense strand.

In one implementation, the antisense strand of the dsRNA agent can also contain at least one motif of three identical modifications on three consecutive nucleotides, where one of the motifs occurs at or near the cleavage site in the antisense strand.

For dsRNA agent having a duplex region of 17-23 nt in length, the cleavage site of the antisense strand is typically around the 10, 11 and 12 positions from the 5'-end. Thus the motifs of three identical modifications may occur at the 9, 10, 11 positions; 10, 11, 12 positions; 11, 12, 13 positions; 12, 13, 14 positions; or 13, 14, 15 positions of the antisense strand, the count starting from the 1^{st} nucleotide from the 5'-end of the antisense strand, or, the count starting from the 1^{st} paired nucleotide within the duplex region from the 5'- end of the antisense strand. The cleavage site in the antisense strand may also change according to the length of the duplex region of the dsRNA from the 5'-end.

The sense strand of the dsRNA agent comprises at least one motif of three identical modifications on three consecutive nucleotides at the cleavage site of the strand; and the antisense strand may have at least one motif of three identical modifications on three consecutive nucleotides at or near the cleavage site of the strand. When the sense strand and the antisense strand form a dsRNA duplex, the sense strand and the antisense strand can be so aligned that one motif of the three nucleotides on the sense strand and one motif of the three nucleotides on the antisense strand have at least one nucleotide overlap, i.e., at least one of the three nucleotides of the motif in the sense strand forms a base pair with at least one of the three nucleotides of the motif in the antisense strand. Alternatively, at least two nucleotides of the motifs from both strands may overlap, or all three nucleotides may overlap.

In one implementation, the sense strand of the dsRNA agent comprises more than one motif of three identical modifications on three consecutive nucleotides. The first motif should occur at or near the cleavage site of the strand and the other motifs may be a wing modifications. The term "wing modification" herein refers to a motif occurring at another portion of the strand that is separated from the motif at or near the cleavage site of the same strand. The wing modification is either adajacent to the first motif or is separated by at least one or more nucleotides. When the motifs are immediately adjacent to each other the chemistry of the motifs are distinct from each other and when the motifs are separated by one or more nucleotide the chemistries of the motifs can be the same or different. Two or more wing modifications may be present. For instance, when two wing modifications are present, the wing modifications may both occur at one end of the duplex region relative to the first motif which is at or near the cleavage site or each of the wing modifications may occur on either side of the first motif.

Like the sense strand, the antisense strand of the dsRNA agent comprises at least two motifs of three identical modifications on three consecutive nucleotides, with at least one of the motifs occurring at or near the cleavage site of the strand. This antisense strand may also contain one or more wing modifications in an alignment similar to the wing modifications that is present on the sense strand.

In one implementation, the wing modification on the sense strand, antisense strand, or both strands of the dsRNA agent typically does not include the first one or two terminal nucleotides at the 3'-end, 5'-end or both ends of the strand.

In another implementation, the wing modification on the sense strand, antisense strand, or both strands of the dsRNA agent typically does not include the first one or two paired nucleotides within the duplex region at the 3'-end, 5'-end or both ends of the strand.

When the sense strand and the antisense strand of the dsRNA agent each contain at least one wing modification, the wing modifications may fall on the same end of the duplex region, and have an overlap of one, two or three nucleotides.

When the sense strand and the antisense strand of the dsRNA agent each contain at least two wing modifications, the sense strand and the antisense strand can be aligned so that two wing modifications each from one strand fall on one end of the duplex region, having an overlap of one, two or three nucleotides; two modifications each from one strand fall on the other end of the duplex region, having an overlap of one, two or three nucleotides.

In one implementation, every nucleotide in the sense strand and antisense strand of the dsRNA agent, including the nucleotides that are part of the motifs, may be modified. Each nucleotide may be modified with the same or different modification which can include one or more alteration of one or both of the non-linking phosphate oxygens and/or of one or more of the linking phosphate oxygens; alteration of a constituent of the ribose sugar, *e.g.,* of the 2' hydroxyl on the ribose sugar; wholesale replacement of the phosphate moiety with "dephospho" linkers; modification or replacement of a naturally occurring base; and replacement or modification of the ribose-phosphate backbone.

As nucleic acids are polymers of subunits, many of the modifications occur at a position which is repeated within a nucleic acid, *e.g.,* a modification of a base, or a phosphate moiety, or a non-linking O of a phosphate moiety. In some cases the modification will occur at all of the subject positions in the nucleic acid but in many cases it will not. By way of example, a modification may only occur at a 3' or 5' terminal position, may only occur in a terminal region, *e.g.,* at a position on a terminal nucleotide or in the last 2, 3, 4, 5, or 10 nucleotides of a strand. A modification may occur in a double strand region, a single strand region, or in both. A modification may occur only in the double strand region of a RNA or may only occur in a single strand region of a RNA. E.g., a phosphorothioate modification at a non-linking O position may only occur at one or both termini, may only occur in a terminal region, *e.g.,* at a position on a terminal nucleotide or in the last 2, 3, 4, 5, or 10 nucleotides of a strand, or may occur in double strand and single strand regions, particularly at termini. The 5' end or ends can be phosphorylated.

It may be possible, *e.g.,* to enhance stability, to include particular bases in overhangs, or to include modified nucleotides or nucleotide surrogates, in single strand overhangs, *e.g.,* in a 5' or 3' overhang, or in both. *E.g.,* it can be desirable to include purine nucleotides in overhangs. In some implementations all or some of the bases in a 3' or 5' overhang may be modified, e.g., with a modification described herein. Modifications can include, *e.g.,* the use of modifications at the 2' position of the ribose sugar with modifications that are known in the art, *e.g.,* the use of deoxyribonucleotides, 2'-deoxy-2'-fluoro (2'-F) or 2'-O-methyl modified instead of the ribosugar of the nucleobase, and modifications in the phosphate group, e.g., phosphorothioate modifications. Overhangs need not be homologous with the target sequence.

In one implementation, each residue of the sense strand and antisense strand is independently modified with LNA, HNA, CeNA, 2'-methoxyethyl, 2'- O-methyl, 2'-O-allyl, 2'-C- allyl, 2'-deoxy, or 2'-fluoro. The strands can contain more than one modification. In one implementation, each residue of the sense strand and antisense strand is independently modified with 2'- O-methyl or 2'-fluoro.

At least two different modifications are typically present on the sense strand and antisense strand. Those two modifications may be the 2'- O-methyl or 2'-fluoro modifications, or others.

In one implementation, the sense strand and antisense strand each contains two differently modified nucleotides selected from 2'-O-methyl or 2'-fluoro.

In one implementation, each residue of the sense strand and antisense strand is independently modified with 2'-O-methyl nucleotide, 2'-deoxyfluoro nucleotide, 2'-O-N-methylacetamido (2'-O-NMA) nucleotide, a 2'-O-dimethylaminoethoxyethyl (2'-O-DMAEOE) nucleotide, 2'-O-aminopropyl (2'-O-AP) nucleotide, or 2'-ara-F nucleotide.

In one implementation, the Na and/or Nb comprise modifications of an alternating pattern. The term "alternating motif' or "alternative pattern" as used herein refers to a motif having one or more modifications, each modification occurring on alternating nucleotides of one strand. The alternating nucleotide may refer to one per every other nucleotide or one per every three nucleotides, or a similar pattern. For example, if A, B and C each represent one type of modification to the nucleotide, the alternating motif can be "ABABABABABAB...," "AABBAABBAABB...," "AABAABAABAAB...," "AAABAAABAAAB...," "AAABBBAAABBB...," or "ABCABCABCABC...," etc.

In one implementation, the Nₐ' and/or Nb' comprise modifications of an alternating pattern. The term "alternating motif' or "alternative pattern" as used herein refers to a motif having one or more modifications, each modification occurring on alternating nucleotides of one strand. The alternating nucleotide may refer to one per every other nucleotide or one per every three nucleotides, or a similar pattern. For example, if A, B and C each represent one type of modification to the nucleotide, the alternating motif can be "ABABABABABAB...," "AABBAABBAABB...," "AABAABAABAAB...," "AAABAAABAAAB...," "AAABBBAAABBB...," or "ABCABCABCABC...," etc.

The type of modifications contained in the alternating motif may be the same or different. For example, if A, B, C, D each represent one type of modification on the nucleotide, the alternating pattern, i.e., modifications on every other nucleotide, may be the same, but each of the sense strand or antisense strand can be selected from several possibilities of modifications within the alternating motif such as "ABABAB...", "ACACAC..." "BDBDBD..." or "CDCDCD...," etc.

In one implementation, the dsRNA agent of the disclosure comprises the modification pattern for the alternating motif on the sense strand relative to the modification pattern for the alternating motif on the antisense strand is shifted. The shift may be such that the modified group of nucleotides of the sense strand corresponds to a differently modified group of nucleotides of the antisense strand and vice versa. For example, the sense strand when paired with the antisense strand in the dsRNA duplex, the alternating motif in the sense strand may start with "ABABAB" from 5'-3' of the strand and the alternating motif in the antisense strand may start with "BABABA" from 3'-5'of the strand within the duplex region. As another example, the alternating motif in the sense strand may start with "AABBAABB" from 5'-3' of the strand and the alternating motif in the antisenese strand may start with "BBAABBAA" from 3'-5' of the strand within the duplex region, so that there is a complete or partial shift of the modification patterns between the sense strand and the antisense strand.

In one implementation, the dsRNA agent of the disclosure comprises the pattern of the alternating motif of 2'-O-methyl modification and 2'-F modification on the sense strand initially has a shift relative to the pattern of the alternating motif of 2'-O-methyl modification and 2'-F modification on the antisense strand initially, i.e., the 2'-O-methyl modified nucleotide on the sense strand base pairs with a 2'-F modified nucleotide on the antisense strand and vice versa. The 1 position of the sense strand may start with the 2'-F modification, and the 1 position of the antisense strand may start with the 2'- O-methyl modification.

The introduction of one or more motifs of three identical modifications on three consecutive nucleotides to the sense strand and/or antisense strand interrupts the initial modification pattern present in the sense strand and/or antisense strand. This interruption of the modification pattern of the sense and/or antisense strand by introducing one or more motifs of three identical modifications on three consecutive nucleotides to the sense and/or antisense strand surprisingly enhances the gene silencing acitivty to the target gene.

In one implementation, when the motif of three identical modifications on three consecutive nucleotides is introduced to any of the strands, the modification of the nucleotide next to the motif is a different modification than the modification of the motif. For example, the portion of the sequence containing the motif is "...NₐYYYN_{b}...," where "Y" represents the modification of the motif of three identical modifications on three consecutive nucleotide, and "Na" and "Nb" represent a modification to the nucleotide next to the motif "YYY" that is different than the modification of Y, and where Nₐ and N_{b} can be the same or different modifications. Altnernatively, Nₐ and/or N_{b} may be present or absent when there is a wing modification present.

The dsRNA agent of the disclosure may further comprise at least one phosphorothioate or methylphosphonate internucleotide linkage. The phosphorothioate or methylphosphonate internucleotide linkage modification may occur on any nucleotide of the sense strand or antisense strand or both in any position of the strand. For instance, the internucleotide linkage modification may occur on every nucleotide on the sense strand and/or antisense strand; each internucleotide linkage modification may occur in an alternating pattern on the sense strand or antisense strand; or the sense strand or antisense strand comprises both internucleotide linkage modifications in an alternating pattern. The alternating pattern of the internucleotide linkage modification on the sense strand may be the same or different from the antisense strand, and the alternating pattern of the internucleotide linkage modification on the sense strand may have a shift relative to the alternating pattern of the internucleotide linkage modification on the antisense strand.

In one implementation, the dsRNA comprises the phosphorothioate or methylphosphonate internucleotide linkage modification in the overhang region. For example, the overhang region comprises two nucleotides having a phosphorothioate or methylphosphonate internucleotide linkage between the two nucleotides. Internucleotide linkage modifications also may be made to link the overhang nucleotides with the terminal paired nucleotides within duplex region. For example, at least 2, 3, 4, or all the overhang nucleotides may be linked through phosphorothioate or methylphosphonate internucleotide linkage, and optionally, there may be additional phosphorothioate or methylphosphonate internucleotide linkages linking the overhang nucleotide with a paired nucleotide that is next to the overhang nucleotide. For instance, there may be at least two phosphorothioate internucleotide linkages between the terminal three nucleotides, in which two of the three nucleotides are overhang nucleotides, and the third is a paried nucleotide next to the overhang nucleotide. Preferably, these terminal three nucleotides may be at the 3'-end of the antisense strand.

In one implementation the sense strand of the dsRNA comprises 1-10 blocks of two to ten phosphorothioate or methylphosphonate internucleotide linkages separated by 1, 2, 3, 4, 5, 6, 7, 8, 9, 10, 11, 12, 13, 14, 15 or 16 phosphate internucleotide linkages, wherein one of the phosphorothioate or methylphosphonate internucleotide linkages is placed at any position in the oligonucleotide sequence and the said sense strand is paired with an antisense strand comprising any combination of phosphorothioate, methylphosphonate and phosphate internucleotide linkages or an antisense strand comprising either phosphorothioate or methylphophonate or phosphate linkage.

In one implementation the antisense strand of the dsRNA comprises two blocks of two phosphorothioate or methylphosphonate internucleotide linkages separated by 1, 2, 3, 4, 5, 6, 7, 8, 9, 10, 11, 12, 13, 14, 15, 16, 17, or 18 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate and phosphate internucleotide linkages or an antisense strand comprising either phosphorothioate or methylphophonate or phosphate linkage.

In one implementation the antisense strand of the dsRNA comprises two blocks of three phosphorothioate or methylphosphonate internucleotide linkages separated by 1, 2, 3, 4, 5, 6, 7, 8, 9, 10, 11, 12, 13, 14, 15 or 16 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate and phosphate internucleotide linkages or an antisense strand comprising either phosphorothioate or methylphophonate or phosphate linkage.

In one implementation the antisense strand of the dsRNA comprises two blocks of four phosphorothioate or methylphosphonate internucleotide linkages separated by 1, 2, 3, 4, 5, 6, 7, 8, 9, 10, 11, 12, 13 or 14 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate and phosphate internucleotide linkages or an antisense strand comprising either phosphorothioate or methylphophonate or phosphate linkage.

In one implementation the antisense strand of the dsRNA comprises two blocks of five phosphorothioate or methylphosphonate internucleotide linkages separated by 1, 2, 3, 4, 5, 6, 7, 8, 9, 10, 11 or 12 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate and phosphate internucleotide linkages or an antisense strand comprising either phosphorothioate or methylphophonate or phosphate linkage.

In one implementation the antisense strand of the dsRNA comprises two blocks of six phosphorothioate or methylphosphonate internucleotide linkages separated by 1, 2, 3, 4, 5, 6, 7, 8, 9 or 10 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate and phosphate internucleotide linkages or an antisense strand comprising either phosphorothioate or methylphophonate or phosphate linkage.

In one implementation the antisense strand of the dsRNA comprises two blocks of seven phosphorothioate or methylphosphonate internucleotide linkages separated by 1, 2, 3, 4, 5, 6, 7 or 8 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate and phosphate internucleotide linkages or an antisense strand comprising either phosphorothioate or methylphophonate or phosphate linkage.

In one implementation the antisense strand of the dsRNA comprises two blocks of eight phosphorothioate or methylphosphonate internucleotide linkages separated by 1, 2, 3, 4, 5 or 6 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate and phosphate internucleotide linkages or an antisense strand comprising either phosphorothioate or methylphophonate or phosphate linkage.

In one implementation the antisense strand of the dsRNA comprises two blocks of nine phosphorothioate or methylphosphonate internucleotide linkages separated by 1, 2, 3 or 4 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate and phosphate internucleotide linkages or an antisense strand comprising either phosphorothioate or methylphophonate or phosphate linkage.

In one implementation, the dsRNA of the disclosure further comprises one or more phosphorothioate or methylphosphonate internucleotide linkage modification within 1-10 of the termini position(s) of the sense and/or antisense strand. For example, at least 2, 3, 4, 5, 6, 7, 8, 9 or 10 nucleotides may be linked through phosphorothioate or methylphosphonate internucleotide linkage at one end or both ends of the sense and/or antisense strand.

In one implementation, the dsRNA of the disclosure further comprises one or more phosphorothioate or methylphosphonate internucleotide linkage modification within 1-10 of the internal region of the duplex of each of the sense and/or antisense strand. For example, at least 2, 3, 4, 5, 6, 7, 8, 9 or 10 nucleotides may be linked through phosphorothioate methylphosphonate internucleotide linkage at position 8-16 of the duplex region counting from the 5'-end of the sense strand; the dsRNA can optionally further comprise one or more phosphorothioate or methylphosphonate internucleotide linkage modification within 1-10 of the termini position(s).

In one implementation, the dsRNA of the disclosure further comprises one to five phosphorothioate or methylphosphonate internucleotide linkage modification(s) within position 1-5 and one to five phosphorothioate or methylphosphonate internucleotide linkage modification(s) within position 18-23 of the sense strand (counting from the 5'-end), and one to five phosphorothioate or methylphosphonate internucleotide linkage modification at positions 1 and 2 and one to five within positions 18-23 of the antisense strand (counting from the 5'-end).

In one implementation, the dsRNA of the disclosure further comprises one phosphorothioate internucleotide linkage modification within position 1-5 and one phosphorothioate or methylphosphonate internucleotide linkage modification within position 18-23 of the sense strand (counting from the 5'-end), and one phosphorothioate internucleotide linkage modification at positions 1 and 2 and two phosphorothioate or methylphosphonate internucleotide linkage modifications within positions 18-23 of the antisense strand (counting from the 5'-end).

In one implementation, the dsRNA of the disclosure further comprises two phosphorothioate internucleotide linkage modifications within position 1-5 and one phosphorothioate internucleotide linkage modification within position 18-23 of the sense strand (counting from the 5'-end), and one phosphorothioate internucleotide linkage modification at positions 1 and 2 and two phosphorothioate internucleotide linkage modifications within positions 18-23 of the antisense strand (counting from the 5'-end).

In one implementation, the dsRNA of the disclosure further comprises two phosphorothioate internucleotide linkage modifications within position 1-5 and two phosphorothioate internucleotide linkage modifications within position 18-23 of the sense strand (counting from the 5'-end), and one phosphorothioate internucleotide linkage modification at positions 1 and 2 and two phosphorothioate internucleotide linkage modifications within positions 18-23 of the antisense strand (counting from the 5'-end).

In one implementation, the dsRNA of the disclosure further comprises two phosphorothioate internucleotide linkage modifications within position 1-5 and two phosphorothioate internucleotide linkage modifications within position 18-23 of the sense strand (counting from the 5'-end), and one phosphorothioate internucleotide linkage modification at positions 1 and 2 and one phosphorothioate internucleotide linkage modification within positions 18-23 of the antisense strand (counting from the 5'-end).

In one implementation, the dsRNA of the disclosure further comprises one phosphorothioate internucleotide linkage modification within position 1-5 and one phosphorothioate internucleotide linkage modification within position 18-23 of the sense strand (counting from the 5'-end), and two phosphorothioate internucleotide linkage modifications at positions 1 and 2 and two phosphorothioate internucleotide linkage modifications within positions 18-23 of the antisense strand (counting from the 5'-end).

In one implementation, the dsRNA of the disclosure further comprises one phosphorothioate internucleotide linkage modification within position 1-5 and one within position 18-23 of the sense strand (counting from the 5'-end), and two phosphorothioate internucleotide linkage modification at positions 1 and 2 and one phosphorothioate internucleotide linkage modification within positions 18-23 of the antisense strand (counting from the 5'-end).

In one implementation, the dsRNA of the disclosure further comprises one phosphorothioate internucleotide linkage modification within position 1-5 (counting from the 5'-end), and two phosphorothioateinternucleotide linkage modifications at positions 1 and 2 and one phosphorothioate internucleotide linkage modification within positions 18-23 of the antisense strand (counting from the 5'-end).

In one implementation, the dsRNA of the disclosure further comprises two phosphorothioate internucleotide linkage modifications within position 1-5 (counting from the 5'-end), and one phosphorothioateinternucleotide linkage modification at positions 1 and 2 and two phosphorothioate internucleotide linkage modifications within positions 18-23 of the antisense strand (counting from the 5'-end).

In one implementation, the dsRNA of the disclosure further comprises two phosphorothioate internucleotide linkage modifications within position 1-5 and one within position 18-23 of the sense strand (counting from the 5'-end), and two phosphorothioate internucleotide linkage modifications at positions 1 and 2 and one phosphorothioate internucleotide linkage modification within positions 18-23 of the antisense strand (counting from the 5'-end).

In one implementation, the dsRNA of the disclosure further comprises two phosphorothioate internucleotide linkage modifications within position 1-5 and one phosphorothioate internucleotide linkage modification within position 18-23 of the sense strand (counting from the 5'-end), and two phosphorothioate internucleotide linkage modifications at positions 1 and 2 and two phosphorothioate internucleotide linkage modifications within positions 18-23 of the antisense strand (counting from the 5'-end).

In one implementation, the dsRNA of the disclosure further comprises two phosphorothioate internucleotide linkage modifications within position 1-5 and one phosphorothioate internucleotide linkage modification within position 18-23 of the sense strand (counting from the 5'-end), and one phosphorothioate internucleotide linkage modification at positions 1 and 2 and two phosphorothioate internucleotide linkage modifications within positions 18-23 of the antisense strand (counting from the 5'-end).

In one implementationt, the dsRNA of the disclosure further comprises two phosphorothioate internucleotide linkage modifications at position 1 and 2, and two phosphorothioate internucleotide linkage modifications at position 20 and 21 of the sense strand (counting from the 5'-end), and one phosphorothioate internucleotide linkage modification at positions 1 and one at position 21 of the antisense strand (counting from the 5'-end).

In one implementation, the dsRNA of the disclosure further comprises one phosphorothioate internucleotide linkage modification at position 1, and one phosphorothioate internucleotide linkage modification at position 21 of the sense strand (counting from the 5'-end), and two phosphorothioate internucleotide linkage modifications at positions 1 and 2 and two phosphorothioate internucleotide linkage modifications at positions 20 and 21 the antisense strand (counting from the 5'-end).

In one eimplementation, the dsRNA of the disclosure further comprises two phosphorothioate internucleotide linkage modifications at position 1 and 2, and two phosphorothioate internucleotide linkage modifications at position 21 and 22 of the sense strand (counting from the 5'-end), and one phosphorothioate internucleotide linkage modification at positions 1 and one phosphorothioate internucleotide linkage modification at position 21 of the antisense strand (counting from the 5'-end).

In one implementation, the dsRNA of the disclosure further comprises one phosphorothioate internucleotide linkage modification at position 1, and one phosphorothioate internucleotide linkage modification at position 21 of the sense strand (counting from the 5'-end), and two phosphorothioate internucleotide linkage modifications at positions 1 and 2 and two phosphorothioate internucleotide linkage modifications at positions 21 and 22 the antisense strand (counting from the 5'-end).

In one eimplementation, the dsRNA of the disclosure further comprises two phosphorothioate internucleotide linkage modifications at position 1 and 2, and two phosphorothioate internucleotide linkage modifications at position 22 and 23 of the sense strand (counting from the 5'-end), and one phosphorothioate internucleotide linkage modification at positions 1 and one phosphorothioate internucleotide linkage modification at position 21 of the antisense strand (counting from the 5'-end).

In one implementation, the dsRNA of the disclosure further comprises one phosphorothioate internucleotide linkage modification at position 1, and one phosphorothioate internucleotide linkage modification at position 21 of the sense strand (counting from the 5'-end), and two phosphorothioate internucleotide linkage modifications at positions 1 and 2 and two phosphorothioate internucleotide linkage modifications at positions 23 and 23 the antisense strand (counting from the 5'-end).

In one implementation, the dsRNA agent of the disclosure comprises mismatch(es) with the target, within the duplex, or combinations thereof. The mistmatch can occur in the overhang region or the duplex region. The base pair can be ranked on the basis of their propensity to promote dissociation or melting (e.g., on the free energy of association or dissociation of a particular pairing, the simplest approach is to examine the pairs on an individual pair basis, though next neighbor or similar analysis can also be used). In terms of promoting dissociation: A:U is preferred over G:C; G:U is preferred over G:C; and I:C is preferred over G:C (I=inosine). Mismatches, e.g., non-canonical or other than canonical pairings (as described elsewhere herein) are preferred over canonical (A:T, A:U, G:C) pairings; and pairings which include a universal base are preferred over canonical pairings.

In one implementation, the dsRNA agent of the disclosure comprises at least one of the first 1, 2, 3, 4, or 5 base pairs within the duplex regions from the 5'- end of the antisense strand can be chosen independently from the group of: A:U, G:U, I:C, and mismatched pairs, e.g., non-canonical or other than canonical pairings or pairings which include a universal base, to promote the dissociation of the antisense strand at the 5'-end of the duplex.

In one implementation, the nucleotide at the 1 position within the duplex region from the 5'-end in the antisense strand is selected from the group consisting of A, dA, dU, U, and dT. Alternatively, at least one of the first 1, 2 or 3 base pair within the duplex region from the 5'- end of the antisense strand is an AU base pair. For example, the first base pair within the duplex region from the 5'- end of the antisense strand is an AU base pair.

In one implementation, the sense strand sequence may be represented by formula (I):

5' nₚ-Nₐ-(XXX)i-N_{b}-YYY-N_{b}-(ZZZ)j-Nₐ-n_{q} 3' (I)

wherein:
i and j are each independently 0 or 1;
p and q are each independently 0-6;
each Na independently represents an oligonucleotide sequence comprising 0-25 modified nucleotides, each sequence comprising at least two differently modified nucleotides;
each Nb independently represents an oligonucleotide sequence comprising 0-10 modified nucleotides;
each nₚ and n_{q} independently represent an overhang nucleotide;
wherein N_{b} and Y do not have the same modification; and
XXX, YYY and ZZZ each independently represent one motif of three identical modifications on three consecutive nucleotides. Preferably YYY is all 2'-F modified nucleotides.

In one implementation, the Na and/or Nb comprise modifications of alternating pattern.

In one implementation, the YYY motif occurs at or near the cleavage site of the sense strand. For example, when the dsRNA agent has a duplex region of 17-23 nucleotide pairs in length, the YYY motif can occur at or the vicinity of the cleavage site (e.g.: can occur at positions 6, 7, 8, 7, 8, 9, 8, 9, 10, 9, 10, 11, 10, 11,12 or 11, 12, 13) of - the sense strand, the count starting from the 1^{st} nucleotide, from the 5'-end; or optionally, the count starting at the 1^{st} paired nucleotide within the duplex region, from the 5'- end.

In one implementation, i is 1 and j is 0, or i is 0 and j is 1, or both i and j are 1. The sense strand can therefore be represented by the following formulas:

5' nₚ-Nₐ-YYY-N_{b}-ZZZ-Nₐ-n_{q} 3' (Ia);

5' nₚ-Nₐ-XXX-N_{b}-YYY-Nₐ-n_{q} 3' (Ib);

or

5' nₚ-Nₐ-XXX-N_{b}-YYY-N_{b}-ZZZ-Nₐ-n_{q} 3' (Ic).

When the sense strand is represented by formula (Ia), Nb represents an oligonucleotide sequence comprising 0-10, 0-7, 0-5, 0-4, 0-2 or 0 modified nucleotides. Each Na independently can represent an oligonucleotide sequence comprising 2-20, 2-15, or 2-10 modified nucleotides.

When the sense strand is represented as formula (Ib), Nb represents an oligonucleotide sequence comprising 0-10, 0-7, 0-10, 0-7, 0-5, 0-4, 0-2 or 0 modified nucleotides. Each Na can independently represent an oligonucleotide sequence comprising 2-20, 2-15, or 2-10 modified nucleotides.

When the sense strand is represented as formula (Ic), each N_{b} independently represents an oligonucleotide sequence comprising 0-10, 0-7, 0-5, 0-4, 0-2 or 0 modified nucleotides. Preferably, Nb is 0, 1, 2, 3, 4, 5 or 6 Each Na can independently represent an oligonucleotide sequence comprising 2-20, 2-15, or 2-10 modified nucleotides.

Each of X, Y and Z may be the same or different from each other.

In one implementation, the antisense strand sequence of the dsRNA may be represented by formula (II): 5' n_{q}'-Nₐ'-(Z'Z'Z')ₖ-N_{b}'-Y'Y'Y'-N_{b}'-(X'X'X'),-N'ₐ-nₚ' 3' (II) wherein:
k and 1 are each independently 0 or 1;
p and q are each independently 0-6;
each Na' independently represents an oligonucleotide sequence comprising 0-25 modified nucleotides, each sequence comprising at least two differently modified nucleotides;
each Nb' independently represents an oligonucleotide sequence comprising 0-10 modified nucleotides;
each nₚ' and n_{q}' independently represent an overhang nucleotide comprising 0-6 nucleotides;
wherein N_{b}' and Y' do not have the same modification;
   and
X'X'X', Y'Y'Y' and Z'Z'Z' each independently represent one motif of three identical modifications on three consecutive nucleotides.

In one implementation, the Na' and/or Nb' comprise modifications of alternating pattern.

The Y'Y'Y' motif occurs at or near the cleavage site of the antisense strand. For example, when the dsRNA agent has a duplex region of 17-23 nt in length, the Y'Y'Y' motif can occur at positions 9, 10, 11;10, 11, 12; 11, 12, 13; 12, 13, 14; or 13, 14, 15 of the antisense strand, with the count starting from the 1^{st} nucleotide, from the 5'-end; or optionally, the count starting at the 1^{st} paired nucleotide within the duplex region, from the 5'- end. Preferably, the Y'Y'Y' motif occurs at positions 11, 12, 13.

In one implementation, Y'Y'Y' motif is all 2'-OMe modified nucleotides.

In one implementation, k is 1 and 1 is 0, or k is 0 and 1 is 1, or both k and 1 are 1.

The antisense strand can therefore be represented by the following formulas:

5' n_{q}'-Nₐ'-Z'Z'Z'-N_{b}'-Y'Y'Y'-Nₐ'-nₚ' 3' (IIa);

5' n_{q}'-Nₐ'-Y'Y'Y'-N_{b}'-X'X'X'-nₚ'3' (IIb);

or

5' n_{q}'-Nₐ'-Z'Z'Z'-N_{b}'-Y'Y'Y'-N_{b}'-X'X'X'-Nₐ'-nₚ' 3' (IIc).

When the antisense strand is represented by formula (IIa), N_{b}' represents an oligonucleotide sequence comprising 0-10, 0-7, 0-10, 0-7, 0-5, 0-4, 0-2 or 0 modified nucleotides. Each Na' independently represents an oligonucleotide sequence comprising 2-20, 2-15, or 2-10 modified nucleotides.

When the antisense strand is represented as formula (IIb), Nb' represents an oligonucleotide sequence comprising 0-10, 0-7, 0-10, 0-7, 0-5, 0-4, 0-2 or 0 modified nucleotides. Each Na' independently represents an oligonucleotide sequence comprising 2-20, 2-15, or 2-10 modified nucleotides.

When the antisense strand is represented as formula (IIc), each Nb' independently represents an oligonucleotide sequence comprising 0-10, 0-7, 0-10, 0-7, 0-5, 0-4, 0-2 or 0 modified nucleotides. Each Na' independently represents an oligonucleotide sequence comprising 2-20, 2-15, or 2-10 modified nucleotides. Preferably, Nb is 0, 1, 2, 3, 4, 5 or 6.

Each of X', Y' and Z' may be the same or different from each other.

Each nucleotide of the sense strand and antisense strand may be independently modified with LNA, HNA, CeNA, 2'-methoxyethyl, 2'-O-methyl, 2'-O-allyl, 2'-C- allyl, or 2'-fluoro. For example, each nucleotide of the sense strand and antisense strand is independently modified with 2'-O-methyl or 2'-fluoro. Each X, Y, Z, X', Y' and Z', in particular, may represent a 2'-O-methyl modification or a 2'-fluoro modification.

In one implementation, the sense strand of the dsRNA agent comprises YYY motif occurring at 9, 10 and 11 positions of the strand when the duplex region is 21 nt, the count starting from the 1^{st} nucleotide from the 5'-end, or optionally, the count starting at the 1^{st} paired nucleotide within the duplex region, from the 5'- end; and Y represents 2'-F modification. The sense strand may additionally contain XXX motif or ZZZ motifs as wing modifications at the opposite end of the duplex region; and XXX and ZZZ each independently represents a 2'-OMe modification or 2'-F modification.

In one implementation the antisense strand may contain Y'Y'Y' motif occurring at positions 11, 12, 13 of the strand, the count starting from the 1^{st} nucleotide from the 5'-end, or optionally, the count starting at the 1^{st} paired nucleotide within the duplex region, from the 5'- end; and Y' represents 2'-O-methyl modification. The antisense strand may additionally contain X'X'X' motif or Z'Z'Z' motifs as wing modifications at the opposite end of the duplex region; and X'X'X' and Z'Z'Z' each independently represents a 2'-OMe modification or 2'-F modification.

The sense strand represented by any one of the above formulas (Ia), (Ib) and (Ic) forms a duplex with a antisense strand being represented by any one of formulas (IIa), (IIb) and (IIc), respectively.

Accordingly, the dsRNA agent may comprise a sense strand and an antisense strand, each strand having 14 to 30 nucleotides, the dsRNA duplex represented by formula (III):
sense: 5' nₚ-Nₐ-(XXX)ᵢ-N_{b}-YYY-N_{b}-(ZZZ)ⱼ-Nₐ-n_{q} 3'
antisense: 3' nₚ'-Nₐ'-(X'X'X')ₖ-N_{b}'-Y'Y'Y'-N_{b}'-(Z'Z'Z')ₗ-Nₐ'-n_{q}' 5' (III) wherein:
   i, j, k, and 1 are each independently 0 or 1;
   p and q are each independently 0-6;
   each Na and Na' independently represents an oligonucleotide sequence comprising 0-25 modified nucleotides, each sequence comprising at least two differently modified nucleotides;
   each Nb and Nb' independently represents an oligonucleotide sequence comprising 0-10 modified nucleotides; wherein
   each nₚ', nₚ, n_{q}', and n_{q} independently represents an overhang nucleotide sequence; and
   XXX, YYY, ZZZ, X'X'X', Y'Y'Y', and Z'Z'Z' each independently represent one motif of three identical modifications on three consecutive nucleotides.

In one implementation, i is 1 and j is 0; or i is 0 and j is 1; or both i and j are 1. In another implementation, k is 1 and 1 is 0; k is 0 and 1 is 1; or both k and 1 are 1.

In one implementation, the dsRNA agent of the disclosure comprises a sense strand and an antisense strand, each strand having 14 to 30 nucleotides, the dsRNA duplex represented by formula (V):

| | |
|---|---|
| sense: | 5' Nₐ-(X X X)ᵢ-N_{b}-Y Y Y -N_{b}-(Z Z Z)ⱼ-Nₐ-n_{q} 3' |
| antisense: | 3' nₚ'-Nₐ'-(X' X' X')ₖ-N_{b}'-Y' Y' Y'-N_{b}'-(Z' Z' Z')ₗ-Nₐ' 5' |
| | (V) |

wherein:
i, j, k, and lare each independently 0 or 1;
p and q are each independently 2;
each Na and Na' independently represents an oligonucleotide sequence comprising 0-25 modified nucleotides,
each sequence comprising at least two differently modified nucleotides;
each Nb and Nb' independently represents an oligonucleotide sequence comprising 0-10 modified nucleotides; wherein
each nₚ', and n_{q} independently represents an overhang nucleotide sequence; and
XXX, YYY, ZZZ, X'X'X', Y'Y'Y', and Z'Z'Z' each independently represent one motif of three identical modifications on three consecutive nucleotides.

In one implementation, i is 1 and j is 0; or i is 0 and j is 1; or both i and j are 1. In another implementation, k is 1 and 1 is 0; k is 0 and 1 is 1; or both k and 1 are 1.

In one implementation, the dsRNA agent of the disclosure comprises a sense strand and an antisense strand, each strand having 14 to 30 nucleotides, the dsRNA duplex represented by formula (Va):

| | |
|---|---|
| sense: | 5' Nₐ-(X X X)ᵢ-N_{b}-Y Y Y -N_{b}-(Z Z Z)ⱼ-Nₐ 3' |
| antisense: | 3' nₚ'-Nₐ'-(X' X' X')ₖ-N_{b}'-Y' Y' Y'-N_{b}'-(Z' Z' Z')ₗ-Nₐ' 5' |
| | (Va) |

wherein:
i, j, k, and lare each independently 0 or 1;
p and q are each independently 2;
each Na and Na' independently represents an oligonucleotide sequence comprising 0-25 modified nucleotides, each sequence comprising at least two differently modified nucleotides;
each Nb and Nb' independently represents an oligonucleotide sequence comprising 0-10 modified nucleotides; wherein
nₚ' represents an overhang nucleotide sequence; and
XXX, YYY, ZZZ, X'X'X', Y'Y'Y', and Z'Z'Z' each independently represent one motif of three identical modifications on three consecutive nucleotides.

Exemplary combinations of the sense strand and antisense strand forming a dsRNA duplex include the formulas below:

When the dsRNA agent is represented by formula (IIIa), each N_{b} and N_{b}' independently represents an oligonucleotide sequence comprising 1-10, 1-7, 1-5 or 1-4 modified nucleotides. Each Na and Na' independently represents an oligonucleotide sequence comprising 2-20, 2-15, or 2-10 modified nucleotides.

When the dsRNA agent is represented as formula (IIIb), each Nb and Nb' independently represents an oligonucleotide sequence comprising 0-10, 0-7, 0-10, 0-7, 0-5, 0-4, 0-2 or 0 modified nucleotides. Each Na and Na' independently represents an oligonucleotide sequence comprising 2-20, 2-15, or 2-10 modified nucleotides.

When the dsRNA agent is represented as formula (IIIc), each Nb and Nb' independently represents an oligonucleotide sequence comprising 0-10, 0-7, 0-10, 0-7, 0-5, 0-4, 0-2 or 0 modified nucleotides. Each Na and Na' independently represents an oligonucleotide sequence comprising 2-20, 2-15, or 2-10 modified nucleotides. Each of Na, Na', Nb and N_{b}' independently comprises modifications of alternating pattern.

Each of X, Y and Z in formulas (III), (IIIa), (IIIb) and (IIIc) may be the same or different from each other.

When the dsRNA agent is represented by formula (III), (IIIa), (IIIb) or (IIIc), at least one of the Y nucleotides may form a base pair with one of the Y' nucleotides. Alternatively, at least two of the Y nucleotides form base pairs with the corresponding Y' nucleotides; or all three of the Y nucleotides all form base pairs with the corresponding Y' nucleotides.

It is understood that Na nucleotides from base pair with Na', Nb nucleotides from base pair with Nb', X nucleotides from base pair with X', Y nucleotides from base pair with Y', and Z nucleotides from base pair with Z'.

When the dsRNA agent is represented by formula (IIIa) or (IIIc), at least one of the Z nucleotides may form a base pair with one of the Z' nucleotides. Alternatively, at least two of the Z nucleotides form base pairs with the corresponding Z' nucleotides; or all three of the Z nucleotides all form base pairs with the corresponding Z' nucleotides.

When the dsRNA agent is represented as formula (IIIb) or (IIIc), at least one of the X nucleotides may form a base pair with one of the X' nucleotides. Alternatively, at least two of the X nucleotides form base pairs with the corresponding X' nucleotides; or all three of the X nucleotides all form base pairs with the corresponding X' nucleotides.

In one implementation, the modification on the Y nucleotide is different than the modification on the Y' nucleotide, the modification on the Z nucleotide is different than the modification on the Z' nucleotide, and/or the modification on the X nucleotide is different than the modification on the X' nucleotide.

In one implementation, the dsRNA agent is a multimer containing at least two duplexes represented by formula (III), (IIIa), (IIIb) or (IIIc), wherein said duplexes are connected by a linker. The linker can be cleavable or non-cleavable. Optionally, said multimer further comprise a ligand. Each of the dsRNA can target the same gene or two different genes; or each of the dsRNA can target same gene at two different target sites.

In one implementationt, the dsRNA agent is a multimer containing three, four, five, six or more duplexes represented by formula (III), (IIIa), (IIIb) or (IIIc), wherein said duplexes are connected by a linker. The linker can be cleavable or non-cleavable. Optionally, said multimer further comprises a ligand. Each of the dsRNA can target the same gene or two different genes; or each of the dsRNA can target same gene at two different target sites.

In one implementation, two dsRNA agent represented by formula (III), (IIIa), (IIIb) or (IIIc) are linked to each other at the 5' end, and one or both of the 3' ends of the are optionally conjugated to to a ligand. Each of the dsRNA can target the same gene or two different genes; or each of the dsRNA can target same gene at two different target sites.

Various publications described multimeric siRNA and can all be used with the dsRNA of the disclosure. Such publications include WO2007/091269, US Patent No. 7858769, WO2010/141511, WO2007/117686, WO2009/014887 and WO2011/031520.

The dsRNA agent that contains conjugations of one or more carbohydrate moieties to a dsRNA agent can optimize one or more properties of the dsRNA agent. In many cases, the carbohydrate moiety will be attached to a modified subunit of the dsRNA agent. E.g., the ribose sugar of one or more ribonucleotide subunits of a dsRNA agent can be replaced with another moiety, e.g., a non-carbohydrate (preferably cyclic) carrier to which is attached a carbohydrate ligand. A ribonucleotide subunit in which the ribose sugar of the subunit has been so replaced is referred to herein as a ribose replacement modification subunit (RRMS). A cyclic carrier may be a carbocyclic ring system, i.e., all ring atoms are carbon atoms, or a heterocyclic ring system, i.e., one or more ring atoms may be a heteroatom, e.g., nitrogen, oxygen, sulfur. The cyclic carrier may be a monocyclic ring system, or may contain two or more rings, e.g. fused rings. The cyclic carrier may be a fully saturated ring system, or it may contain one or more double bonds.

The ligand may be attached to the polynucleotide via a carrier. The carriers include (i) at least one "backbone attachment point," preferably two "backbone attachment points" and (ii) at least one "tethering attachment point." A "backbone attachment point" as used herein refers to a functional group, e.g. a hydroxyl group, or generally, a bond available for, and that is suitable for incorporation of the carrier into the backbone, e.g., the phosphate, or modified phosphate, e.g., sulfur containing, backbone, of a ribonucleic acid. A "tethering attachment point" (TAP) in some implementations refers to a constituent ring atom of the cyclic carrier, e.g., a carbon atom or a heteroatom (distinct from an atom which provides a backbone attachment point), that connects a selected moiety. The moiety can be, e.g., a carbohydrate, e.g. monosaccharide, disaccharide, trisaccharide, tetrasaccharide, oligosaccharide and polysaccharide. Optionally, the selected moiety is connected by an intervening tether to the cyclic carrier. Thus, the cyclic carrier will often include a functional group, e.g., an amino group, or generally, provide a bond, that is suitable for incorporation or tethering of another chemical entity, e.g., a ligand to the constituent ring.

In an implementation the dsRNA of the disclosure is conjugated to a ligand via a carrier, wherein the carrier can be cyclic group or acyclic group; preferably, the cyclic group is selected from pyrrolidinyl, pyrazolinyl, pyrazolidinyl, imidazolinyl, imidazolidinyl, piperidinyl, piperazinyl, [1,3]dioxolane, oxazolidinyl, isoxazolidinyl, morpholinyl, thiazolidinyl, isothiazolidinyl, quinoxalinyl, pyridazinonyl, tetrahydrofuryl and and decalin; preferably, the acyclic group is selected from serinol backbone or diethanolamine backbone.

The double-stranded RNA (dsRNA) agent of the disclosure may optionally be conjugated to one or more ligands. The ligand can be attached to the sense strand, antisense strand or both strands, at the 3'-end, 5'-end or both ends. For instance, the ligand may be conjugated to the sense strand, in particular, the 3'-end of the sense strand.

### Ligands

A wide variety of entities can be coupled to the oligonucleotides of the present disclosure. Preferred moieties are ligands, which are coupled, preferably covalently, either directly or indirectly via an intervening tether.

In preferred implementation, a ligand alters the distribution, targeting or lifetime of the molecule into which it is incorporated. In preferred implementations a ligand provides an enhanced affinity for a selected target, e.g., molecule, cell or cell type, compartment, receptor *e.g.,* a cellular or organ compartment, tissue, organ or region of the body, as, e.g., compared to a species absent such a ligand. Ligands providing enhanced affinity for a selected target are also termed targeting ligands.

Some ligands can have endosomolytic properties. The endosomolytic ligands promote the lysis of the endosome and/or transport of the composition of the disclosure, or its components, from the endosome to the cytoplasm of the cell. The endosomolytic ligand may be a polyanionic peptide or peptidomimetic which shows pH-dependent membrane activity and fusogenicity. In one disclosure, the endosomolytic ligand assumes its active conformation at endosomal pH. The "active" conformation is that conformation in which the endosomolytic ligand promotes lysis of the endosome and/or transport of the composition of the disclosure, or its components, from the endosome to the cytoplasm of the cell. Exemplary endosomolytic ligands include the GALA peptide (Subbarao et al., Biochemistry, 1987, 26: 2964-2972), the EALA peptide (Vogel et al., J. Am. Chem. Soc., 1996, 118: 1581-1586), and their derivatives (Turk et al., Biochem. Biophys. Acta, 2002, 1559: 56-68). In one implementation, the endosomolytic component may contain a chemical group (e.g., an amino acid) which will undergo a change in charge or protonation in response to a change in pH. The endosomolytic component may be linear or branched.

Ligands can improve transport, hybridization, and specificity properties and may also improve nuclease resistance of the resultant natural or modified oligoribonucleotide, or a polymeric molecule comprising any combination of monomers described herein and/or natural or modified ribonucleotides.

Ligands in general can include therapeutic modifiers, e.g., for enhancing uptake; diagnostic compounds or reporter groups e.g., for monitoring distribution; cross-linking agents; and nuclease-resistance conferring moieties. General examples include lipids, steroids, vitamins, sugars, proteins, peptides, polyamines, and peptide mimics.

Ligands can include a naturally occurring substance, such as a protein (e.g., human serum albumin (HSA), low-density lipoprotein (LDL), high-density lipoprotein (HDL), or globulin); a carbohydrate (e.g., a dextran, pullulan, chitin, chitosan, inulin, cyclodextrin or hyaluronic acid); or a lipid. The ligand may also be a recombinant or synthetic molecule, such as a synthetic polymer, e.g., a synthetic polyamino acid, an oligonucleotide (e.g. an aptamer). Examples of polyamino acids include polyamino acid is a polylysine (PLL), poly L-aspartic acid, poly L-glutamic acid, styrene-maleic acid anhydride copolymer, poly(L-lactide-co-glycolied) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacryllic acid), N-isopropylacrylamide polymers, or polyphosphazine. Example of polyamines include: polyethylenimine, polylysine (PLL), spermine, spermidine, polyamine, pseudopeptide-polyamine, peptidomimetic polyamine, dendrimer polyamine, arginine, amidine, protamine, cationic lipid, cationic porphyrin, quaternary salt of a polyamine, or an alpha helical peptide.

Ligands can also include targeting groups, e.g., a cell or tissue targeting agent, e.g., a lectin, glycoprotein, lipid or protein, e.g., an antibody, that binds to a specified cell type such as a kidney cell. A targeting group can be a thyrotropin, melanotropin, lectin, glycoprotein, surfactant protein A, Mucin carbohydrate, multivalent lactose, multivalent galactose, N-acetyl-galactosamine, N-acetyl-gulucosamine multivalent mannose, multivalent fucose, glycosylated polyaminoacids, multivalent galactose, transferrin, bisphosphonate, polyglutamate, polyaspartate, a lipid, cholesterol, a steroid, bile acid, folate, vitamin B 12, biotin, an RGD peptide, an RGD peptide mimetic or an aptamer. Table 2 shows some examples of targeting ligands and their associated receptors.

Other examples of ligands include dyes, intercalating agents (e.g. acridines), cross-linkers (e.g. psoralene, mitomycin C), porphyrins (TPPC4, texaphyrin, Sapphyrin), polycyclic aromatic hydrocarbons (e.g., phenazine, dihydrophenazine), artificial endonucleases or a chelator (e.g. EDTA), lipophilic molecules, e.g, cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid,O3-(oleoyl)lithocholic acid, O3-(oleoyl)cholenic acid, dimethoxytrityl, or phenoxazine)and peptide conjugates (e.g., antennapedia peptide, Tat peptide), alkylating agents, phosphate, amino, mercapto, PEG *(e.g.,* PEG-40K), MPEG, [MPEG]₂, polyamino, alkyl, substituted alkyl, radiolabeled markers, enzymes, haptens (*e.g.* biotin), transport/absorption facilitators *(e.g.,* aspirin, vitamin E, folic acid), synthetic ribonucleases (e.g., imidazole, bisimidazole, histamine, imidazole clusters, acridine-imidazole conjugates, Eu3+ complexes of tetraazamacrocycles), dinitrophenyl, HRP, or AP.

Ligands can be proteins, e.g., glycoproteins, or peptides, e.g., molecules having a specific affinity for a co-ligand, or antibodies e.g., an antibody, that binds to a specified cell type such as a cancer cell, endothelial cell, or bone cell. Ligands may also include hormones and hormone receptors. They can also include non-peptidic species, such as lipids, lectins, carbohydrates, vitamins, cofactors, multivalent lactose, multivalent galactose, N-acetyl-galactosamine, N-acetyl-gulucosamine multivalent mannose, multivalent fucose, or aptamers. The ligand can be, for example, a lipopolysaccharide, an activator of p38 MAP kinase, or an activator of NF-κB.

The ligand can be a substance, e.g, a drug, which can increase the uptake of the iRNA agent into the cell, for example, by disrupting the cell's cytoskeleton, e.g., by disrupting the cell's microtubules, microfilaments, and/or intermediate filaments. The drug can be, for example, taxon, vincristine, vinblastine, cytochalasin, nocodazole, japlakinolide, latrunculin A, phalloidin, swinholide A, indanocine, or myoservin.

The ligand can increase the uptake of the oligonucleotide into the cell by activating an inflammatory response, for example. Exemplary ligands that would have such an effect include tumor necrosis factor alpha (TNFalpha), interleukin-1 beta, or gamma interferon.

In one implementation, the ligand is a lipid or lipid-based molecule. Such a lipid or lipid-based molecule preferably binds a serum protein, e.g., human serum albumin (HSA). An HSA binding ligand allows for distribution of the conjugate to a target tissue, e.g., a non-kidney target tissue of the body. For example, the target tissue can be the liver, including parenchymal cells of the liver. Other molecules that can bind HSA can also be used as ligands. For example, naproxen or aspirin can be used. A lipid or lipid-based ligand can (a) increase resistance to degradation of the conjugate, (b) increase targeting or transport into a target cell or cell membrane, and/or (c) can be used to adjust binding to a serum protein, e.g., HSA.

A lipid based ligand can be used to modulate, e.g., control the binding of the conjugate to a target tissue. For example, a lipid or lipid-based ligand that binds to HSA more strongly will be less likely to be targeted to the kidney and therefore less likely to be cleared from the body. A lipid or lipid-based ligand that binds to HSA less strongly can be used to target the conjugate to the kidney.

In a preferred implementation, the lipid based ligand binds HSA. Preferably, it binds HSA with a sufficient affinity such that the conjugate will be preferably distributed to a non-kidney tissue. However, it is preferred that the affinity not be so strong that the HSA-ligand binding cannot be reversed.

In another preferred implementation, the lipid based ligand binds HSA weakly or not at all, such that the conjugate will be preferably distributed to the kidney. Other moieties that target to kidney cells can also be used in place of or in addition to the lipid based ligand.

In another aspect, the ligand is a moiety, e.g., a vitamin, which is taken up by a target cell, e.g., a proliferating cell. These are particularly useful for treating disorders characterized by unwanted cell proliferation, e.g., of the malignant or non-malignant type, e.g., cancer cells. Exemplary vitamins include vitamin A, E, and K. Other exemplary vitamins include B vitamins, e.g., folic acid, B12, riboflavin, biotin, pyridoxal or other vitamins or nutrients taken up by cancer cells. Also included are HAS, low density lipoprotein (LDL) and high-density lipoprotein (HDL).

In another implementation, the ligand is a cell-permeation agent, preferably a helical cell-permeation agent. Preferably, the agent is amphipathic. An exemplary agent is a peptide such as tat or antennopedia. If the agent is a peptide, it can be modified, including a peptidylmimetic, invertomers, non-peptide or pseudo-peptide linkages, and use of D-amino acids. The helical agent is preferably an alpha-helical agent, which preferably has a lipophilic and a lipophobic phase.

The ligand can be a peptide or peptidomimetic. A peptidomimetic (also referred to herein as an oligopeptidomimetic) is a molecule capable of folding into a defined three-dimensional structure similar to a natural peptide. The peptide or peptidomimetic moiety can be about 5-50 amino acids long, *e.g.,* about 5, 10, 15, 20, 25, 30, 35, 40, 45, or 50 amino acids long. A peptide or peptidomimetic can be, for example, a cell permeation peptide, cationic peptide, amphipathic peptide, or hydrophobic peptide (e.g., consisting primarily of Tyr, Trp or Phe). The peptide moiety can be a dendrimer peptide, constrained peptide or crosslinked peptide. In another alternative, the peptide moiety can include a hydrophobic membrane translocation sequence (MTS). An exemplary hydrophobic MTS-containing peptide is RFGF having the amino acid sequence AAVALLPAVLLALLAP. An RFGF analogue (e.g., amino acid sequence AALLPVLLAAP) containing a hydrophobic MTS can also be a targeting moiety. The peptide moiety can be a "delivery" peptide, which can carry large polar molecules including peptides, oligonucleotides, and protein across cell membranes. For example, sequences from the HIV Tat protein (GRKKRRQRRRPPQ) and the Drosophila Antennapedia protein (RQIKIWFQNRRMKWKK) have been found to be capable of functioning as delivery peptides. A peptide or peptidomimetic can be encoded by a random sequence of DNA, such as a peptide identified from a phage-display library, or one-bead-one-compound (OBOC) combinatorial library (Lam et al., Nature, 354:82-84, 1991). Preferably the peptide or peptidomimetic tethered to an iRNA agent via an incorporated monomer unit is a cell targeting peptide such as an arginine-glycine-aspartic acid (RGD)-peptide, or RGD mimic. A peptide moiety can range in length from about 5 amino acids to about 40 amino acids. The peptide moieties can have a structural modification, such as to increase stability or direct conformational properties. Any of the structural modifications described below can be utilized. An RGD peptide moiety can be used to target a tumor cell, such as an endothelial tumor cell or a breast cancer tumor cell (Zitzmann et al., Cancer Res., 62:5139-43, 2002). An RGD peptide can facilitate targeting of an iRNA agent to tumors of a variety of other tissues, including the lung, kidney, spleen, or liver (Aoki et al., Cancer Gene Therapy 8:783-787, 2001). Preferably, the RGD peptide will facilitate targeting of an iRNA agent to the kidney. The RGD peptide can be linear or cyclic, and can be modified, e.g., glycosylated or methylated to facilitate targeting to specific tissues. For example, a glycosylated RGD peptide can deliver an iRNA agent to a tumor cell expressing α_{V}β₃ (Haubner et al., Jour. Nucl. Med., 42:326-336, 2001). Peptides that target markers enriched in proliferating cells can be used. E.g., RGD containing peptides and peptidomimetics can target cancer cells, in particular cells that exhibit an integrin. Thus, one could use RGD peptides, cyclic peptides containing RGD, RGD peptides that include D-amino acids, as well as synthetic RGD mimics. In addition to RGD, one can use other moieties that target the integrin ligand. Generally, such ligands can be used to control proliferating cells and angiogeneis. Preferred conjugates of this type lignads that targets PECAM-1, VEGF, or other cancer gene, e.g., a cancer gene described herein.

A "cell permeation peptide" is capable of permeating a cell, e.g., a microbial cell, such as a bacterial or fungal cell, or a mammalian cell, such as a human cell. A microbial cell-permeating peptide can be, for example, an α-helical linear peptide (e.g., LL-37 or Ceropin PI), a disulfide bond-containing peptide (e.g., α-defensin, β-defensin or bactenecin), or a peptide containing only one or two dominating amino acids (e.g., PR-39 or indolicidin). A cell permeation peptide can also include a nuclear localization signal (NLS). For example, a cell permeation peptide can be a bipartite amphipathic peptide, such as MPG, which is derived from the fusion peptide domain of HIV-1 gp41 and the NLS of SV40 large T antigen (Simeoni et al., Nucl. Acids Res. 31:2717-2724, 2003).

In one implementation, a targeting peptide can be an amphipathic α-helical peptide. Exemplary amphipathic α-helical peptides include, but are not limited to, cecropins, lycotoxins, paradaxins, buforin, CPF, bombinin-like peptide (BLP), cathelicidins, ceratotoxins, S. clava peptides, hagfish intestinal antimicrobial peptides (HFIAPs), magainines, brevinins-2, dermaseptins, melittins, pleurocidin, H₂A peptides, Xenopus peptides, esculentinis-1, and caerins. A number of factors will preferably be considered to maintain the integrity of helix stability. For example, a maximum number of helix stabilization residues will be utilized (e.g., leu, ala, or lys), and a minimum number helix destabilization residues will be utilized (e.g., proline, or cyclic monomeric units. The capping residue will be considered (for example Gly is an exemplary N-capping residue and/or C-terminal amidation can be used to provide an extra H-bond to stabilize the helix. Formation of salt bridges between residues with opposite charges, separated by i ± 3, or i ± 4 positions can provide stability. For example, cationic residues such as lysine, arginine, homo-arginine, ornithine or histidine can form salt bridges with the anionic residues glutamate or aspartate.

Peptide and peptidomimetic ligands include those having naturally occurring or modified peptides, e.g., D or L peptides; α, β, or γ peptides; N-methyl peptides; azapeptides; peptides having one or more amide, i.e., peptide, linkages replaced with one or more urea, thiourea, carbamate, or sulfonyl urea linkages; or cyclic peptides.

The targeting ligand can be any ligand that is capable of targeting a specific receptor. Examples are: folate, GalNAc, galactose, mannose, mannose-6P, clusters of sugars such as GalNAc cluster, mannose cluster, galactose cluster, or an apatamer. A cluster is a combination of two or more sugar units. The targeting ligands also include integrin receptor ligands, Chemokine receptor ligands, transferrin, biotin, serotonin receptor ligands, PSMA, endothelin, GCPII, somatostatin, LDL and HDL ligands. The ligands can also be based on nucleic acid, e.g., an aptamer. The aptamer can be unmodified or have any combination of modifications disclosed herein.

Endosomal release agents include imidazoles, poly or oligoimidazoles, PEIs, peptides, fusogenic peptides, polycaboxylates, polyacations, masked oligo or poly cations or anions, acetals, polyacetals, ketals/polyketyals, orthoesters, polymers with masked or unmasked cationic or anionic charges, dendrimers with masked or unmasked cationic or anionic charges.

PK modulator stands for pharmacokinetic modulator. PK modulator include lipophiles, bile acids, steroids, phospholipid analogues, peptides, protein binding agents, PEG, vitamins etc. Examplary PK modulator include, but are not limited to, cholesterol, fatty acids, cholic acid, lithocholic acid, dialkylglycerides, diacylglyceride, phospholipids, sphingolipids, naproxen, ibuprofen, vitamin E, biotin etc. Oligonucleotides that comprise a number of phosphorothioate linkages are also known to bind to serum protein, thus short oligonucleotides, e.g. oligonucleotides of about 5 bases, 10 bases, 15 bases or 20 bases, comprising multiple of phosphorothioate linkages in the backbaone are also amenable to the present disclosure as ligands (e.g. as PK modulating ligands).

In addition, aptamers that bind serum components (e.g. serum proteins) are also amenable to the present disclosure as PK modulating ligands.

Other ligand conjugates amenable to the disclosure are described in U.S. Patent Applications USSN: 10/916,185, filed August 10, 2004; USSN: 10/946,873, filed September 21, 2004; USSN: 10/833,934, filed August 3, 2007; USSN: 11/115,989 filed April 27, 2005 and USSN: 11/944,227 filed November 21, 2007.

When two or more ligands are present, the ligands can all have same properties, all have different properties or some ligands have the same properties while others have different properties. For example, a ligand can have targeting properties, have endosomolytic activity or have PK modulating properties. In a preferred implementation, all the ligands have different properties.

Ligands can be coupled to the oligonucleotides at various places, for example, 3'-end, 5'-end, and/or at an internal position. In preferred implementations, the ligand is attached to the oligonucleotides *via* an intervening tether, e.g. a carrier described herein. The ligand or tethered ligand may be present on a monomer when said monomer is incorporated into the growing strand. In some implementations, the ligand may be incorporated via coupling to a "precursor" monomer after said "precursor" monomer has been incorporated into the growing strand. For example, a monomer having, e.g., an amino-terminated tether (i.e., having no associated ligand), e.g., TAP-(CH₂)ₙNH₂ may be incorporated into a growing oligonucelotide strand. In a subsequent operation, i.e., after incorporation of the precursor monomer into the strand, a ligand having an electrophilic group, e.g., a pentafluorophenyl ester or aldehyde group, can subsequently be attached to the precursor monomer by coupling the electrophilic group of the ligand with the terminal nucleophilic group of the precursor monomer's tether.

In another example, a monomer having a chemical group suitable for taking part in Click Chemistry reaction may be incorporated e.g., an azide or alkyne terminated tether/linker. In a subsequent operation, i.e., after incorporation of the precursor monomer into the strand, a ligand having complementary chemical group, e.g. an alkyne or azide can be attached to the precursor monomer by coupling the alkyne and the azide together.

For double- stranded oligonucleotides, ligands can be attached to one or both strands. In some implementations, a double-stranded iRNA agent contains a ligand conjugated to the sense strand. In other implementations, a double-stranded iRNA agent contains a ligand conjugated to the antisense strand.

In some implementations, ligand can be conjugated to nucleobases, sugar moieties, or internucleosidic linkages of nucleic acid molecules. Conjugation to purine nucleobases or derivatives thereof can occur at any position including, endocyclic and exocyclic atoms. In some implementations, the 2-, 6-, 7-, or 8-positions of a purine nucleobase are attached to a conjugate moiety. Conjugation to pyrimidine nucleobases or derivatives thereof can also occur at any position. In some implementations, the 2-, 5-, and 6-positions of a pyrimidine nucleobase can be substituted with a conjugate moiety. Conjugation to sugar moieties of nucleosides can occur at any carbon atom. Example carbon atoms of a sugar moiety that can be attached to a conjugate moiety include the 2', 3', and 5' carbon atoms. The 1' position can also be attached to a conjugate moiety, such as in an abasic residue. Internucleosidic linkages can also bear conjugate moieties. For phosphorus-containing linkages (e.g., phosphodiester, phosphorothioate, phosphorodithiotate, phosphoroamidate, and the like), the conjugate moiety can be attached directly to the phosphorus atom or to an O, N, or S atom bound to the phosphorus atom. For amine- or amide-containing internucleosidic linkages (e.g., PNA), the conjugate moiety can be attached to the nitrogen atom of the amine or amide or to an adjacent carbon atom.

Any suitable ligand in the field of RNA interference may be used, although the ligand is typically a carbohydrate e.g. monosaccharide (such as GalNAc), disaccharide, trisaccharide, tetrasaccharide, polysaccharide.

Linkers that conjugate the ligand to the nucleic acid include those discussed above. For example, the ligand can be one or more GalNAc (*N*-acetylglucosamine) derivatives attached through a bivalent or trivalent branched linker.

In one implementation, the dsRNA of the disclosure is conjugated to a bivalent and trivalent branched linkers include the structures shown in any of formula (IV) - (VII): 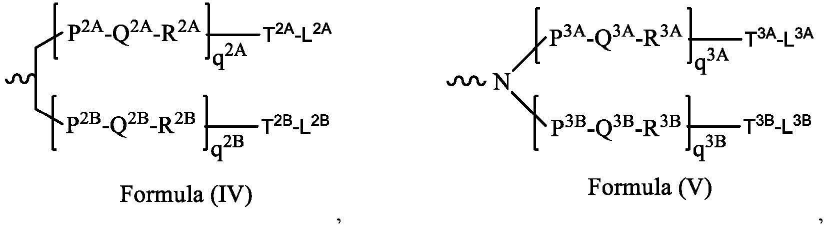 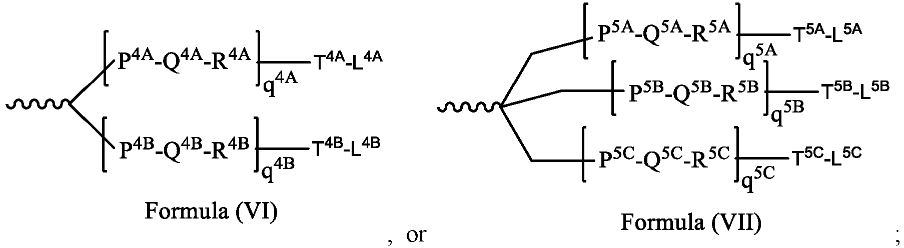 wherein:
q^{2A}, q^{2B}, q^{3A}, q^{3B}, q^{4A}, q^{4B} q^{5A}, q^{5B} and q^{5C} represent independently for each occurrence 0-20 and wherein the repeating unit can be the same or different;
P^{2A}, P^{2B}, P^{3A}, P^{3B}, P^{4A}, P^{4B}, P^{5A}, P^{5B}, P^{5C}, T^{2A}, T^{2B}, T^{3A}, T^{3B}, T^{4A}, T^{4B}, T^{4A}, T^{5B}, T^{5c} are each independently for each occurrence absent, CO, NH, O, S, OC(O), NHC(O), CH₂, CH₂NH or CH₂O;
Q^{2A}, Q^{2B}, Q^{3A}, Q^{3B}, Q^{4A}, Q^{4B}, Q^{5A}, Q^{5B}, Q^{5C} are independently for each occurrence absent, alkylene, substituted alkylene wherin one or more methylenes can be interrupted or terminated by one or more of O, S, S(O), SO₂, N(R^{N}), C(R')=C(R"), C=C or C(O);
R^{2A}, R^{2B}, R^{3A}, R^{3B}, R^{4A}, R^{4B}, R^{5A}, R^{5B}, R^{5C} are each independently for each occurrence absent, NH, O, S, CH₂, C(O)O, C(O)NH, NHCH(R^{a})C(O), -C(O)-CH(R^{a})-NH-, CO, CH=N-O 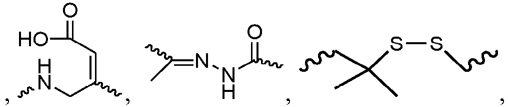 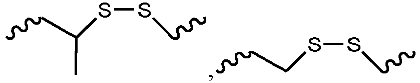
or heterocyclyl;
L^{2A}, L^{2B}, L^{3A}, L^{3B}, L^{4A}, L^{4B}, L^{5A}, L^{5B} and L^{5C} represent the ligand; i.e. each independently for each occurrence a monosaccharide (such as GalNAc), disaccharide, trisaccharide, tetrasaccharide, oligosaccharide, or polysaccharide; and
R^{a} is H or amino acid side chain.

Trivalent conjugating GalNAc derivatives are particularly useful for use with RNAi agents for inhibiting the expression of a target gene, such as those of formula (VII): 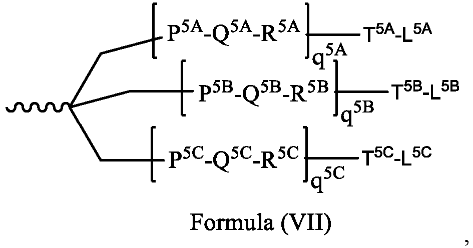 wherein L^{5A}, L^{5B} and L^{5C} represent a monosaccharide, such as GalNAc derivative.

Examples of suitable bivalent and trivalent branched linker groups conjugating GalNAc derivatives include, but are not limited to, the following compounds: 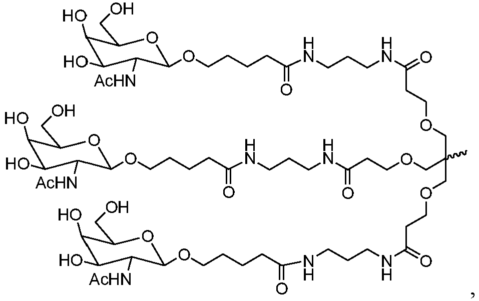 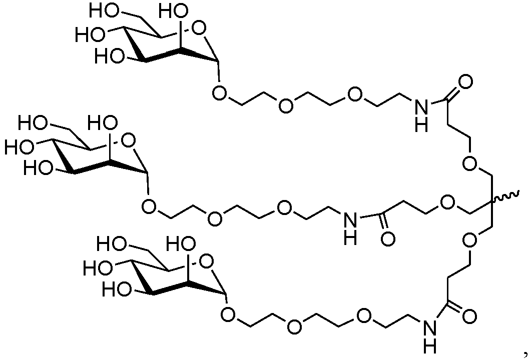 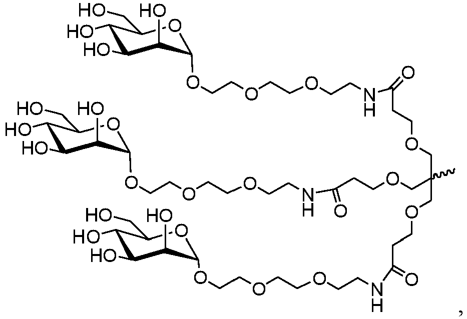 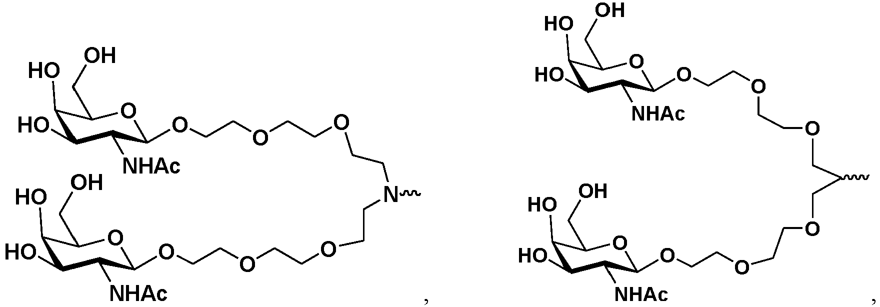 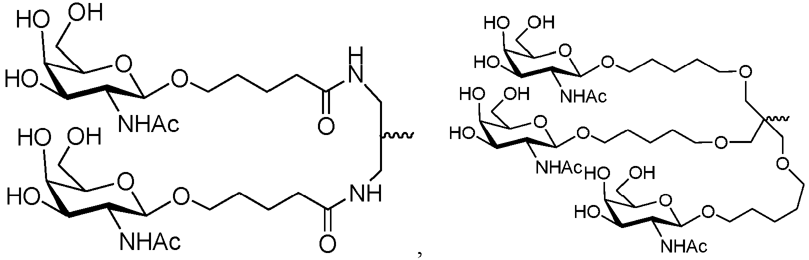 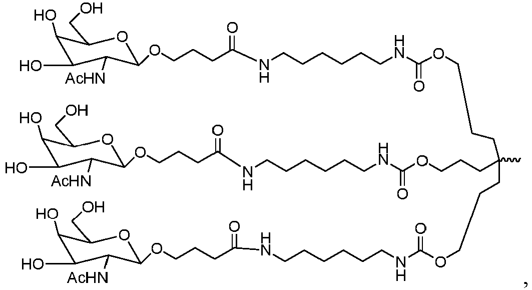 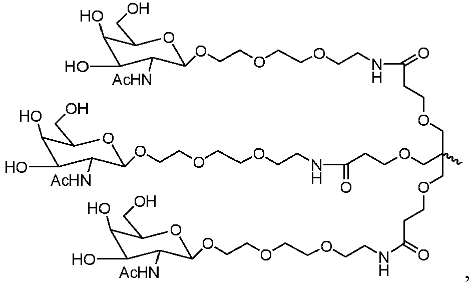 or

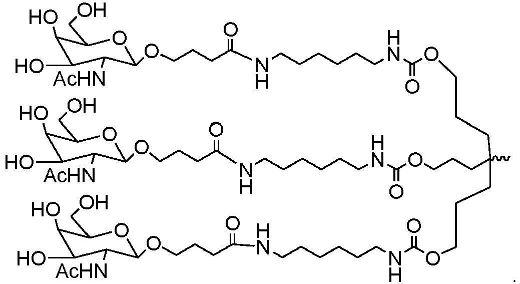

### Definitions

As used herein, the terms "dsRNA", "siRNA", and "iRNA agent" are used interchangeably to agents that can mediate silencing of a target RNA, *e.g.,* mRNA, *e.g.,* a transcript of a gene that encodes a protein. For convenience, such mRNA is also referred to herein as mRNA to be silenced. Such a gene is also referred to as a target gene. In general, the RNA to be silenced is an endogenous gene or a pathogen gene. In addition, RNAs other than mRNA, e.g., tRNAs, and viral RNAs, can also be targeted.

As used herein, the phrase "mediates RNAi" refers to the ability to silence, in a sequence specific manner, a target RNA. While not wishing to be bound by theory, it is believed that silencing uses the RNAi machinery or process and a guide RNA, *e.g.,* an siRNA agent of 21 to 23 nucleotides.

As used herein, "specifically hybridizable" and "complementary" are terms which are used to indicate a sufficient degree of complementarity such that stable and specific binding occurs between a compound of the disclosure and a target RNA molecule. Specific binding requires a sufficient degree of complementarity to avoid non-specific binding of the oligomeric compound to non-target sequences under conditions in which specific binding is desired, *i.e.,* under physiological conditions in the case of assays or therapeutic treatment, or in the case of *in vitro* assays, under conditions in which the assays are performed. The non-target sequences typically differ by at least 5 nucleotides.

In one implementation, a dsRNA agent of the disclosure is "sufficiently complementary" to a target RNA, *e.g.,* a target mRNA, such that the dsRNA agent silences production of protein encoded by the target mRNA. In another implementation, the dsRNA agent of the disclosure is "exactly complementary" to a target RNA, *e.g.,* the target RNA and the dsRNA duplex agent anneal, for example to form a hybrid made exclusively of Watson-Crick base pairs in the region of exact complementarity. A "sufficiently complementary" target RNA can include an internal region *(e.g.,* of at least 10 nucleotides) that is exactly complementary to a target RNA. Moreover, in some implementations, the dsRNA agent of the disclosure specifically discriminates a single-nucleotide difference. In this case, the dsRNA agent only mediates RNAi if exact complementary is found in the region (e.g., within 7 nucleotides of) the single-nucleotide difference.

As used herein, the term "oligonucleotide" refers to a nucleic acid molecule (RNA or DNA) for example of length less than 100, 200, 300, or 400 nucleotides.

The term "halo" refers to any radical of fluorine, chlorine, bromine or iodine. The term "alkyl" refers to saturated and unsaturated non-aromatic hydrocarbon chains that may be a straight chain or branched chain, containing the indicated number of carbon atoms (these include without limitation propyl, allyl, or propargyl), which may be optionally inserted with N, O, or S. For example, C₁-C₁₀ indicates that the group may have from 1 to 10 (inclusive) carbon atoms in it. The term "alkoxy" refers to an -O-alkyl radical. The term "alkylene" refers to a divalent alkyl (*i.e.,* -R-). The term "alkylenedioxo" refers to a divalent species of the structure -O-R-O-, in which R represents an alkylene. The term "aminoalkyl" refers to an alkyl substituted with an amino The term "mercapto" refers to an -SH radical. The term "thioalkoxy" refers to an - S-alkyl radical.

The term "aryl" refers to a 6-carbon monocyclic or 10-carbon bicyclic aromatic ring system wherein 0, 1, 2, 3, or 4 atoms of each ring may be substituted by a substituent. Examples of aryl groups include phenyl, naphthyl and the like. The term "arylalkyl" or the term "aralkyl" refers to alkyl substituted with an aryl. The term "arylalkoxy" refers to an alkoxy substituted with aryl.

The term "cycloalkyl" as employed herein includes saturated and partially unsaturated cyclic hydrocarbon groups having 3 to 12 carbons, for example, 3 to 8 carbons, and, for example, 3 to 6 carbons, wherein the cycloalkyl group additionally may be optionally substituted. Cycloalkyl groups include, without limitation, cyclopropyl, cyclobutyl, cyclopentyl, cyclopentenyl, cyclohexyl, cyclohexenyl, cycloheptyl, and cyclooctyl.

The term "heteroaryl" refers to an aromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S *(e.g.,* carbon atoms and 1-3, 1-6, or 1-9 heteroatoms of N, O, or S if monocyclic, bicyclic, or tricyclic, respectively), wherein 0, 1, 2, 3, or 4 atoms of each ring may be substituted by a substituent. Examples of heteroaryl groups include pyridyl, furyl or furanyl, imidazolyl, benzimidazolyl, pyrimidinyl, thiophenyl or thienyl, quinolinyl, indolyl, thiazolyl, and the like. The term "heteroarylalkyl" or the term "heteroaralkyl" refers to an alkyl substituted with a heteroaryl. The term "heteroarylalkoxy" refers to an alkoxy substituted with heteroaryl.

The term "heterocyclyl" refers to a nonaromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S *(e.g.,* carbon atoms and 1-3, 1-6, or 1-9 heteroatoms of N, O, or S if monocyclic, bicyclic, or tricyclic, respectively), wherein 0, 1, 2 or 3 atoms of each ring may be substituted by a substituent. Examples of heterocyclyl groups include trizolyl, tetrazolyl, piperazinyl, pyrrolidinyl, dioxanyl, morpholinyl, tetrahydrofuranyl, and the like.

The term "oxo" refers to an oxygen atom, which forms a carbonyl when attached to carbon, an N-oxide when attached to nitrogen, and a sulfoxide or sulfone when attached to sulfur.

The term "acyl" refers to an alkylcarbonyl, cycloalkylcarbonyl, arylcarbonyl, heterocyclylcarbonyl, or heteroarylcarbonyl substituent, any of which may be further substituted by substituents.

The term "substituted" refers to the replacement of one or more hydrogen radicals in a given structure with the radical of a specified substituent including, but not limited to: halo, alkyl, alkenyl, alkynyl, aryl, heterocyclyl, thiol, alkylthio, arylthio, alkylthioalkyl, arylthioalkyl, alkylsulfonyl, alkylsulfonylalkyl, arylsulfonylalkyl, alkoxy, aryloxy, aralkoxy, aminocarbonyl, alkylaminocarbonyl, arylaminocarbonyl, alkoxycarbonyl, aryloxycarbonyl, haloalkyl, amino, trifluoromethyl, cyano, nitro, alkylamino, arylamino, alkylaminoalkyl, arylaminoalkyl, aminoalkylamino, hydroxy, alkoxyalkyl, carboxyalkyl, alkoxycarbonylalkyl, aminocarbonylalkyl, acyl, aralkoxycarbonyl, carboxylic acid, sulfonic acid, sulfonyl, phosphonic acid, aryl, heteroaryl, heterocyclic, and aliphatic. It is understood that the substituent can be further substituted.

### Cleavable Linking Groups

A cleavable linking group is one which is sufficiently stable outside the cell, but which upon entry into a target cell is cleaved to release the two parts the linker is holding together. In a preferred implementation, the cleavable linking group is cleaved at least 10 times or more, preferably at least 100 times faster in the target cell or under a first reference condition (which can, e.g., be selected to mimic or represent intracellular conditions) than in the blood of a subject, or under a second reference condition (which can, e.g., be selected to mimic or represent conditions found in the blood or serum).

Cleavable linking groups are susceptible to cleavage agents, e.g., pH, redox potential or the presence of degradative molecules. Generally, cleavage agents are more prevalent or found at higher levels or activities inside cells than in serum or blood. Examples of such degradative agents include: redox agents which are selected for particular substrates or which have no substrate specificity, including, e.g., oxidative or reductive enzymes or reductive agents such as mercaptans, present in cells, that can degrade a redox cleavable linking group by reduction; esterases; endosomes or agents that can create an acidic environment, e.g., those that result in a pH of five or lower; enzymes that can hydrolyze or degrade an acid cleavable linking group by acting as a general acid, peptidases (which can be substrate specific), and phosphatases.

A cleavable linkage group, such as a disulfide bond can be susceptible to pH. The pH of human serum is 7.4, while the average intracellular pH is slightly lower, ranging from about 7.1-7.3. Endosomes have a more acidic pH, in the range of 5.5-6.0, and lysosomes have an even more acidic pH at around 5.0. Some linkers will have a cleavable linking group that is cleaved at a preferred pH, thereby releasing the cationic lipid from the ligand inside the cell, or into the desired compartment of the cell.

A linker can include a cleavable linking group that is cleavable by a particular enzyme. The type of cleavable linking group incorporated into a linker can depend on the cell to be targeted. For example, liver targeting ligands can be linked to the cationic lipids through a linker that includes an ester group. Liver cells are rich in esterases, and therefore the linker will be cleaved more efficiently in liver cells than in cell types that are not esterase-rich. Other cell-types rich in esterases include cells of the lung, renal cortex, and testis.

Linkers that contain peptide bonds can be used when targeting cell types rich in peptidases, such as liver cells and synoviocytes.

In general, the suitability of a candidate cleavable linking group can be evaluated by testing the ability of a degradative agent (or condition) to cleave the candidate linking group. It will also be desirable to also test the candidate cleavable linking group for the ability to resist cleavage in the blood or when in contact with other non-target tissue. Thus one can determine the relative susceptibility to cleavage between a first and a second condition, where the first is selected to be indicative of cleavage in a target cell and the second is selected to be indicative of cleavage in other tissues or biological fluids, e.g., blood or serum. The evaluations can be carried out in cell free systems, in cells, in cell culture, in organ or tissue culture, or in whole animals. It may be useful to make initial evaluations in cell-free or culture conditions and to confirm by further evaluations in whole animals. In preferred implementations, useful candidate compounds are cleaved at least 2, 4, 10 or 100 times faster in the cell (or under in vitro conditions selected to mimic intracellular conditions) as compared to blood or serum (or under in vitro conditions selected to mimic extracellular conditions).

### Redox cleavable linking groups

One class of cleavable linking groups are redox cleavable linking groups that are cleaved upon reduction or oxidation. An example of reductively cleavable linking group is a disulphide linking group (-S-S-). To determine if a candidate cleavable linking group is a suitable "reductively cleavable linking group," or for example is suitable for use with a particular iRNA moiety and particular targeting agent one can look to methods described herein. For example, a candidate can be evaluated by incubation with dithiothreitol (DTT), or other reducing agent using reagents know in the art, which mimic the rate of cleavage which would be observed in a cell, e.g., a target cell. The candidates can also be evaluated under conditions which are selected to mimic blood or serum conditions. In a preferred implementation, candidate compounds are cleaved by at most 10% in the blood. In preferred implementations, useful candidate compounds are degraded at least 2, 4, 10 or 100 times faster in the cell (or under in vitro conditions selected to mimic intracellular conditions) as compared to blood (or under in vitro conditions selected to mimic extracellular conditions). The rate of cleavage of candidate compounds can be determined using standard enzyme kinetics assays under conditions chosen to mimic intracellular media and compared to conditions chosen to mimic extracellular media.

### Phosphate-based cleavable linking groups

Phosphate-based cleavable linking groups are cleaved by agents that degrade or hydrolyze the phosphate group. An example of an agent that cleaves phosphate groups in cells are enzymes such as phosphatases in cells. Examples of phosphate-based linking groups are -O-P(O)(ORk)-O-, -O-P(S)(ORk)-O-, -O-P(S)(SRk)-O-, -S-P(O)(ORk)-O-, - O-P(O)(ORk)-S-, -S-P(O)(ORk)-S-, -O-P(S)(ORk)-S-, -S-P(S)(ORk)-O-, -O-P(O)(Rk)-O-, -O-P(S)(Rk)-O-, -S-P(O)(Rk)-O-, -S-P(S)(Rk)-O-, -S-P(O)(Rk)-S-, -O-P(S)( Rk)-S-. Preferred implementations are -O-P(O)(OH)-O-, -O-P(S)(OH)-O-, -O-P(S)(SH)-O-, -S-P(O)(OH)-O-, -O-P(O)(OH)-S-, -S-P(O)(OH)-S-, -O-P(S)(OH)-S-, -S-P(S)(OH)-O-, -OP(O)(H)-O-, -O-P(S)(H)-O-, -S-P(O)(H)-O-, -S-P(S)(H)-O-, -S-P(O)(H)-S-, -O-P(S)(H)-S-. A preferred implementation is -O-P(O)(OH)-O-. These candidates can be evaluated using methods analogous to those described above.

### Acid cleavable linking groups

Acid cleavable linking groups are linking groups that are cleaved under acidic conditions. In preferred implementations acid cleavable linking groups are cleaved in an acidic environment with a pH of about 6.5 or lower (e.g., about 6.0, 5.5, 5.0, or lower), or by agents such as enzymes that can act as a general acid. In a cell, specific low pH organelles, such as endosomes and lysosomes can provide a cleaving environment for acid cleavable linking groups. Examples of acid cleavable linking groups include but are not limited to hydrazones, esters, and esters of amino acids. Acid cleavable groups can have the general formula -C=NN-, C(O)O, or -OC(O). A preferred implementation is when the carbon attached to the oxygen of the ester (the alkoxy group) is an aryl group, substituted alkyl group, or tertiary alkyl group such as dimethyl pentyl or t-butyl. These candidates can be evaluated using methods analogous to those described above.

### Ester-based linking groups

Ester-based cleavable linking groups are cleaved by enzymes such as esterases and amidases in cells. Examples of ester-based cleavable linking groups include but are not limited to esters of alkylene, alkenylene and alkynylene groups. Ester cleavable linking groups have the general formula -C(O)O-, or -OC(O)-. These candidates can be evaluated using methods analogous to those described above.

### Peptide-based cleaving groups

Peptide-based cleavable linking groups are cleaved by enzymes such as peptidases and proteases in cells. Peptide-based cleavable linking groups are peptide bonds formed between amino acids to yield oligopeptides (e.g., dipeptides, tripeptides etc.) and polypeptides. Peptide-based cleavable groups do not include the amide group (-C(O)NH-). The amide group can be formed between any alkylene, alkenylene or alkynelene. A peptide bond is a special type of amide bond formed between amino acids to yield peptides and proteins. The peptide based cleavage group is generally limited to the peptide bond (i.e., the amide bond) formed between amino acids yielding peptides and proteins and does not include the entire amide functional group. Peptide-based cleavable linking groups have the general formula - NHCHR^{A}C(O)NHCHR^{B}C(O)-, where R^{A} and R^{B} are the R groups of the two adjacent amino acids. These candidates can be evaluated using methods analogous to those described above. As used herein, "carbohydrate" refers to a compound which is either a carbohydrate per se made up of one or more monosaccharide units having at least 6 carbon atoms (which may be linear, branched or cyclic) with an oxygen, nitrogen or sulfur atom bonded to each carbon atom; or a compound having as a part thereof a carbohydrate moiety made up of one or more monosaccharide units each having at least six carbon atoms (which may be linear, branched or cyclic), with an oxygen, nitrogen or sulfur atom bonded to each carbon atom. Representative carbohydrates include the sugars (mono-, di-, tri- and oligosaccharides containing from about 4-9 monosaccharide units), and polysaccharides such as starches, glycogen, cellulose and polysaccharide gums. Specific monosaccharides include C₅ and above (preferably C₅ -C₈) sugars; di- and trisaccharides include sugars having two or three monosaccharide units (preferably C₅ -C₈).

### Alternative implementations

In another implementation, the disclosure relates to a dsRNA agent capable of inhibiting the expression of a target gene. The dsRNA agent comprises a sense strand and an antisense strand, each strand having 14 to 30 nucleotides. The sense strand contains at least one motif of three identical modifications on three consecutive nucleotides, where at least one of the motifs occurs at or near the cleavage site in the antisense strand. Every nucleotide in the sense strand and antisense strand has been modified. The modifications on sense strand and antisense strand each independently comprises at least two different modifications.

In another implementation, the disclosure relates to a dsRNA agent capable of inhibiting the expression of a target gene. The dsRNA agent comprises a sense strand and an antisense strand, each strand having 14 to 30 nucleotides. The sense strand contains at least one motif of three identical modifications on three consecutive nucleotides, where at least one of the motifs occurs at or near the cleavage site in the antisense strand. The antisense strand contains at least one motif of three identical modifications on three consecutive nucleotides. The modification pattern of the antisense strand is shifted by one or more nucleotides relative to the modification pattern of the sense strand.

In another implementation, the disclosure relates to a dsRNA agent capable of inhibiting the expression of a target gene. The dsRNA agent comprises a sense strand and an antisense strand, each strand having 14 to 30 nucleotides. The sense strand contains at least two motifs of three identical modifications on three consecutive nucleotides, when at least one of the motifs occurs at the cleavage site in the strand and at least one of the motifs occurs at another portion of the strand that is separated from the motif at the cleavage site by at least one nucleotide. The antisense strand contains at least one motif of three identical modifications on three consecutive nucleotides, where at least one of the motifs occurs at or near the cleavage site in the strand and at least one of the motifs occurs at another portion of the strand that is separated from the motif at or near cleavage site by at least one nucleotide.

In another implementation, the disclosure relates to a dsRNA agent capable of inhibiting the expression of a target gene. The dsRNA agent comprises a sense strand and an antisense strand, each strand having 14 to 30 nucleotides. The sense strand contains at least two motifs of three identical modifications on three consecutive nucleotides, where at least one of the motifs occurs at the cleavage site in the strand and at least one of the motifs occurs at another portion of the strand that is separated from the motif at the cleavage site by at least one nucleotide. The antisense strand contains at least one motif of three identical modifications on three consecutive nucleotides, where at least one of the motifs occurs at or near the cleavage site in the strand and at least one of the motifs occurs at another portion of the strand that is separated from the motif at or near cleavage site by at least one nucleotide. The modification in the motif occurring at the cleavage site in the sense strand is different than the modification in the motif occurring at or near the cleavage site in the antisense strand. In another implementation, the disclosure relates to a dsRNA agent capable of inhibiting the expression of a target gene. The dsRNA agent comprises a sense strand and an antisense strand, each strand having 12 to 30 nucleotides. The sense strand contains at least one motif of three 2'-F modifications on three consecutive nucleotides, where at least one of the motifs occurs at the cleavage site in the strand. The antisense strand contains at least one motif of three 2'-O-methyl modifications on three consecutive nucleotides.

The sense strand may further comprises one or more motifs of three identical modifications on three consecutive nucleotides, where the one or more additional motifs occur at another portion of the strand that is separated from the three 2'-F modifications at the cleavage site by at least one nucleotide. The antisense strand may further comprises one or more motifs of three identical modifications on three consecutive nucleotides, where the one or more additional motifs occur at another portion of the strand that is separated from the three 2'-O-methyl modifications by at least one nucleotide. At least one of the nucleotides having a 2'-F modification may form a base pair with one of the nucleotides having a 2'-O-methyl modification.

In one eimplementation, the dsRNA of the disclosure is administered in buffer.

In one implementation, siRNA compounds described herein can be formulated for administration to a subject. A formulated siRNA composition can assume a variety of states. In some examples, the composition is at least partially crystalline, uniformly crystalline, and/or anhydrous (e.g., less than 80, 50, 30, 20, or 10% water). In another example, the siRNA is in an aqueous phase, *e.g.,* in a solution that includes water.

The aqueous phase or the crystalline compositions can, *e.g.,* be incorporated into a delivery vehicle, e.g., a liposome (particularly for the aqueous phase) or a particle *(e.g.,* a microparticle as can be appropriate for a crystalline composition). Generally, the siRNA composition is formulated in a manner that is compatible with the intended method of administration, as described herein. For example, in particular implementations the composition is prepared by at least one of the following methods: spray drying, lyophilization, vacuum drying, evaporation, fluid bed drying, or a combination of these techniques; or sonication with a lipid, freeze-drying, condensation and other self-assembly.

A siRNA preparation can be formulated in combination with another agent, *e.g*., another therapeutic agent or an agent that stabilizes a siRNA, *e.g.,* a protein that complexes with siRNA to form an iRNP. Still other agents include chelators, e.g., EDTA *(e.g.,* to remove divalent cations such as Mg²⁺), salts, RNAse inhibitors *(e.g.,* a broad specificity RNAse inhibitor such as RNAsin) and so forth.

In one implementation, the siRNA preparation includes another siNA compound, *e.g.,* a second siRNA that can mediate RNAi with respect to a second gene, or with respect to the same gene. Still other preparation can include at least 3, 5, ten, twenty, fifty, or a hundred or more different siRNA species. Such siRNAs can mediate RNAi with respect to a similar number of different genes.

In one implementation, the siRNA preparation includes at least a second therapeutic agent *( e.g.,* an agent other than a RNA or a DNA). For example, a siRNA composition for the treatment of a viral disease, e.g., HIV, might include a known antiviral agent *(e.g.,* a protease inhibitor or reverse transcriptase inhibitor). In another example, a siRNA composition for the treatment of a cancer might further comprise a chemotherapeutic agent.

Exemplary formulations are discussed below.

*Liposomes.* For ease of exposition the formulations, compositions and methods in this section are discussed largely with regard to unmodified siRNA compounds. It may be understood, however, that these formulations, compositions and methods can be practiced with other siRNA compounds, e.g., modified siRNAs, and such practice is within the disclosure. An siRNA compound, *e.g.,* a double-stranded siRNA compound, or ssiRNA compound, *(e.g.,* a precursor, *e.g.,* a larger siRNA compound which can be processed into a ssiRNA compound, or a DNA which encodes an siRNA compound, e.g., a double-stranded siRNA compound, or ssiRNA compound, or precursor thereof) preparation can be formulated for delivery in a membranous molecular assembly, *e.g*., a liposome or a micelle. As used herein, the term "liposome" refers to a vesicle composed of amphiphilic lipids arranged in at least one bilayer, *e.g*., one bilayer or a plurality of bilayers. Liposomes include unilamellar and multilamellar vesicles that have a membrane formed from a lipophilic material and an aqueous interior. The aqueous portion contains the siRNA composition. The lipophilic material isolates the aqueous interior from an aqueous exterior, which typically does not include the siRNA composition, although in some examples, it may. Liposomes are useful for the transfer and delivery of active ingredients to the site of action. Because the liposomal membrane is structurally similar to biological membranes, when liposomes are applied to a tissue, the liposomal bilayer fuses with bilayer of the cellular membranes. As the merging of the liposome and cell progresses, the internal aqueous contents that include the siRNA are delivered into the cell where the siRNA can specifically bind to a target RNA and can mediate RNAi. In some cases the liposomes are also specifically targeted, *e.g.,* to direct the siRNA to particular cell types.

A liposome containing a siRNA can be prepared by a variety of methods. In one example, the lipid component of a liposome is dissolved in a detergent so that micelles are formed with the lipid component. For example, the lipid component can be an amphipathic cationic lipid or lipid conjugate. The detergent can have a high critical micelle concentration and may be nonionic. Exemplary detergents include cholate, CHAPS, octylglucoside, deoxycholate, and lauroyl sarcosine. The siRNA preparation is then added to the micelles that include the lipid component. The cationic groups on the lipid interact with the siRNA and condense around the siRNA to form a liposome. After condensation, the detergent is removed, *e.g*., by dialysis, to yield a liposomal preparation of siRNA.

If necessary a carrier compound that assists in condensation can be added during the condensation reaction, *e.g*., by controlled addition. For example, the carrier compound can be a polymer other than a nucleic acid (*e.g*., spermine or spermidine). pH can also adjusted to favor condensation.

Further description of methods for producing stable polynucleotide delivery vehicles, which incorporate a polynucleotide/cationic lipid complex as structural components of the delivery vehicle, are described in, *e.g.,* WO 96/37194. Liposome formation can also include one or more aspects of exemplary methods described in Feigner, P. L. et al., Proc. Natl. Acad. Sci., USA 8:7413-7417, 1987; U.S. Pat. No. 4,897,355; U.S. Pat. No. 5,171,678; Bangham, et al. M. Mol. Biol. 23:238, 1965; Olson, et al. Biochim. Biophys. Acta 557:9, 1979; Szoka, et al. Proc. Natl. Acad. Sci. 75: 4194, 1978; Mayhew, et al. Biochim. Biophys. Acta 775:169, 1984; Kim, et al. Biochim. Biophys. Acta 728:339, 1983; and Fukunaga, et al. Endocrinol. 115:757, 1984. Commonly used techniques for preparing lipid aggregates of appropriate size for use as delivery vehicles include sonication and freeze-thaw plus extrusion (see, *e.g.,* Mayer, et al. Biochim. Biophys. Acta 858:161, 1986). Microfluidization can be used when consistently small (50 to 200 nm) and relatively uniform aggregates are desired (Mayhew, et al. Biochim. Biophys. Acta 775:169, 1984). These methods are readily adapted to packaging siRNA preparations into liposomes.

Liposomes that are pH-sensitive or negatively-charged entrap nucleic acid molecules rather than complex with them. Since both the nucleic acid molecules and the lipid are similarly charged, repulsion rather than complex formation occurs. Nevertheless, some nucleic acid molecules are entrapped within the aqueous interior of these liposomes. pH-sensitive liposomes have been used to deliver DNA encoding the thymidine kinase gene to cell monolayers in culture. Expression of the exogenous gene was detected in the target cells (Zhou et al., Journal of Controlled Release, 19, (1992) 269-274).

One major type of liposomal composition includes phospholipids other than naturally-derived phosphatidylcholine. Neutral liposome compositions, for example, can be formed from dimyristoyl phosphatidylcholine (DMPC) or dipalmitoyl phosphatidylcholine (DPPC). Anionic liposome compositions generally are formed from dimyristoyl phosphatidylglycerol, while anionic fusogenic liposomes are formed primarily from dioleoyl phosphatidylethanolamine (DOPE). Another type of liposomal composition is formed from phosphatidylcholine (PC) such as, for example, soybean PC, and egg PC. Another type is formed from mixtures of phospholipid and/or phosphatidylcholine and/or cholesterol.

Examples of other methods to introduce liposomes into cells *in vitro* and include U.S. Pat. No. 5,283,185; U.S. Pat. No. 5,171,678; WO 94/00569; WO 93/24640; WO 91/16024; Feigner, J. Biol. Chem. 269:2550, 1994; Nabel, Proc. Natl. Acad. Sci. 90:11307, 1993; Nabel, Human Gene Ther. 3:649, 1992; Gershon, Biochem. 32:7143, 1993; and Strauss EMBO J. 11:417, 1992.

In one implementation, cationic liposomes are used. Cationic liposomes possess the advantage of being able to fuse to the cell membrane. Non-cationic liposomes, although not able to fuse as efficiently with the plasma membrane, are taken up by macrophages *in vivo* and can be used to deliver siRNAs to macrophages.

Further advantages of liposomes include: liposomes obtained from natural phospholipids are biocompatible and biodegradable; liposomes can incorporate a wide range of water and lipid soluble drugs; liposomes can protect encapsulated siRNAs in their internal compartments from metabolism and degradation (Rosoff, in "Pharmaceutical Dosage Forms," Lieberman, Rieger and Banker (Eds.), 1988, volume 1, p. 245). Important considerations in the preparation of liposome formulations are the lipid surface charge, vesicle size and the aqueous volume of the liposomes.

A positively charged synthetic cationic lipid, N-[1-(2,3-dioleyloxy)propyl]-N,N,N-trimethylammonium chloride (DOTMA) can be used to form small liposomes that interact spontaneously with nucleic acid to form lipid-nucleic acid complexes which are capable of fusing with the negatively charged lipids of the cell membranes of tissue culture cells, resulting in delivery of siRNA (see, *e.g.,* Feigner, P. L. et al., Proc. Natl. Acad. Sci., USA 8:7413-7417, 1987 and U.S. Pat. No. 4,897,355 for a description of DOTMA and its use with DNA).

A DOTMA analogue, 1,2-bis(oleoyloxy)-3-(trimethylammonia)propane (DOTAP) can be used in combination with a phospholipid to form DNA-complexing vesicles. Lipofectin^{™} Bethesda Research Laboratories, Gaithersburg, Md.) is an effective agent for the delivery of highly anionic nucleic acids into living tissue culture cells that comprise positively charged DOTMA liposomes which interact spontaneously with negatively charged polynucleotides to form complexes. When enough positively charged liposomes are used, the net charge on the resulting complexes is also positive. Positively charged complexes prepared in this way spontaneously attach to negatively charged cell surfaces, fuse with the plasma membrane, and efficiently deliver functional nucleic acids into, for example, tissue culture cells. Another commercially available cationic lipid, 1,2-bis(oleoyloxy)-3,3-(trimethylammonia)propane ("DOTAP") (Boehringer Mannheim, Indianapolis, Indiana) differs from DOTMA in that the oleoyl moieties are linked by ester, rather than ether linkages.

Other reported cationic lipid compounds include those that have been conjugated to a variety of moieties including, for example, carboxyspermine which has been conjugated to one of two types of lipids and includes compounds such as 5-carboxyspermylglycine dioctaoleoylamide ("DOGS") (Transfectam^{™}, Promega, Madison, Wisconsin) and dipalmitoylphosphatidylethanolamine 5-carboxyspermyl-amide ("DPPES") (see, *e.g.,* U.S. Pat. No. 5,171,678).

Another cationic lipid conjugate includes derivatization of the lipid with cholesterol ("DC-Chol") which has been formulated into liposomes in combination with DOPE (See, Gao, X. and Huang, L., Biochim. Biophys. Res. Commun. 179:280, 1991). Lipopolylysine, made by conjugating polylysine to DOPE, has been reported to be effective for transfection in the presence of serum (Zhou, X. et al., Biochim. Biophys. Acta 1065:8, 1991). For certain cell lines, these liposomes containing conjugated cationic lipids, are said to exhibit lower toxicity and provide more efficient transfection than the DOTMA-containing compositions. Other commercially available cationic lipid products include DMRIE and DMRIE-HP (Vical, La Jolla, California) and Lipofectamine (DOSPA) (Life Technology, Inc., Gaithersburg, Maryland). Other cationic lipids suitable for the delivery of oligonucleotides are described in WO 98/39359 and WO 96/37194.

Liposomal formulations are particularly suited for topical administration, liposomes present several advantages over other formulations. Such advantages include reduced side effects related to high systemic absorption of the administered drug, increased accumulation of the administered drug at the desired target, and the ability to administer siRNA, into the skin. In some implementations, liposomes are used for delivering siRNA to epidermal cells and also to enhance the penetration of siRNA into dermal tissues, *e.g*., into skin. For example, the liposomes can be applied topically. Topical delivery of drugs formulated as liposomes to the skin has been documented (see, *e.g.,* Weiner et al., Journal of Drug Targeting, 1992, vol. 2,405-410 and du Plessis et al., Antiviral Research, 18, 1992, 259-265; Mannino, R. J. and Fould-Fogerite, S., Biotechniques 6:682-690, 1988; Itani, T. et al. Gene 56:267-276. 1987; Nicolau, C. et al. Meth. Enz. 149:157-176, 1987; Straubinger, R. M. and Papahadjopoulos, D. Meth. Enz. 101:512-527, 1983; Wang, C. Y. and Huang, L., Proc. Natl. Acad. Sci. USA 84:7851-7855, 1987).

Non-ionic liposomal systems have also been examined to determine their utility in the delivery of drugs to the skin, in particular systems comprising non-ionic surfactant and cholesterol. Non-ionic liposomal formulations comprising Novasome I (glyceryl dilaurate/cholesterol/polyoxyethylene-10-stearyl ether) and Novasome II (glyceryl distearate/ cholesterol/polyoxyethylene-10-stearyl ether) were used to deliver a drug into the dermis of mouse skin. Such formulations with siRNA are useful for treating a dermatological disorder.

Liposomes that include siRNA can be made highly deformable. Such deformability can enable the liposomes to penetrate through pore that are smaller than the average radius of the liposome. For example, transfersomes are a type of deformable liposomes. Transferosomes can be made by adding surface edge activators, usually surfactants, to a standard liposomal composition. Transfersomes that include siRNA can be delivered, for example, subcutaneously by infection in order to deliver siRNA to keratinocytes in the skin. In order to cross intact mammalian skin, lipid vesicles must pass through a series of fine pores, each with a diameter less than 50 nm, under the influence of a suitable transdermal gradient. In addition, due to the lipid properties, these transferosomes can be self-optimizing (adaptive to the shape of pores, *e.g.,* in the skin), self-repairing, and can frequently reach their targets without fragmenting, and often self-loading.

Other formulations amenable to the present disclosure are described in United States provisional application serial nos. 61/018,616, filed January 2, 2008; 61/018,611, filed January 2, 2008; 61/039,748, filed March 26, 2008; 61/047,087, filed April 22, 2008 and 61/051,528, filed May 8, 2008. PCT application no PCT/US2007/080331, filed October 3, 2007 also describes formulations that are amenable to the present disclosure.

*Surfactants.* For ease of exposition the formulations, compositions and methods in this section are discussed largely with regard to unmodified siRNA compounds. It may be understood, however, that these formulations, compositions and methods can be practiced with other siRNA compounds, *e.g*., modified siRNA compounds, and such practice is within the scope of the disclosure. Surfactants find wide application in formulations such as emulsions (including microemulsions) and liposomes (see above). siRNA (or a precursor, *e.g.,* a larger dsiRNA which can be processed into a siRNA, or a DNA which encodes a siRNA or precursor) compositions can include a surfactant. In one implementation, the siRNA is formulated as an emulsion that includes a surfactant. The most common way of classifying and ranking the properties of the many different types of surfactants, both natural and synthetic, is by the use of the hydrophile/lipophile balance (HLB). The nature of the hydrophilic group provides the most useful means for categorizing the different surfactants used in formulations (Rieger, in "Pharmaceutical Dosage Forms," Marcel Dekker, Inc., New York, NY, 1988, p. 285).

If the surfactant molecule is not ionized, it is classified as a nonionic surfactant. Nonionic surfactants find wide application in pharmaceutical products and are usable over a wide range of pH values. In general their HLB values range from 2 to about 18 depending on their structure. Nonionic surfactants include nonionic esters such as ethylene glycol esters, propylene glycol esters, glyceryl esters, polyglyceryl esters, sorbitan esters, sucrose esters, and ethoxylated esters. Nonionic alkanolamides and ethers such as fatty alcohol ethoxylates, propoxylated alcohols, and ethoxylated/propoxylated block polymers are also included in this class. The polyoxyethylene surfactants are the most popular members of the nonionic surfactant class.

If the surfactant molecule carries a negative charge when it is dissolved or dispersed in water, the surfactant is classified as anionic. Anionic surfactants include carboxylates such as soaps, acyl lactylates, acyl amides of amino acids, esters of sulfuric acid such as alkyl sulfates and ethoxylated alkyl sulfates, sulfonates such as alkyl benzene sulfonates, acyl isethionates, acyl taurates and sulfosuccinates, and phosphates. The most important members of the anionic surfactant class are the alkyl sulfates and the soaps.

If the surfactant molecule carries a positive charge when it is dissolved or dispersed in water, the surfactant is classified as cationic. Cationic surfactants include quaternary ammonium salts and ethoxylated amines. The quaternary ammonium salts are the most used members of this class.

If the surfactant molecule has the ability to carry either a positive or negative charge, the surfactant is classified as amphoteric. Amphoteric surfactants include acrylic acid derivatives, substituted alkylamides, N-alkylbetaines and phosphatides.

The use of surfactants in drug products, formulations and in emulsions has been reviewed (Rieger, in "Pharmaceutical Dosage Forms," Marcel Dekker, Inc., New York, NY, 1988, p. 285).

*Micelles and other Membranous Formulations.* For ease of exposition the micelles and other formulations, compositions and methods in this section are discussed largely with regard to unmodified siRNA compounds. It may be understood, however, that these micelles and other formulations, compositions and methods can be practiced with other siRNA compounds, *e.g*., modified siRNA compounds, and such practice is within the disclosure. The siRNA compound, *e.g.,* a double-stranded siRNA compound, or ssiRNA compound, *(e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof)) composition can be provided as a micellar formulation. "Micelles" are defined herein as a particular type of molecular assembly in which amphipathic molecules are arranged in a spherical structure such that all the hydrophobic portions of the molecules are directed inward, leaving the hydrophilic portions in contact with the surrounding aqueous phase. The converse arrangement exists if the environment is hydrophobic.

A mixed micellar formulation suitable for delivery through transdermal membranes may be prepared by mixing an aqueous solution of the siRNA composition, an alkali metal C₈ to C₂₂ alkyl sulphate, and a micelle forming compounds. Exemplary micelle forming compounds include lecithin, hyaluronic acid, pharmaceutically acceptable salts of hyaluronic acid, glycolic acid, lactic acid, chamomile extract, cucumber extract, oleic acid, linoleic acid, linolenic acid, monoolein, monooleates, monolaurates, borage oil, evening of primrose oil, menthol, trihydroxy oxo cholanyl glycine and pharmaceutically acceptable salts thereof, glycerin, polyglycerin, lysine, polylysine, triolein, polyoxyethylene ethers and analogues thereof, polidocanol alkyl ethers and analogues thereof, chenodeoxycholate, deoxycholate, and mixtures thereof. The micelle forming compounds may be added at the same time or after addition of the alkali metal alkyl sulphate. Mixed micelles will form with substantially any kind of mixing of the ingredients but vigorous mixing in order to provide smaller size micelles.

In one method a first micellar composition is prepared which contains the siRNA composition and at least the alkali metal alkyl sulphate. The first micellar composition is then mixed with at least three micelle forming compounds to form a mixed micellar composition. In another method, the micellar composition is prepared by mixing the siRNA composition, the alkali metal alkyl sulphate and at least one of the micelle forming compounds, followed by addition of the remaining micelle forming compounds, with vigorous mixing.

Phenol and/or m-cresol may be added to the mixed micellar composition to stabilize the formulation and protect against bacterial growth. Alternatively, phenol and/or m-cresol may be added with the micelle forming ingredients. An isotonic agent such as glycerin may also be added after formation of the mixed micellar composition.

For delivery of the micellar formulation as a spray, the formulation can be put into an aerosol dispenser and the dispenser is charged with a propellant. The propellant, which is under pressure, is in liquid form in the dispenser. The ratios of the ingredients are adjusted so that the aqueous and propellant phases become one, *i.e.,* there is one phase. If there are two phases, it is necessary to shake the dispenser prior to dispensing a portion of the contents, *e.g*., through a metered valve. The dispensed dose of pharmaceutical agent is propelled from the metered valve in a fine spray.

Propellants may include hydrogen-containing chlorofluorocarbons, hydrogen-containing fluorocarbons, dimethyl ether and diethyl ether. In certain implementations, HFA 134a (1,1,1,2 tetrafluoroethane) may be used.

The specific concentrations of the essential ingredients can be determined by relatively straightforward experimentation. For absorption through the oral cavities, it is often desirable to increase, *e.g*., at least double or triple, the dosage for through injection or administration through the gastrointestinal tract.

*Particles.* For ease of exposition the particles, formulations, compositions and methods in this section are discussed largely with regard to modified siRNA compounds. It may be understood, however, that these particles, formulations, compositions and methods can be practiced with other siRNA compounds, *e.g*., unmodified siRNA compounds, and such practice is within the disclosure. In another implementation, an siRNA compound, *e.g.,* a double-stranded siRNA compound, or ssiRNA compound, *(e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) preparations may be incorporated into a particle, *e.g.,* a microparticle. Microparticles can be produced by spray-drying, but may also be produced by other methods including lyophilization, evaporation, fluid bed drying, vacuum drying, or a combination of these techniques.

### Pharmaceutical compositions

The iRNA agents of the disclosuremay be formulated for pharmaceutical use. Pharmaceutically acceptable compositions comprise a therapeutically-effective amount of one or more of the the dsRNA agents in any of the preceding implementations, taken alone or formulated together with one or more pharmaceutically acceptable carriers (additives), excipient and/or diluents.

The pharmaceutical compositions may be specially formulated for administration in solid or liquid form, including those adapted for the following: (1) oral administration, for example, drenches (aqueous or non-aqueous solutions or suspensions), tablets, *e.g*., those targeted for buccal, sublingual, and systemic absorption, boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; or (8) nasally. Delivery using subcutaneous or intravenous methods can be particularly advantageous.

The phrase "therapeutically-effective amount" as used herein means that amount of a compound, material, or composition comprising a compound of the disclosure which is effective for producing some desired therapeutic effect in at least a sub-population of cells in an animal at a reasonable benefit/risk ratio applicable to any medical treatment.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, manufacturing aid (*e.g*., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) lubricating agents, such as magnesium state, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; (22) bulking agents, such as polypeptides and amino acids (23) serum component, such as serum albumin, HDL and LDL; and (22) other non-toxic compatible substances employed in pharmaceutical formulations.

The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred per cent, this amount will range from about 0.1 per cent to about ninety-nine percent of active ingredient, preferably from about 5 per cent to about 70 per cent, most preferably from about 10 per cent to about 30 per cent.

In certain implementations, a formulation of the present disclosure comprises an excipient selected from the group consisting of cyclodextrins, celluloses, liposomes, micelle forming agents, *e.g*., bile acids, and polymeric carriers, *e.g*., polyesters and polyanhydrides; and a compound of the present disclosure. In certain implementations, an aforementioned formulation renders orally bioavailable a compound of the present disclosure.

iRNA agent preparation can be formulated in combination with another agent, *e.g.,* another therapeutic agent or an agent that stabilizes a iRNA, *e.g.,* a protein that complexes with iRNA to form an iRNP. Still other agents include chelators, *e.g*., EDTA *(e.g.,* to remove divalent cations such as Mg²⁺), salts, RNAse inhibitors *(e.g.,* a broad specificity RNAse inhibitor such as RNAsin) and so forth.

Methods of preparing these formulations or compositions include the step of bringing into association a compound of the present disclosure with the carrier and, optionally, one or more accessory ingredients. In general, the formulations are prepared by uniformly and intimately bringing into association a compound of the present disclosure with liquid carriers, or finely divided solid carriers, or both, and then, if necessary, shaping the product.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

The compounds according to the disclosure may be formulated for administration in any convenient way for use in human or veterinary medicine, by analogy with other pharmaceuticals.

The term "treatment" is intended to encompass also prophylaxis, therapy and cure. The patient receiving this treatment is any animal in need, including primates, in particular humans, and other mammals such as equines, cattle, swine and sheep; and poultry and pets in general.

Double-stranded RNAi agents are produced in a cell *in vivo, e.g.,* from exogenous DNA templates that are delivered into the cell. For example, the DNA templates can be inserted into vectors and used as gene therapy vectors. Gene therapy vectors can be delivered to a subject by, for example, intravenous injection, local administration (U.S. Pat. No. 5,328,470), or by stereotactic injection (see, *e.g.,* Chen et al. (1994) Proc. Natl. Acad. Sci. USA 91:3054-3057). The pharmaceutical preparation of the gene therapy vector can include the gene therapy vector in an acceptable diluent, or can comprise a slow release matrix in which the gene delivery vehicle is imbedded. The DNA templates, for example, can include two transcription units, one that produces a transcript that includes the top strand of a dsRNA agent and one that produces a transcript that includes the bottom strand of a dsRNA agent. When the templates are transcribed, the dsRNA agent is produced, and processed into siRNA agent fragments that mediate gene silencing.

### Routes of Delivery

A composition that includes an iRNA can be delivered to a subject by a variety of routes. Exemplary routes include: intravenous, subcutaneous, topical, rectal, anal, vaginal, nasal, pulmonary, ocular.

The iRNA molecules of the disclosure can be incorporated into pharmaceutical compositions suitable for administration. Such compositions typically include one or more species of iRNA and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The compositions of the present disclosure may be administered in a number of ways depending upon whether local or systemic treatment is desired and upon the area to be treated. Administration may be topical (including ophthalmic, vaginal, rectal, intranasal, transdermal), oral or parenteral. Parenteral administration includes intravenous drip, subcutaneous, intraperitoneal or intramuscular injection, or intrathecal or intraventricular administration.

The route and site of administration may be chosen to enhance targeting. For example, to target muscle cells, intramuscular injection into the muscles of interest would be a logical choice. Lung cells might be targeted by administering the iRNA in aerosol form. The vascular endothelial cells could be targeted by coating a balloon catheter with the iRNA and mechanically introducing the DNA.

### Dosage

In one implementation, the disclosure features a method of administering a dsRNA agent, *e.g.,* a siRNA agent, to a subject *(e.g.,* a human subject). The method includes administering a unit dose of the dsRNA agent, *e.g.,* a siRNA agent, *e.g.,* double stranded siRNA agent that (a) the double-stranded part is 14-30 nucleotides (nt) long, for example, 21-23 nt, (b) is complementary to a target RNA *(e.g.,* an endogenous or pathogen target RNA), and, optionally, (c) includes at least one 3' overhang 1-5 nucleotide long. In one implementation, the unit dose is less than 10 mg per kg of bodyweight, or less than 10, 5, 2, 1, 0.5, 0.1, 0.05, 0.01, 0.005, 0.001, 0.0005, 0.0001, 0.00005 or 0.00001 mg per kg of bodyweight, and less than 200 nmole of RNA agent *(e.g.,* about 4.4 × 10¹⁶ copies) per kg of bodyweight, or less than 1500, 750, 300, 150, 75, 15, 7.5, 1.5, 0.75, 0.15, 0.075, 0.015, 0.0075, 0.0015, 0.00075, 0.00015 nmole of RNA agent per kg of bodyweight.

The defined amount can be an amount effective to treat or prevent a disease or disorder, *e.g.,* a disease or disorder associated with the target RNA. The unit dose, for example, can be administered by injection (*e.g*., intravenous, subcutaneous or intramuscular), an inhaled dose, or a topical application. In some implementations dosages may be less than 10, 5, 2, 1, or 0.1 mg/kg of body weight.

In some implementations, the unit dose is administered less frequently than once a day, *e.g.,* less than every 2, 4, 8 or 30 days. In another implementation, the unit dose is not administered with a frequency (*e.g*., not a regular frequency). For example, the unit dose may be administered a single time.

In one implementation, the effective dose is administered with other traditional therapeutic modalities. In one implementation, the subject has a viral infection and the modality is an antiviral agent other than a dsRNA agent, *e.g.,* other than a siRNA agent. In another implementation, the subject has atherosclerosis and the effective dose of a dsRNA agent, *e.g.,* a siRNA agent, is administered in combination with, *e.g.,* after surgical intervention, *e.g*., angioplasty.

In one implementation, a subject is administered an initial dose and one or more maintenance doses of a dsRNA agent, *e.g.,* a siRNA agent, *(e.g.,* a precursor, *e.g.,* a larger dsRNA agent which can be processed into a siRNA agent, or a DNA which encodes a dsRNA agent, *e.g.,* a siRNA agent, or precursor thereof). The maintenance dose or doses can be the same or lower than the initial dose, *e.g.,* one-half less of the initial dose. A maintenance regimen can include treating the subject with a dose or doses ranging from 0.01 µg to 15 mg/kg of body weight per day, *e.g.,* 10, 1, 0.1, 0.01, 0.001, or 0.00001 mg per kg of bodyweight per day. The maintenance doses are, for example, administered no more than once every 2, 5, 10, or 30 days. Further, the treatment regimen may last for a period of time which will vary depending upon the nature of the particular disease, its severity and the overall condition of the patient. In certain implementations the dosage may be delivered no more than once per day, e.g., no more than once per 24, 36, 48, or more hours, e.g., no more than once for every 5 or 8 days. Following treatment, the patient can be monitored for changes in his condition and for alleviation of the symptoms of the disease state. The dosage of the compound may either be increased in the event the patient does not respond significantly to current dosage levels, or the dose may be decreased if an alleviation of the symptoms of the disease state is observed, if the disease state has been ablated, or if undesired side-effects are observed.

The effective dose can be administered in a single dose or in two or more doses, as desired or considered appropriate under the specific circumstances. If desired to facilitate repeated or frequent infusions, implantation of a delivery device, e.g., a pump, semi-permanent stent (e.g., intravenous, intraperitoneal, intracisternal or intracapsular), or reservoir may be advisable.

In one implementation, the composition includes a plurality of dsRNA agent species. In another implementation, the dsRNA agent species has sequences that are nonoverlapping and non-adjacent to another species with respect to a naturally occurring target sequence. In another implementation, the plurality of dsRNA agent species is specific for different naturally occurring target genes. In another implementation, the dsRNA agent is allele specific.

The dsRNA agents of the disclosure described herein can be administered to mammals, particularly large mammals such as nonhuman primates or humans in a number of ways.

In one implementation, the administration of the dsRNA agent, *e.g.,* a siRNA agent, composition is parenteral, *e.g.,* intravenous *(e.g.,* as a bolus or as a diffusible infusion), intradermal, intraperitoneal, intramuscular, intrathecal, intraventricular, intracranial, subcutaneous, transmucosal, buccal, sublingual, endoscopic, rectal, oral, vaginal, topical, pulmonary, intranasal, urethral or ocular. Administration can be provided by the subject or by another person, *e.g.,* a health care provider. The medication can be provided in measured doses or in a dispenser which delivers a metered dose. Selected modes of delivery are discussed in more detail below.

The disclosure provides methods, compositions, and kits, for rectal administration or delivery of dsRNA agents described herein

### Methods of inhibiting expression of the target gene

Implementations of the disclosure also relate to methods for inhibiting the expression of a target gene. The method comprises the step of administering the dsRNA agents in any of the preceding implementations, in an amount sufficient to inhibit expression of the target gene.

Another implementation the disclosure relates to a method of modulating the expression of a target gene in a cell, comprising providing to said cell a dsRNA agent of this disclosure. In one implementation, the target gene is selected from the group consisting of Factor VII, Eg5, PCSK9, TPX2, apoB, SAA, TTR, RSV, PDGF beta gene, Erb-B gene, Src gene, CRK gene, GRB2 gene, RAS gene, MEKK gene, JNK gene, RAF gene, Erk1/2 gene, PCNA(p21) gene, MYB gene, JUN gene, FOS gene, BCL-2 gene, hepciden, Activated Protein C, Cyclin D gene, VEGF gene, EGFR gene, Cyclin A gene, Cyclin E gene, WNT-1 gene, beta-catenin gene, c-MET gene, PKC gene, NFKB gene, STAT3 gene, survivin gene, Her2/Neu gene, topoisomerase I gene, topoisomerase II alpha gene, mutations in the p73 gene, mutations in the p21(WAF1/CIP1) gene, mutations in the p27(KIP1) gene, mutations in the PPM1D gene, mutations in the RAS gene, mutations in the caveolin I gene, mutations in the MIB I gene, mutations in the MTAI gene, mutations in the M68 gene, mutations in tumor suppressor genes, and mutations in the p53 tumor suppressor gene.

The invention is further illustrated by the following examples, which should not be construed as further limiting.

### EXAMPLES

### Example 1. In vitro screening of siRNA duplexes

### Cell culture and transfections:

Human Hep3B cells or rat H.II.4.E cells (ATCC, Manassas, VA) were grown to near confluence at 37 °C in an atmosphere of 5% CO₂ in RPMI (ATCC) supplemented with 10% FBS, streptomycin, and glutamine (ATCC) before being released from the plate by trypsinization. Transfection was carried out by adding 14.8 µl of Opti-MEM plus 0.2 µl of Lipofectamine RNAiMax per well (Invitrogen, Carlsbad CA. cat # 13778-150) to 5 µl of siRNA duplexes per well into a 96-well plate and incubated at room temperature for 15 minutes. 80 µl of complete growth media without antibiotic containing ~2 ×10⁴ Hep3B cells were then added to the siRNA mixture. Cells were incubated for either 24 or 120 hours prior to RNA purification. Single dose experiments were performed at 10nM and 0.1nM final duplex concentration and dose response experiments were done using 8, 4 fold serial dilutions with a maximum dose of 10nM final duplex concentration.

### Total RNA isolation using DYNABEADS mRNA Isolation Kit (Invitrogen. part #: 610-12):

Cells were harvested and lysed in 150 µl of Lysis/Binding Buffer then mixed for 5 minute at 850rpm using an Eppendorf Thermomixer (the mixing speed was the same throughout the process). Ten microliters of magnetic beads and 80 µl Lysis/Binding Buffer mixture were added to a round bottom plate and mixed for 1 minute. Magnetic beads were captured using magnetic stand and the supernatant was removed without disturbing the beads. After removing supernatant, the lysed cells were added to the remaining beads and mixed for 5 minutes. After removing supernatant, magnetic beads were washed 2 times with 150 µl Wash Buffer A and mixed for 1 minute. Beads were capture again and supernatant removed. Beads were then washed with 150 µl Wash Buffer B, captured and supernatant was removed. Beads were next washed with 150 µl Elution Buffer, captured and supernatant removed. Beads were allowed to dry for 2 minutes. After drying, 50 µl of Elution Buffer was added and mixed for 5 minutes at 70°C. Beads were captured on magnet for 5 minutes. 40 µl of supernatant was removed and added to another 96 well plate.

### cDNA synthesis using ABI High capacity cDNA reverse transcription kit (Applied Biosystems, Foster City, CA, Cat #4368813):

A master mix of 1 µl 10X Buffer, 0.4µl 25X dNTPs, 1µl Random primers, 0.5 µl Reverse Transcriptase, 0.5 µl RNase inhibitor and 1.6µl of H₂O per reaction were added into 5 µl total RNA. cDNA was generated using a Bio-Rad C-1000 or S-1000 thermal cycler (Hercules, CA) through the following steps: 25 °C 10 min, 37 °C 120 min, 85 °C 5 sec, 4 °C hold.

### Real time PCR:

2µl of cDNA were added to a master mix containing 0.5µl GAPDH TaqMan Probe (Applied Biosystems Cat #4326317E (human) Cat # 4308313 (rodent)), 0.5µl TTR TaqMan probe (Applied Biosystems cat # HS00174914 _m1 (human) cat # Rn00562124_m1 (rat)) and 5µl Lightcycler 480 probe master mix (Roche Cat #04887301001) per well in a 384 well plate (Roche cat # 04887301001). Real time PCR was done in a Roche LC 480 Real Time PCR machine (Roche). Each duplex was tested in at least two independent transfections and each transfection was assayed in duplicate, unless otherwise noted.

To calculate relative fold change, real time data were analyzed using the ΔΔCt method and normalized to assays performed with cells transfected with 10nM AD-1955, or mock transfected cells. IC₅₀S were calculated using a 4 parameter fit model using XLFit and normalized to cells transfected with AD-1955 or naive cells over the same dose range, or to its own lowest dose. IC₅₀S were calculated for each individual transfection as well as in combination, where a single IC₅₀ was fit to the data from both transfections.

The results of gene silencing of the exemplary siRNA duplex with various motif modifications of the disclosure are shown in the table below.

### Example 2. RNA Synthesis and Duplex Annealing

### 1. Oligonucleotide Synthesis:

All oligonucleotides were synthesized on an AKTAoligopilot synthesizer or an ABI 394 synthsizer. Commercially available controlled pore glass solid support (dT-CPG, 500Ǻ, Prime Synthesis) and RNA phosphoramidites with standard protecting groups, 5' -O-dimethoxytrityl N6-benzoyl-2' -*t*-butyldimethylsilyl-adenosine-3'-*O*-N,N' - diisopropyl-2-cyanoethylphosphoramidite, 5'-*O*-dimethoxytrityl-N4-acetyl-2'-*t*-butyldimethylsilyl-cytidine-3'-*O*-N,N'-diisopropyl-2-cyanoethylphosphoramidite, 5'-*O*-dimethoxytrityl-N2--isobutryl-2'-*t*-butyldimethylsilyl-guanosine-3'-*O*-N,N'-diisopropyl-2-cyanoethylphosphoramidite, and 5'-*O*-dimethoxytrityl-2'-*t*-butyldimethylsilyl-uridine-3'-*O*-N,N'-diisopropyl-2-cyanoethylphosphoramidite (Pierce Nucleic Acids Technologies) were used for the oligonucleotide synthesis unless otherwise specified. The 2'-F phosphoramidites, 5'-*O*-dimethoxytrityl-N4-acetyl-2'-fluro-cytidine-3'-*O*N,N'-diisopropyl-2-cyanoethyl-phosphoramidite and 5'-*O*-dimethoxytrityl-2'-flurouridine-3'-O-N,N'-diisopropyl-2-cyanoethyl-phosphoramidite were purchased from (Promega). All phosphoramidites were used at a concentration of 0.2M in acetonitrile (CH₃CN) except for guanosine which was used at 0.2M concentration in 10% THF/ANC (v/v). Coupling/recycling time of 16 minutes was used. The activator was 5-ethyl thiotetrazole (0.75M, American International Chemicals), for the PO-oxidation Iodine/Water/Pyridine was used and the PS-oxidation PADS (2 %) in 2,6-lutidine/ACN (1:1 v/v) was used..

Ligand conjugated strands were synthesized using solid support containing the corresponding ligand. For example, the introduction of carbohydrate moiety/ligand (for e.g., GalNAc) at the 3'-end of a sequence was achieved by starting the synthesis with the corresponding carbohydrate solid support. Similarly a cholesterol moiety at the 3'-end was introduced by starting the synthesis on the cholesterol support. In general, the ligand moiety was tethered to *trans-*4-hydroxyprolinol via a tether of choice as described in the previous examples to obtain a hydroxyprolinol-ligand moiety. The hydroxyprolinol-ligand moiety was then coupled to a solid support via a succinate linker or was converted to phosphoramidite via standard phosphitylation conditions to obtain the desired carbohydrate conjugate building blocks. Fluorophore labeled siRNAs were synthesized from the corresponding phosphoramidite or solid support, purchased from Biosearch Technologies. The oleyl lithocholic (GalNAc)₃ polymer support made in house at a loading of 38.6 µmol/gram. The Mannose (Man)₃ polymer support was also made in house at a loading of 42.0 µmol/gram.

Conjugation of the ligand of choice at desired position, for example at the 5'-end of the sequence, was achieved by coupling of the corresponding phosphoramidite to the growing chain under standard phosphoramidite coupling conditions unless otherwise specified. An extended 15 min coupling of 0.1M solution of phosphoramidite in anhydrous CH₃CN in the presence of 5-(ethylthio)-1*H*-tetrazole activator to a solid bound oligonucleotide. Oxidation of the internucleotide phosphite to the phosphate was carried out using standard iodine-water as reported (1) or by treatment with *tert*-butyl hydroperoxide/acetonitrile/water (10: 87: 3) with 10 min oxidation wait time conjugated oligonucleotide. Phosphorothioate was introduced by the oxidation of phosphite to phosphorothioate by using a sulfur transfer reagent such as DDTT (purchased from AM Chemicals), PADS and or Beaucage reagent The cholesterol phosphoramidite was synthesized in house, and used at a concentration of 0.1 M in dichloromethane. Coupling time for the cholesterol phosphoramidite was 16 minutes.

### 2. Deprotection-I (Nucleobase Deprotection)

After completion of synthesis, the support was transferred to a 100 ml glass bottle (VWR). The oligonucleotide was cleaved from the support with simultaneous deprotection of base and phosphate groups with 80 mL of a mixture of ethanolic ammonia [ammonia: ethanol (3:1)] for 6.5h at 55°C. The bottle was cooled briefly on ice and then the ethanolic ammonia mixture was filtered into a new 250 ml bottle. The CPG was washed with 2 × 40 mL portions of ethanol/water (1:1 v/v). The volume of the mixture was then reduced to ~ 30 ml by roto-vap. The mixture was then frozen on dry ice and dried under vacuum on a speed vac.

### 3. Deprotection-II (Removal of 2' TBDMS group)

The dried residue was resuspended in 26 ml of triethylamine, triethylamine trihydrofluoride (TEA.3HF) or pyridine-HF and DMSO (3:4:6) and heated at 60°C for 90 minutes to remove the *tert*-butyldimethylsilyl (TBDMS) groups at the 2' position. The reaction was then quenched with 50 ml of 20mM sodium acetate and pH adjusted to 6.5, and stored in freezer until purification.

### 4. Analysis

The oligoncuelotides were analyzed by high-performance liquid chromatography (HPLC) prior to purification and selection of buffer and column depends on nature of the sequence and or conjugated ligand.

### 5. HPLC Purification

The ligand conjugated oligonucleotides were purified reverse phase preparative HPLC. The unconjugated oligonucleotides were purified by anion-exchange HPLC on a TSK gel column packed in house. The buffers were 20 mM sodium phosphate (pH 8.5) in 10% CH₃CN (buffer A) and 20 mM sodium phosphate (pH 8.5) in 10% CH₃CN, 1M NaBr (buffer B). Fractions containing full-length oligonucleotides were pooled, desalted, and lyophilized. Approximately 0.15 OD of desalted oligonucleotidess were diluted in water to 150 µl and then pipetted in special vials for CGE and LC/MS analysis. Compounds were finally analyzed by LC-ESMS and CGE.

### 6. siRNA preparation

For the preparation of siRNA, equimolar amounts of sense and antisense strand were heated in 1×PBS at 95°C for 5 min and slowly cooled to room temperature. Integrity of the duplex was confirmed by HPLC analysis.

**Table 2. ANGPTL3 modified duplex**

| Duplex ID | S ID | Sense strand (S) | AS ID | Antisense strand (AS) | % of mRNA remained conc. of siRNA | | | IC50 (nM) |
|---|---|---|---|---|---|---|---|---|
| | | | | | 1 nM | 0.1 nM | 0.01 nM | |
| D1000 | S1000 | AfuGfuAfaCfcAfAfGfaGfuAfuUfcCfasu | AS1000 | AfUfgGfaAfuAfcUfcuuGfgUfuAfcAfusGfsa | 0.03 | 0.1 | 0.47 | 0.006 |
| D1001 | S1001 | AfsuGfuAfaCfcAfAfGfaGfuAfuucCfasUf | AS1001 | aUfsgGfAfAfuAfcUfcuuGfgUfuAfcAfusGfsa | 0.03 | 0.10 | 0.49 | 0.0065 |
| D1002 | S1002 | AfuGfuAfaCfcAfAfGfaGfuAfuucCfasUf | AS1002 | aUfgGfAfAfuAfcUfcuuGfgsUfuAfcAfusGfsa | 0.04 | 0.10 | 0.46 | 0.0068 |
| D1003 | S1003 | AfuGfuAfaCfcAfAfGfaGfuAfuucCfasUf | AS1003 | aUfgGfAfAfuAfcUfcuuGfgUfsuAfcAfusGfsa | 0.05 | 0.12 | 0.56 | 0.0073 |
| D1004 | S1004 | aUGuaACccAGagUAuuCCasu | AS1004 | AUggAAuaCUcuUGguUAcaUsGsa | 0.07 | 0.13 | 0.44 | 0.008 |
| D1005 | S1005 | AfuGfuAfaCfcAfAfGfaGfuAfuucCfasUf | AS1005 | aUfgGfAfAfuAfcUfcuuGfgsUfsuAfcAfusGfsa | 0.06 | 0.11 | 0.53 | 0.0093 |
| D1006 | S1006 | AfuGfuAfAfccAfAfGfaGfuAfuUfcCfasUf | AS1006 | aUfgGfaAfuAfcUfcuuGfGfuuAfcAfusGfsa | 0.05 | 0.16 | 0.55 | 0.0095 |
| D1007 | S1007 | AfuGfuAfAfCfcAfAfGfaGfuAfuUfcCfasUf | AS1007 | aUfgGfaAfuAfcUfcuuGfguuAfcAfusGfsa | 0.05 | 0.14 | 0.48 | 0.0098 |
| D1008 | S1008 | auguaaccaadGadGudAudAcdGasu | AS1008 | aUfgGfaAfuAfcUfcUfuGfgUfuAfcAfusGfsa | 0.07 | 0.11 | 0.33 | 0.010 |
| D1009 | S1009 | | AS1009 | uCfuugGfuUfaCfaugAfaAfuccCfasUfsc | 0.03 | 0.14 | 0.56 | 0.0101 |
| D1010 | S1010 | UfgGfgauUfuCfAfUfgUfaAfcCfaAfgsAf | AS1010 | uCfuUfgGfuUfaCfaugAfaAfUfCfcCfasUfsc | 0.03 | 0.14 | 0.65 | 0.0101 |
| D1011 | S1011 | aUfGfuAfAfccAfAfGfaGfuAfuUfcCfasUf | AS1011 | aUfgGfaAfuAfcUfcuuGfGfuuAfcaUfsgsa | 0.06 | 0.10 | 0.55 | 0.011 |
| D1012 | S1012 | UfgGfgAfuUfuCfAfUfgUfaacCfaAfgsAf | AS1012 | uCfuUfgGfUfUfaCfaugAfaAfuCfcCfasUfsc | 0.04 | 0.13 | 0.54 | 0.0114 |
| D1013 | S1013 | auguaaccaadGadGudAudAcdGasu | AS1013 | aUfgGfaAfuAfcUfcUfugdGudTadCadTsgsa | 0.11 | 0.19 | 0.49 | 0.011 |
| D1014 | S1014 | AfuGfuaaCfcAfAfGfaGfuAfuUfcCfasUf | AS1014 | aUfgGfaAfuAfcUfcuuGfgUfUfAfcAfusGfsa | 0.04 | 0.16 | 0.59 | 0.013 |
| D1015 | S1015 | AfuguAfaccAfaGfdAGfdTAdTudCcdAsu | AS1015 | dAUdGgdAadTAfdCUfcUfuGfgUfuAfcAfusGfsa | 0.07 | 0.15 | 0.51 | 0.013 |
| D1016 | S1016 | auGfuAfaCfcAfAfGfaGfuAfuUfcCfasUf | AS1016 | aUfgGfaAfuAfcUfcuuGfgUfuAfcAfUfsGfsa | 0.05 | 0.14 | 0.64 | 0.013 |
| D1017 | S1017 | UfGfggAfuUfuCfAfUfgUfAfAfcCfaAfgsAf | AS1017 | uCfuUfgGfuuaCfaugAfaAfuCfCfcasUfsc | 0.09 | 0.41 | 0.74 | 0.0133 |
| D1018 | S1018 | AfuguAfaCfcAfAfGfaGfuAfuUfcCfasUf | AS1018 | aUfgGfaAfuAfcUfcuuGfgUfuAfCfAfusGfsa | 0.03 | 0.14 | 0.61 | 0.014 |
| D1019 | S1019 | AfuGfuAfaccAfAfGfaGfuAfuUfcCfasUf | AS1019 | aUfgGfaAfuAfcUfcuuGfGfUfuAfcAfusGfsa | 0.02 | 0.2 | 0.7 | 0.014 |
| D1020 | S1020 | AfsuGfuAfaCfcAfAfGfaGfuAfuucCfasUf | AS1020 | asUfsgGfAfAfuAfcUfcuuGfgUfuAfcAfusGfsa | 0.04 | 0.16 | 0.67 | 0.0156 |
| D1021 | S1021 | aUfguAfAfccAfAfgagUfaUfuCfcasUf | AS1021 | aUfGfgAfaUfaCfUfCfuuGfGfuuAfCfaUfsgsa | 0.11 | 0.24 | 0.64 | 0.016 |
| D1022 | S1022 | | AS1022 | udCdTugdGdTuadCdAugdAdAaudCdCcasdTsc | 0.08 | 0.27 | 0.64 | 0.0161 |
| D1023 | S1023 | AfsuGfuAfaCfcAfAfGfaGfuAfuucCfasUf | AS1023 | aUfgsGfAfAfuAfcUfcuuGfgUfuAfcAfusGfsa | 0.03 | 0.19 | 0.63 | 0.0163 |
| D1024 | S1024 | UfgGfgAfuUfuCfAfUfguaAfcCfaAfgsAf | AS1024 | uCfuUfgGfuUfAfCfaugAfaAfuCfcCfasUfsc | 0.05 | 0.25 | 0.69 | 0.0164 |
| D1025 | S1025 | UfgGfgAfuUfuCfAfUfgUfAfAfcCfaAfgsAf | AS1025 | uCfuUfgGfuuaCfaugAfaAfuCfcCfasUfsc | 0.04 | 0.18 | 0.75 | 0.0166 |
| D1026 | S1026 | UfgGfgAfuUfuCfAfUfgUfaAfcCfaAfgsAf | AS1026 | uCfuUfgGfuUfaCfaugAfaAfuCfcCfasUfsc | 0.04 | 0.19 | 0.66 | 0.0178 |
| D1027 | S1027 | UfgGfgAfuUfuCfAfUfgUfaAfccaAfgsAf | AS1027 | uCfuUfGfGfuUfaCfaugAfaAfuCfcCfasUfsc | 0.04 | 0.19 | 0.69 | 0.018 |
| D1028 | S1028 | | AS1028 | adTdGgadAdTacdTdCuudGdGuudAdCausdGsa | 0.15 | 0.29 | 0.72 | 0.018 |
| D1029 | S1029 | | AS1029 | | 0.1 | 0.27 | 0.61 | 0.018 |
| D1030 | S1030 | UfgGfGfAfuuuCfAfUfgUfaAfcCfaAfgsAf | AS1030 | uCfuUfgGfuUfaCfaugAfAfAfuccCfasUfsc | 0.04 | 0.21 | 0.64 | 0.0187 |
| D1031 | S1031 | AfuGfuAfAfccAfAfGfAfGfuAfuuccAfsu | AS1031 | AfUfGfGfAfAfuAfCfUfCfUfuGfGfuuAfcAfusGfsa | 0.06 | 0.15 | 0.62 | 0.019 |
| D1032 | S1032 | AfsuGfuAfaCfcAfAfGfaGfuAfuucCfasUf | AS1032 | asUfgGfAfAfuAfcUfcuuGfgUfsuAfcAfusGfsa | 0.09 | 0.34 | 0.78 | 0.021 |
| D1033 | S1033 | UfgGfgAfuUfuCfaUfGfUfaacCfaAfgsAf | AS1033 | uCfuUfgGfUfUfacaUfgAfaAfuCfcCfasUfsc | 0.06 | 0.26 | 0.57 | 0.0212 |
| D1034 | S1034 | AfuGfuAfAfccAfaGfaGfuAfuUfcCfasUf | AS1034 | aUfgGfaAfuAfcUfcUfuGfGfuuAfcAfusGfsa | 0.11 | 0.39 | 0.82 | 0.0216 |
| D1035 | S1035 | UfgGfgAfuuuCfAfUfgUfaAfcCfaAfgsAf | AS1035 | uCfuUfgGfuUfaCfaugAfAfAfuCfcCfasUfsc | 0.04 | 0.16 | 0.56 | 0.0222 |
| D1036 | S1036 | UfgGfGfAfuUfuCfaUfgUfaAfcCfAfAfgsAf | AS1036 | uCfuugGfuUfaCfaUfgAfaAfuccCfasUfsc | 0.06 | 0.31 | 0.78 | 0.0234 |
| D1037 | S1037 | UfgGfGfAfuUfuCfAfUfgUfaAfcCfaAfgsAf | AS1037 | uCfuUfgGfuUfaCfaugAfaAfuccCfasUfsc | 0.03 | 0.14 | 0.62 | 0.0235 |
| D1038 | S1038 | UfGfggAfUfuuCfAfugUfAfacCfAfagsAf | AS1038 | uCfUfugGfUfuaCfAfugAfAfauCfCfcasUfsc | 0.09 | 0.39 | 0.78 | 0.0239 |
| D1039 | S1039 | AfuGfuAfaCfcAfAfGfaGfuAfuucCfasUf | AS1039 | aUfgGfAfAfuAfcUfcuuGfgUfuAfcAfusGfsa | 0.03 | 0.14 | 0.59 | 0.025 |
| D1040 | S1040 | AfuGfuAfaCfcAfAfGfaGfuAfuUfccasUf | AS1040 | aUfGfGfaAfuAfcUfcuuGfgUfuAfcAfusGfsa | 0.03 | 0.13 | 0.56 | 0.025 |
| D1041 | S1041 | AfsuGfuAfaCfcAfAfGfaGfuAfuucCfasUf | AS1041 | asUfgGfAfAfuAfcUfcuuGfgUfuAfcAfusGfsa | 0.06 | 0.27 | 0.79 | 0.0252 |
| D1042 | S1042 | UfgGfgAfuuuCfAfUfgUfAfAfcCfaAfgsAf | AS1042 | uCfuUfgGfuuaCfaugAfAfAfuCfeCfasUfse | 0.05 | 0.27 | 0.67 | 0.0259 |
| D1043 | S1043 | AfuGfuAfaCfcAfAfGfaGfuauUfcCfasUf | AS1043 | aUfgGfaAfUfAfcUfcuuGfgUfuAfcAfusGfsa | 0.02 | 0.16 | 0.63 | 0.027 |
| D1044 | S1044 | AfsuGfuAfaCfcAfAfGfaGfuAfuucCfasUf | AS1044 | asUfgGfAfAfuAfcUfcuuGfgsUfsuAfcAfusGfsa | 0.06 | 0.30 | 0.81 | 0.0271 |
| D1045 | S1045 | aUfguAfAfccAfAfgaGfGfauUfCfcasUf | AS1045 | aUfGfgaAfUfacUfCfuuGfGfuuAfCfaUfsgsa | 0.12 | 0.29 | 0.8 | 0.028 |
| D1046 | S1046 | AfuGfuAfaCfcAfAfGfaguAfuUfcCfasUf | AS1046 | aUfgGfaAfuAfCfUfcuuGfgUfuAfcAfusGfsa | 0.03 | 0.15 | 0.59 | 0.030 |
| D1047 | S1047 | UfgGfGfAfuUfuCfaUfgUfAfAfcCfaAfgsAf | AS1047 | uCfuUfgGfuuaCfaUfgAfaAfuccCfasUfsc | 0.08 | 0.44 | 0.83 | 0.0324 |
| D1048 | S1048 | AfuGfuAfaCfcAfAfGfaGfuAfuUfcCfasUf | AS1048 | aUfgGfaAfuAfcUfcuuGfgUfuAfcAfusGfsa | 0.07 | 0.23 | 0.67 | 0.036 |
| D1049 | S1049 | AfuGfuAfAfccAfAfGfAfGfuAfuuccAfsu | AS1049 | | 0.08 | 0.23 | 0.73 | 0.037 |
| D1050 | S1050 | UfgGfgAfuuuCfaUfgUfaAfcCfAfAfgsAf | AS1050 | uCfuugGfuUfaCfaUfgAfAfAfuCfcCfasUfsc | 0.06 | 0.29 | 0.78 | 0.0372 |
| D1051 | S1051 | AfuGfuAfaccaagaguAfuUfcCfasUf | AS1051 | aUfgGfaAfudAcdTcdTudGgdTuAfcAfusgsa | 0.12 | 0.41 | 0.86 | 0.040 |
| D1052 | S1052 | AfuguAfaccAfaGfdAGfdTAdTUdCcdAsu | AS1052 | aUfgGfaAfuAfcUfcUfuGfgUfuAfcAfusGfsa | 0.1 | 0.22 | 0.72 | 0.042 |
| D1053 | S1053 | AfuguAfaccAfaGfdAGfdTAdTUdCcdAsu | AS1053 | dAUdGGdAAfuAfcUfcUfuGfGfUfuAfCfAfusGfsa | 0.09 | 0.31 | 0.69 | 0.044 |
| D1054 | S1054 | | AS1054 | adTdGGfaAfudAdCUfcUfuGfgUfuAfcAfusGfsa | 0.1 | 0.45 | 0.75 | 0.047 |
| D1055 | S1055 | AfuguAfaccAfaGfaGfdTAdTUdCcdAsu | AS1055 | dAUdGGdAadTAfcUfcUfuGfgUfuAfcAfusGfsa | 0.12 | 0.26 | 0.7 | 0.049 |
| D1056 | S1056 | AuGuAaCcAaGaGuAuUcCasU | AS1056 | aUgGaAuAcUcUuGgUuAcAusGsa | 0.08 | 0.24 | 0.65 | 0.050 |
| D1057 | S1057 | AfuguAfaccAfagaGfuauUfccasUf | AS1057 | aUfGfGfaAfUfAfcUfCfUfuGfGfUfuAfCfAfusGfsa | 0.14 | 0.42 | 0.62 | 0.051 |
| D1058 | S1058 | AfuGfuAfaccaagaguAfuUfcCfasUf | AS1058 | aUfgGfaAfudAcdTcdTudGgdTuAfcAfusGfsa | 0.12 | 0.36 | 0.86 | 0.053 |
| D1059 | S1059 | AfuguAfaccAfaGfdAGfdTAdTUdCcdAsu | AS1059 | dAUdGGdAadTAfdCUfcUfuGfgUfuAfcAfusGfsa | 0.09 | 0.27 | 0.7 | 0.054 |
| D1060 | S1060 | adTgudAdAccdAdAgagdTadTudCcasdT | AS1060 | adTdGgdAadTadCdTdCuudGdGuudAdCadTsgsa | 0.11 | 0.37 | 0.66 | 0.056 |
| D1061 | S1061 | | AS1061 | adTdGGfaAfuAfdCdTcUfuGfgUfuAfcAfusGfsa | 0.1 | 0.31 | 0.77 | 0.059 |
| D1062 | S1062 | AfuguAfaccAfaGfdAGfdTAdTudCcdAsu | AS1062 | aUfgGfaAfuAfcUfcUfuGfgUfuAfcAfusGfsa | 0.1 | 0.27 | 0.65 | 0.059 |
| D1063 | S1063 | | AS1063 | dAdTggdAdAuadCdTcudTdGgudTdAcadTsdGsa | 0.12 | 0.44 | 0.82 | 0.064 |
| D1064 | S1064 | | AS1064 | adTdGGfaAfdTdAcUfcUfuGfgUfuAfcAfusGfsa | 0.12 | 0.32 | 0.83 | 0.064 |
| D1065 | S1065 | AfuguAfaccAfaGfaGfdTAdTudCcdAsu | AS1065 | dAUdGgdAadTAfcUfcUfuGfgUfuAfcAfusGfsa | 0.13 | 0.34 | 0.72 | 0.066 |
| D1066 | S1066 | | AS1066 | adTdGGfadAdTAfcUfcUfuGfgUfuAfcAfusGfsa | 0.11 | 0.33 | 0.72 | 0.067 |
| D1067 | S1067 | AfuguAfaccAfaGfaGfdTAdTUdCcdAsu | AS1067 | aUfgGfaAfuAfcUfcUfuGfgUfuAfcAfusGfsa | 0.11 | 0.37 | 0.62 | 0.070 |
| D1068 | S1068 | AfuguAfaccAfaGfaGfdTAdTUdCcdAsu | AS1068 | dAUdGGdAAuAfcUfcUfuGfGfUfuAfCfAfusGfsa | 0.16 | 0.33 | 0.64 | 0.072 |
| D1069 | S1069 | aUfGfuaAfCfccAfGfagUfAfuuCfCfasu | AS1069 | AfUfggAfAfuaCfUfcuUfGfguUfAfcaUfsGfsa | 0.14 | 0.43 | 0.73 | 0.074 |
| D1070 | S1070 | AfuGfuAfaCfCfAfaGfaguAfuUfcCfasUf | AS1070 | aUfgGfaAfuAfCfUfcUfuggUfuAfcAfusGfsa | 0.08 | 0.42 | 0.94 | 0.075 |
| D1071 | S1071 | UfgGfgAfuuuCfaUfgUfaAfcCfaAfgsAf | AS1071 | uCfuUfgGfuUfaCfaUfgAfAfAfuCfcCfasUfsc | 0.14 | 0.28 | 0.83 | 0.0797 |
| D1072 | S1072 | AfuGfuAfaCfcAfaGfAfGfuauUfcCfasUf | AS1072 | aUfgGfaAfUfAfcucUfuGfgUfuAfcAfusGfsa | 0.05 | 0.26 | 0.8 | 0.082 |
| D1073 | S1073 | | AS1073 | aUfgGfadAdTdAdCUfcUfuGfgUfuAfcAfusGfsa | 0.12 | 0.41 | 0.73 | 0.083 |
| D1074 | S1074 | AfUfguAfAfccAfAfgaGfUfauUfCfcasUf | AS1074 | aUfGfgaAfUfacUfCfuuGfGfuuAfCfausGfsa | 0.14 | 0.44 | 0.75 | 0.086 |
| D1075 | S1075 | AfuGfuAfaCfcAfaGfaGfuAfuUfcCfasUf | AS1075 | aUfgGfdAdAdTdAcUfcUfuGfgUfuAfcAfusGfsa | 0.1 | 0.41 | 0.72 | 0.088 |
| D1076 | S1076 | | AS1076 | aUfgdGdAdAdTAfcUfcUfuGfgUfuAfcAfusGfsa | 0.15 | 0.45 | 0.86 | 0.088 |
| D1077 | S1077 | AfuGfuAfaCfcAfaGfaGfuAfuUfcCfasu | AS1077 | AfUfgGfaAfuAfcUfcUfuGfgUfuAfcAfusGfsa | 0.08 | 0.46 | 0.95 | 0.092 |
| D1078 | S1078 | AfuGfuAfaCfcAfaGfaGfuAfuUfcCfasUf | AS1078 | dAUdGGdAadTAfcUfcUfuGfgUfuAfcAfusGfsa | 0.09 | 0.32 | 0.76 | 0.093 |
| D1079 | S1079 | AfuguAfaccAfaGfaGfdTadTudCcdAsu | AS1079 | dAudGgdAadTAfcUfcUfuGfgUfuAfcAfusGfsa | 0.14 | 0.38 | 0.76 | 0.095 |
| D1080 | S1080 | AfuGfuAfaCfcAfaGfAfGfuAfuucCfasUf | AS1080 | aUfgGfAfAfuAfcucUfuGfgUfuAfcAfusGfsa | 0.05 | 0.42 | 0.86 | 0.099 |
| D1081 | S1081 | | AS1081 | dAdTdGdGaAfuAfcUfcUfuGfgUfuAfcAfusGfsa | 0.17 | 0.47 | 0.9 | 0.105 |
| D1082 | S1082 | AfuGfuAfaccaagaguAfuUfcCfasUf | AS1082 | aUfgGfaAfudACfudCUfudGGfudTAfcAfusgsa | 0.12 | 0.44 | 0.83 | 0.106 |
| D1083 | S1083 | AfuGfuAfaCfcAfaGfaGfuAfuUfcCfasUf | AS1083 | adTdGGfaAfdTdAcUfcUfuGfgUfuAfcAfusGfsa | 0.11 | 0.34 | 0.74 | 0.109 |
| D1084 | S1084 | AfuGfuAfAfCfcAfaGfaGfuauUfcCfasUf | AS1084 | aUfgGfaAfUfAfcUfcUfuGfguuAfcAfusGfsa | 0.1 | 0.45 | 0.93 | 0.117 |
| D1085 | S1085 | AfuGfUfAfaCfcAfaGfaGfuauUfcCfasUf | AS1085 | aUfgGfaAfUfAfcUfcUfuGfgUfuacAfusGfsa | 0.07 | 0.42 | 0.78 | 0.120 |
| D1086 | S1086 | aUfguAfAfccAfAfgaGfuAfuUfcCfasUf | AS1086 | aUfgGfaAfuAfcUfCfuuGfGfuuAfCfaUfsgsa | 0.17 | 0.45 | 0.83 | 0.1197 |
| D1087 | S1087 | AfuGfuAfaCfcAfaGfaGfUfAfuUfcCfasu | AS1087 | AfUfgGfaAfuacUfcUfuGfgUfuAfcAfusGfsa | 0.05 | 0.3 | 0.7 | 0.120 |
| D1088 | S1088 | AfuGfuAfaCfcAfaGfaGfuAfuUfcCfasUf | AS1088 | aUfgGfaAfuAfcUfcUfuGfgUfuAfcAfusgsa | 0.11 | 0.46 | 0.8 | 0.120 |
| D1089 | S1089 | AfuGfuAfaCfcAfaGfaGfUfAfuUfcCfasUf | AS1089 | aUfgGfaAfuacUfcUfuGfgUfuAfcAfusGfsa | 0.14 | 0.49 | 0.85 | 0.122 |
| D1090 | S1090 | AfuGfuAfaCfcAfaGfaGfuauUfcCfasUf | AS1090 | aUfgGfaAfUfAfcUfcUfuGfgUfuAfcAfusGfsa | 0.1 | 0.41 | 0.85 | 0.125 |
| D1091 | S1091 | AfuguAfaccAfaGfaGfdTAdTudCcdAsu | AS1091 | aUfgGfaAfuAfcUfcUfuGfgUfuAfcAfusGfsa | 0.16 | 0.38 | 0.77 | 0.125 |
| D1092 | S1092 | AfuGfuAfaCfcAfaGfAfGfuAfuUfcCfasu | AS1092 | AfUfgGfaAfuAfcucUfuGfgUfuAfcAfusGfsa | 0.05 | 0.31 | 0.93 | 0.126 |
| D1093 | S1093 | auGfuAfaCfcAfaGfAfGfuAfuUfcCfasUf | AS1093 | aUfgGfaAfuAfcucUfuGfgUfuAfcAfUfsGfsa | 0.06 | 0.33 | 0.9 | 0.135 |
| D1094 | S1094 | AfuGfuAfaCfcAfaGfaGfUfAfuUfccasUf | AS1094 | aUfGfGfaAfuacUfcUfuGfgUfuAfcAfusGfsa | 0.07 | 0.39 | 0.85 | 0.142 |
| D1095 | S1095 | AfuGfuAfaCfcAfaGfAfGfuAfuUfcCfasUf | AS1095 | aUfgGfaAfuAfcucUfuGfgUfuAfcAfusGfsa | 0.09 | 0.39 | 0.76 | 0.146 |
| D1096 | S1096 | AfuGfuAfaCfcAfaGfaGfUfAfuucCfasUf | AS1096 | aUfgGfAfAfuacUfcUfuGfgUfuAfcAfusGfsa | 0.06 | 0.38 | 0.85 | 0.147 |
| D1097 | S1097 | AfuGfUfAfaCfcAfaGfaGfuAfuucCfasUf | AS1097 | aUfgGfAfAfuAfcUfcUfuGfgUfuacAfusGfsa | 0.12 | 0.47 | 0.87 | 0.147 |
| D1098 | S1098 | AfuGfuAfaCfcAfaGfaGfuAfUfUfccasUf | AS1098 | aUfGfGfaauAfcUfcUfuGfgUfuAfcAfusGfsa | 0.06 | 0.42 | 0.85 | 0.151 |
| D1099 | S1099 | AfuGfuAfaCfcAfaGfaGfuAfuUfcCfasUf | AS1099 | dAUdGGdAadTAfdCUfcUfuGfgUfuAfcAfusGfsa | 0.16 | 0.41 | 0.85 | 0.152 |
| D1100 | S1100 | AfuguAfaccAfaGfaGfuAfuUfcCfasUf | AS1100 | aUfgGfaAfuAfcUfcUfuGfgUfuAfcAfusGfsa | 0.15 | 0.48 | 0.72 | 0.152 |
| D1101 | S1101 | AfuGfuAfaCfcAfaGfAfGfuAfuUfccasUf | AS1101 | aUfGfGfaAfuAfcucUfuGfgUfuAfcAfusGfsa | 0.06 | 0.38 | 0.94 | 0.158 |
| D1102 | S1102 | AfuGfuAfaccaagaguAfuUfcCfasUf | AS1102 | aUfgGfaAfuAfdCuCfdTuGfdGuUfacAfusGfsa | 0.21 | 0.45 | 0.89 | 0.162 |
| D1103 | S1103 | AfuGfuaaCfCfAfaGfaGfuAfuUfcCfasUf | AS1103 | aUfgGfaAfuAfcUfcUfuggUfUfAfcAfusGfsa | 0.14 | 0.49 | 0.95 | 0.163 |
| D1104 | S1104 | AfuGfuAfaccAfaGfaGfUfAfuUfcCfasUf | AS1104 | aUfgGfaAfuacUfcUfuGfGfUfuAfcAfusGfsa | 0.06 | 0.36 | 0.92 | 0.163 |
| D1105 | S1105 | AfuGfuAfaCfcAfaGfaGfuAfuucCfasUf | AS1105 | aUfgGfAfAfuAfcUfcUfuGfgUfuAfcAfusGfsa | 0.1 | 0.45 | 0.84 | 0.167 |
| D1106 | S1106 | AfuGfuaaCfcAfaGfAfGfuAfuUfcCfasUf | AS1106 | aUfgGfaAfuAfcucUfuGfgUfUfAfcAfusGfsa | 0.09 | 0.43 | 0.91 | 0.170 |
| D1107 | S1107 | AfuGfuAfaccAfaGfAfGfuAfuUfcCfasUf | AS1107 | aUfgGfaAfuAfcucUfuGfGfUfuAfcAfusGfsa | 0.09 | 0.46 | 1 | 0.171 |
| D1108 | S1108 | AfuguAfaccAfaGfaGfdTadTudCcdAsu | AS1108 | aUfgGfaAfuAfcUfcUfuGfgUfuAfcAfusGfsa | 0.11 | 0.39 | 0.71 | 0.176 |
| D1109 | S1109 | AfuGfUfAfaCfcAfaGfaGfuAfuUfccasUf | AS1109 | aUfGfGfaAfuAfcUfcUfuGfgUfuacAfusGfsa | 0.1 | 0.43 | 0.9 | 0.180 |
| D1110 | S1110 | AfuGfuAfaCfcAfaGfaguAfUfUfcCfasUf | AS1110 | aUfgGfaauAfCfUfcUfuGfgUfuAfcAfusGfsa | 0.06 | 0.42 | 0.88 | 0.182 |
| D1111 | S1111 | AfuGfuAfaCfcAfaGfaGfuAfuUfcCfasUf | AS1111 | dAUdGGdAAuAfcUfcUfuGfGfUfuAfCfAfusGfsa | 0.18 | 0.49 | 0.79 | 0.183 |
| D1112 | S1112 | AfuGfUfAfaccAfaGfaGfuAfuUfcCfasUf | AS1112 | aUfgGfaAfuAfcUfcUfuGfGfUfuacAfusGfsa | 0.14 | 0.48 | 0.85 | 0.195 |
| D1113 | S1113 | AfuGfuAfaCfcAfaGfaguAfuUfcCfasUf | AS1113 | aUfgGfaAfuAfCfUfcUfuGfgUfuAfcAfusGfsa | 0.09 | 0.41 | 0.85 | 0.201 |
| D1114 | S1114 | auGfuAfaCfcAfaGfaGfUfAfuUfcCfasUf | AS1114 | aUfgGfaAfuacUfcUfuGfgUfuAfcAfUfsGfsa | 0.05 | 0.44 | 0.94 | 0.201 |
| D1115 | S1115 | AfuguAfaCfcAfaGfaGfUfAfuUfcCfasUf | AS1115 | aUfgGfaAfuacUfcUfuGfgUfuAfCfAfusGfsa | 0.08 | 0.41 | 0.96 | 0.204 |
| D1116 | S1116 | AfuGfuAfaCfcAfaGfaGfuAfuUfcCfasUf | AS1116 | adTdGGfadAdTAfcUfcUfuGfgUfuAfcAfusGfsa | 0.15 | 0.47 | 0.79 | 0.208 |
| D1117 | S1117 | AfuGfuaaCfcAfaGfaGfUfAfuUfcCfasUf | AS1117 | aUfgGfaAfuacUfcUfuGfgUfUfAfcAfusGfsa | 0.08 | 0.42 | 0.92 | 0.224 |
| D1118 | S1118 | auguaaccaagaguauuccasu | AS1118 | | 0.19 | 0.5 | 0.87 | 0.303 |
| D1119 | S1119 | AfuGfuAfaCfcAfaGfaGfuAfuUfcCfasUf | AS1119 | aUfgGfaAfuAfcUfcUfuGfgUfuAfcAfusGfsa | 0.14 | 0.55 | 0.89 | |
| D1120 | S1120 | AfuGfuAfaCfcAfaGfaGfuAfuUfcCfasUf | AS1120 | aUfgGfaAfuAfcUfcUfuGfgUfuAfcAfusGfsa | 0.19 | 0.63 | 0.72 | |
| D1121 | S1121 | AfuGfuAfaccAfaGfaGfuAfuUfcCfasUf | AS1121 | aUfgGfaAfuAfcUfcUfuGfGfUfuAfcAfusGfsa | 0.14 | 0.61 | 0.91 | |
| D1122 | S1122 | AfUfGfuAfaCfcAfaGfaGfuAfuUfccasUf | AS1122 | aUfGfGfaAfuAfcUfcUfuGfgUfuAfcausGfsa | 0.14 | 0.54 | 0.95 | |
| D1123 | S1123 | auGfuAfAfCfcAfaGfaGfuAfuUfcCfasUf | AS1123 | aUfgGfaAfuAfcUfcUfuGfguuAfcAfUfsGfsa | 0.13 | 0.61 | 0.97 | |
| D1124 | S1124 | AfuGfuAfaCfcAfaGfaGfuAfUfUfcCfasUf | AS1124 | aUfgGfaauAfcUfcUfuGfgUfuAfcAfusGfsa | 0.14 | 0.56 | 0.94 | |
| D1125 | S1125 | AfuGfuAfaCfcaaGfaGfuAfuUfcCfasUf | AS1125 | aUfgGfaAfuAfcUfcUfUfGfgUfuAfcAfusGfsa | 0.21 | 0.74 | 0.95 | |
| D1126 | S1126 | AfUfGfuAfaCfcAfaGfaGfuAfuucCfasUf | AS1126 | aUfgGfAfAfuAfcUfcUfuGfgUfuAfcausGfsa | 0.2 | 0.69 | 0.91 | |
| D1127 | S1127 | AfuguAfAfCfcAfaGfaGfuAfuUfcCfasUf | AS1127 | aUfgGfaAfuAfcUfcUfuGfguuAfCfAfusGfsa | 0.17 | 0.7 | 0.96 | |
| D1128 | S1128 | AfUfGfuAfaCfcAfaGfaGfuAfuUfcCfasUf | AS1128 | aUfgGfaAfuAfcUfcUfuGfgUfuAfcausGfsa | 0.19 | 0.62 | 0.85 | |
| D1129 | S1129 | AfuGfuAfaCfcAfaGfaGfuAfuUfCfCfasUf | AS1129 | aUfggaAfuAfcUfcUfuGfgUfuAfcAfusGfsa | 0.23 | 0.76 | 0.98 | |
| D1130 | S1130 | AfuGfuAfaCfcAfagaGfuAfuUfcCfasUf | AS1130 | aUfgGfaAfuAfcUfCfUfuGfgUfuAfcAfusGfsa | 0.21 | 0.64 | 0.9 | |
| D1131 | S1131 | AfuGfuAfAfCfcaaGfaGfuAfuUfcCfasUf | AS1131 | aUfgGfaAfuAfcUfcUfUfGfguuAfcAfusGfsa | 0.17 | 0.7 | 1.01 | |
| D1132 | S1132 | AfuGfUfAfaCfcAfaGfaGfuAfuUfcCfasUf | AS1132 | aUfgGfaAfuAfcUfcUfuGfgUfuacAfusGfsa | 0.17 | 0.58 | 0.87 | |
| D1133 | S1133 | AfuGfuAfaCfcAfaGfaGfuAfuUfcCfAfsUf | AS1133 | augGfaAfuAfcUfcUfuGfgUfuAfcAfusGfsa | 0.33 | 0.89 | 1.05 | |
| D1134 | S1134 | AfUfGfuAfaCfcAfaGfaguAfuUfcCfasUf | AS1134 | aUfgGfaAfuAfCfUfcUfuGfgUfuAfcausGfsa | 0.16 | 0.64 | 0.96 | |
| D1135 | S1135 | AfuGfUfAfaCfcAfaGfaguAfuUfcCfasUf | AS1135 | aUfgGfaAfuAfCfUfcUfuGfgUfuacAfusGfsa | 0.12 | 0.53 | 0.96 | |
| D1136 | S1136 | AfuGfuAfAfCfcAfagaGfuAfuUfcCfasUf | AS1136 | aUfgGfaAfuAfcUfCfUfuGfguuAfcAfusGfsa | 0.16 | 0.58 | 0.98 | |
| D1137 | S1137 | AfuGfuAfAfCfcAfaGfaGfuAfuUfcCfasUf | AS1137 | aUfgGfaAfuAfcUfcUfuGfguuAfcAfusGfsa | 0.16 | 0.6 | 0.91 | |
| D1138 | S1138 | AfuGfuAfaCfcAfaGfaGfuAfuUfcCfasUf | AS1138 | aUfgGfaAfuAfcUfcUfuGfgUfuAfcAfusGfsAf | 0.1 | 0.54 | 0.91 | |
| D1139 | S1139 | AfUfGfuAfaCfcAfagaGfuAfuUfcCfasUf | AS1139 | aUfgGfaAfuAfcUfCfUfuGfgUfuAfcausGfsa | 0.24 | 0.68 | 0.98 | |
| D1140 | S1140 | AfuGfUfAfaCfcAfagaGfuAfuUfcCfasUf | AS1140 | aUfgGfaAfuAfcUfCfUfuGfgUfuacAfusGfsa | 0.13 | 0.75 | 0.9 | |
| D1141 | S1141 | AfuGfuAfAfCfcAfaGfaguAfuUfcCfasUf | AS1141 | aUfgGfaAfuAfCfUfcUfuGfguuAfcAfusGfsa | 0.15 | 0.52 | 1.05 | |
| D1142 | S1142 | AfuGfuAfaCfCfAfaGfaGfuAfuUfcCfasUf | AS1142 | aUfgGfaAfuAfcUfcUfuggUfuAfcAfusGfsa | 0.16 | 0.66 | 0.89 | |
| D1143 | S1143 | auGfuAfaCfcAfaGfaGfuAfuUfcCfasUf | AS1143 | aUfgGfaAfuAfcUfcUfuGfgUfuAfcAfUfsGfsa | 0.12 | 0.51 | 0.89 | |
| D1144 | S1144 | AfUfGfuAfaCfcaaGfaGfuAfuUfcCfasUf | AS1144 | aUfgGfaAfuAfcUfcUfUfGfgUfuAfcausGfsa | 0.25 | 0.71 | 0.95 | |
| D1145 | S1145 | AfuGfUfAfaCfcaaGfaGfuAfuUfcCfasUf | AS1145 | aUfgGfaAfuAfcUfcUfUfGfgUfuacAfusGfsa | 0.17 | 0.74 | 0.98 | |
| D1146 | S1146 | AfuguAfaCfcAfaGfaGfuAfuUfcCfasUf | AS1146 | aUfgGfaAfuAfcUfcUfuGfgUfuAfCfAfusGfsa | 0.11 | 0.51 | 0.86 | |
| D1147 | S1147 | AfuGfuAfaCfcAfaGfaGfuAfuUfccasUf | AS1147 | aUfGfGfaAfuAfcUfcUfuGfgUfuAfcAfusGfsa | 0.1 | 0.52 | 0.83 | |
| D1148 | S1148 | AfUfGfuAfaccAfaGfaGfuAfuUfcCfasUf | AS1148 | aUfgGfaAfuAfcUfcUfuGfGfUfuAfcausGfsa | 0.14 | 0.63 | 0.98 | |
| D1149 | S1149 | AfuGfuAfAfCfcAfaGfaGfuAfuucCfasUf | AS1149 | aUfgGfAfAfuAfcUfcUfuGfguuAfcAfusGfsa | 0.13 | 0.58 | 0.88 | |
| D1150 | S1150 | AfuGfuaaCfcAfaGfaGfuAfuUfcCfasUf | AS1150 | aUfgGfaAfuAfcUfcUfuGfgUfUfAfcAfusGfsa | 0.15 | 0.62 | 0.94 | |
| D1151 | S1151 | AfUfGfuaaCfcAfaGfaGfuAfuUfcCfasUf | AS1151 | aUfgGfaAfuAfcUfcUfuGfgUfUfAfcausGfsa | 0.18 | 0.73 | 0.94 | |
| D1152 | S1152 | auGfUfAfaCfcAfaGfaGfuAfuUfcCfasUf | AS1152 | aUfgGfaAfuAfcUfcUfuGfgUfuacAfUfsGfsa | 0.13 | 0.53 | 0.97 | |
| D1153 | S1153 | AfuGfuAfAfCfcAfaGfaGfuAfuUfccasUf | AS1153 | aUfGfGfaAfuAfcUfcUfuGfguuAfcAfusGfsa | 0.13 | 0.53 | 0.98 | |
| D1154 | S1154 | UfgGfgAfuUfuCfaUfgUfaAfcCfaAfgsAf | AS1154 | uCfuUfgGfuUfaCfaUfgAfaAfuCfcCfasUfsc | 0.09 | 0.5 | 0.78 | |
| D1155 | S1155 | UfgGfGfAfuuuCfaUfgUfAfAfcCfaAfgsAf | AS1155 | uCfuUfgGfuuaCfaUfgAfAfAfuccCfasUfsc | 0.13 | 0.62 | 0.89 | |
| D1156 | S1156 | UfgGfgAfuuuCfaUfGfUfaAfcCfaAfgsAf | AS1156 | uCfuUfgGfuUfacaUfgAfAfAfuCfcCfasUfsc | 0.12 | 0.65 | 0.85 | |
| D1157 | S1157 | UfgGfgAfuUfuCfaUfgUfAfAfcCfaAfgsAf | AS1157 | uCfuUfgGfuuaCfaUfgAfaAfuCfcCfasUfsc | 0.11 | 0.54 | 0.85 | |
| D1158 | S1158 | UfgGfgAfuuuCfaUfgUfAfAfcCfaAfgsAf | AS1158 | uCfuUfgGfuuaCfaUfgAfAfAfuCfcCfasUfsc | 0.13 | 0.53 | 0.8 | |
| D1159 | S1159 | UfGfggAfUfuUfcAfuGfuAfAfccAfAfgsAf | AS1159 | uCfuuGfGfuuAfcAfuGaAfauCfCfcasUfsc | 0.59 | 0.89 | 0.81 | |
| D1160 | S1160 | UfGfggAfUfuuCfaUfgUfAfAfcCfaAfgsAf | AS1160 | uCfuUfgGfuuaCfaUfgAfAfauCfCfcasUfsc | 0.16 | 0.72 | 0.9 | |
| D1161 | S1161 | UfgGfgAfuUfucaUfGfUfaAfcCfaAfgsAf | AS1161 | uCfuUfgGfuUfacaUfGfAfaAfuCfcCfasUfsc | 0.27 | 0.69 | 0.86 | |
| D1162 | S1162 | AfuGfuAfaCfcaaGfaGfUfAfuUfcCfasUf | AS1162 | aUfgGfaAfuacUfcUfUfGfgUfuAfcAfusGfsa | 0.12 | 0.6 | 0.95 | |
| D1163 | S1163 | AfuGfuAfaccAfaGfaGfuAfUfUfcCfasUf | AS1163 | aUfgGfaauAfcUfcUfuGfGfUfuAfcAfusGfsa | 0.05 | 0.56 | 1.02 | |
| D1164 | S1164 | AfuGfuAfaCfcAfagaGfUfAfuUfcCfasUf | AS1164 | aUfgGfaAfuacUfCfUfuGfgUfuAfcAfusGfsa | 0.13 | 0.55 | 1 | |
| D1165 | S1165 | AfuGfuAfaCfcaaGfaGfuAfUfUfcCfasUf | AS1165 | aUfgGfaauAfcUfcUfUfGfgUfuAfcAfusGfsa | 0.09 | 0.6 | 0.97 | |
| D1166 | S1166 | AfuguAfaCfCfAfaGfaGfuAfuUfcCfasUf | AS1166 | aUfgGfaAfuAfcUfcUfuggUfuAfCfAfusGfsa | 0.15 | 0.59 | 0.91 | |
| D1167 | S1167 | AfuGfuAfaCfcAfagaGfuAfUfUfcCfasUf | AS1167 | aUfgGfaauAfcUfCfUfuGfgUfuAfcAfusGfsa | 0.11 | 0.59 | 1 | |
| D1168 | S1168 | AfuGfuAfaCfCfAfagaGfuAfuUfcCfasUf | AS1168 | aUfgGfaAfuAfcUfCfUfuggUfuAfcAfusGfsa | 0.13 | 0.57 | 0.94 | |
| D1169 | S1169 | auGfuAfaCfcAfaGfaGfuAfUfUfcCfasUf | AS1169 | aUfgGfaauAfcUfcUfuGfgUfuAfcAfUfsGfsa | 0.08 | 0.5 | 0.9 | |
| D1170 | S1170 | AfuguAfaCfcAfaGfaGfuAfUfUfcCfasUf | AS1170 | aUfgGfaauAfcUfcUfuGfgUfuAfCfAfusGfsa | 0.06 | 0.53 | 0.91 | |
| D1171 | S1171 | auGfuAfaCfcAfaGfaGfuAfuUfCfCfasUf | AS1171 | aUfggaAfuAfcUfcUfuGfgUfuAfcAfUfsGfsa | 0.07 | 0.56 | 0.89 | |
| D1172 | S1172 | AfuGfuAfaCfCfAfaGfaGfuAfuucCfasUf | AS1172 | aUfgGfAfAfuAfcUfcUfuggUfuAfcAfusGfsa | 0.13 | 0.59 | 0.98 | |
| D1173 | S1173 | AfuGfuAfaCfcaaGfAfGfuAfuUfcCfasUf | AS1173 | aUfgGfaAfuAfcucUfUfGfgUfuAfcAfusGfsa | 0.2 | 0.65 | 1.03 | |
| D1174 | S1174 | AfuGfuaaCfcAfaGfaGfuAfUfUfcCfasUf | AS1174 | aUfgGfaauAfcUfcUfuGfgUfUfAfcAfusGfsa | 0.07 | 0.51 | 0.95 | |
| D1175 | S1175 | AfuguAfaCfcAfaGfaGfuAfuUfCfCfasUf | AS1175 | aUfggaAfuAfcUfcUfuGfgUfuAfCfAfusGfsa | 0.2 | 0.53 | 0.76 | |
| D1176 | S1176 | auGfuAfaCfcAfaGfaGfuAfuUfcCfAfsUf | AS1176 | augGfaAfuAfcUfcUfuGfgUfuAfcAfusGfsa | 0.74 | 0.98 | 0.81 | |
| D1177 | S1177 | AfuGfuAfaCfcAfaGfaGfuAfuucCfAfsUf | AS1177 | augGfAfAfuAfcUfcUfuGfgUfuAfcAfusGfsa | 0.43 | 0.64 | 0.88 | |
| D1178 | S1178 | auguaaccAfaGfaGfuAfuUfcCfasUf | AS1178 | aUfgGfaAfuAfcUfcUfuGfgUfuAfcAfusGfsa | 0.17 | 0.49 | 0.81 | |
| D1179 | S1179 | AfuGfuaaCfcAfaGfaGfuAfuUfCfCfasUf | AS1179 | aUfggaAfuAfcUfcUfuGfgUfUfAfcAfusGfsa | 0.22 | 0.65 | 0.73 | |
| D1180 | S1180 | AfuguAfaCfcAfaGfaGfuAfuUfcCfAfsUf | AS1180 | augGfaAfuAfcUfcUfuGfgUfuAfcAfUfsGfsa | 0.6 | 1.09 | 0.8 | |
| D1181 | S1181 | auGfuAfaCfcAfaGfaGfuAfuUfccasu | AS1181 | aUfgGfaAfuAfcUfcUfuGfgUfuAfcAfusGfsa | 0.3 | 0.78 | 0.78 | |
| D1182 | S1182 | auguaaeeaaGfaGfuAfuUfeCfasUf | AS1182 | aUfgGfaAfuAfcUfcUfuGfgUfuAfcAfusGfsa | 0.35 | 0.73 | 0.84 | |
| D1183 | S1183 | AfuGfuAfaccAfaGfaGfuAfuUfCfCfasUf | AS1183 | aUfggaAfuAfcUfcUfuGfGfUfuAfcAfusGfsa | 0.19 | 0.6 | 0.94 | |
| D1184 | S1184 | AfuGfuaaCfcAfaGfaGfuAfuUfcCfAfsUf | AS1184 | augGfaAfuAfcUfcUfuGfgUfuAfCfAfusGfsa | 0.61 | 1.08 | 0.8 | |
| D1185 | S1185 | auGfuAfaCfcAfaGfaGfuAfuuccasu | AS1185 | aUfgGfaAfuAfcUfcUfuGfgUfuAfcAfusGfsa | 0.16 | 0.52 | 0.72 | |
| D1186 | S1186 | auguaaccaagaGfuAfuUfcCfasUf | AS1186 | aUfgGfaAfuAfcUfcUfuGfgUfuAfcAfusGfsa | 0.2 | 0.53 | 0.74 | |
| D1187 | S1187 | AfuGfuAfaCfcaaGfaGfuAfuUfCfCfasUf | AS1187 | aUfggaAfuAfcUfcUfUfGfgUfuAfcAfusGfsa | 0.34 | 0.66 | 0.85 | |
| D1188 | S1188 | AfuGfuAfaccAfaGfaGfuAfuUfcCfAfsUf | AS1188 | augGfaAfuAfcUfcUfuGfgUfUfAfcAfusGfsa | 0.61 | 0.98 | 1.02 | |
| D1189 | S1189 | AfuGfuAfaCfcAfaGfaGfuAfuuccasu | AS1189 | aUfgGfaAfuAfcUfcUfuGfgUfuAfcAfusGfsa | 0.3 | 0.73 | 0.85 | |
| D1190 | S1190 | auguaaccaagaguauuccasu | AS1190 | aUfgGfaAfuAfcUfcUfuGfgUfuAfcAfusGfsa | 0.28 | 0.69 | 0.78 | |
| D1191 | S1191 | AfuGfuAfaCfcAfaGfaGfuAfuUfcCfasUf | AS1191 | aUfgGfaAfuAfcUfcUfugdGudTadCadTsgsa | 0.33 | 0.88 | 0.64 | |
| D1192 | S1192 | AfuGfuAfaCfcAfagaGfuAfuUfCfCfasUf | AS1192 | aUfggaAfuAfcUfCfUfuGfgUfuAfcAfusGfsa | 0.31 | 0.64 | 0.83 | |
| D1193 | S1193 | AfuGfuAfaCfcaaGfaGfuAfuUfcCfAfsUf | AS1193 | augGfaAfuAfcUfcUfuGfGfUfuAfcAfusGfsa | 0.64 | 0.82 | 0.92 | |
| D1194 | S1194 | AfuGfuAfaCfcAfaGfaGfuauuccasu | AS1194 | aUfgGfaAfuAfcUfcUfuGfgUfuAfcAfusGfsa | 0.21 | 0.62 | 0.77 | |
| D1195 | S1195 | AfuGfuAfaCfcAfaGfaGfuAfuUfcCfasUf | AS1195 | aUfgGfaAfuAfcUfcUfuGfGfUfuAfCfAfusGfsa | 0.17 | 0.7 | 0.95 | |
| D1196 | S1196 | AfuGfuAfaCfcAfaGfaguAfuUfCfCfasUf | AS1196 | aUfggaAfuAfCfUfcUfuGfgUfuAfcAfusGfsa | 0.19 | 0.71 | 0.65 | |
| D1197 | S1197 | AfuGfuAfaCfcAfagaGfuAfuUfcCfAfsUf | AS1197 | augGfaAfuAfcUfcUfUfGfgUfuAfcAfusGfsa | 0.64 | 0.82 | 0.93 | |
| D1198 | S1198 | auguAfaCfcAfaGfaGfuAfuUfccasu | AS1198 | aUfgGfaAfuAfcUfcUfuGfgUfuAfcAfusGfsa | 0.19 | 0.65 | 0.72 | |
| D1199 | S1199 | AfuGfuAfaCfcAfaGfaGfuauUfCfCfasUf | AS1199 | aUfggaAfUfAfcUfcUfuGfgUfuAfcAfusGfsa | 0.15 | 0.52 | 0.64 | |
| D1200 | S1200 | AfuGfuAfaCfcAfaGfaguAfuUfcCfAfsUf | AS1200 | augGfaAfuAfcUfCfUfuGfgUfuAfcAfusGfsa | 0.48 | 0.74 | 0.92 | |
| D1201 | S1201 | auguAfaCfcAfaGfaGfuAfuUfcCfasu | AS1201 | aUfgGfaAfuAfcUfcUfuGfgUfuAfcAfusGfsa | 0.17 | 0.71 | 0.77 | |
| D1202 | S1202 | AfuGfuAfaCfcAfaGfaGfuauUfcCfAfsUf | AS1202 | augGfaAfuAfCfUfcUfuGfgUfuAfcAfusGfsa | 0.43 | 0.69 | 0.85 | |
| D1203 | S1203 | auguaaCfcAfaGfaGfuAfuUfcCfasUf | AS1203 | aUfgGfaAfuAfcUfcUfuGfgUfuAfcAfusGfsa | 0.14 | 0.61 | 0.76 | |
| D1204 | S1204 | AfuGfuAfaCfcAfaGfaGfuAfuUfcCfasUf | AS1204 | adTdGGfaAfudAdCUfcUfuGfgUfuAfcAfusGfsa | 0.16 | 0.56 | 0.89 | |
| D1205 | S1205 | | AS1205 | aUfgGfdAdAdTdAcUfcUfuGfgUfuAfcAfusGfsa | 0.13 | 0.57 | 0.9 | |
| D1206 | S1206 | AfuGfuAfaCfcAfaGfaGfuAfuUfcCfasUf | AS1206 | adTdGdGdAAfuAfcUfcUfuGfgUfuAfcAfusGfsa | 0.29 | 0.73 | 0.89 | |
| D1207 | S1207 | AfuGfuAfaCfcAfaGfaGfuAfuUfcCfasUf | AS1207 | adTdGGfaAfuAfdCdTcUfuGfgUfuAfcAfusGfsa | 0.16 | 0.56 | 0.78 | |
| D1208 | S1208 | AfuGfuAfaCfcAfaGfaGfuAfuUfcCfasUf | AS1208 | aUfdGdGdAdAuAfcUfcUfuGfgUfuAfcAfusGfsa | 0.22 | 0.67 | 0.89 | |
| D1209 | S1209 | AfuguAfaccAfaGfaGfuAfuUfcCfasUf | AS1209 | aUfgGfaAfuAfcUfcUfuGfGfUfuAfCfAfusGfsa | 0.14 | 0.55 | 0.78 | |
| D1210 | S1210 | AfuGfuAfaCfcAfaGfaGfuAfuUfcCfasUf | AS1210 | aUfgdGdAdAdTAfcUfcUfuGfgUfuAfcAfusGfsa | 0.14 | 0.5 | 0.84 | |
| D1211 | S1211 | AfuGfuAfaCfcAfaGfaGfuAfuUfcCfasUf | AS1211 | aUfgGfadAdTdAdCUfcUfuGfgUfuAfcAfusGfsa | 0.14 | 0.59 | 0.72 | |
| D1212 | S1212 | auguaaccaaGfaGfuAfuUfcCfasUf | AS1212 | aUfgGfaAfuAfcUfcUfugdGudTadCadTsgsa | 0.21 | 0.74 | 0.77 | |
| D1213 | S1213 | | AS1213 | adTdGdGdAAfuAfcUfcUfuGfgUfuAfcAfusGfsa | 0.15 | 0.53 | 0.91 | |
| D1214 | S1214 | aUfgUfaAfcCfaAfgAfgUfaUfuCfcAfsu | AS1214 | aUfgGfaAfuAfcUfcUfuGfgUfuAfcAfusGfsa | 0.12 | 0.71 | 0.87 | |
| D1215 | S1215 | | AS1215 | aUfdGdGdAdAuAfcUfcUfuGfgUfuAfcAfusGfsa | 0.18 | 0.67 | 0.97 | |
| D1216 | S1216 | AfuGfuAfaccaagaguAfuUfcCfasUf | AS1216 | aUfgGfaAfuacucuuggUfuAfcAfusgsa | 0.36 | 0.87 | 1.07 | |
| D1217 | S1217 | AfuGfuAfaccaagaguAfuUfcCfasUf | AS1217 | aUfgGfaAfuAfCfUfCfUfuGfGfuuAfcAfusgsa | 0.37 | 0.73 | 1.03 | |
| D1218 | S1218 | AfUfguAfAfccAfAfgaGfUfauUfCfcasUf | AS1218 | aUfGfgaAfUfacUfCfuuGfGfuuAfCfausGfsa | 0.23 | 0.42 | 0.84 | |
| D1219 | S1219 | AfuGfuAfaccaagaguAfuUfcCfasUf | AS1219 | aUfgGfaAfuaCfUfcUfUfgGfuuAfcAfusgsa | 0.43 | 0.71 | 1.03 | |
| D1220 | S1220 | AfuGfuAfaccaagaguAfuUfcCfasUf | AS1220 | aUfgGfaAfuAfcUfCfUfuGfGfuuAfcAfusgsa | 0.37 | 0.63 | 0.99 | |
| D1221 | S1221 | AfuGfuAfaccaagaguAfuUfcCfasUf | AS1221 | aUfgGfaAfuAfcUfCfUfuGfGfuUfacAfusgsa | 0.29 | 0.84 | 0.88 | |
| D1222 | S1222 | AfuGfuAfaccaagaguAfuUfcCfasUf | AS1222 | aUfgGfaAfuaCfuCfuUfgGfuuAfcAfusgsa | 0.31 | 0.8 | 0.99 | |
| D1223 | S1223 | auGfuAfAfccAfaGfagUfaUfUfcCfasUf | AS1223 | aUfgGfaaUfaCfUfcUfuGfGfuuAfcAfAfsgsa | 0.09 | 0.52 | 0.82 | |
| D1224 | S1224 | AfuGfuAfaccaagaguAfuUfcCfasUf | AS1224 | aUfgGfaAfuadCudCudTgdGuuAfcAfusgsa | 0.22 | 0.79 | 1 | |
| D1225 | S1225 | auGfuaAfccAfagAfguAfuuCfcasUf | AS1225 | aUfGfgAfAfuAfCfuCfUfuGfGfuUfAfcAfUfsGfsa | 0.31 | 0.76 | 0.84 | |
| D1226 | S1226 | AfuGfuAfaccaagaguAfuUfcCfasUf | AS1226 | aUfgGfaAfuadCUfcdTUfgdGuuAfcAfusgsa | 0.26 | 0.64 | 0.87 | |
| D1227 | S1227 | augUfaacCfaagAfguaUfuccAfsu | AS1227 | aUfgGfAfaUfAfCfuCfUfUfgGfUfUfaCfAfUfsGfsa | 0.33 | 0.79 | 0.81 | |
| D1228 | S1228 | AfuGfuAfaCfcAfaGfaGfuAfuUfcCfasUf | AS1228 | aUfgGfaAfuAfcUfcUfuGfgUfuAfcAfusGfsa | 0.464 | 0.932 | 0.978 | |
| D1229 | S1229 | AfuGfuAfaCfcAfaGfaGfuAfuUfcCfasUf | AS1229 | aUfgGfaAfuAfcUfcUfuGfgUfuAfcAfusGfsa | 0.453 | 1.047 | 1.178 | |
| D1230 | S1230 | AfuGfuAfaCfcAfaGfaGfuAfuUfcCfasUf | AS1230 | aUfgGfaAfuAfcUfcUfuGfgUfuAfcAfusGfsa | 0.831 | 0.967 | 1.151 | |
| D1231 | S1231 | auGfuAfAfCfcAfaGfaGfuAfuUfcCfasu | AS1231 | AfUfgGfaAfuAfcUfcUfuGfguuAfcAfUfsGfsa | 0.09 | 0.5 | 1.07 | |
| D1232 | S1232 | AfuGfuAfaCfCfAfaGfaGfuAfuUfcCfasu | AS1232 | AfUfgGfaAfuAfcUfcUfuggUfuAfcAfusGfsa | 0.11 | 0.54 | 1.1 | |
| D1233 | S1233 | AfuGfuAfaCfcAfaGfaGfuAfuUfCfCfasu | AS1233 | AfUfggaAfuAfcUfcUfuGfgUfuAfcAfusGfsa | 0.19 | 0.61 | 0.74 | |
| D1234 | S1234 | aUfgUfaAfcCfaAfgAfgUfaUfuCfcAfsu | AS1234 | AfuGfgAfaUfaCfuCfuUfgGfuUfaCfaUfsgsAf | 0.22 | 0.61 | 0.98 | |
| D1235 | S1235 | aUfgUfaAfcCfaAfgAfgUfaUfuCfcAfsu | AS1235 | AfuGfgAfaUfaCfuCfuUfgGfuUfaCfaUfsgsAf | 0.27 | 0.69 | 0.92 | |
| D1236 | S1236 | AfuGfuAfaCfcAfaGfaGfuAfuUfcCfasUf | AS1236 | AfuGfgAfaUfaCfuCfuUfgGfuUfaCfaUfsgsAf | 0.54 | 1.08 | 0.8 | |
| D1237 | S1237 | augUfaAfccaAfgAfguaUfuCfcasu | AS1237 | AfUfGfgAfaUfAfCfuCfuUfGfGfuUfaCfAfUfsgsa | 0.29 | 0.61 | 0.79 | |
| D1238 | S1238 | AfugUfaAfccaAfgAfguaUfuCfcasu | AS1238 | AfUfGfgAfaUfAfCfuCfuUfGfGfuUfaCfAfusgsa | 0.31 | 0.6 | 0.88 | |
| D1239 | S1239 | AfuGfuAfaCfcAfaGfaGfuAfuUfcCfasUf | AS1239 | dAUdGGdAauAfcUfcUfuGfgUfuAfcAfusGfsa | 0.2 | 0.67 | 0.85 | |
| D1240 | S1240 | AfuGfuAfaCfcAfaGfaGfuAfuUfcCfasUf | AS1240 | dAUdGgdAauAfcUfcUfuGfgUfuAfcAfusGfsa | 0.23 | 0.58 | 0.68 | |
| D1241 | S1241 | AfuGfuAfaCfcAfaGfaGfuAfuUfcCfasUf | AS1241 | dAudGgdAauAfcUfcUfuGfgUfuAfcAfusGfsa | 0.25 | 0.65 | 0.78 | |
| D1242 | S1242 | AfuGfuAfaCfcAfaGfaGfuAfuUfcCfasUf | AS1242 | dAUdGgdAadTAfcUfcUfuGfgUfuAfcAfusGfsa | 0.18 | 0.64 | 0.84 | |
| D1243 | S1243 | AfuGfuAfaCfcAfaGfaGfuAfuUfcCfasUf | AS1243 | dAUdGGdAAfuAfcUfcUfuGfGfUfuAfCfAfusGfsa | 0.19 | 0.72 | 0.87 | |
| D1244 | S1244 | AfuGfuAfaCfcAfaGfaGfuAfuUfcCfasUf | AS1244 | dAUdGgdAadTAfdCUfcUfuGfgUfuAfcAfusGfsa | 0.16 | 0.55 | 0.8 | |
| D1245 | S1245 | AfuGfuAfaCfcAfaGfaGfuAfuUfcCfasUf | AS1245 | dAUdGGdAAuAfcUfcUfuGfgUfuAfcAfusGfsa | 0.22 | 0.51 | 0.9 | |
| D1246 | S1246 | AfuGfuAfaCfcAfaGfaGfuAfuUfcCfasUf | AS1246 | dAudGgdAadTAfcUfcUfuGfgUfuAfcAfusGfsa | 0.27 | 0.78 | 0.66 | |
| D1247 | S1247 | AfuGfuAfaCfcAfaGfaGfuAfuUfcCfasUf | AS1247 | dAdTdGdGaAfuAfcUfcUfuGfgUfuAfcAfusGfsa | 0.16 | 0.57 | 0.97 | |
| D1248 | S1248 | AfacaAfuguUfcUfuGfdCUdCudAudAsa | AS1248 | dTUdAudAgdAGfcAfaGfaAfcAfcUfgUfusUfsu | 0.06 | 0.09 | 0.36 | 0.0047 |
| D1249 | S1249 | AfaCfaGfuGfuUfcUfuGfCfUfcUfaUfasa | AS1249 | UfUfaUfaGfagcAfaGfaAfcAfcUfgUfusUfsu | 0.06 | 0.10 | 0.47 | 0.005 |
| D1250 | S1250 | AfaCfaGfuGfuUfcUfugcUfcUfAfUfasAf | AS1250 | uUfauaGfaGfCfAfaGfaAfcAfcUfgUfusUfsu | 0.07 | 0.14 | 0.55 | 0.005 |
| D1251 | S1251 | AfaCfaGfuGfuUfcUfuGfcucUfAfUfasAf | AS1251 | uUfauaGfAfGfcAfaGfaAfcAfcUfgUfusUfsu | 0.07 | 0.14 | 0.49 | 0.006 |
| D1252 | S1252 | cAGuGuucuuGcucuAuAAdTdT | AS1252 | UuAuAGAGcAAGAAcACUGdTdT | | | | 0.006 |
| D1253 | S1253 | AfaCfaGfuGfuUfcUfugcUfCfUfaUfasAf | AS1253 | uUfaUfagaGfCfAfaGfaAfcAfcUfgUfusUfsu | 0.05 | 0.12 | 0.43 | 0.006 |
| D1254 | S1254 | AfaCfaGfuGfuUfCfUfuGfcUfcUfaUfasa | AS1254 | UfUfaUfaGfaGfcAfagaAfcAfcUfgUfusUfsu | 0.06 | 0.13 | 0.39 | 0.006 |
| D1255 | S1255 | AfaCfaGfuGfuUfcUfuGfcUfCfUfaUfasa | AS1255 | UfUfaUfagaGfcAfaGfaAfcAfcUfgUfusUfsu | 0.08 | 0.17 | 0.48 | 0.007 |
| D1256 | S1256 | AfaCfaGfuGfuUfcUfUfGfcUfcUfaUfasa | AS1256 | UfUfaUfaGfaGfcaaGfaAfcAfcUfgUfusUfsu | 0.08 | 0.14 | 0.40 | 0.007 |
| D1257 | S1257 | AfaCfaGfuGfuUfcUfuGfcUfCfUfaUfasAf | AS1257 | uUfaUfagaGfcAfaGfaAfcAfcUfgUfusUfsUf | 0.07 | 0.12 | 0.40 | 0.007 |
| D1258 | S1258 | AfaCfaguGfuUfCfUfuGfcUfcUfaUfasAf | AS1258 | uUfaUfaGfaGfcAfagaAfcAfCfUfgUfusUfsu | 0.08 | 0.13 | 0.41 | 0.007 |
| D1259 | S1259 | AfaCfAfGfuGfuUfcUfuGfcucUfaUfasAf | AS1259 | uUfaUfaGfAfGfcAfaGfaAfcAfcugUfusUfsu | 0.05 | 0.11 | 0.35 | 0.008 |
| D1260 | S1260 | AfacaGfuGfuUfCfUfuGfcUfcUfaUfasAf | AS1260 | uUfaUfaGfaGfcAfagaAfcAfcUfGfUfusUfsu | 0.06 | 0.12 | 0.40 | 0.008 |
| D1261 | S1261 | AfacaGfuGfuUfcUfuGfcUfCfUfaUfasAf | AS1261 | uUfaUfagaGfcAfaGfaAfcAfcUfGfUfusUfsu | 0.06 | 0.13 | 0.42 | 0.008 |
| D1262 | S1262 | AfaCfaGfuGfuUfcUfuGfcucUfaUfasAf | AS1262 | uUfaUfaGfAfGfcAfaGfaAfcAfcUfgUfusUfsu | 0.06 | 0.13 | 0.37 | 0.008 |
| D1263 | S1263 | cAGuGuucuuGcucuAuAAdTdT | AS1263 | UuAuAGAGcAAGAAcACUGdTdT | | | | 0.008 |
| D1264 | S1264 | AfaCfaGfuGfuUfcUfuGfCfUfcUfauasAf | AS1264 | uUfAfUfaGfagcAfaGfaAfcAfcUfgUfusUfsu | 0.07 | 0.12 | 0.50 | 0.008 |
| D1265 | S1265 | AfaCfaGfuguUfCfUfuGfcUfcUfaUfasAf | AS1265 | uUfaUfaGfaGfcAfagaAfCfAfcUfgUfusUfsu | 0.12 | 0.13 | 0.48 | 0.009 |
| D1266 | S1266 | AfacaGfuGfuUfcUfuGfcUfcUfAfUfasAf | AS1266 | uUfauaGfaGfcAfaGfaAfcAfcUfGfUfusUfsu | 0.07 | 0.15 | 0.51 | 0.009 |
| D1267 | S1267 | AfacaAfuguUfcUfuGfdCudCudAudAsa | AS1267 | dTudAudAgdAGfcAfaGfaAfcAfcAfgUfusUfsu | 0.06 | 0.14 | 0.48 | 0.0088 |
| D1268 | S1268 | AfaCfaGfuGfuUfCfUfuGfcucUfaUfasAf | AS1268 | uUfaUfaGfAfGfcAfagaAfcAfcUfgUfusUfsu | 0.05 | 0.09 | 0.35 | 0.009 |
| D1269 | S1269 | cAGuGuucuuGcucuAuAAdTdT | AS1269 | UuAuAGAGcAAGAAcACUGdTdT | | | | 0.009 |
| D1270 | S1270 | aaCfaGfuGfuUfcUfuGfcUfCfUfaUfasAf | AS1270 | uUfaUfagaGfcAfaGfaAfcAfcUfgUfUfsUfsu | 0.07 | 0.14 | 0.49 | 0.009 |
| D1271 | S1271 | AfaCfaGfUfGfuUfcUfuGfcucUfaUfasAf | AS1271 | uUfaUfaGfAfGfcAfaGfaAfcacUfgUfusUfsu | 0.06 | 0.10 | 0.36 | 0.009 |
| D1272 | S1272 | cAGuGuucuuGcucuAuAAdTdT | AS1272 | UuAuAGAGcAAGAAcACUGdTdT | | | | 0.009 |
| D1273 | S1273 | AfaCfaGfUfGfuUfcUfuGfcUfcUfaUfasAf | AS1273 | uUfaUfaGfaGfcAfaGfaAfcacUfgUfusUfsUf | 0.06 | 0.13 | 0.51 | 0.009 |
| D1274 | S1274 | AfaCfaGfuGfuUfCfUfuGfcUfcuaUfasAf | AS1274 | uUfaUfAfGfaGfcAfagaAfcAfcUfgUfusUfsu | 0.06 | 0.12 | 0.46 | 0.010 |
| D1275 | S1275 | cAGuGuucuuGcucuAuAAdTdT | AS1275 | UuAuAGAGcAAGAAcACUGdTdT | | | | 0.010 |
| D1276 | S1276 | AfaCfaGfuGfuUfCfUfuGfcUfcUfauasAf | AS1276 | uUfAfUfaGfaGfcAfagaAfcAfcUfgUfusUfsu | 0.06 | 0.14 | 0.47 | 0.010 |
| D1277 | S1277 | AfaCfaguGfuUfcUfuGfcUfCfUfaUfasAf | AS1277 | uUfaUfagaGfcAfaGfaAfcAfCfUfgUfusUfsu | 0.07 | 0.15 | 0.50 | 0.010 |
| D1278 | S1278 | AfaCfaGfuGfuUfCfUfugcUfcUfaUfasAf | AS1278 | uUfaUfaGfaGfCfAfagaAfcAfcUfgUfusUfsu | 0.06 | 0.13 | 0.43 | 0.010 |
| D1279 | S1279 | cAGuGuucuuGcucuAuAAdTdT | AS1279 | UuAuAGAGcAAGAAcACUGdTdT | | | | 0.010 |
| D1280 | S1280 | AfaCfaGfuGfuUfcUfuGfcUfcUfaUfasa | AS1280 | UfUfaUfaGfaGfcAfaGfaAfcAfcUfgUfususu | 0.06 | 0.14 | 0.45 | 0.010 |
| D1281 | S1281 | AfaCfAfGfuGfuUfcUfuGfcUfcUfaUfasa | AS1281 | UfUfaUfaGfaGfcAfaGfaAfcAfcugUfusUfsu | 0.07 | 0.18 | 0.46 | 0.011 |
| D1282 | S1282 | AfaCfaGfuGfuUfcUfuGfcUfcUfaUfasAf | AS1282 | uUfaUfaGfaGfcAfaGfaAfcAfcUfgUfusUfsu | 0.07 | 0.15 | 0.55 | 0.011 |
| D1283 | S1283 | AfaCfaGfuGfuUfcUfuGfcucUfaUfasAf | AS1283 | uUfaUfaGfAfGfcAfaGfaAfcAfcUfgUfususu | 0.07 | 0.12 | 0.45 | 0.011 |
| D1284 | S1284 | AfacaGfuGfuUfcUfuGfcUfcUfaUfasAf | AS1284 | uUfaUfaGfaGfcAfaGfaAfcAfcUfGfUfusUfsu | 0.06 | 0.13 | 0.48 | 0.011 |
| D1285 | S1285 | AfAfCfaGfuGfuUfcUfuGfcucUfaUfasAf | AS1285 | uUfaUfaGfAfGfcAfaGfaAfcAfcUfguusUfsu | 0.06 | 0.11 | 0.40 | 0.011 |
| D1286 | S1286 | AfaCfAfGfuGfuUfcUfuGfcUfcUfauasAf | AS1286 | uUfAfUfaGfaGfcAfaGfaAfcAfcugUfusUfsu | 0.06 | 0.16 | 0.47 | 0.011 |
| D1287 | S1287 | AfaCfaGfuGfuUfcUfugcUfcUfaUfasAf | AS1287 | uUfaUfaGfaGfCfAfaGfaAfcAfcUfgUfususu | 0.07 | 0.19 | 0.46 | 0.012 |
| D1288 | S1288 | AfaCfaGfuGfuUfcUfugcUfcUfaUfasAf | AS1288 | uUfaUfaGfaGfCfAfaGfaAfcAfcUfgUfusUfsu | 0.06 | 0.17 | 0.46 | 0.012 |
| D1289 | S1289 | AfaCfaGfuGfuUfcUfUfGfcucUfaUfasAf | AS1289 | uUfaUfaGfAfGfcaaGfaAfcAfcUfgUfusUfsu | 0.05 | 0.09 | 0.31 | 0.012 |
| D1290 | S1290 | AfAfCfaGfuGfuUfcUfuGfcUfcUfaUfasa | AS1290 | UfUfaUfaGfaGfcAfaGfaAfcAfcUfguusUfsu | 0.06 | 0.16 | 0.49 | 0.013 |
| D1291 | S1291 | AfaCfaGfuGfuUfCfUfuGfcUfcUfaUfasAf | AS1291 | uUfaUfaGfaGfcAfagaAfcAfcUfgUfusUfsUf | 0.06 | 0.11 | 0.32 | 0.013 |
| D1292 | S1292 | AfaCfAfGfuGfuUfcUfugcUfcUfaUfasAf | AS1292 | uUfaUfaGfaGfCfAfaGfaAfcAfcugUfusUfsu | 0.06 | 0.14 | 0.44 | 0.013 |
| D1293 | S1293 | AfaCfaGfUfGfuUfcUfuGfcUfcUfaUfasa | AS1293 | UfUfaUfaGfaGfcAfaGfaAfcacUfgUfusUfsu | 0.07 | 0.16 | 0.39 | 0.013 |
| D1294 | S1294 | AfaCfAfGfuGfuUfcUfuGfcUfcuaUfasAf | AS1294 | uUfaUfAfGfaGfcAfaGfaAfcAfcugUfusUfsu | 0.07 | 0.18 | 0.41 | 0.014 |
| D1295 | S1295 | AfaCfaGfUfGfuUfcUfuGfcUfcuaUfasAf | AS1295 | uUfaUfAfGfaGfcAfaGfaAfcacUfgUfusUfsu | 0.07 | 0.18 | 0.47 | 0.014 |
| D1296 | S1296 | | AS1296 | dTdTaudAdGagdCdAagdAdAcadCdTgudTsdTsu | 0.12 | 0.21 | 0.68 | 0.0146 |
| D1297 | S1297 | AfacaGfUfGfuUfcUfuGfcUfcUfaUfasAf | AS1297 | uUfaUfaGfaGfcAfaGfaAfcacUfGfUfusUfsu | 0.06 | 0.15 | 0.50 | 0.016 |
| D1298 | S1298 | AfaCfaGfUfGfuUfcUfuGfcUfcUfauasAf | AS1298 | uUfAfUfaGfaGfcAfaGfaAfcacUfgUfusUfsu | 0.08 | 0.17 | 0.50 | 0.016 |
| D1299 | S1299 | AfaCfaguGfuUfcUfuGfcUfcUfaUfasAf | AS1299 | uUfaUfaGfaGfcAfaGfaAfcAfCfUfgUfususu | 0.07 | 0.16 | 0.50 | 0.018 |
| D1300 | S1300 | AfaCfaGfuGfuUfcUfUfGfcUfcUfauasAf | AS1300 | uUfAfUfaGfaGfcaaGfaAfcAfcUfgUfusUfsu | 0.06 | 0.12 | 0.43 | 0.020 |
| D1301 | S1301 | AfaCfaGfUfGfuUfcUfugcUfcUfaUfasAf | AS1301 | uUfaUfaGfaGfCfAfaGfaAfcacUfgUfusUfsu | 0.07 | 0.17 | 0.45 | 0.021 |
| D1302 | S1302 | AfaCfaGfuguUfcUfUfGfcUfcUfaUfasAf | AS1302 | uUfaUfaGfaGfcaaGfaAfCfAfcUfgUfusUfsu | 0.06 | 0.14 | 0.49 | 0.021 |
| D1303 | S1303 | AfAfCfaguGfuUfcUfuGfcUfcUfaUfasAf | AS1303 | uUfaUfaGfaGfcAfaGfaAfcAfCfUfguusUfsu | 0.07 | 0.24 | 0.51 | 0.022 |
| D1304 | S1304 | AfaCfaGfuGfuucUfuGfcUfcUfaUfasAf | AS1304 | uUfaUfaGfaGfcAfaGfAfAfcAfcUfgUfususu | 0.09 | 0.27 | 0.47 | 0.033 |
| D1305 | S1305 | | AS1305 | udTadTdAgadGdCaadGdAacdAdCugdTdTsusu | 0.19 | 0.36 | 0.86 | 0.045 |
| D1306 | S1306 | AfacaGfuguUfcUfuGfdCUdCUdAudAsa | AS1306 | dTUdAUdAGfaGfcAfaGfaAfCfAfcUfGfUfusUfsu | 0.08 | 0.22 | 0.61 | |
| D1307 | S1307 | | AS1307 | dTUdAUdAGfaGfcAfaGfaAfCfAfcUfGfUfusUfsu | 0.13 | 0.39 | 0.84 | |
| D1308 | S1308 | AfacaGfuguUfcUfuGfdCUdCUdAudAsa | AS1308 | dTUdAUdAgdAGfcAfaGfaAfcAfcUfgUfusUfsu | 0.09 | 0.13 | 0.48 | |
| D1309 | S1309 | | AS1309 | dTUdAUdAgdAGfdCAfaGfaAfcAfcUfgUfusUfsu | 0.07 | 0.13 | 0.58 | |
| D1310 | S1310 | AfacaAfuguUfcUfdTGfdCUdCudAudAsa | AS1310 | dTUdAudAgdAGfdCAfaGfaAfcAfcAfgUfusUfsu | 0.07 | 0.14 | 0.55 | |
| D1311 | S1311 | | AS1311 | dTdTdAdTaGfaGfcAfaGfaAfcAfcAfgUfusUfsu | 0.10 | 0.30 | 0.66 | |
| D1312 | S1312 | AfacaGfuguUfcUfuGfdCUdCUdAudAsa | AS1312 | dTUdAUdAgdAGfcAfaGfaAfcAfcUfgUfusUfsu | 0.09 | 0.13 | 0.48 | |
| D1313 | S1313 | AfAfCfaGfuGfuucUfuGfcUfcUfaUfasAf | AS1313 | uUfaUfaGfaGfcAfaGfAfAfcAfcUfguusUfsu | 0.14 | 0.38 | 0.74 | |
| D1314 | S1314 | AfaCfaGfuGfuUfcUfuGfcUfcUfaUfasAf | AS1314 | uUfaUfaGfaGfcAfaGfaAfcAfcUfgUfusUfsu | 0.07 | 0.19 | 0.54 | |
| D1315 | S1315 | AfaCfaGfuGfuUfcUfuGfcUfcUfaUfasAf | AS1315 | uUfaUfaGfaGfcAfaGfaAfcAfcUfgUfusUfsu | 0.07 | 0.15 | 0.55 | |
| D1316 | S1316 | AfaCfaGfuGfuUfcUfuGfcUfcUfauasAf | AS1316 | uUfAfUfaGfaGfcAfaGfaAfcAfcUfgUfususu | 0.07 | 0.16 | 0.53 | |
| D1317 | S1317 | AfacaGfuGfuUfcUfuGfcUfcUfaUfasAf | AS1317 | uUfaUfaGfaGfcAfaGfaAfcAfcUfGfUfususu | 0.07 | 0.16 | 0.55 | |
| D1318 | S1318 | AfAfCfaGfuguUfcUfuGfcUfcUfaUfasAf | AS1318 | uUfaUfaGfaGfcAfaGfaAfCfAfcUfguusUfsu | 0.10 | 0.32 | 0.61 | |
| D1319 | S1319 | AfaCfaGfuGfuUfcUfuGfcUfcUfaUfasAf | AS1319 | uUfaUfaGfaGfcAfaGfaAfcAfcUfgUfususu | 0.08 | 0.16 | 0.53 | |
| D1320 | S1320 | AfaCfaGfuGfuUfcUfuGfcUfcUfaUfasAf | AS1320 | uUfaUfaGfaGfcAfaGfaAfcAfcUfgUfususu | 0.08 | 0.16 | 0.61 | |
| D1321 | S1321 | AfaCfaGfuGfuUfcUfuGfcUfCfUfaUfasAf | AS1321 | uUfaUfagaGfcAfaGfaAfcAfcUfgUfusUfsu | 0.06 | 0.14 | 0.58 | |
| D1322 | S1322 | AfaCfaGfuGfuUfcuuGfcUfcUfaUfasAf | AS1322 | uUfaUfaGfaGfcAfAfGfaAfcAfcUfgUfusUfsu | 0.15 | 0.49 | 0.84 | |
| D1323 | S1323 | AfaCfaGfuGfuUfcUfuGfcUfcuaUfasAf | AS1323 | uUfaUfAfGfaGfcAfaGfaAfcAfcUfgUfususu | 0.07 | 0.20 | 0.62 | |
| D1324 | S1324 | AfAfCfaGfuGfuUfcUfuGfcUfcUfaUfasAf | AS1324 | uUfaUfaGfaGfcAfaGfaAfcAfcUfguusUfsu | 0.08 | 0.25 | 0.78 | |
| D1325 | S1325 | AfAfCfaGfuGfuUfcUfuGfcUfcUfaUfasAf | AS1325 | uUfaUfaGfaGfcAfaGfaAfcAfcUfguusUfsu | 0.08 | 0.18 | 0.80 | |
| D1326 | S1326 | AfaCfaGfuGfuUfcUfuGfcUfcUfAfUfasAf | AS1326 | uUfauaGfaGfcAfaGfaAfcAfcUfgUfusUfsu | 0.07 | 0.21 | 0.66 | |
| D1327 | S1327 | AfaCfaGfuGfuucUfuGfcUfcUfaUfasAf | AS1327 | uUfaUfaGfaGfcAfaGfAfAfcAfcUfgUfusUfsu | 0.10 | 0.31 | 0.70 | |
| D1328 | S1328 | AfAfCfaGfuGfuUfcUfuGfcUfcUfauasAf | AS1328 | uUfAfUfaGfaGfcAfaGfaAfcAfcUfguusUfsu | 0.07 | 0.15 | 0.55 | |
| D1329 | S1329 | AfaCfAfGfuGfuUfcUfuGfcUfcUfaUfasAf | AS1329 | uUfaUfaGfaGfcAfaGfaAfcAfcugUfusUfsu | 0.08 | 0.19 | 0.71 | |
| D1330 | S1330 | AfaCfaGfuGfuUfcUfuGfcUfcUfaUfAfsAf | AS1330 | uuaUfaGfaGfcAfaGfaAfcAfcUfgUfusUfsu | 0.09 | 0.27 | 0.76 | |
| D1331 | S1331 | AfaCfaGfuguUfcUfuGfcUfcUfaUfasAf | AS1331 | uUfaUfaGfaGfcAfaGfaAfCfAfcUfgUfusUfsu | 0.07 | 0.21 | 0.65 | |
| D1332 | S1332 | AfAfCfaGfuGfuUfcUfuGfcUfcuaUfasAf | AS1332 | uUfaUfAfGfaGfcAfaGfaAfcAfcUfguusUfsu | 0.07 | 0.17 | 0.53 | |
| D1333 | S1333 | AfaCfaGfUfGfuUfcUfuGfcUfcUfaUfasAf | AS1333 | uUfaUfaGfaGfcAfaGfaAfcacUfgUfusUfsu | 0.08 | 0.25 | 0.73 | |
| D1334 | S1334 | AfaCfaguGfuUfcUfuGfcUfcUfaUfasAf | AS1334 | uUfaUfaGfaGfcAfaGfaAfcAfCfUfgUfusUfsu | 0.07 | 0.18 | 0.54 | |
| D1335 | S1335 | AfaCfaGfuGfuUfcuuGfcUfcUfaUfasAf | AS1335 | uUfaUfaGfaGfcAfAfGfaAfcAfcUfgUfususu | 0.14 | 0.38 | 0.57 | |
| D1336 | S1336 | AfaCfaGfuGfUfUfcUfuGfcUfcUfaUfasAf | AS1336 | uUfaUfaGfaGfcAfaGfaacAfcUfgUfusUfsu | 0.16 | 0.50 | 0.96 | |
| D1337 | S1337 | AfaCfaGfuGfuUfcUfuGfcUfcUfauasAf | AS1337 | uUfAfUfaGfaGfcAfaGfaAfcAfcUfgUfusUfsu | 0.08 | 0.19 | 0.54 | |
| D1338 | S1338 | AfAfCfaGfuGfuUfcUfugcUfcUfaUfasAf | AS1338 | uUfaUfaGfaGfCfAfaGfaAfcAfcUfguusUfsu | 0.08 | 0.20 | 0.69 | |
| D1339 | S1339 | AfaCfaGfuGfuUfCfUfuGfcUfcUfaUfasAf | AS1339 | uUfaUfaGfaGfcAfagaAfcAfcUfgUfusUfsu | 0.07 | 0.16 | 0.55 | |
| D1340 | S1340 | AfaCfaGfuGfuUfcUfuGfcUfcuaUfasAf | AS1340 | uUfaUfAfGfaGfcAfaGfaAfcAfcUfgUfusUfsu | 0.08 | 0.17 | 0.57 | |
| D1341 | S1341 | AfaCfaGfuguUfcUfuGfcUfcUfaUfasAf | AS1341 | uUfaUfaGfaGfcAfaGfaAfCfAfcUfgUfususu | 0.08 | 0.22 | 0.63 | |
| D1342 | S1342 | AfAfCfaGfuGfuUfcuuGfcUfcUfaUfasAf | AS1342 | uUfaUfaGfaGfcAfAfGfaAfcAfcUfguusUfsu | 0.21 | 0.56 | 0.86 | |
| D1343 | S1343 | AfacaGfuGfUfUfcUfuGfcUfcUfaUfasAf | AS1343 | uUfaUfaGfaGfcAfaGfaacAfcUfGfUfusUfsu | 0.14 | 0.37 | 0.73 | |
| D1344 | S1344 | AfaCfaGfuGfuucUfUfGfcUfcUfaUfasAf | AS1344 | uUfaUfaGfaGfcaaGfAfAfcAfcUfgUfusUfsu | 0.08 | 0.20 | 0.66 | |
| D1345 | S1345 | AfaCfAfGfuGfuUfcuuGfcUfcUfaUfasAf | AS1345 | uUfaUfaGfaGfcAfAfGfaAfcAfcugUfusUfsu | 0.12 | 0.34 | 0.73 | |
| D1346 | S1346 | AfaCfaGfuGfUfUfcUfuGfcUfcUfauasAf | AS1346 | uUfAfUfaGfaGfcAfaGfaacAfcUfgUfusUfsu | 0.16 | 0.42 | 0.90 | |
| D1347 | S1347 | AfaCfaGfuGfUfUfcUfuGfcUfcUfaUfasAf | AS1347 | uUfaUfaGfaGfcAfaGfaacAfcUfgUfusUfsUf | 0.17 | 0.43 | 0.85 | |
| D1348 | S1348 | AfaCfAfGfuGfuucUfuGfcUfcUfaUfasAf | AS1348 | uUfaUfaGfaGfcAfaGfAfAfcAfcugUfusUfsu | 0.08 | 0.21 | 0.58 | |
| D1349 | S1349 | AfaCfaGfuGfUfUfcUfuGfcUfcuaUfasAf | AS1349 | uUfaUfAfGfaGfcAfaGfaacAfcUfgUfusUfsu | 0.21 | 0.39 | 0.88 | |
| D1350 | S1350 | AfaCfaguGfuUfcUfUfGfcUfcUfaUfasAf | AS1350 | uUfaUfaGfaGfcaaGfaAfcAfCfUfgUfusUfsu | 0.06 | 0.13 | 0.52 | |
| D1351 | S1351 | AfaCfAfGfuguUfcUfuGfcUfcUfaUfasAf | AS1351 | uUfaUfaGfaGfcAfaGfaAfCfAfcugUfusUfsu | 0.08 | 0.21 | 0.58 | |
| D1352 | S1352 | AfaCfaGfUfGfuUfcuuGfcUfcUfaUfasAf | AS1352 | uUfaUfaGfaGfcAfAfGfaAfcacUfgUfusUfsu | 0.18 | 0.49 | 0.84 | |
| D1353 | S1353 | AfaCfaGfuGfUfUfcUfuGfcucUfaUfasAf | AS1353 | uUfaUfaGfAfGfcAfaGfaacAfcUfgUfusUfsu | 0.11 | 0.25 | 0.68 | |
| D1354 | S1354 | AfacaGfuGfuUfcUfUfGfcUfcUfaUfasAf | AS1354 | uUfaUfaGfaGfcaaGfaAfcAfcUfGfUfusUfsu | 0.07 | 0.15 | 0.52 | |
| D1355 | S1355 | AfaCfaGfUfGfuucUfuGfcUfcUfaUfasAf | AS1355 | uUfaUfaGfaGfcAfaGfAfAfcacUfgUfusUfsu | 0.10 | 0.26 | 0.63 | |
| D1356 | S1356 | AfaCfaGfuGfUfUfcUfugcUfcUfaUfasAf | AS1356 | uUfaUfaGfaGfCfAfaGfaacAfcUfgUfusUfsu | 0.16 | 0.33 | 0.79 | |
| D1357 | S1357 | AfaCfAfGfuGfuUfcUfuGfcUfcUfaUfasAf | AS1357 | uUfaUfaGfaGfcAfaGfaAfcAfcugUfusUfsUf | 0.09 | 0.19 | 0.51 | |
| D1358 | S1358 | AfaCfaGfuGfUfUfcuuGfcUfcUfaUfasAf | AS1358 | uUfaUfaGfaGfcAfAfGfaacAfcUfgUfusUfsu | 0.22 | 0.48 | 0.71 | |
| D1359 | S1359 | AfaCfaGfuGfuUfcUfUfGfcUfcUfaUfasAf | AS1359 | uUfaUfaGfaGfcaaGfaAfcAfcUfgUfusUfsUf | 0.10 | 0.17 | 0.61 | |
| D1360 | S1360 | AfaCfaguGfUfUfcUfuGfcUfcUfaUfasAf | AS1360 | uUfaUfaGfaGfcAfaGfaacAfCfUfgUfusUfsu | 0.14 | 0.40 | 0.87 | |
| D1361 | S1361 | AfaCfaGfuGfuUfcUfUfGfcUfcuaUfasAf | AS1361 | uUfaUfAfGfaGfcaaGfaAfcAfcUfgUfusUfsu | 0.07 | 0.14 | 0.52 | |
| D1362 | S1362 | aaCfaGfuGfuUfcUfuGfCfUfcUfaUfasAf | AS1362 | uUfaUfaGfagcAfaGfaAfcAfcUfgUfUfsUfsu | 0.10 | 0.28 | 0.81 | |
| D1363 | S1363 | AfaCfaGfuGfuucUfuGfcUfcUfAfUfasAf | AS1363 | uUfauaGfaGfcAfaGfAfAfcAfcUfgUfusUfsu | 0.06 | 0.16 | 0.68 | |
| D1364 | S1364 | AfaCfaGfuGfuUfcUfugcUfcUfaUfAfsAf | AS1364 | uuaUfaGfaGfCfAfaGfaAfcAfcUfgUfusUfsu | 0.09 | 0.26 | 0.67 | |
| D1365 | S1365 | aacaguguucuugcucuauasa | AS1365 | uUfaUfaGfaGfcAfaGfaAfcAfcUfgUfusUfsu | 0.20 | 0.59 | 0.95 | |
| D1366 | S1366 | AfaCfaGfuGfuUfcUfuGfCfUfcUfauasAf | AS1366 | uUfAfUfaGfagcAfaGfaAfcAfcUfgUfusUfsu | 0.06 | 0.13 | 0.53 | |
| D1367 | S1367 | AfaCfaGfuGfuUfcUfuGfCfUfcUfaUfasAf | AS1367 | uUfaUfaGfagcAfaGfaAfcAfcUfgUfusUfsUf | 0.08 | 0.16 | 0.53 | |
| D1368 | S1368 | AfaCfaGfuguUfcUfuGfcUfcUfAfUfasAf | AS1368 | uUfauaGfaGfcAfaGfaAfCfAfcUfgUfusUfsu | 0.07 | 0.15 | 0.54 | |
| D1369 | S1369 | AfaCfaGfuGfuUfcuuGfcUfcUfaUfAfsAf | AS1369 | uuaUfaGfaGfcAfAfGfaAfcAfcUfgUfusUfsu | 0.23 | 0.56 | 0.89 | |
| D1370 | S1370 | AfaCfaGfuGfuUfcUfuGfCfUfcuaUfasAf | AS1370 | uUfaUfAfGfagcAfaGfaAfcAfcUfgUfusUfsu | 0.06 | 0.12 | 0.55 | |
| D1371 | S1371 | AfaCfaGfuGfuUfcUfuGfCfUfcuaUfasAf | AS1371 | uUfaUfAfGfagcAfaGfaAfcAfcUfgUfusUfsu | 0.07 | 0.18 | 0.58 | |
| D1372 | S1372 | AfaCfaguGfuUfcUfuGfcUfcUfAfUfasAf | AS1372 | uUfauaGfaGfcAfaGfaAfcAfCfUfgUfusUfsu | 0.06 | 0.15 | 0.56 | |
| D1373 | S1373 | AfaCfaGfuGfuucUfuGfcUfcUfaUfAfsAf | AS1373 | uuaUfaGfaGfcAfaGfAfAfcAfcUfgUfusUfsu | 0.21 | 0.51 | 0.89 | |
| D1374 | S1374 | AfacaGfuguUfcUfuGfcUfcUfaUfasAf | AS1374 | uUfaUfaGfaGfcAfaGfaAfCfAfcUfGfUfusUfsu | 0.08 | 0.21 | 0.64 | |
| D1375 | S1375 | AfaCfaGfuGfuUfcuuGfCfUfcUfaUfasAf | AS1375 | uUfaUfaGfagcAfAfGfaAfcAfcUfgUfusUfsu | 0.15 | 0.40 | 0.94 | |
| D1376 | S1376 | AfaCfaGfuGfuUfcuuGfCfUfcUfaUfasAf | AS1376 | uUfaUfaGfagcAfAfGfaAfcAfcUfgUfusUfsu | 0.13 | 0.40 | 0.96 | |
| D1377 | S1377 | AfaCfaGfuGfuUfcUfuGfcUfCfUfauasAf | AS1377 | uUfAfUfagaGfcAfaGfaAfcAfcUfgUfusUfsu | 0.08 | 0.17 | 0.64 | |
| D1378 | S1378 | AfaCfaGfuguUfcUfuGfcUfcUfaUfAfsAf | AS1378 | uuaUfaGfaGfcAfaGfaAfCfAfcUfgUfusUfsu | 0.18 | 0.50 | 0.97 | |
| D1379 | S1379 | AfaCfaGfuGfuucUfuGfCfUfcUfaUfasAf | AS1379 | uUfaUfaGfagcAfaGfAfAfcAfcUfgUfusUfsu | 0.08 | 0.24 | 0.79 | |
| D1380 | S1380 | aaCfaGfuGfuUfcUfuGfcUfcUfAfUfasAf | AS1380 | uUfauaGfaGfcAfaGfaAfcAfcUfgUfUfsUfsu | 0.07 | 0.14 | 0.58 | |
| D1381 | S1381 | AfaCfaguGfuUfcUfuGfcUfcUfaUfAfsAf | AS1381 | uuaUfaGfaGfcAfaGfaAfcAfCfUfgUfusUfsu | 0.11 | 0.34 | 0.96 | |
| D1382 | S1382 | AfaCfaGfuguUfcUfuGfCfUfcUfaUfasAf | AS1382 | uUfaUfaGfagcAfaGfaAfCfAfcUfgUfusUfsu | 0.08 | 0.18 | 0.69 | |
| D1383 | S1383 | AfaCfaGfuGfuUfcuuGfcUfCfUfaUfasAf | AS1383 | uUfaUfagaGfcAfAfGfaAfcAfcUfgUfusUfsu | 0.14 | 0.38 | 0.85 | |
| D1384 | S1384 | AfaCfaGfuGfuUfcUfuGfcUfcUfAfUfasAf | AS1384 | uUfauaGfaGfcAfaGfaAfcAfcUfgUfusUfsUf | 0.07 | 0.16 | 0.54 | |
| D1385 | S1385 | AfacaGfuGfuUfcUfuGfcUfcUfaUfAfsAf | AS1385 | uuaUfaGfaGfcAfaGfaAfcAfcUfGfUfusUfsu | 0.08 | 0.20 | 0.75 | |
| D1386 | S1386 | aacaguguucUfuGfcUcUaudAsa | AS1386 | uUfdAUdAGfaGfcAfaGfaadCadCudGdTdTsusu | 0.25 | 0.56 | 0.90 | |
| D1387 | S1387 | AfaCfaguGfuUfcUfuGfCfUfcUfaUfasAf | AS1387 | uUfaUfaGfagcAfaGfaAfcAfCfUfgUfusUfsu | 0.08 | 0.19 | 0.70 | |
| D1388 | S1388 | AfaCfaGfuGfuucUfuGfcUfCfUfaUfasAf | AS1388 | uUfaUfagaGfcAfaGfAfAfcAfcUfgUfusUfsu | 0.08 | 0.14 | 0.60 | |
| D1389 | S1389 | AfaCfaGfuGfuUfcUfuGfcUfcuaUfAfsAf | AS1389 | uuaUfAfGfaGfcAfaGfaAfcAfcUfgUfusUfsu | 0.08 | 0.19 | 0.62 | |
| D1390 | S1390 | aaCfaGfuGfuUfcUfuGfcUfcUfaUfAfsAf | AS1390 | uuaUfaGfaGfcAfaGfaAfcAfcUfgUfUfsUfsu | 0.08 | 0.27 | 0.76 | |
| D1391 | S1391 | aacaguguucdTudGcdTcdTadTasa | AS1391 | uUfdAUdAGfaGfcAfaGfaadCadCudGudTsusu | 0.18 | 0.36 | 0.81 | |
| D1392 | S1392 | AfacaGfuGfuUfcUfuGfCfUfcUfaUfasAf | AS1392 | uUfaUfaGfagcAfaGfaAfcAfcUfGfUfusUfsu | 0.07 | 0.17 | 0.55 | |
| D1393 | S1393 | AfaCfaGfuguUfcUfuGfcUfCfUfaUfasAf | AS1393 | uUfaUfagaGfcAfaGfaAfCfAfcUfgUfusUfsu | 0.07 | 0.15 | 0.57 | |
| D1394 | S1394 | AfaCfaGfuGfuUfcuuGfcUfcUfAfUfasAf | AS1394 | uUfauaGfaGfcAfAfGfaAfcAfcUfgUfusUfsu | 0.26 | 0.68 | 1.06 | |
| D1395 | S1395 | AfaCfaGfuGfuUfcUfuGfcucUfaUfAfsAf | AS1395 | uuaUfaGfAfGfcAfaGfaAfcAfcUfgUfusUfsu | 0.06 | 0.18 | 0.58 | |
| D1396 | S1396 | AfaCfaGfuGfuUfcUfuGfcUfcUfaUfAfsAf | AS1396 | uuaUfaGfaGfcAfaGfaAfcAfcUfgUfusUfsUf | 0.09 | 0.27 | 0.73 | |
| D1397 | S1397 | | AS1397 | uUfadTdAdGdAGfcAfaGfaGfcAfcAfgUfusUfsu | 0.20 | 0.51 | 0.73 | |
| D1398 | S1398 | AfacaGfuguUfcuuGfcucUfauasAf | AS1398 | uUfAfUfaGfAfGfcAfAfGfaAfCfAfcUfGfUfusUfsu | 0.13 | 0.34 | 0.86 | |
| D1399 | S1399 | dAacadGugudTcuudGcucdTauasdA | AS1399 | | 0.24 | 0.42 | 0.82 | |
| D1400 | S1400 | | AS1400 | uUfaUfdAdGdAdGcAfaGfaGfcAfcAfgUfusUfsu | 0.49 | 0.85 | 0.78 | |
| D1401 | S1401 | | AS1401 | uUfaUfadGdAdGdCAfaGfaGfcAfcAfgUfusUfsu | 0.67 | 0.83 | 0.85 | |
| D1402 | S1402 | aaCfAfguGfUfucUfUfgcUfCfuaUfAfsa | AS1402 | uUfaUfAfgaGfCfaaGfAfacAfCfugUfUfsusu | 0.18 | 0.47 | 0.80 | |
| D1403 | S1403 | | AS1403 | udTdAUfadGdAGfcAfaGfaGfcAfcAfgUfusUfsu | 0.73 | 0.89 | 0.77 | |
| D1404 | S1404 | aacAgugUucuUgcuCuauAsa | AS1404 | uUaUAgAGCaAGAaCACuGUUsusu | 0.12 | 0.39 | 0.79 | |
| D1405 | S1405 | AacaGuguUcuuGcucUauasA | AS1405 | uUAUaGAGcAAGaACAcUGUusUsu | 0.12 | 0.37 | 0.77 | |
| D1406 | S1406 | | AS1406 | udTdAUfaGfadGdCAfaGfaGfcAfcAfgUfusUfsu | 0.59 | 0.93 | 0.89 | |
| D1407 | S1407 | aACagUGuuCUugCUcuAUasa | AS1407 | UUauAGagCAagAAcaCUguUsUsu | 0.09 | 0.16 | 0.55 | |
| D1408 | S1408 | | AS1408 | udTdAdTdAGfaGfcAfaGfaAfcAfcAfgUfusUfsu | 0.22 | 0.64 | 0.86 | |
| D1409 | S1409 | aaCAguGUucUUgcUCuaUAsa | AS1409 | uUaUAgaGCaaGAacACugUUsusu | 0.13 | 0.31 | 0.76 | |
| D1410 | S1410 | | AS1410 | udTdAdTdAdGaGfcAfaGfaGfcAfcAfgUfusUfsu | 0.77 | 0.94 | 0.93 | |
| D1411 | S1411 | aacAfgugUfucuUfgcuCfuauAfsa | AS1411 | uUfaUfAfgAfGfCfaAfGfAfaCfAfCfuGfUfUfsusu | 0.23 | 0.53 | 1.04 | |
| D1412 | S1412 | aacdAgugdTucudTgcudCuaudAsa | AS1412 | | 0.30 | 0.64 | 0.90 | |
| D1413 | S1413 | AfaCfaGfuGfuUfcUfuGfcUfcUfaUfasa | AS1413 | UfUfaUfaGfaGfcAfaGfaAfcAfcUfgUfusUfsu | 0.09 | 0.19 | 0.63 | |
| D1414 | S1414 | AfaCfaGfuGfUfUfcUfuGfcUfcUfaUfasa | AS1414 | UfUfaUfaGfaGfcAfaGfaacAfcUfgUfusUfsu | 0.11 | 0.28 | 0.66 | |
| D1415 | S1415 | AfaCfaGfuGfuUfcUfuGfCfUfcUfaUfasa | AS1415 | UfUfaUfaGfagcAfaGfaAfcAfcUfgUfusUfsu | 0.06 | 0.13 | 0.53 | |
| D1416 | S1416 | aacaguguucuugcucuauasa | AS1416 | | 0.20 | 0.53 | 0.99 | |
| D1417 | S1417 | AfaCfaGfuGfuUfcUfuGfcUfcUfAfUfasa | AS1417 | UfUfauaGfaGfcAfaGfaAfcAfcUfgUfusUfsu | 0.07 | 0.17 | 0.53 | |
| D1418 | S1418 | aAfCfagUfGfuuCfUfugCfUfcuAfUfasa | AS1418 | UfUfauAfGfagCfAfagAfAfcaCfUfguUfsUfsu | 0.08 | 0.20 | 0.70 | |
| D1419 | S1419 | AfaCfAfGfuGfuUfcUfuGfcUfcUfaUfasAf | AS1419 | uUfaUfaGfaGfcAfaGfaAfcAfcugUfusUfsUf | 0.08 | 0.20 | 0.70 | |

### Example 3: In vitro silencing activity with various chemical modifications on TTR siRNA

The IC₅₀ for each modified siRNA is determined in Hep3B cells by standard reverse transfection using Lipofectamine RNAiMAX. In brief, reverse transfection is carried out by adding 5 µL of Opti-MEM to 5 µL of siRNA duplex per well into a 96-well plate along with 10 µL of Opti-MEM plus 0.5 µL of Lipofectamine RNAiMax per well (Invitrogen, Carlsbad CA. cat # 13778-150) and incubating at room temperature for 15-20 minutes. Following incubation, 100 µL of complete growth media without antibiotic containing 12,000-15,000 Hep3B cells is then added to each well. Cells are incubated for 24 hours at 37°C in an atmosphere of 5% CO2 prior to lysis and analysis of ApoB and GAPDH mRNA by bDNA (Quantigene). Seven different siRNA concentrations ranging from 10nM to 0.6pM are assessed for IC₅₀ determination and ApoB/GAPDH for ApoB transfected cells is normalized to cells transfected with 10nM Luc siRNA.

| **Abbreviation** | **Nucleotide(s)** |
|---|---|
| Af | 2'-F-adenosine |
| Cf | 2'-F-cytidine |
| Gf | 2'-F-guanosine |
| Uf | 2'-F-uridine |
| A | adenosine |
| C | cytidine |
| G | guanosine |
| U | uridine |
| a | 2'-O-methyladenosine |
| c | 2'-O-methylcytidine |
| g | 2'-O-methylguanosine |
| u | 2'-O-methyluridine |
| dT | 2'-deoxythymidine |
| s | phosphorothioate linkage |

**Table 3. ANGPTL3 modified duplex**

| Duplex ID | Sense ID | SS seq | AS ID | AS seq | RNAimax, Hep3b | | |
|---|---|---|---|---|---|---|---|
| | | | | | 10nM | 0.1n M | 0.025n M |
| D2000 | S2000 | UfcAfcAfaUfuAfAfGfcUfcCfuUfcUfuUf | A2000 | aAfaGfaAfgGfaGfcuuAfaUfuGfuGfasAfsc | 0.036 | 0.274 | 0.233 |
| D2001 | S2001 | UfuAfuUfgUfuCfCfUfcUfaGfuUfaUfuUf | A2001 | aAfaUfaAfcUfaGfaggAfaCfaAfuAfasAfsa | 0.044 | 0.278 | 0.247 |
| D2002 | S2002 | GfcUfaUfgUfuAfGfAfcGfaUfgUfaAfaAf | A2002 | uUfuUfaCfaUfcGfucuAfaCfaUfaGfcsAfsa | 0.062 | 0.474 | 0.449 |
| D2003 | S2003 | GfgAfcAfuGfgUfCfUfuAfaAfgAfcUfuUf | A2003 | aAfaGfuCfuUfuAfagaCfcAfuGfuCfcsCfsa | 0.303 | 1.042 | 0.912 |
| D2004 | S2004 | CfaAfaAfaCfuCfAfAfcAfuAfuUfuGfaUf | A2004 | aUfcAfaAfuAfuGfuugAfgUfuUfuUfgsAfsa | 0.102 | 0.623 | 0.499 |
| D2005 | S2005 | AfcCfaGfuGfaAfAfUfcAfaAfgAfaGfaAf | A2005 | uUfcUfuCfuUfuGfauuUfcAfcUfgGfusUfsu | 0.124 | 0.901 | 0.756 |
| D2006 | S2006 | CfaCfaAfuUfaAfGfCfuCfcUfuCfuUfuUf | A2006 | aAfaAfgAfaGfgAfgcuUfaAfuUfgUfgsAfsa | 0.069 | 0.269 | 0.244 |
| D2007 | S2007 | CfuAfuGfuUfaGfAfCfgAfuGfuAfaAfaAf | A2007 | uUfuUfuAfcAfuCfgucUfaAfcAfuAfgsCfsa | 0.052 | 0.622 | 0.589 |
| D2008 | S2008 | UfcAfaCfaUfaUfUfUfgAfuCfaGfuCfuUf | A2008 | aAfgAfcUfgAfuCfaaaUfaUfgUfuGfasGfsu | 0.133 | 0.798 | 0.785 |
| D2009 | S2009 | AfaCfuGfaGfaAfGfAfaCfuAfcAfuAfuAf | A2009 | uAfuAfuGfuAfgUfucuUfcUfcAfgUfusCfsc | 0.097 | 0.671 | 0.528 |
| D2010 | S2010 | AfcAfaUfuAfaGfCfUfcCfuUfcUfuUfuUf | A2010 | aAfaAfaGfaAfgGfagcUfuAfaUfuGfusGfsa | 0.145 | 0.308 | 0.293 |
| D2011 | S2011 | CfuCfcAfgAfgCfCfAfaAfaUfcAfaGfaUf | A2011 | aUfcUfuGfaUfuUfuggCfuCfuGfgAfgsAfsu | 0.122 | 0.882 | 0.938 |
| D2012 | S2012 | CfgAfuGfuAfaAfAfAfuUfuUfaGfcCfaAf | A2012 | uUfgGfcUfaAfaAfuuuUfuAfcAfuCfgsUfsc | 0.102 | 0.843 | 0.733 |
| D2013 | S2013 | GfuCfuUfaAfaGfAfCfuUfuGfuCfcAfuAf | A2013 | uAfuGfgAfcAfaAfgucUfuUfaAfgAfcsCfsa | 1.133 | 1.105 | 1.022 |
| D2014 | S2014 | CfaAfcAfuAfuUfUfGfaUfcAfgUfcUfuUf | A2014 | aAfaGfaCfuGfaUfcaaAfuAfuGfuUfgsAfsg | 0.077 | 0.413 | 0.450 |
| D2015 | S2015 | AfcUfgAfgAfaGfAfAfcUfaCfaUfaUfaAf | A2015 | uUfaUfaUfgUfaGfuucUfuCfuCfaGfusUfsc | 0.055 | 0.293 | 0.364 |
| D2016 | S2016 | CfcAfgAfgCfcAfAfAfaUfcAfaGfaUfuUf | A2016 | aAfaUfcUfuGfaUfuuuGfgCfuCfuGfgsAfsg | 0.080 | 0.650 | 0.499 |
| D2017 | S2017 | GfaUfgUfaAfaAfAfUfuUfuAfgCfcAfaUf | A2017 | aUfuGfgCfuAfaAfauuUfuUfaCfaUfcsGfsu | 0.076 | 0.605 | 0.579 |
| D2018 | S2018 | UfcUfuAfaAfgAfCfUfuUfgUfcCfaUfaAf | A2018 | uUfaUfgGfaCfaAfaguCfuUfuAfaGfasCfsc | 1.326 | 1.098 | 0.927 |
| D2019 | S2019 | AfaCfaUfaUfuUfGfAfuCfaGfuCfuUfuUf | A2019 | aAfaAfgAfcUfgAfucaAfaUfaUfgUfusGfsa | 0.047 | 0.560 | 0.477 |
| D2020 | S2020 | CfuGfaGfaAfgAfAfCfuAfcAfuAfuAfaAf | A2020 | uUfuAfuAfuGfuAfguuCfuUfcUfcAfgsUfsu | 0.066 | 0.690 | 0.681 |
| D2021 | S2021 | AfaUfuAfaGfcUfCfCfuUfcUfuUfuUfaUf | A2021 | aUfaAfaAfaGfaAfggaGfcUfuAfaUfusGfsu | 0.041 | 0.611 | 0.251 |
| D2022 | S2022 | AfaAfuCfaAfgAfUfUfuGfcUfaUfgUfuAf | A2022 | uAfaCfaUfaGfcAfaauCfuUfgAfuUfusUfsg | 0.053 | 0.555 | 0.516 |
| D2023 | S2023 | UfuCfaGfuUfgGfGfAfcAfuGfgUfcUfuAf | A2023 | uAfaGfaCfcAfuGfuccCfaAfcUfgAfasGfsg | 0.779 | 1.045 | 0.963 |
| D2024 | S2024 | GfgGfcCfaAfaUfUfAfaUfgAfcAfuAfuUf | A2024 | aAfuAfuGfuCfaUfuaaUfuUfgGfcCfcsUfsu | 1.487 | 0.949 | 0.883 |
| D2025 | S2025 | AfcAfuAfuUfuGfAfUfcAfgUfcUfuUfuUf | A2025 | aAfaAfaGfaCfuGfaucAfaAfuAfuGfusUfsg | 0.043 | 0.432 | 0.477 |
| D2026 | S2026 | AfgAfaCfuAfcAfUfAfuAfaAfcUfaCfaAf | A2026 | uUfgUfaGfuUfuAfuauGfuAfgUfuCfusUfsc | 0.324 | 1.042 | 0.905 |
| D2027 | S2027 | AfuUfaAfgCfuCfCfUfuCfuUfuUfuAfuUf | A2027 | aAfuAfaAfaAfgAfaggAfgCfuUfaAfusUfsg | 0.042 | 0.283 | 0.224 |
| D2028 | S2028 | AfgAfuUfuGfcUfAfUfgUfuAfgAfcGfaUf | A2028 | aUfcGfuCfuAfaCfauaGfcAfaAfuCfusUfsg | 0.349 | 0.936 | 0.896 |
| D2029 | S2029 | UfcAfgUfuGfgGfAfCfaUfgGfuCfuUfaAf | A2029 | uUfaAfgAfcCfaUfgucCfcAfaCfuGfasAfsg | 0.914 | 0.907 | 0.944 |
| D2030 | S2030 | GfgCfcAfaAfuUfAfAfuGfaCfaUfaUfuUf | A2030 | aAfaUfaUfgUfcAfuuaAfuUfuGfgCfcsCfsu | 0.047 | 0.353 | 0.326 |
| D2031 | S2031 | CfaUfaUfuUfgAfUfCfaGfuCfuUfuUfuAf | A2031 | uAfaAfaAfgAfcUfgauCfaAfaUfaUfgsUfsu | 0.110 | 0.867 | 0.842 |
| D2032 | S2032 | UfaCfaUfaUfaAfAfCfuAfcAfaGfuCfaAf | A2032 | uUfgAfcUfuGfuAfguuUfaUfaUfgUfasGfsu | 0.200 | 0.699 | 0.656 |
| D2033 | S2033 | UfuUfuAfuUfgUfUfCfcUfcUfaGfuUfaUf | A2033 | aUfaAfcUfaGfaGfgaaCfaAfuAfaAfasAfsg | 0.050 | 0.218 | 0.192 |
| D2034 | S2034 | UfuGfcUfaUfgUfUfAfgAfcGfaUfgUfaAf | A2034 | uUfaCfaUfcGfuCfuaaCfaUfaGfcAfasAfsu | 0.096 | 0.792 | 0.640 |
| D2035 | S2035 | CfaGfuUfgGfgAfCfAfuGfgUfcUfuAfaAf | A2035 | uUfuAfaGfaCfcAfuguCfcCfaAfcUfgsAfsa | 0.127 | 0.936 | 0.890 |
| D2036 | S2036 | AfaAfuUfaAfuGfAfCfaUfaUfuUfcAfaAf | A2036 | uUfuGfaAfaUfaUfgucAfuUfaAfuUfusGfsg | 0.061 | 0.683 | 0.668 |
| D2037 | S2037 | GfaUfcAfgUfcUfUfUfuUfaUfgAfuCfuAf | A2037 | uAfgAfuCfaUfaAfaaaGfaCfuGfaUfcsAfsa | 0.157 | 1.010 | 0.723 |
| D2038 | S2038 | AfcAfuAfuAfaAfCfUfaCfaAfgUfcAfaAf | A2038 | uUfuGfaCfuUfgUfaguUfuAfuAfuGfusAfsg | 0.047 | 0.532 | 0.525 |
| D2039 | S2039 | UfuUfaUfuGfuUfCfCfuCfuAfgUfuAfuUf | A2039 | aAfuAfaCfuAfgAfggaAfcAfaUfaAfasAfsa | 0.031 | 0.505 | 0.238 |
| D2040 | S2040 | UfgCfuAfuGfuUfAfGfaCfgAfuGfuAfaAf | A2040 | uUfuAfcAfuCfgUfcuaAfcAfuAfgCfasAfsa | 0.056 | 0.484 | 0.408 |
| D2041 | S2041 | GfgGfaCfaUfgGfUfCfuUfaAfaGfaCfuUf | A2041 | aAfgUfcUfuUfaAfgacCfaUfgUfcCfcsAfsa | 0.570 | 0.999 | 0.994 |
| D2042 | S2042 | UfgAfcAfuAfuUfUfCfaAfaAfaCfuCfaAf | A2042 | uUfgAfgUfuUfuUfgaaAfuAfuGfuCfasUfsu | 0.065 | 0.870 | 0.728 |
| D2043 | S2043 | AfuCfaGfuCfuUfUfUfuAfuGfaUfcUfaUf | A2043 | aUfaGfaUfcAfuAfaaaAfgAfcUfgAfusCfsa | 0.048 | 0.362 | 0.282 |
| D2044 | S2044 | CfaUfaUfaAfaCfUfAfcAfaGfuCfaAfaAf | A2044 | uUfuUfgAfcUfuGfuagUfuUfaUfaUfgsUfsa | 0.314 | 0.904 | 0.937 |
| D2045 | S2045 | CfuUfgAfaCfuCfAfAfcUfcAfaAfaCfuUf | A2045 | aAfgUfuUfuGfaGfuugAfgUfuCfaAfgsUfsg | 0.060 | 0.295 | 0.251 |
| D2046 | S2046 | CfuAfcUfuCfaAfCfAfaAfaAfgUfgAfaAf | A2046 | uUfuCfaCfuUfuUfuguUfgAfaGfuAfgsAfsa | 0.052 | 0.570 | 0.599 |
| D2047 | S2047 | AfaGfaGfcAfaCfUfAfaCfuAfaCfuUfaAf | A2047 | uUfaAfgUfuAfgUfuagUfuGfcUfcUfusCfsu | 0.028 | 0.369 | 0.381 |
| D2048 | S2048 | AfaAfcAfaGfaUfAfAfuAfgCfaUfcAfaAf | A2048 | uUfuGfaUfgCfuAfuuaUfcUfuGfuUfusUfsu | 0.039 | 0.227 | 0.204 |
| D2049 | S2049 | GfcAfuAfgUfcAfAfAfuAfaAfaGfaAfaUf | A2049 | aUfuUfcUfuUfuAfuuuGfaCfuAfuGfcsUfsg | 0.032 | 0.437 | 0.422 |
| D2050 | S2050 | AfuAfuAfaAfcUfAfCfaAfgUfcAfaAfaAf | A2050 | uUfuUfuGfaCfuUfguaGfuUfuAfuAfusGfsu | 0.297 | 0.946 | 0.850 |
| D2051 | S2051 | GfaAfcUfcAfaCfUfCfaAfaAfcUfuGfaAf | A2051 | uUfcAfaGfuUfuUfgagUfuGfaGfuUfcsAfsa | 0.179 | 0.929 | 0.884 |
| D2052 | S2052 | UfaCfuUfcAfaCfAfAfaAfaGfuGfaAfaUf | A2052 | aUfuUfcAfcUfuUfuugUfuGfaAfgUfasGfsa | 0.091 | 0.536 | 0.524 |
| D2053 | S2053 | AfgAfgCfaAfcUfAfAfcUfaAfcUfuAfaUf | A2053 | aUfuAfaGfuUfaGfuuaGfuUfgCfuCfusUfsc | 0.086 | 0.611 | 0.621 |
| D2054 | S2054 | GfaUfaAfuAfgCfAfUfcAfaAfgAfcCfuUf | A2054 | aAfgGfuCfuUfuGfaugCfuAfuUfaUfcsUfsu | 0.058 | 0.676 | 0.591 |
| D2055 | S2055 | CfaUfaGfuCfaAfAfUfaAfaAfgAfaAfuAf | A2055 | uAfuUfuCfuUfuUfauuUfgAfcUfaUfgsCfsu | 0.048 | 0.630 | 0.674 |
| D2056 | S2056 | UfaUfaAfaCfuAfCfAfaGfuCfaAfaAfaUf | A2056 | aUfuUfuUfgAfcUfuguAfgUfuUfaUfasUfsg | 0.072 | 0.534 | 0.459 |
| D2057 | S2057 | AfaCfuCfaAfcUfCfAfaAfaCfuUfgAfaAf | A2057 | uUfuCfaAfgUfuUfugaGfuUfgAfgUfusCfsa | 0.161 | 0.864 | 0.775 |
| D2058 | S2058 | AfcUfuCfaAfcAfAfAfaAfgUfgAfaAfuAf | A2058 | uAfuUfuCfaCfuUfuuuGfuUfgAfaGfusAfsg | 0.198 | 0.969 | 0.865 |
| D2059 | S2059 | GfaGfcAfaCfuAfAfCfuAfaCfuUfaAfuUf | A2059 | aAfuUfaAfgUfuAfguuAfgUfuGfcUfcsUfsu | 0.031 | 0.253 | 0.210 |
| D2060 | S2060 | AfaCfcAfaCfaGfCfAfuAfgUfcAfaAfuAf | A2060 | uAfuUfuGfaCfuAfugcUfgUfuGfgUfusUfsa | 0.035 | 0.561 | 0.569 |
| D2061 | S2061 | AfgUfcAfaAfuAfAfAfaGfaAfaUfaGfaAf | A2061 | uUfcUfaUfuUfcUfuuuAfuUfuGfaCfusAfsu | 0.057 | 0.668 | 0.386 |
| D2062 | S2062 | AfgUfcAfaAfaAfUfGfaAfgAfgGfuAfaAf | A2062 | uUfuAfcCfuCfuUfcauUfuUfuGfaCfusUfsg | 0.720 | 1.017 | 0.924 |
| D2063 | S2063 | CfuUfgAfaAfgCfCfUfcCfuAfgAfaGfaAf | A2063 | uUfcUfuCfuAfgGfaggCfuUfuCfaAfgsUfsu | 0.324 | 1.020 | 0.963 |
| D2064 | S2064 | CfuUfcAfaCfaAfAfAfaGfuGfaAfaUfaUf | A2064 | aUfaUfuUfcAfcUfuuuUfgUfuGfaAfgsUfsa | 0.048 | 0.549 | 0.531 |
| D2065 | S2065 | CfaAfcUfaAfcUfAfAfcUfuAfaUfuCfaAf | A2065 | uUfgAfaUfuAfaGfuuaGfuUfaGfuUfgsCfsu | 0.046 | 0.739 | 0.649 |
| D2066 | S2066 | AfcCfaAfcAfgCfAfUfaGfuCfaAfaUfaAf | A2066 | uUfaUfuUfgAfcUfaugCfuGfuUfgGfusUfsu | 0.076 | 0.840 | 0.777 |
| D2067 | S2067 | GfaAfcCfcAfcAfGfAfaAfuUfuCfuCfuAf | A2067 | uAfgAfgAfaAfuUfucuGfuGfgGfuUfcsUfsu | 0.103 | 0.916 | 0.808 |
| D2068 | S2068 | GfaAfuAfuGfuCfAfCfuUfgAfaCfuCfaAf | A2068 | uUfgAfgUfuCfaAfgugAfcAfuAfuUfcsUfsu | 0.046 | 0.532 | 0.520 |
| D2069 | S2069 | UfgAfaAfgCfcUfCfCfuAfgAfaGfaAfaAf | A2069 | uUfuUfcUfuCfuAfggaGfgCfuUfuCfasAfsg | 0.067 | 0.894 | 0.822 |
| D2070 | S2070 | UfuCfaAfcAfaAfAfAfgUfgAfaAfuAfuUf | A2070 | aAfuAfuUfuCfaCfuuuUfuGfuUfgAfasGfsu | 0.052 | 0.557 | 0.395 |
| D2071 | S2071 | AfaCfuAfaCfuAfAfCfuUfaAfuUfcAfaAf | A2071 | uUfuGfaAfuUfaAfguuAfgUfuAfgUfusGfsc | 0.025 | 0.220 | 0.232 |
| D2072 | S2072 | CfcAfaCfaGfcAfUfAfgUfcAfaAfuAfaAf | A2072 | uUfuAfuUfuGfaCfuauGfcUfgUfuGfgsUfsu | 0.293 | 0.923 | 0.899 |
| D2073 | S2073 | AfaCfcCfaCfaGfAfAfaUfuUfcUfcUfaUf | A2073 | aUfaGfaGfaAfaUfuucUfgUfgGfgUfusCfsu | 0.021 | 0.375 | 0.356 |
| D2074 | S2074 | UfgUfcAfcUfuGfAfAfcUfcAfaCfuCfaAf | A2074 | uUfgAfgUfuGfaGfuucAfaGfuGfaCfasUfsa | 0.052 | 0.402 | 0.513 |
| D2075 | S2075 | GfaAfaGfcCfuCfCfUfaGfaAfgAfaAfaAf | A2075 | uUfuUfuCfuUfcUfaggAfgGfcUfuUfcsAfsa | 0.171 | 0.904 | 0.893 |
| D2076 | S2076 | AfaUfaUfuUfaGfAfAfgAfgCfaAfcUfaAf | A2076 | uUfaGfuUfgCfuCfuucUfaAfaUfaUfusUfsc | 0.142 | 0.614 | 0.688 |
| D2077 | S2077 | AfcUfaAfcUfaAfCfUfuAfaUfuCfaAfaAf | A2077 | uUfuUfgAfaUfuAfaguUfaGfuUfaGfusUfsg | 0.020 | 0.312 | 0.316 |
| D2078 | S2078 | CfaAfcAfgCfaUfAfGfuCfaAfaUfaAfaAf | A2078 | uUfuUfaUfuUfgAfcuaUfgCfuGfuUfgsGfsu | 0.026 | 0.313 | 0.393 |
| D2079 | S2079 | CfcAfcAfgAfaAfUfUfuCfuCfuAfuCfuUf | A2079 | aAfgAfuAfgAfgAfaauUfuCfuGfuGfgsGfsu | 0.012 | 0.596 | 0.345 |
| D2080 | S2080 | GfuCfaCfuUfgAfAfCfuCfaAfcUfcAfaAf | A2080 | uUfuGfaGfuUfgAfguuCfaAfgUfgAfcsAfsu | 0.054 | 0.503 | 0.456 |
| D2081 | S2081 | CfuCfcUfaGfaAfGfAfaAfaAfaUfuCfuAf | A2081 | uAfgAfaUfuUfuUfucuUfcUfaGfgAfgsGfsc | 0.050 | 0.596 | 0.531 |
| D2082 | S2082 | AfuUfuAfgAfaGfAfGfcAfaCfuAfaCfuAf | A2082 | uAfgUfuAfgUfuGfcucUfuCfuAfaAfusAfsu | 0.064 | 0.806 | 0.928 |
| D2083 | S2083 | CfuAfaCfuAfaCfUfUfaAfuUfcAfaAfaUf | A2083 | aUfuUfuGfaAfuUfaagUfuAfgUfuAfgsUfsu | 0.056 | 0.844 | 0.761 |
| D2084 | S2084 | CfaGfcAfuAfgUfCfAfaAfuAfaAfaGfaAf | A2084 | uUfcUfuUfuAfuUfugaCfuAfuGfcUfgsUfsu | 0.046 | 0.859 | 0.756 |
| D2085 | S2085 | GfaAfaUfaAfgAfAfAfuGfuAfaAfaCfaUf | A2085 | aUfgUfuUfuAfcAfuuuCfuUfaUfuUfcsAfsu | 0.039 | 0.615 | 0.612 |
| D2086 | S2086 | UfcAfcUfuGfaAfCfUfcAfaCfuCfaAfaAf | A2086 | uUfuUfgAfgUfuGfaguUfcAfaGfuGfasCfsa | 0.057 | 0.724 | 0.663 |
| D2087 | S2087 | UfcUfaCfuUfcAfAfCfaAfaAfaGfuGfaAf | A2087 | uUfcAfcUfuUfuUfguuGfaAfgUfaGfasAfsu | 0.732 | 1.028 | 0.915 |
| D2088 | S2088 | UfuUfaGfaAfgAfGfCfaAfcUfaAfcUfaAf | A2088 | uUfaGfuUfaGfuUfgcuCfuUfcUfaAfasUfsa | 0.061 | 0.795 | 0.785 |
| D2089 | S2089 | AfaAfaCfaAfgAfUfAfaUfaGfcAfuCfaAf | A2089 | uUfgAfuGfcUfaUfuauCfuUfgUfuUfusUfsc | 0.330 | 1.017 | 0.865 |
| D2090 | S2090 | AfgCfaUfaGfuCfAfAfaUfaAfaAfgAfaAf | A2090 | uUfuCfuUfuUfaUfuugAfcUfaUfgCfusGfsu | 0.038 | 0.606 | 0.589 |
| D2091 | S2091 | AfgAfcCfcAfgCfAfAfcUfcUfcAfaGfuUf | A2091 | aAfcUfuGfaGfaGfuugCfuGfgGfuCfusGfsa | 0.301 | 0.850 | 0.753 |
| D2092 | S2092 | AfgUfcCfaUfgGfAfCfaUfuAfaUfuCfaAf | A2092 | uUfgAfaUfuAfaUfgucCfaUfgGfaCfusAfsc | 0.407 | 0.791 | 0.726 |
| D2093 | S2093 | GfaUfgGfaUfcAfCfAfaAfaCfuUfcAfaUf | A2093 | aUfuGfaAfgUfuUfuguGfaUfcCfaUfcsUfsa | 0.120 | 0.658 | 0.654 |
| D2094 | S2094 | CfuAfgAfgAfaGfAfUfaUfaCfuCfcAfuAf | A2094 | uAfuGfgAfgUfaUfaucUfuCfuCfuAfgsGfsc | 0.071 | 0.610 | 0.645 |
| D2095 | S2095 | AfaAfgAfcAfaCfAfAfaCfaUfuAfuAfuUf | A2095 | aAfuAfuAfaUfgUfuugUfuGfuCfuUfusCfsc | 0.029 | 0.306 | 0.461 |
| D2096 | S2096 | CfaUfuAfuAfuUfGfAfaUfaUfuCfuUfuUf | A2096 | aAfaAfgAfaUfaUfucaAfuAfuAfaUfgsUfsu | 0.031 | 0.510 | 0.595 |
| D2097 | S2097 | GfaCfcCfaGfcAfAfCfuCfuCfaAfgUfuUf | A2097 | aAfaCfuUfgAfgAfguuGfcUfgGfgUfcsUfsg | 0.075 | 0.697 | 0.845 |
| D2098 | S2098 | GfgAfuCfaCfaAfAfAfcUfuCfaAfuGfaAf | A2098 | uUfcAfuUfgAfaGfuuuUfgUfgAfuCfcsAfsu | 0.130 | 0.831 | 0.951 |
| D2099 | S2099 | GfaAfgAfuAfuAfCfUfcCfaUfaGfuGfaAf | A2099 | uUfcAfcUfaUfgGfaguAfuAfuCfuUfcsUfsc | 0.058 | 0.828 | 0.938 |
| D2100 | S2100 | GfaCfaAfcAfaAfCfAfuUfaUfaUfuGfaAf | A2100 | uUfcAfaUfaUfaAfuguUfuGfuUfgUfcsUfsu | 0.026 | 0.564 | 0.856 |
| D2101 | S2101 | GfgGfaAfaUfcAfCfGfaAfaCfcAfaCfuAf | A2101 | uAfgUfuGfgUfuUfcguGfaUfuUfcCfcsAfsa | 0.314 | 0.948 | 1.033 |
| D2102 | S2102 | AfcCfcAfgCfaAfCfUfcUfcAfaGfuUfuUf | A2102 | aAfaAfcUfuGfaGfaguUfgCfuGfgGfusCfsu | 0.033 | 0.448 | 0.675 |
| D2103 | S2103 | GfgAfcAfuUfaAfUfUfcAfaCfaUfcGfaAf | A2103 | uUfcGfaUfgUfuGfaauUfaAfuGfuCfcsAfsu | 0.156 | 0.897 | 0.912 |
| D2104 | S2104 | GfaUfcAfcAfaAfAfCfuUfcAfaUfgAfaAf | A2104 | uUfuCfaUfuGfaAfguuUfuGfuGfaUfcsCfsa | 0.056 | 0.619 | 0.769 |
| D2105 | S2105 | AfcUfcCfaUfaGfUfGfaAfgCfaAfuCfuAf | A2105 | uAfgAfuUfgCfuUfcacUfaUfgGfaGfusAfsu | 0.100 | 0.823 | 0.925 |
| D2106 | S2106 | AfcAfaCfaAfaCfAfUfuAfuAfuUfgAfaUf | A2106 | aUfuCfaAfuAfuAfaugUfuUfgUfuGfusCfsu | 0.035 | 0.565 | 0.843 |
| D2107 | S2107 | GfgAfaAfuCfaCfGfAfaAfcCfaAfcUfaUf | A2107 | aUfaGfuUfgGfuUfucgUfgAfuUfuCfcsCfsa | 0.076 | 0.701 | 0.890 |
| D2108 | S2108 | CfcCfaGfcAfaCfUfCfuCfaAfgUfuUfuUf | A2108 | aAfaAfaCfuUfgAfgagUfuGfcUfgGfgsUfsc | 0.057 | 0.626 | 0.884 |
| D2109 | S2109 | GfaCfaUfuAfaUfUfCfaAfcAfuCfgAfaUf | A2109 | aUfuCfgAfuGfuUfgaaUfuAfaUfgUfcsCfsa | 0.160 | 0.873 | 1.012 |
| D2110 | S2110 | AfaCfgUfgGfgAfGfAfaCfuAfcAfaAfuAf | A2110 | uAfuUfuGfuAfgUfucuCfcCfaCfgUfusUfsc | 0.101 | 0.881 | 0.981 |
| D2111 | S2111 | CfuCfcAfuAfgUfGfAfaGfcAfaUfcUfaAf | A2111 | uUfaGfaUfuGfcUfucaCfuAfuGfgAfgsUfsa | 0.026 | 0.435 | 0.691 |
| D2112 | S2112 | CfaAfcAfaAfcAfUfUfaUfaUfuGfaAfuAf | A2112 | uAfuUfcAfaUfaUfaauGfuUfuGfuUfgsUfsc | 0.154 | 0.882 | 1.091 |
| D2113 | S2113 | GfaAfaUfcAfcGfAfAfaCfcAfaCfuAfuAf | A2113 | uAfuAfgUfuGfgUfuucGfuGfaUfuUfcsCfsc | 0.045 | 0.764 | 1.004 |
| D2114 | S2114 | CfuCfuCfaAfgUfUfUfuUfcAfuGfuCfuAf | A2114 | uAfgAfcAfuGfaAfaaaCfuUfgAfgAfgsUfsu | 0.105 | 0.925 | 0.988 |
| D2115 | S2115 | AfcAfuUfaAfuUfCfAfaCfaUfcGfaAfuAf | A2115 | uAfuUfcGfaUfgUfugaAfuUfaAfuGfusCfsc | 0.114 | 0.919 | 0.905 |
| D2116 | S2116 | GfgGfaGfaAfcUfAfCfaAfaUfaUfgGfuUf | A2116 | aAfcCfaUfaUfuUfguaGfuUfcUfcCfcsAfsc | 0.234 | 1.023 | 0.951 |
| D2117 | S2117 | UfcCfaUfaGfuGfAfAfgCfaAfuCfuAfaUf | A2117 | aUfuAfgAfuUfgCfuucAfcUfaUfgGfasGfsu | 0.033 | 0.566 | 0.778 |
| D2118 | S2118 | AfaCfaAfaCfaUfUfAfuAfuUfgAfaUfaUf | A2118 | aUfaUfuCfaAfuAfuaaUfgUfuUfgUfusGfsu | 0.031 | 0.535 | 0.785 |
| D2119 | S2119 | UfgGfcAfaUfgUfCfCfcCfaAfuGfcAfaUf | A2119 | aUfuGfcAfuUfgGfggaCfaUfuGfcCfasGfsu | 0.065 | 0.815 | 0.967 |
| D2120 | S2120 | UfcAfgGfuAfgUfCfCfaUfgGfaCfaUfuAf | A2120 | uAfaUfgUfcCfaUfggaCfuAfcCfuGfasUfsa | 0.223 | 0.825 | 0.924 |
| D2121 | S2121 | UfuAfaUfuCfaAfCfAfuCfgAfaUfaGfaUf | A2121 | aUfcUfaUfuCfgAfuguUfgAfaUfuAfasUfsg | 0.083 | 0.781 | 0.915 |
| D2122 | S2122 | GfgAfgAfaCfuAfCfAfaAfuAfuGfgUfuUf | A2122 | aAfaCfcAfuAfuUfuguAfgUfuCfuCfcsCfsa | 0.079 | 0.680 | 0.767 |
| D2123 | S2123 | CfcAfuAfgUfgAfAfGfcAfaUfcUfaAfuUf | A2123 | aAfuUfaGfaUfuGfcuuCfaCfuAfuGfgsAfsg | 0.026 | 0.537 | 0.793 |
| D2124 | S2124 | AfcAfaAfcAfuUfAfUfaUfuGfaAfuAfuUf | A2124 | aAfuAfuUfcAfaUfauaAfuGfuUfuGfusUfsg | 0.044 | 0.680 | 0.828 |
| D2125 | S2125 | AfaUfgCfaAfuCfCfCfgGfaAfaAfcAfaAf | A2125 | uUfuGfuUfuUfcCfgggAfuUfgCfaUfusGfsg | 0.349 | 0.971 | 1.005 |
| D2126 | S2126 | CfaGfgUfaGfuCfCfAfuGfgAfcAfuUfaAf | A2126 | uUfaAfuGfuCfcAfuggAfcUfaCfcUfgsAfsu | 0.070 | 0.548 | 0.546 |
| D2127 | S2127 | UfuCfaAfcAfuCfGfAfaUfaGfaUfgGfaUf | A2127 | aUfcCfaUfcUfaUfucgAfuGfuUfgAfasUfsu | 0.225 | 0.958 | 0.967 |
| D2128 | S2128 | GfuUfgGfgCfcUfAfGfaGfaAfgAfuAfuAf | A2128 | uAfuAfuCfuUfcUfcuaGfgCfcCfaAfcsCfsa | 0.765 | 0.969 | 0.922 |
| D2129 | S2129 | CfaUfaGfuGfaAfGfCfaAfuCfuAfaUfuAf | A2129 | uAfaUfuAfgAfuUfgcuUfcAfcUfaUfgsGfsa | 0.028 | 0.583 | 0.777 |
| D2130 | S2130 | AfaCfaUfuAfuAfUfUfgAfaUfaUfuCfuUf | A2130 | aAfgAfaUfaUfuCfaauAfuAfaUfgUfusUfsg | 0.249 | 0.916 | 0.981 |
| D2131 | S2131 | GfcAfaUfcCfcGfGfAfaAfaCfaAfaGfaUf | A2131 | aUfcUfuUfgUfuUfuccGfgGfaUfuGfcsAfsu | 0.435 | 1.002 | 1.019 |
| D2132 | S2132 | GfgUfaGfuCfcAfUfGfgAfcAfuUfaAfuUf | A2132 | aAfuUfaAfuGfuCfcauGfgAfcUfaCfcsUfsg | 0.427 | 0.988 | 0.918 |
| D2133 | S2133 | AfuCfgAfaUfaGfAfUfgGfaUfcAfcAfaAf | A2133 | uUfuGfuGfaUfcCfaucUfaUfuCfgAfusGfsu | 0.170 | 0.706 | 0.890 |
| D2134 | S2134 | CfcUfaGfaGfaAfGfAfuAfuAfcUfcCfaUf | A2134 | aUfgGfaGfuAfuAfucuUfcUfcUfaGfgsCfsc | 0.033 | 0.543 | 0.733 |
| D2135 | S2135 | GfuUfgGfaAfgAfCfUfgGfaAfaGfaCfaAf | A2135 | uUfgUfcUfuUfcCfaguCfuUfcCfaAfcsUfsc | 0.137 | 0.975 | 0.944 |
| D2136 | S2136 | AfcAfuUfaUfaUfUfGfaAfuAfuUfcUfuUf | A2136 | aAfaGfaAfuAfuUfcaaUfaUfaAfuGfusUfsu | 0.114 | 0.882 | 0.940 |
| D2137 | S2137 | CfaAfuCfcCfgGfAfAfaAfcAfaAfgAfuUf | A2137 | aAfuCfuUfuGfuUfuucCfgGfgAfuUfgsCfsa | 0.155 | 0.755 | 0.686 |
| D2138 | S2138 | CfuAfcUfuGfgGfAfUfcAfcAfaAfgCfaAf | A2138 | uUfgCfuUfuGfuGfaucCfcAfaGfuAfgsAfsa | 0.196 | 0.825 | 0.658 |
| D2139 | S2139 | AfcAfaCfcUfaAfAfUfgGfuAfaAfuAfuAf | A2139 | uAfuAfuUfuAfcCfauuUfaGfgUfuGfusUfsu | 0.133 | 0.704 | 0.671 |
| D2140 | S2140 | AfuCfcAfuCfcAfAfCfaGfaUfuCfaGfaAf | A2140 | uUfcUfgAfaUfcUfguuGfgAfuGfgAfusCfsa | 0.184 | 0.775 | 0.658 |
| D2141 | S2141 | AfaCfuGfaGfgCfAfAfaUfuUfaAfaAfgAf | A2141 | uCfuUfuUfaAfaUfuugCfcUfcAfgUfusCfsa | 0.076 | 0.682 | 0.777 |
| D2142 | S2142 | AfgAfgUfaUfgUfGfUfaAfaAfaUfcUfgUf | A2142 | aCfaGfaUfuUfuUfacaCfaUfaCfuCfusGfsu | 0.448 | 0.659 | 0.761 |
| D2143 | S2143 | AfaUfcCfcGfgAfAfAfaCfaAfaGfaUfuUf | A2143 | aAfaUfcUfuUfgUfuuuCfcGfgGfaUfusGfsc | 0.097 | 0.844 | 0.924 |
| D2144 | S2144 | UfaCfuUfgGfgAfUfCfaCfaAfaGfcAfaAf | A2144 | uUfuGfcUfuUfgUfgauCfcCfaAfgUfasGfsa | 0.084 | 0.875 | 0.947 |
| D2145 | S2145 | CfaAfcCfuAfaAfUfGfgUfaAfaUfaUfaAf | A2145 | uUfaUfaUfuUfaCfcauUfuAfgGfuUfgsUfsu | 0.104 | 0.811 | 0.814 |
| D2146 | S2146 | UfuGfaAfuGfaAfCfUfgAfgGfcAfaAfuUf | A2146 | aAfuUfuGfcCfuCfaguUfcAfuUfcAfasAfsg | 0.046 | 0.549 | 0.680 |
| D2147 | S2147 | AfcUfgAfgGfcAfAfAfuUfuAfaAfaGfgAf | A2147 | uCfcUfuUfuAfaAfuuuGfcCfuCfaGfusUfsc | 0.079 | 0.890 | 1.005 |
| D2148 | S2148 | GfaGfuAfuGfuGfUfAfaAfaAfuCfuGfuAf | A2148 | uAfcAfgAfuUfuUfuacAfcAfuAfcUfcsUfsg | 0.497 | 0.676 | 0.783 |
| D2149 | S2149 | AfcUfuGfgGfaUfCfAfcAfaAfgCfaAfaAf | A2149 | uUfuUfgCfuUfuGfugaUfcCfcAfaGfusAfsg | 0.049 | 0.699 | 0.907 |
| D2150 | S2150 | AfuGfgUfaAfaUfAfUfaAfcAfaAfcCfaAf | A2150 | uUfgGfuUfuGfuUfauaUfuUfaCfcAfusUfsu | 0.093 | 0.928 | 0.941 |
| D2151 | S2151 | UfgAfaUfgAfaCfUfGfaGfgCfaAfaUfuUf | A2151 | aAfaUfuUfgCfcUfcagUfuCfaUfuCfasAfsa | 0.201 | 0.736 | 0.885 |
| D2152 | S2152 | CfuGfaGfgCfaAfAfUfuUfaAfaAfgGfcAf | A2152 | uGfcCfuUfuUfaAfauuUfgCfcUfcAfgsUfsu | 0.071 | 0.938 | 0.872 |
| D2153 | S2153 | AfgUfaUfgUfgUfAfAfaAfaUfcUfgUfaAf | A2153 | uUfaCfaGfaUfuUfuuaCfaCfaUfaCfusCfsu | 0.504 | 0.816 | 0.689 |
| D2154 | S2154 | GfaAfaAfcAfaAfGfAfuUfuGfgUfgUfuUf | A2154 | aAfaCfaCfcAfaAfucuUfuGfuUfuUfcsCfsg | 0.061 | 0.723 | 0.922 |
| D2155 | S2155 | AfgUfgUfgGfaGfAfAfaAfcAfaCfcUfaAf | A2155 | uUfaGfgUfuGfuUfuucUfcCfaCfaCfusCfsa | 0.071 | 0.689 | 0.869 |
| D2156 | S2156 | GfuCfuCfaAfaAfUfGfgAfaGfgUfuAfuAf | A2156 | uAfuAfaCfcUfuCfcauUfuUfgAfgAfcsUfsu | 0.133 | 0.643 | 0.974 |
| D2157 | S2157 | GfaAfuGfaAfcUfGfAfgGfcAfaAfuUfuAf | A2157 | uAfaAfuUfuGfcCfucaGfuUfcAfuUfcsAfsa | 0.204 | 0.751 | 1.008 |
| D2158 | S2158 | UfgAfgGfcAfaAfUfUfuAfaAfaGfgCfaAf | A2158 | uUfgCfcUfuUfuAfaauUfuGfcCfuCfasGfsu | 0.089 | 0.820 | 0.937 |
| D2159 | S2159 | GfuAfuGfuGfuAfAfAfaAfuCfuGfuAfaUf | A2159 | aUfuAfcAfgAfuUfuuuAfcAfcAfuAfcsUfsc | 0.535 | 0.697 | 0.788 |
| D2160 | S2160 | AfaAfaCfaAfaGfAfUfuUfgGfuGfuUfuUf | A2160 | aAfaAfcAfcCfaAfaucUfuUfgUfuUfusCfsc | 0.297 | 0.954 | 1.004 |
| D2161 | S2161 | GfuGfuGfgAfgAfAfAfaCfaAfcCfuAfaAf | A2161 | uUfuAfgGfuUfgUfuuuCfuCfcAfcAfcsUfsc | 0.178 | 0.872 | 0.918 |
| D2162 | S2162 | AfuGfgAfaGfgUfUfAfuAfcUfcUfaUfaAf | A2162 | uUfaUfaGfaGfuAfuaaCfcUfuCfcAfusUfsu | 0.026 | 0.489 | 0.890 |
| D2163 | S2163 | AfaUfgAfaCfuGfAfGfgCfaAfaUfuUfaAf | A2163 | uUfaAfaUfuUfgCfcucAfgUfuCfaUfusCfsa | 0.111 | 0.789 | 0.859 |
| D2164 | S2164 | GfaGfgCfaAfaUfUfUfaAfaAfgGfcAfaUf | A2164 | aUfuGfcCfuUfuUfaaaUfuUfgCfcUfcsAfsg | 0.241 | 0.956 | 0.869 |
| D2165 | S2165 | UfaUfgUfgUfaAfAfAfaUfcUfgUfaAfuAf | A2165 | uAfuUfaCfaGfaUfuuuUfaCfaCfaUfasCfsu | 0.571 | 0.762 | 0.931 |
| D2166 | S2166 | AfcAfaAfgAfuUfUfGfgUfgUfuUfuCfuAf | A2166 | uAfgAfaAfaCfaCfcaaAfuCfuUfuGfusUfsu | 0.106 | 0.981 | 0.924 |
| D2167 | S2167 | UfgUfgGfaGfaAfAfAfcAfaCfcUfaAfaUf | A2167 | aUfuUfaGfgUfuGfuuuUfcUfcCfaCfasCfsu | 0.064 | 0.765 | 0.902 |
| D2168 | S2168 | UfgGfaAfgGfuUfAfUfaCfuCfuAfuAfaAf | A2168 | uUfuAfuAfgAfgUfauaAfcCfuUfcCfasUfsu | 0.029 | 0.675 | 0.859 |
| D2169 | S2169 | AfuGfaAfcUfgAfGfGfcAfaAfuUfuAfaAf | A2169 | uUfuAfaAfuUfuGfccuCfaGfuUfcAfusUfsc | 0.054 | 0.733 | 0.843 |
| D2170 | S2170 | AfgGfcAfaAfuUfUfAfaAfaGfgCfaAfuAf | A2170 | uAfuUfgCfcUfuUfuaaAfuUfuGfcCfusCfsa | 0.075 | 0.754 | 0.881 |
| D2171 | S2171 | AfaGfaUfuUfgGfUfGfuUfuUfcUfaCfuUf | A2171 | aAfgUfaGfaAfaAfcacCfaAfaUfcUfusUfsg | 0.303 | 1.065 | 0.977 |
| D2172 | S2172 | AfaAfcAfaCfcUfAfAfaUfgGfuAfaAfuAf | A2172 | uAfuUfuAfcCfaUfuuaGfgUfuGfuUfusUfsc | 0.101 | 0.855 | 0.880 |
| D2173 | S2173 | AfuAfcUfcUfaUfAfAfaAfuCfaAfcCfaAf | A2173 | uUfgGfuUfgAfuUfuuaUfaGfaGfuAfusAfsa | 0.107 | 0.961 | 0.960 |
| D2174 | S2174 | UfgAfaCfuGfaGfGfCfaAfaUfuUfaAfaAf | A2174 | uUfuUfaAfaUfuUfgccUfcAfgUfuCfasUfsu | 0.078 | 0.714 | 0.878 |
| D2175 | S2175 | GfgCfaAfaUfuUfAfAfaAfgGfcAfaUfaAf | A2175 | uUfaUfuGfcCfuUfuuaAfaUfuUfgCfcsUfsc | 0.054 | 0.767 | 0.918 |
| D2176 | S2176 | UfuUfuCfuAfcUfUfGfgGfaUfcAfcAfaAf | A2176 | uUfuGfuGfaUfcCfcaaGfuAfgAfaAfasCfsa | 0.915 | 1.030 | 0.916 |
| D2177 | S2177 | AfaCfaAfcCfuAfAfAfuGfgUfaAfaUfaUf | A2177 | aUfaUfuUfaCfcAfuuuAfgGfuUfgUfusUfsu | 0.042 | 0.260 | 0.448 |
| D2178 | S2178 | UfaCfuCfuAfuAfAfAfaUfcAfaCfcAfaAf | A2178 | uUfuGfgUfuGfaUfuuuAfuAfgAfgUfasUfsa | 0.063 | 0.897 | 0.869 |
| D2179 | S2179 | GfaAfcUfgAfgGfCfAfaAfuUfuAfaAfaAf | A2179 | uUfuUfuAfaAfuUfugcCfuCfaGfuUfcsAfsu | 0.178 | 0.858 | 0.869 |
| D2180 | S2180 | CfaGfaGfuAfuGfUfGfuAfaAfaAfuCfuUf | A2180 | aAfgAfuUfuUfuAfcacAfuAfcUfcUfgsUfsg | 0.436 | 0.677 | 0.813 |

### Example 4: In vitro silencing activity with various chemical modifications on ANGPTL3 siRNA

### Cell culture and transfections

Hep3B cells (ATCC, Manassas, VA) were grown to near confluence at 37°C in an atmosphere of 5% CO₂ in RPMI (ATCC) supplemented with 10% FBS, streptomycin, and glutamine (ATCC) before being released from the plate by trypsinization. Transfection was carried out by adding 14.8 µl of Opti-MEM plus 0.2µl of Lipofectamine RNAiMax per well (Invitrogen, Carlsbad CA. cat # 13778-150) to 5 µl of siRNA duplexes per well into a 96-well plate and incubated at room temperature for 15 minutes. 80 µl of complete growth media without antibiotic containing ~2×10⁴ Hep3B cells were then added to the siRNA mixture. Cells were incubated for either 24 or 120 hours prior to RNA purification. Single dose experiments were performed at 10nM and 0.1nM final duplex concentration and dose response experiments were done at 10, 1, 0.5, 0.1,0.05, 0.01, 0.005, 0.001, 0.0005, 0.0001, 0.00005 and 0.00001 nM final duplex concentration unless otherwise stated.

### cDNA synthesis using ABI High capacity cDNA reverse transcription kit (Applied Biosystems, Foster City, CA, Cat #4368813)

A master mix of 2µl 10X Buffer, 0.8 µl 25X dNTPs, 2µl Random primers, 1µl Reverse Transcriptase, 1 µl RNase inhibitor and 3.2µl of H₂O per reaction were added into 10µl total RNA. cDNA was generated using a Bio-Rad C-1000 or S-1000 thermal cycler (Hercules, CA) through the following steps: 25°C 10 min, 37°C 120 min, 85°C 5 sec, 4°C hold.

### Real time PCR

2µl of cDNA was added to a master mix containing 0.5µl GAPDH TaqMan Probe (Applied Biosystems Cat #4326317E), 0.5µl ANGPTL TaqMan probe (Applied Biosystems cat # Hs00205581_ml) and 5µl Lightcycler 480 probe master mix (Roche Cat #04887301001) per well in a 384 well 50 plates (Roche cat # 04887301001). Real time PCR was done in an ABI 7900HT Real Time PCR system (Applied Biosystems) using the ΔΔCt(RQ) assay. Each duplex was tested in two independent transfections, and each transfection was assayed in duplicate, unless otherwise noted in the summary tables.

To calculate relative fold change, real time data was analyzed using the ΔΔCt method and normalized to assays performed with cells transfected with 10nM AD-1955, or mock transfected cells. IC₅₀s were calculated using a 4 parameter fit model using XLFit and normalized to cells transfected with AD-1955 or naïve cells over the same dose range, or to its own lowest dose. AD-1955 sequence, used as a negative control, targets luciferase and has the following sequence: sense: cuuAcGcuGAGuAcuucGAdTsdT;
antisense: UCGAAGuACUcAGCGuAAGdTsdT.

## Claims

1. A double-stranded RNAi agent capable of inhibiting the expression of a target gene, comprising a sense strand and an antisense strand, each strand having 14 to 30 nucleotides, wherein the duplex is represented by formula (III):
sense:
5' nₚ-Nₐ-(XXX)ᵢ-N_{b}-YYY-N_{b}-(ZZZ)ⱼ-Nₐ-n_{q}3
,
antisense:
3' n_{g}'-Nₐ'-(X'X'X')ₖ-N_{b}'-Y'Y'Y'-N_{b}'-(Z'Z'Z')₁-Nₐ'-n_{q}' 5' (III)
wherein:
i, j, k, and l are each independently 0 or 1;
p and q are each independently 0-6;
each Nₐ and Nₐ' independently represents an oligonucleotide sequence comprising 0-25 nucleotides which are either modified or unmodified or combinations thereof, each sequence comprising at least two differently modified nucleotides, each N_{b} and N_{b}' independently represents an oligonucleotide sequence comprising 0-10 nucleotides which are either modified or unmodified or combinations thereof;
each nₚ, nₚ', n_{q} and n_{q}' independently represents an overhang nucleotide sequence comprising 0-6 nucleotides; and
XXX, YYY, ZZZ, X'X'X', Y'Y'Y', and Z'Z'Z' each independently represent one motif of three identical modifications on three consecutive nucleotides;
the Y'Y'Y' motif occurs at the 11, 12 and 13 positions of the antisense strand from the 5'-end;
the Y' is 2'-OMe;
said agent further comprises at least one phosphorothioate internucleotide linkage, wherein the antisense strand comprises two blocks of two phosphorothioate internucleotide linkages separated by 1, 2, 3, 4, 5, 6, 7, 8, 9, 10, 11, 12, 13, 14, 15, 16, 17 or 18 phosphate internucleotide linkages, and said antisense strand is paired with a sense strand comprising any combination of phosphorothioate, methylphosphonate and phosphate internucleotide linkages;
and
wherein the modification on Nb is different than the modification on Y and the modification on Nb' is different than the modification on Y'
provided that the double-stranded RNAi is not a double-stranded RNAi consisting of
Sense strand: GfsgsUfuAfaCfaCfCfAfuUfuAfcUfuCfaAfL96
Antisense strand: usUfsgAfaGfuAfaAfuggUfgUfuAfaCfcsasg,
Sense strand: GfsgsUfuAfaCfaCfCfAfuUf(Uhd)AfcUfuCfaAfL96
Antisense strand: usUfsgAfaGfuAfaAfuggUfgUfuAfaCfcsasg, and
Sense strand: GfqUfuAfaCfaCfCfAfuUfuAfcUfuCfaAfL96
Antisense strand: usUfsgAfaGfuAfaAfuggUfgUfuAfaCfcsasg, wherein
Gf is 2-'-fluoroguanosine-3'--phosphate,
g is 2"-O-methylquanosine-3"-phosphate,
Uf is 2-'-fluorouridine-3'-phosphate,
u is 2'-O-methyluridine-3'-phosphate,
Af is 2-'-fluoroadenosine-3'-phosphate,
a is 2'-O-methyladenosine-3'-phosphate,
Cf is 2'-fluorocytidine-3'-phosphate,
c is 2'-O-methylcytidine-3'-phosphate,
(Uhd) is 2'-O-hexadecyl-uridine-3-'-phosphate,
s is phosphorothioate linkage, and
L96 is N-[tris(GalNAc-alkyl)-amidodecanoyl)]-4-hydroxyprolinol (Hyp-(GalNAc-alkyl)₃).

2. The double-stranded RNAi agent of claim 1, wherein
(a) i is 1; j is 1; or both i and j are 1; or
(b) k is 1; l is 1; or both k and I are 1.

3. The double-stranded RNAi agent of claim 1 or 2, wherein the YYY motif occurs at or near the cleavage site of the sense strand.

4. The double-stranded RNAi agent of claim 1, wherein
(a) the duplex region is 17-30 nucleotide pairs in length, 17-19 nucleotide pairs in length, or 27-30 nucleotide pairs in length, and/or
(b) each strand has 17-30 nucleotides.

5. The double-stranded RNAi agent of claim 1, wherein the modifications on the nucleotides are selected from the group consisting of LNA, HNA, CeNA, 2'-methoxyethyl, 2'-O-alkyl, 2'-O-allyl, 2'-C- allyl, 2'-fluoro, 2'-deoxy, and combinations thereof, or from the group consisting of 2'-O-methyl nucleotide, 2'-deoxyfluoro nucleotide, 2'-O-N-methylacetamido (2'-O-NMA) nucleotide, a 2'-O-dimethylaminoethoxyethyl (2'-O-DMAEOE) nucleotide, 2'-O-aminopropyl (2'-O-AP) nucleotide, 2'-ara-F, and combinations thereof, wherein preferably the nucleotides are modified with either 2'-OCH₃ or 2'-F.

6. The double-stranded RNAi agent of any one of claims 1 to 5, further comprising at least one ligand, wherein preferably the ligand
(a) is one or more GalNAc derivatives attached through a bivalent or trivalent branched linker; and/or
(b) is attached to the 3' end of the sense strand.

7. The double-stranded RNAi agent of any one of claims 1 to 6, wherein
(a) the nucleotide at the 1 position of the 5'-end of the duplex in the antisense strand is selected from the group consisting of A, dA, dU, U, and dT; and/or
(b) the base pair at the 1 position of the 5'-end of the duplex is an AU base pair.

8. The double-stranded RNAi agent of any one of claims 1 to 7, wherein the Y nucleotides contain a 2'-fluoro modification.

9. A pharmaceutical composition comprising the double-stranded RNAi agent according to any one of the preceding claims alone or in combination with a pharmaceutically acceptable carrier or excipient.

10. A double-stranded RNAi agent according to any one of the preceding claims for use in a method of therapy, the method comprising inhibiting the expression of a target gene.

11. The agent for use of claim 10, wherein the double-stranded RNAi agent is to be administered through subcutaneous or intravenous administration.

12. An agent as defined in any one of claims 1 to 8 for use in a method of therapy, the method comprising delivering said agent to a specific target in a subject.

13. The agent for use of claim 12, wherein said agent is to be administered through intramuscular, intrabronchial, intrapleural, intraperitoneal, intraarterial, lymphatic, intravenous, subcutaneous, or cerebrospinal administration, or combinations thereof.

## Patentansprüche

1. Doppelsträngiges RNAi-Agens, das die Expression eines Zielgens inhibieren kann, umfassend einen Sinnstrang und eine Antisinnstrang, wobei jeder Strang 14 bis 30 Nukleotide hat, wobei der Doppelstrang durch die Formel (III) repräsentiert ist:
Sinn:
5' nₚ-Nₐ -(XXX)ᵢ-N_{b}-YYY-N_{b}-(ZZZ)ⱼ-Nₐ-n_{q}3'
Antisinn:
3' nₚ'-Nₐ'-(X'X'X')ₖ-N_{b}'-Y'Y'Y'-N_{b}'-(Z'Z'Z')ₗ-Nₐ'-n_{q}' 5' (III)
wobei:
i, j, k und l unabhängig voneinander 0 oder 1 sind;
p und q unabhängig voneinander 0-6 sind;
jedes Nₐ und Nₐ unabhängig voneinander eine Oligonukleotidsequenz repräsentiert, die 0-25 Nukleotide umfasst, die entweder modifiziert oder unmodifiziert oder eine Kombination davon sind, wobei jede Sequenz mindestens zwei unterschiedlich modifizierte Nukleotide umfasst, jedes N_{b} und N_{b}' unabhängig voneinander eine Oligonukleotidsequenz repräsentiert, die 0-10 Nukleotide umfasst, die entweder modifiziert oder unmodifiziert oder eine Kombination davon sind,
jedes nₚ, nₚ', n_{q}, und n_{q}' unabhängig voneinander eine Überhang-Nukleotidsequenz repräsentiert, die 0-6 Nukleotide umfasst; und
XXX, YYY, ZZZ, X'X'X', Y'Y'Y' und Z'Z'Z' jeweils unabhängig voneinander ein Motiv von drei identischen Modifikationen an drei aufeinanderfolgenden Nukleotiden repräsentieren;
das Y'Y'Y'-Motiv an den Positionen 11, 12 und 13 des Antisinnstrangs ausgehend vom 5'-Ende vorkommt;
Y' 2'-OMe ist;
das Agens weiterhin mindestens eine Phosphorothioat-Internukleotid-Verknüpfung umfasst, wobei der Antisinnstrang zwei Blöcke von zwei Phosphorothioat-Internukleotid-Verknüpfungen umfasst, die durch 1, 2, 3, 4, 5, 6, 7, 8, 9, 10, 11, 12, 13, 14, 15, 16, 17 oder 18 Phosphat-Internukleotid-Verknüpfungen getrennt sind, und der Antisinnstrang mit einem Sinnstrang gepaart ist, der eine beliebige Kombination von Phosphorothioat-,
Methylphosphonat- und Phosphat-Internukleotid-Verknüpfungen umfasst,
und
wobei die Modifikation an Nb unterschiedlich zu der Modifikation an Y ist und die Modifikation an Nb' unterschiedlich zu der Modifikation an Y' ist,
unter der Vorrausetzung, dass die doppelsträngige RNAi keine doppelsträngige RNAi ist, die besteht aus
Sinnstrang: GfsgsUfuAfaCfaCfCfAfuUfuAfcUfuCfaAfL96
Antisinnstrang: usUfsgAfaGfuAfaAfuggUfgUfuAfaCfcsasg,
Sinnstrang: GfsgsUfuAfaCfaCfCfAfuUf(Uhd)AfcUfuCfaAfL96
Antisinnstrang: usUfsgAfaGfuAfaAfuggUfgUfuAfaCfcsasg, und
Sinnstrang: GfgUfuAfaCfaCfCfAfuUfuAfcUfuCfaAfL96
Antisinnstrang: usUfsgAfaGfuAfaAfuggUfgUfuAfaCfcsasg, wobei
Gf 2 '-fluorocytidine-3'- phosphate,
g 2'-O-methylguanosine-3' -phosphate,
Uf 2 '-fluorouridine-3'-phosphate,
u 2'-O-methyluridine-3'-phosphate,
Af 2 '-fluoroadenosine-3'-phosphate,
a 2'-O-methyladenosine-3'-phosphate,
Cf 2'-fluorocytidine-3'-phosphate,
c 2'-O-methylcytosine-3'-phosphate,
(Uhd) 2'-O-hexadecyl-uridine-3 '-phosphate,
s phosphorothioate linkage, und
L96 N-[tris(GalNAc-alkyl)-amidodecanoyl)]-4-hydroxyprolinol Hyp-(GalNAc-alkyl)₃ ist.

2. Doppelsträngiges RNAi-Agens nach Anspruch 1, wobei
(a) i 1 ist, j 1 ist; oder sowohl i als auch j 1 sind; oder
(b) k 1 ist, l 1 ist; oder sowohl k als auch l 1 sind.

3. Doppelsträngiges RNAi-Agens nach Anspruch 1 oder 2, wobei das Motiv YYY an oder in der Nähe der Spaltstelle des Sinnstrangs vorkommt.

4. Doppelsträngiges RNAi-Agens nach Anspruch 1, wobei
(a) der Doppelstrang der Region 17-30 Nukleotidpaare lang, 17-19 Nukleotidpaare lang oder 27-30 Nukleotidpaare lang ist, und/oder
(b) jeder Strang 17-30 Nukleotide hat.

5. Doppelsträngiges RNAi-Agens nach Anspruch 1, wobei die Modifikationen an den Nukleotiden ausgewählt ist aus der Gruppe bestehend aus LNA, HNA, CeNA, 2'-Methoxyethyl, 2'-O-Alkyl, 2'-O-Allyl, 2'-C-Allyl, 2'-Fluor, 2'-Deoxy, und Kombinationen davon, oder aus der Gruppe bestehend aus 2'-O-Methyl-Nukleotid, 2'-Deoxyfluor-Nukleotid, 2'-O-N-Methylacetamido (2'-O-NMA)-Nukleotid, einem 2'-O-Dimethylaminoethoxyethyl (2'-O-DMAEOE)-Nukleotid, 2'-O-Aminopropyl (2'-O-AP)-Nukleotid, 2'-Ara-F, und Kombinationen davon, wobei die Nukleotide bevorzugt entweder mit 2'-OCH₃ oder 2'-F modifiziert sind.

6. Doppelsträngiges RNAi-Agens nach einem der Ansprüche 1 bis 5, weiterhin umfassend mindestens einen Liganden, wobei der Ligand bevorzugt
(i) ein oder mehrere GalNac-Derivate ist, das/die durch einen bivalenten der trivalenten verzweigte Linker verknüpft ist/sind; und/oder
(ii) am 3'-Ende des Sinnstrangs verknüpft ist.

7. Doppelsträngiges RNAi-Agens nach einem der Ansprüche 1 bis 6, wobei
(a) das Nukleotid an der 1 Position des 5'-Endes des Doppelstrangs im Antisinnstrang aus der Gruppe bestehend aus A, dA, dU, U und dT ausgewählt ist; und/oder
(b) das Basenpaar an der 1 Position des 5'-Endes des Duplexes ein AU Basenpaar ist.

8. Doppelsträngiges RNAi-Agens nach einem der Ansprüche 1 bis 7, wobei die Y-Nukleotide eine 2'-Fluor-Modifikation enthalten.

9. Pharmazeutische Zusammensetzung, umfassend das doppelsträngige RNAi-Agens nach einem der vorherigen Ansprüche, alleine oder in Kombination mit einem pharmazeutisch verträglichen Träger oder Exzipienten.

10. Doppelsträngiges RNAi-Agens nach einem der vorherigen Ansprüche zur Verwendung in einem therapeutischen Verfahren, wobei das Verfahren die Inhibition der Expression eines Zielgens umfasst.

11. Agens zur Verwendung nach Anspruch 10, wobei das doppelsträngige RNAi-Agens subkutan oder intravenös zu verabreichen ist.

12. Doppelsträngiges RNAi-Agens nach einem der Ansprüche 1 bis 8 zur Verwendung in einem therapeutischen Verfahren, wobei das Verfahren die Abgabe des Agens an ein spezifisches Ziel in einem Individuum umfasst.

13. Agens zur Verwendung nach Anspruch 12, wobei das Agens intramuskulär, intrabronchial, intrapleural, intraperitoneal, intraarteriell, lymphatisch, intravenös, subkutan, oder cerebrospinal zu verabreichen ist oder durch Kombinationen davon.

## Revendications

1. Agent à ARNi double brin capable d'inhiber l'expression d'un gène cible, comprenant un brin sens et un brin antisens, chaque brin ayant 14 à 30 nucléotides, dans lequel le duplex est représenté par la formule (III) :
sens :
5'nₚ-Nₐ-(XXX)ᵢ-N_{b}-YYY-N_{b}-(ZZZⱼ-N,-n_{q}3'
antisens :
3' np'-Nₐ'-(X'X'X')ₖ-N_{b}'-Y'Y'Y'-N_{b}'-(Z'Z'Z')ₗ-Nₐ'-n_{q}' 5' (III)
dans lequel :
chacun de i, j, k et l vaut indépendamment 0 ou 1 ;
chacun de p et q vaut indépendamment de 0 à 6 ;
chaque Nₐ et Nₐ' représente indépendamment une séquence oligonucléotidique comprenant 0 à 25 nucléotides qui sont soit modifiés soit non modifiés soit une combinaison de ceux-ci, chaque séquence comprenant au moins deux nucléotides différemment modifiés, chaque N_{b} et N_{b}' représente indépendamment une séquence oligonucléotidique comprenant 0 à 10 nucléotides qui sont soit modifiés soit non modifiés soit une combinaison de ceux-ci ;
chaque nₚ, nₚ', n_{q} et n_{q}' représente indépendamment une séquence de nucléotides en surplomb comprenant 0 à 6 nucléotides ; et
chacun de XXX, YYY, ZZZ, X'X'X', Y'Y'Y', et Z'Z'Z' représente indépendamment un motif donné de trois modifications identiques sur trois nucléotides consécutifs ;
le motif Y'Y'Y' apparaît aux positions 11, 12 et 13 du brin antisens à partir de l'extrémité 5' ;
Y' est 2'-OMe ;
ledit agent comprend en outre au moins une liaison inter-nucléotidique phosphorothioate, dans lequel ledit brin antisens comprend deux blocs de deux liaisons inter-nucléotidiques phosphorothioate séparées par 1, 2, 3, 4, 5, 6, 7, 8, 9, 10, 11, 12, 13, 14, 15, 16, 17 ou 18 liaisons inter-nucléotidiques phosphate, et ledit brin antisens est apparié à un brin sens comprenant n'importe quelle combinaison de liaisons inter-nucléotidiques phosphorothioate, méthylphosphonate et phosphate ; et
dans lequel la modification sur Nb est différente de la modification sur Y et la modification sur Nb' est différente de la modification sur Y',
à condition que l'ARNi double brin ne soit pas un ARNi double brin consistant en
brin sens: GfsgsUfuAfaCfaCfCfAfuUfuAfcUfuCfaAfL96
brin antisens: usUfsgAfaGfuAfaAfuggUfgUfuAfaCfcsasg,
brin sens: GfsgsUfuAfaCfaCfCfAfuUf(Uhd)AfcUfuCfaAfL96
brin antisens: usUfsgAfaGfuAfaAfuggUfgUfuAfaCfcsasg, et
brin sens: GfgUfuAfaCfaCfCfAfuUfuAfcUfuCfaAfL96
brin antisens: usUfsgAfaGfuAfaAfuggUfgUfuAfaCfcsasg, dans lequel
Gf est 2'-fluoroguanosine-3'-phosphate,
g est 2'-O-méthylguanosine-3'-phosphate,
Uf est 2'-fluorouridine-3'-phosphate,
u est 2'-O-méthyluridine-3'-phosphate,
Af est 2'-fluoroadénosine-3'-phosphate,
a est 2'-O-méthyladénosine-3'-phosphate,
Cf est 2'-fluorocytidine-3'-phosphate,
c est 2'-O-méthylcytidine-3'-phosphate,
(Uhd) est 2'-O-hexadécyl-uridine-3'-phosphate,
s est une liaison phosphorothioate, et
L96 est N-[tris(GalNAc-alkyl)-amidodécanoyl)]-4-hydroxyprolinol (Hyp-(GalNAc-alkyl)3).

2. Agent à ARNi double brin selon la revendication 1, dans lequel
(a) i vaut 1 ; j vaut 1 ; ou i et j valent tous deux 1 ; ou
(b) k vaut 1 ; l vaut 1 ; ou k et l valent tous deux 1.

3. Agent à ARNi double brin selon la revendication 1 ou 2, dans lequel le motif YYY apparaît au niveau ou à proximité du site de coupure du brin sens.

4. Agent à ARNi double brin selon la revendication 1, dans lequel
(a) la région duplex a une longueur de 17 à 30 paires de nucléotides, une longueur de 17 à 19 paires de nucléotides, ou une longueur de 27 à 30 paires de nucléotides, et/ou
(b) chaque brin a 17 à 30 nucléotides.

5. Agent à ARNi double brin selon la revendication 1, dans lequel les modifications sur les nucléotides sont choisies dans le groupe constitué par LNA, HNA, CeNA, 2'-méthoxyéthyle, 2'-O-alkyle, 2'-O-allyle, 2'-C-allyle, 2'-fluoro, 2'-désoxy, et leurs combinaisons, ou dans le groupe constitué par un nucléotide 2'-O-méthylé, un nucléotide 2'-désoxyfluoré, un nucléotide 2'-O-N-méthylacétamidé (2'-O-NMA), un nucléotide 2'-O-diméthylaminoéthoxyéthylé (2'-O-DMAEOE), un nucléotide 2'-O-aminopropylé (2'-O-AP), 2'-ara-F, et leurs combinaisons, dans lequel de préférence les nucléotides sont modifiés par soit 2'-OCH₃ soit 2'-F.

6. Agent à ARNi double brin selon l'une quelconque des revendications 1 à 5, comprenant en outre au moins un ligand, dans lequel de préférence le ligand
(a) est un ou plusieurs dérivés de GalNAc attachés par l'intermédiaire d'un lieur ramifié divalent ou trivalent ; et/ou
(b) est attaché à l'extrémité 3' du brin sens.

7. Agent à ARNi double brin selon l'une quelconque des revendications 1 à 6, dans lequel
(a) le nucléotide à la position 1 de l'extrémité 5' du duplex dans le brin antisens est choisi dans le groupe constitué par A, dA, dU, U, et dT ; et/ou
(b) la paire de bases à la position 1 de l'extrémité 5' du duplex est une paire de bases AU.

8. Agent à ARNi double brin selon l'une quelconque des revendications 1 à 7, dans lequel les nucléotides Y contiennent une modification 2'-fluoro.

9. Composition pharmaceutique comprenant l'agent à ARNi double brin selon l'une quelconque des revendications précédentes, seul ou en combinaison avec un véhicule ou excipient pharmaceutiquement acceptable.

10. Agent à ARNi double brin selon l'une quelconque des revendications précédentes, pour une utilisation dans une méthode de thérapie, la méthode comprenant l'inhibition de l'expression d'un gène cible.

11. Agent pour une utilisation selon la revendication 10, dans lequel l'agent à ARNi double brin est destiné à être administré par voie sous-cutanée ou intraveineuse.

12. Agent selon l'une quelconque des revendications 1 à 8 pour une utilisation dans une méthode de thérapie, la méthode comprenant la délivrance dudit agent à une cible spécifique chez un sujet.

13. Agent pour une utilisation selon la revendication 12, lequel agent est destiné à être administré par voie intramusculaire, intrabronchique, intrapleurale, intrapéritonéale, intra-artérielle, lymphatique, intraveineuse, sous-cutanée ou cérébrospinale, ou leurs combinaisons.


### SEQUENCE LISTING

<110> ALNYLAM PHARMACEUTICALS, INC.
<120> MODIFIED RNAI AGENTS
<130> X1807 EP/1
<150> US 61/561,710
   <151> 2011-11-18
<160> 1208
<170> PatentIn version 3.5
<210> 1
   <211> 16
   <212> PRT
   <213> Unknown
<220>
   <223> Description of Unknown: Exemplary hydrophobic membrane translocation peptide
<400> 1
   Ala Ala Val Ala Leu Leu Pro Ala Val Leu Leu Ala Leu Leu Ala Pro
   1               5                   10                  15
<210> 2
   <211> 11
   <212> PRT
   <213> Unknown
<220>
   <223> Description of Unknown: RFGF analogue peptide
<400> 2
   Ala Ala Leu Leu Pro Val Leu Leu Ala Ala Pro
   1               5                   10
<210> 3
   <211> 13
   <212> PRT
   <213> Human immunodeficiency virus
<400> 3
   Gly Arg Lys Lys Arg Arg Gln Arg Arg Arg Pro Pro Gln
   1               5                   10
<210> 4
   <211> 16
   <212> PRT
   <213> Drosophila sp.
<400> 4
   Arg Gln Ile Lys Ile Trp Phe Gln Asn Arg Arg Met Lys Trp Lys Lys
   1               5                   10                  15
<210> 5
   <211> 21
   <212> RNA
   <213> Artificial Sequence
<220>
   <223> Description of Artificial Sequence: Synthetic oligonucleotide
<400> 5
   auguaaccaa gaguauucca u 21
<210> 6
   <211> 21
   <212> RNA
   <213> Artificial Sequence
<220>
   <223> Description of Artificial Sequence: Synthetic oligonucleotide
<400> 6
   auguaaccaa gaguauucca u 21
<210> 7
   <211> 21
   <212> RNA
   <213> Artificial Sequence
<220>
   <223> Description of Artificial Sequence: Synthetic oligonucleotide
<400> 7
   auguaaccaa gaguauucca u 21
<210> 8
   <211> 21
   <212> RNA
   <213> Artificial Sequence
<220>
   <223> Description of Artificial Sequence: Synthetic oligonucleotide
<400> 8
   auguaaccaa gaguauucca u 21
<210> 9
   <211> 21
   <212> RNA
   <213> Artificial Sequence
<220>
   <223> Description of Artificial Sequence: Synthetic oligonucleotide
<400> 9
   auguaaccca gaguauucca u 21
<210> 10
   <211> 21
   <212> RNA
   <213> Artificial Sequence
<220>
   <223> Description of Artificial Sequence: Synthetic oligonucleotide
<400> 10
   auguaaccaa gaguauucca u 21
<210> 11
   <211> 21
   <212> RNA
   <213> Artificial Sequence
<220>
   <223> Description of Artificial Sequence: Synthetic oligonucleotide
<400> 11
   auguaaccaa gaguauucca u 21
<210> 12
   <211> 21
   <212> RNA
   <213> Artificial Sequence
<220>
   <223> Description of Artificial Sequence: Synthetic oligonucleotide
<400> 12
   auguaaccaa gaguauucca u 21
<210> 13
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 13
   auguaaccaa gaguauacga u 21
<210> 14
   <211> 21
   <212> RNA
   <213> Artificial Sequence
<220>
   <223> Description of Artificial Sequence: Synthetic oligonucleotide
<400> 14
   ugggauuuca uguaaccaag a 21
<210> 15
   <211> 21
   <212> RNA
   <213> Artificial Sequence
<220>
   <223> Description of Artificial Sequence: Synthetic oligonucleotide
<400> 15
   ugggauuuca uguaaccaag a 21
<210> 16
   <211> 21
   <212> RNA
   <213> Artificial Sequence
<220>
   <223> Description of Artificial Sequence: Synthetic oligonucleotide
<400> 16
   auguaaccaa gaguauucca u 21
<210> 17
   <211> 21
   <212> RNA
   <213> Artificial Sequence
<220>
   <223> Description of Artificial Sequence: Synthetic oligonucleotide
<400> 17
   ugggauuuca uguaaccaag a 21
<210> 18
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 18
   auguaaccaa gaguauacga u 21
<210> 19
   <211> 21
   <212> RNA
   <213> Artificial Sequence
<220>
   <223> Description of Artificial Sequence: Synthetic oligonucleotide
<400> 19
   auguaaccaa gaguauucca u 21
<210> 20
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 20
   auguaaccaa gagtatucca u 21
<210> 21
   <211> 21
   <212> RNA
   <213> Artificial Sequence
<220>
   <223> Description of Artificial Sequence: Synthetic oligonucleotide
<400> 21
   auguaaccaa gaguauucca u 21
<210> 22
   <211> 21
   <212> RNA
   <213> Artificial Sequence
<220>
   <223> Description of Artificial Sequence: Synthetic oligonucleotide
<400> 22
   ugggauuuca uguaaccaag a 21
<210> 23
   <211> 21
   <212> RNA
   <213> Artificial Sequence
<220>
   <223> Description of Artificial Sequence: Synthetic oligonucleotide
<400> 23
   auguaaccaa gaguauucca u 21
<210> 24
   <211> 21
   <212> RNA
   <213> Artificial Sequence
<220>
   <223> Description of Artificial Sequence: Synthetic oligonucleotide
<400> 24
   auguaaccaa gaguauucca u 21
<210> 25
   <211> 21
   <212> RNA
   <213> Artificial Sequence
<220>
   <223> Description of Artificial Sequence: Synthetic oligonucleotide
<400> 25
   auguaaccaa gaguauucca u 21
<210> 26
   <211> 21
   <212> RNA
   <213> Artificial Sequence
<220>
   <223> Description of Artificial Sequence: Synthetic oligonucleotide
<400> 26
   auguaaccaa gaguauucca u 21
<210> 27
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 27
   tgggatuuca ugtaaccaag a 21
<210> 28
   <211> 21
   <212> RNA
   <213> Artificial Sequence
<220>
   <223> Description of Artificial Sequence: Synthetic oligonucleotide
<400> 28
   auguaaccaa gaguauucca u 21
<210> 29
   <211> 21
   <212> RNA
   <213> Artificial Sequence
<220>
   <223> Description of Artificial Sequence: Synthetic oligonucleotide
<400> 29
   ugggauuuca uguaaccaag a 21
<210> 30
   <211> 21
   <212> RNA
   <213> Artificial Sequence
<220>
   <223> Description of Artificial Sequence: Synthetic oligonucleotide
<400> 30
   ugggauuuca uguaaccaag a 21
<210> 31
   <211> 21
   <212> RNA
   <213> Artificial Sequence
<220>
   <223> Description of Artificial Sequence: Synthetic oligonucleotide
<400> 31
   ugggauuuca uguaaccaag a 21
<210> 32
   <211> 21
   <212> RNA
   <213> Artificial Sequence
<220>
   <223> Description of Artificial Sequence: Synthetic oligonucleotide
<400> 32
   ugggauuuca uguaaccaag a 21
<210> 33
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 33
   atguaaccaa gagtautcca t 21
<210> 34
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 34
   atgtaaccaa gagtatucca u 21
<210> 35
   <211> 21
   <212> RNA
   <213> Artificial Sequence
<220>
   <223> Description of Artificial Sequence: Synthetic oligonucleotide
<400> 35
   ugggauuuca uguaaccaag a 21
<210> 36
   <211> 21
   <212> RNA
   <213> Artificial Sequence
<220>
   <223> Description of Artificial Sequence: Synthetic oligonucleotide
<400> 36
   auguaaccaa gaguauucca u 21
<210> 37
   <211> 21
   <212> RNA
   <213> Artificial Sequence
<220>
   <223> Description of Artificial Sequence: Synthetic oligonucleotide
<400> 37
   auguaaccaa gaguauucca u 21
<210> 38
   <211> 21
   <212> RNA
   <213> Artificial Sequence
<220>
   <223> Description of Artificial Sequence: Synthetic oligonucleotide
<400> 38
   ugggauuuca uguaaccaag a 21
<210> 39
   <211> 21
   <212> RNA
   <213> Artificial Sequence
<220>
   <223> Description of Artificial Sequence: Synthetic oligonucleotide
<400> 39
   auguaaccaa gaguauucca u 21
<210> 40
   <211> 21
   <212> RNA
   <213> Artificial Sequence
<220>
   <223> Description of Artificial Sequence: Synthetic oligonucleotide
<400> 40
   ugggauuuca uguaaccaag a 21
<210> 41
   <211> 21
   <212> RNA
   <213> Artificial Sequence
<220>
   <223> Description of Artificial Sequence: Synthetic oligonucleotide
<400> 41
   ugggauuuca uguaaccaag a 21
<210> 42
   <211> 21
   <212> RNA
   <213> Artificial Sequence
<220>
   <223> Description of Artificial Sequence: Synthetic oligonucleotide
<400> 42
   ugggauuuca uguaaccaag a 21
<210> 43
   <211> 21
   <212> RNA
   <213> Artificial Sequence
<220>
   <223> Description of Artificial Sequence: Synthetic oligonucleotide
<400> 43
   ugggauuuca uguaaccaag a 21
<210> 44
   <211> 21
   <212> RNA
   <213> Artificial Sequence
<220>
   <223> Description of Artificial Sequence: Synthetic oligonucleotide
<400> 44
   auguaaccaa gaguauucca u 21
<210> 45
   <211> 21
   <212> RNA
   <213> Artificial Sequence
<220>
   <223> Description of Artificial Sequence: Synthetic oligonucleotide
<400> 45
   auguaaccaa gaguauucca u 21
<210> 46
   <211> 21
   <212> RNA
   <213> Artificial Sequence
<220>
   <223> Description of Artificial Sequence: Synthetic oligonucleotide
<400> 46
   auguaaccaa gaguauucca u 21
<210> 47
   <211> 21
   <212> RNA
   <213> Artificial Sequence
<220>
   <223> Description of Artificial Sequence: Synthetic oligonucleotide
<400> 47
   ugggauuuca uguaaccaag a 21
<210> 48
   <211> 21
   <212> RNA
   <213> Artificial Sequence
<220>
   <223> Description of Artificial Sequence: Synthetic oligonucleotide
<400> 48
   auguaaccaa gaguauucca u 21
<210> 49
   <211> 21
   <212> RNA
   <213> Artificial Sequence
<220>
   <223> Description of Artificial Sequence: Synthetic oligonucleotide
<400> 49
   auguaaccaa gaguauucca u 21
<210> 50
   <211> 21
   <212> RNA
   <213> Artificial Sequence
<220>
   <223> Description of Artificial Sequence: Synthetic oligonucleotide
<400> 50
   auguaaccaa gaggauucca u 21
<210> 51
   <211> 21
   <212> RNA
   <213> Artificial Sequence
<220>
   <223> Description of Artificial Sequence: Synthetic oligonucleotide
<400> 51
   auguaaccaa gaguauucca u 21
<210> 52
   <211> 21
   <212> RNA
   <213> Artificial Sequence
<220>
   <223> Description of Artificial Sequence: Synthetic oligonucleotide
<400> 52
   ugggauuuca uguaaccaag a 21
<210> 53
   <211> 21
   <212> RNA
   <213> Artificial Sequence
<220>
   <223> Description of Artificial Sequence: Synthetic oligonucleotide
<400> 53
   auguaaccaa gaguauucca u 21
<210> 54
   <211> 21
   <212> RNA
   <213> Artificial Sequence
<220>
   <223> Description of Artificial Sequence: Synthetic oligonucleotide
<400> 54
   auguaaccaa gaguauucca u 21
<210> 55
   <211> 21
   <212> RNA
   <213> Artificial Sequence
<220>
   <223> Description of Artificial Sequence: Synthetic oligonucleotide
<400> 55
   ugggauuuca uguaaccaag a 21
<210> 56
   <211> 21
   <212> RNA
   <213> Artificial Sequence
<220>
   <223> Description of Artificial Sequence: Synthetic oligonucleotide
<400> 56
   auguaaccaa gaguauucca u 21
<210> 57
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 57
   auguaaccaa gagtatucca u 21
<210> 58
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 58
   auguaaccaa gagtatucca u 21
<210> 59
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 59
   auguaaccaa gagtauucca u 21
<210> 60
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 60
   auguaaccaa gagtatucca u 21
<210> 61
   <211> 21
   <212> RNA
   <213> Artificial Sequence
<220>
   <223> Description of Artificial Sequence: Synthetic oligonucleotide
<400> 61
   auguaaccaa gaguauucca u 21
<210> 62
   <211> 21
   <212> RNA
   <213> Artificial Sequence
<220>
   <223> Description of Artificial Sequence: Synthetic oligonucleotide
<400> 62
   auguaaccaa gaguauucca u 21
<210> 63
   <211> 21
   <212> RNA
   <213> Artificial Sequence
<220>
   <223> Description of Artificial Sequence: Synthetic oligonucleotide
<400> 63
   auguaaccaa gaguauucca u 21
<210> 64
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 64
   auguaaccaa gagtatucca u 21
<210> 65
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 65
   atguaaccaa gagtatucca t 21
<210> 66
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 66
   auguaaccaa gaguauucca u 21
<210> 67
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 67
   auguaaccaa gagtatucca u 21
<210> 68
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 68
   atguaaccca gagtauucca u 21
<210> 69
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 69
   auguaaccaa gagtauucca u 21
<210> 70
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 70
   auguaaccaa gagtatucca u 21
<210> 71
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 71
   auguaaccaa gaguatucca u 21
<210> 72
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 72
   auguaaccaa gagtatucca u 21
<210> 73
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 73
   auguaaccaa gagtatucca u 21
<210> 74
   <211> 21
   <212> RNA
   <213> Artificial Sequence
<220>
   <223> Description of Artificial Sequence: Synthetic oligonucleotide
<400> 74
   auguaaccca gaguauucca u 21
<210> 75
   <211> 21
   <212> RNA
   <213> Artificial Sequence
<220>
   <223> Description of Artificial Sequence: Synthetic oligonucleotide
<400> 75
   auguaaccaa gaguauucca u 21
<210> 76
   <211> 21
   <212> RNA
   <213> Artificial Sequence
<220>
   <223> Description of Artificial Sequence: Synthetic oligonucleotide
<400> 76
   ugggauuuca uguaaccaag a 21
<210> 77
   <211> 21
   <212> RNA
   <213> Artificial Sequence
<220>
   <223> Description of Artificial Sequence: Synthetic oligonucleotide
<400> 77
   auguaaccaa gaguauucca u 21
<210> 78
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 78
   auguaaccaa gagtatucca u 21
<210> 79
   <211> 21
   <212> RNA
   <213> Artificial Sequence
<220>
   <223> Description of Artificial Sequence: Synthetic oligonucleotide
<400> 79
   auguaaccaa gaguauucca u 21
<210> 80
   <211> 21
   <212> RNA
   <213> Artificial Sequence
<220>
   <223> Description of Artificial Sequence: Synthetic oligonucleotide
<400> 80
   auguaaccaa gaguauucca u 21
<210> 81
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 81
   auguaaccaa gaguattcca u 21
<210> 82
   <211> 21
   <212> RNA
   <213> Artificial Sequence
<220>
   <223> Description of Artificial Sequence: Synthetic oligonucleotide
<400> 82
   auguaaccaa gaguauucca u 21
<210> 83
   <211> 21
   <212> RNA
   <213> Artificial Sequence
<220>
   <223> Description of Artificial Sequence: Synthetic oligonucleotide
<400> 83
   auguaaccaa gaguauucca u 21
<210> 84
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 84
   auguaaccaa gagtatucca u 21
<210> 85
   <211> 21
   <212> RNA
   <213> Artificial Sequence
<220>
   <223> Description of Artificial Sequence: Synthetic oligonucleotide
<400> 85
   auguaaccaa gaguauucca u 21
<210> 86
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 86
   auguaaccaa gaguauucca t 21
<210> 87
   <211> 21
   <212> RNA
   <213> Artificial Sequence
<220>
   <223> Description of Artificial Sequence: Synthetic oligonucleotide
<400> 87
   auguaaccaa gaguauucca u 21
<210> 88
   <211> 21
   <212> RNA
   <213> Artificial Sequence
<220>
   <223> Description of Artificial Sequence: Synthetic oligonucleotide
<400> 88
   auguaaccaa gaguauucca u 21
<210> 89
   <211> 21
   <212> RNA
   <213> Artificial Sequence
<220>
   <223> Description of Artificial Sequence: Synthetic oligonucleotide
<400> 89
   auguaaccaa gaguauucca u 21
<210> 90
   <211> 21
   <212> RNA
   <213> Artificial Sequence
<220>
   <223> Description of Artificial Sequence: Synthetic oligonucleotide
<400> 90
   auguaaccaa gaguauucca u 21
<210> 91
   <211> 21
   <212> RNA
   <213> Artificial Sequence
<220>
   <223> Description of Artificial Sequence: Synthetic oligonucleotide
<400> 91
   auguaaccaa gaguauucca u 21
<210> 92
   <211> 21
   <212> RNA
   <213> Artificial Sequence
<220>
   <223> Description of Artificial Sequence: Synthetic oligonucleotide
<400> 92
   auguaaccaa gaguauucca u 21
<210> 93
   <211> 21
   <212> RNA
   <213> Artificial Sequence
<220>
   <223> Description of Artificial Sequence: Synthetic oligonucleotide
<400> 93
   auguaaccaa gaguauucca u 21
<210> 94
   <211> 21
   <212> RNA
   <213> Artificial Sequence
<220>
   <223> Description of Artificial Sequence: Synthetic oligonucleotide
<400> 94
   auguaaccaa gaguauucca u 21
<210> 95
   <211> 21
   <212> RNA
   <213> Artificial Sequence
<220>
   <223> Description of Artificial Sequence: Synthetic oligonucleotide
<400> 95
   auguaaccaa gaguauucca u 21
<210> 96
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 96
   auguaaccaa gagtatucca u 21
<210> 97
   <211> 21
   <212> RNA
   <213> Artificial Sequence
<220>
   <223> Description of Artificial Sequence: Synthetic oligonucleotide
<400> 97
   auguaaccaa gaguauucca u 21
<210> 98
   <211> 21
   <212> RNA
   <213> Artificial Sequence
<220>
   <223> Description of Artificial Sequence: Synthetic oligonucleotide
<400> 98
   auguaaccaa gaguauucca u 21
<210> 99
   <211> 21
   <212> RNA
   <213> Artificial Sequence
<220>
   <223> Description of Artificial Sequence: Synthetic oligonucleotide
<400> 99
   auguaaccaa gaguauucca u 21
<210> 100
   <211> 21
   <212> RNA
   <213> Artificial Sequence
<220>
   <223> Description of Artificial Sequence: Synthetic oligonucleotide
<400> 100
   auguaaccaa gaguauucca u 21
<210> 101
   <211> 21
   <212> RNA
   <213> Artificial Sequence
<220>
   <223> Description of Artificial Sequence: Synthetic oligonucleotide
<400> 101
   auguaaccaa gaguauucca u 21
<210> 102
   <211> 21
   <212> RNA
   <213> Artificial Sequence
<220>
   <223> Description of Artificial Sequence: Synthetic oligonucleotide
<400> 102
   auguaaccaa gaguauucca u 21
<210> 103
   <211> 21
   <212> RNA
   <213> Artificial Sequence
<220>
   <223> Description of Artificial Sequence: Synthetic oligonucleotide
<400> 103
   auguaaccaa gaguauucca u 21
<210> 104
   <211> 21
   <212> RNA
   <213> Artificial Sequence
<220>
   <223> Description of Artificial Sequence: Synthetic oligonucleotide
<400> 104
   auguaaccaa gaguauucca u 21
<210> 105
   <211> 21
   <212> RNA
   <213> Artificial Sequence
<220>
   <223> Description of Artificial Sequence: Synthetic oligonucleotide
<400> 105
   auguaaccaa gaguauucca u 21
<210> 106
   <211> 21
   <212> RNA
   <213> Artificial Sequence
<220>
   <223> Description of Artificial Sequence: Synthetic oligonucleotide
<400> 106
   auguaaccaa gaguauucca u 21
<210> 107
   <211> 21
   <212> RNA
   <213> Artificial Sequence
<220>
   <223> Description of Artificial Sequence: Synthetic oligonucleotide
<400> 107
   auguaaccaa gaguauucca u 21
<210> 108
   <211> 21
   <212> RNA
   <213> Artificial Sequence
<220>
   <223> Description of Artificial Sequence: Synthetic oligonucleotide
<400> 108
   auguaaccaa gaguauucca u 21
<210> 109
   <211> 21
   <212> RNA
   <213> Artificial Sequence
<220>
   <223> Description of Artificial Sequence: Synthetic oligonucleotide
<400> 109
   auguaaccaa gaguauucca u 21
<210> 110
   <211> 21
   <212> RNA
   <213> Artificial Sequence
<220>
   <223> Description of Artificial Sequence: Synthetic oligonucleotide
<400> 110
   auguaaccaa gaguauucca u 21
<210> 111
   <211> 21
   <212> RNA
   <213> Artificial Sequence
<220>
   <223> Description of Artificial Sequence: Synthetic oligonucleotide
<400> 111
   auguaaccaa gaguauucca u 21
<210> 112
   <211> 21
   <212> RNA
   <213> Artificial Sequence
<220>
   <223> Description of Artificial Sequence: Synthetic oligonucleotide
<400> 112
   auguaaccaa gaguauucca u 21
<210> 113
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 113
   auguaaccaa gagtatucca u 21
<210> 114
   <211> 21
   <212> RNA
   <213> Artificial Sequence
<220>
   <223> Description of Artificial Sequence: Synthetic oligonucleotide
<400> 114
   auguaaccaa gaguauucca u 21
<210> 115
   <211> 21
   <212> RNA
   <213> Artificial Sequence
<220>
   <223> Description of Artificial Sequence: Synthetic oligonucleotide
<400> 115
   auguaaccaa gaguauucca u 21
<210> 116
   <211> 21
   <212> RNA
   <213> Artificial Sequence
<220>
   <223> Description of Artificial Sequence: Synthetic oligonucleotide
<400> 116
   auguaaccaa gaguauucca u 21
<210> 117
   <211> 21
   <212> RNA
   <213> Artificial Sequence
<220>
   <223> Description of Artificial Sequence: Synthetic oligonucleotide
<400> 117
   auguaaccaa gaguauucca u 21
<210> 118
   <211> 21
   <212> RNA
   <213> Artificial Sequence
<220>
   <223> Description of Artificial Sequence: Synthetic oligonucleotide
<400> 118
   auguaaccaa gaguauucca u 21
<210> 119
   <211> 21
   <212> RNA
   <213> Artificial Sequence
<220>
   <223> Description of Artificial Sequence: Synthetic oligonucleotide
<400> 119
   auguaaccaa gaguauucca u 21
<210> 120
   <211> 21
   <212> RNA
   <213> Artificial Sequence
<220>
   <223> Description of Artificial Sequence: Synthetic oligonucleotide
<400> 120
   auguaaccaa gaguauucca u 21
<210> 121
   <211> 21
   <212> RNA
   <213> Artificial Sequence
<220>
   <223> Description of Artificial Sequence: Synthetic oligonucleotide
<400> 121
   auguaaccaa gaguauucca u 21
<210> 122
   <211> 21
   <212> RNA
   <213> Artificial Sequence
<220>
   <223> Description of Artificial Sequence: Synthetic oligonucleotide
<400> 122
   auguaaccaa gaguauucca u 21
<210> 123
   <211> 21
   <212> RNA
   <213> Artificial Sequence
<220>
   <223> Description of Artificial Sequence: Synthetic oligonucleotide
<400> 123
   auguaaccaa gaguauucca u 21
<210> 124
   <211> 21
   <212> RNA
   <213> Artificial Sequence
<220>
   <223> Description of Artificial Sequence: Synthetic oligonucleotide
<400> 124
   auguaaccaa gaguauucca u 21
<210> 125
   <211> 21
   <212> RNA
   <213> Artificial Sequence
<220>
   <223> Description of Artificial Sequence: Synthetic oligonucleotide
<400> 125
   auguaaccaa gaguauucca u 21
<210> 126
   <211> 21
   <212> RNA
   <213> Artificial Sequence
<220>
   <223> Description of Artificial Sequence: Synthetic oligonucleotide
<400> 126
   auguaaccaa gaguauucca u 21
<210> 127
   <211> 21
   <212> RNA
   <213> Artificial Sequence
<220>
   <223> Description of Artificial Sequence: Synthetic oligonucleotide
<400> 127
   auguaaccaa gaguauucca u 21
<210> 128
   <211> 21
   <212> RNA
   <213> Artificial Sequence
<220>
   <223> Description of Artificial Sequence: Synthetic oligonucleotide
<400> 128
   auguaaccaa gaguauucca u 21
<210> 129
   <211> 21
   <212> RNA
   <213> Artificial Sequence
<220>
   <223> Description of Artificial Sequence: Synthetic oligonucleotide
<400> 129
   auguaaccaa gaguauucca u 21
<210> 130
   <211> 21
   <212> RNA
   <213> Artificial Sequence
<220>
   <223> Description of Artificial Sequence: Synthetic oligonucleotide
<400> 130
   auguaaccaa gaguauucca u 21
<210> 131
   <211> 21
   <212> RNA
   <213> Artificial Sequence
<220>
   <223> Description of Artificial Sequence: Synthetic oligonucleotide
<400> 131
   auguaaccaa gaguauucca u 21
<210> 132
   <211> 21
   <212> RNA
   <213> Artificial Sequence
<220>
   <223> Description of Artificial Sequence: Synthetic oligonucleotide
<400> 132
   auguaaccaa gaguauucca u 21
<210> 133
   <211> 21
   <212> RNA
   <213> Artificial Sequence
<220>
   <223> Description of Artificial Sequence: Synthetic oligonucleotide
<400> 133
   auguaaccaa gaguauucca u 21
<210> 134
   <211> 21
   <212> RNA
   <213> Artificial Sequence
<220>
   <223> Description of Artificial Sequence: Synthetic oligonucleotide
<400> 134
   auguaaccaa gaguauucca u 21
<210> 135
   <211> 21
   <212> RNA
   <213> Artificial Sequence
<220>
   <223> Description of Artificial Sequence: Synthetic oligonucleotide
<400> 135
   auguaaccaa gaguauucca u 21
<210> 136
   <211> 21
   <212> RNA
   <213> Artificial Sequence
<220>
   <223> Description of Artificial Sequence: Synthetic oligonucleotide
<400> 136
   auguaaccaa gaguauucca u 21
<210> 137
   <211> 21
   <212> RNA
   <213> Artificial Sequence
<220>
   <223> Description of Artificial Sequence: Synthetic oligonucleotide
<400> 137
   auguaaccaa gaguauucca u 21
<210> 138
   <211> 21
   <212> RNA
   <213> Artificial Sequence
<220>
   <223> Description of Artificial Sequence: Synthetic oligonucleotide
<400> 138
   auguaaccaa gaguauucca u 21
<210> 139
   <211> 21
   <212> RNA
   <213> Artificial Sequence
<220>
   <223> Description of Artificial Sequence: Synthetic oligonucleotide
<400> 139
   auguaaccaa gaguauucca u 21
<210> 140
   <211> 21
   <212> RNA
   <213> Artificial Sequence
<220>
   <223> Description of Artificial Sequence: Synthetic oligonucleotide
<400> 140
   auguaaccaa gaguauucca u 21
<210> 141
   <211> 21
   <212> RNA
   <213> Artificial Sequence
<220>
   <223> Description of Artificial Sequence: Synthetic oligonucleotide
<400> 141
   auguaaccaa gaguauucca u 21
<210> 142
   <211> 21
   <212> RNA
   <213> Artificial Sequence
<220>
   <223> Description of Artificial Sequence: Synthetic oligonucleotide
<400> 142
   auguaaccaa gaguauucca u 21
<210> 143
   <211> 21
   <212> RNA
   <213> Artificial Sequence
<220>
   <223> Description of Artificial Sequence: Synthetic oligonucleotide
<400> 143
   auguaaccaa gaguauucca u 21
<210> 144
   <211> 21
   <212> RNA
   <213> Artificial Sequence
<220>
   <223> Description of Artificial Sequence: Synthetic oligonucleotide
<400> 144
   auguaaccaa gaguauucca u 21
<210> 145
   <211> 21
   <212> RNA
   <213> Artificial Sequence
<220>
   <223> Description of Artificial Sequence: Synthetic oligonucleotide
<400> 145
   auguaaccaa gaguauucca u 21
<210> 146
   <211> 21
   <212> RNA
   <213> Artificial Sequence
<220>
   <223> Description of Artificial Sequence: Synthetic oligonucleotide
<400> 146
   auguaaccaa gaguauucca u 21
<210> 147
   <211> 21
   <212> RNA
   <213> Artificial Sequence
<220>
   <223> Description of Artificial Sequence: Synthetic oligonucleotide
<400> 147
   auguaaccaa gaguauucca u 21
<210> 148
   <211> 21
   <212> RNA
   <213> Artificial Sequence
<220>
   <223> Description of Artificial Sequence: Synthetic oligonucleotide
<400> 148
   auguaaccaa gaguauucca u 21
<210> 149
   <211> 21
   <212> RNA
   <213> Artificial Sequence
<220>
   <223> Description of Artificial Sequence: Synthetic oligonucleotide
<400> 149
   auguaaccaa gaguauucca u 21
<210> 150
   <211> 21
   <212> RNA
   <213> Artificial Sequence
<220>
   <223> Description of Artificial Sequence: Synthetic oligonucleotide
<400> 150
   auguaaccaa gaguauucca u 21
<210> 151
   <211> 21
   <212> RNA
   <213> Artificial Sequence
<220>
   <223> Description of Artificial Sequence: Synthetic oligonucleotide
<400> 151
   auguaaccaa gaguauucca u 21
<210> 152
   <211> 21
   <212> RNA
   <213> Artificial Sequence
<220>
   <223> Description of Artificial Sequence: Synthetic oligonucleotide
<400> 152
   auguaaccaa gaguauucca u 21
<210> 153
   <211> 21
   <212> RNA
   <213> Artificial Sequence
<220>
   <223> Description of Artificial Sequence: Synthetic oligonucleotide
<400> 153
   auguaaccaa gaguauucca u 21
<210> 154
   <211> 21
   <212> RNA
   <213> Artificial Sequence
<220>
   <223> Description of Artificial Sequence: Synthetic oligonucleotide
<400> 154
   auguaaccaa gaguauucca u 21
<210> 155
   <211> 21
   <212> RNA
   <213> Artificial Sequence
<220>
   <223> Description of Artificial Sequence: Synthetic oligonucleotide
<400> 155
   auguaaccaa gaguauucca u 21
<210> 156
   <211> 21
   <212> RNA
   <213> Artificial Sequence
<220>
   <223> Description of Artificial Sequence: Synthetic oligonucleotide
<400> 156
   auguaaccaa gaguauucca u 21
<210> 157
   <211> 21
   <212> RNA
   <213> Artificial Sequence
<220>
   <223> Description of Artificial Sequence: Synthetic oligonucleotide
<400> 157
   auguaaccaa gaguauucca u 21
<210> 158
   <211> 21
   <212> RNA
   <213> Artificial Sequence
<220>
   <223> Description of Artificial Sequence: Synthetic oligonucleotide
<400> 158
   auguaaccaa gaguauucca u 21
<210> 159
   <211> 21
   <212> RNA
   <213> Artificial Sequence
<220>
   <223> Description of Artificial Sequence: Synthetic oligonucleotide
<400> 159
   ugggauuuca uguaaccaag a 21
<210> 160
   <211> 21
   <212> RNA
   <213> Artificial Sequence
<220>
   <223> Description of Artificial Sequence: Synthetic oligonucleotide
<400> 160
   ugggauuuca uguaaccaag a 21
<210> 161
   <211> 21
   <212> RNA
   <213> Artificial Sequence
<220>
   <223> Description of Artificial Sequence: Synthetic oligonucleotide
<400> 161
   ugggauuuca uguaaccaag a 21
<210> 162
   <211> 21
   <212> RNA
   <213> Artificial Sequence
<220>
   <223> Description of Artificial Sequence: Synthetic oligonucleotide
<400> 162
   ugggauuuca uguaaccaag a 21
<210> 163
   <211> 21
   <212> RNA
   <213> Artificial Sequence
<220>
   <223> Description of Artificial Sequence: Synthetic oligonucleotide
<400> 163
   ugggauuuca uguaaccaag a 21
<210> 164
   <211> 21
   <212> RNA
   <213> Artificial Sequence
<220>
   <223> Description of Artificial Sequence: Synthetic oligonucleotide
<400> 164
   ugggauuuca uguaaccaag a 21
<210> 165
   <211> 21
   <212> RNA
   <213> Artificial Sequence
<220>
   <223> Description of Artificial Sequence: Synthetic oligonucleotide
<400> 165
   ugggauuuca uguaaccaag a 21
<210> 166
   <211> 21
   <212> RNA
   <213> Artificial Sequence
<220>
   <223> Description of Artificial Sequence: Synthetic oligonucleotide
<400> 166
   ugggauuuca uguaaccaag a 21
<210> 167
   <211> 21
   <212> RNA
   <213> Artificial Sequence
<220>
   <223> Description of Artificial Sequence: Synthetic oligonucleotide
<400> 167
   auguaaccaa gaguauucca u 21
<210> 168
   <211> 21
   <212> RNA
   <213> Artificial Sequence
<220>
   <223> Description of Artificial Sequence: Synthetic oligonucleotide
<400> 168
   auguaaccaa gaguauucca u 21
<210> 169
   <211> 21
   <212> RNA
   <213> Artificial Sequence
<220>
   <223> Description of Artificial Sequence: Synthetic oligonucleotide
<400> 169
   auguaaccaa gaguauucca u 21
<210> 170
   <211> 21
   <212> RNA
   <213> Artificial Sequence
<220>
   <223> Description of Artificial Sequence: Synthetic oligonucleotide
<400> 170
   auguaaccaa gaguauucca u 21
<210> 171
   <211> 21
   <212> RNA
   <213> Artificial Sequence
<220>
   <223> Description of Artificial Sequence: Synthetic oligonucleotide
<400> 171
   auguaaccaa gaguauucca u 21
<210> 172
   <211> 21
   <212> RNA
   <213> Artificial Sequence
<220>
   <223> Description of Artificial Sequence: Synthetic oligonucleotide
<400> 172
   auguaaccaa gaguauucca u 21
<210> 173
   <211> 21
   <212> RNA
   <213> Artificial Sequence
<220>
   <223> Description of Artificial Sequence: Synthetic oligonucleotide
<400> 173
   auguaaccaa gaguauucca u 21
<210> 174
   <211> 21
   <212> RNA
   <213> Artificial Sequence
<220>
   <223> Description of Artificial Sequence: Synthetic oligonucleotide
<400> 174
   auguaaccaa gaguauucca u 21
<210> 175
   <211> 21
   <212> RNA
   <213> Artificial Sequence
<220>
   <223> Description of Artificial Sequence: Synthetic oligonucleotide
<400> 175
   auguaaccaa gaguauucca u 21
<210> 176
   <211> 21
   <212> RNA
   <213> Artificial Sequence
<220>
   <223> Description of Artificial Sequence: Synthetic oligonucleotide
<400> 176
   auguaaccaa gaguauucca u 21
<210> 177
   <211> 21
   <212> RNA
   <213> Artificial Sequence
<220>
   <223> Description of Artificial Sequence: Synthetic oligonucleotide
<400> 177
   auguaaccaa gaguauucca u 21
<210> 178
   <211> 21
   <212> RNA
   <213> Artificial Sequence
<220>
   <223> Description of Artificial Sequence: Synthetic oligonucleotide
<400> 178
   auguaaccaa gaguauucca u 21
<210> 179
   <211> 21
   <212> RNA
   <213> Artificial Sequence
<220>
   <223> Description of Artificial Sequence: Synthetic oligonucleotide
<400> 179
   auguaaccaa gaguauucca u 21
<210> 180
   <211> 21
   <212> RNA
   <213> Artificial Sequence
<220>
   <223> Description of Artificial Sequence: Synthetic oligonucleotide
<400> 180
   auguaaccaa gaguauucca u 21
<210> 181
   <211> 21
   <212> RNA
   <213> Artificial Sequence
<220>
   <223> Description of Artificial Sequence: Synthetic oligonucleotide
<400> 181
   auguaaccaa gaguauucca u 21
<210> 182
   <211> 21
   <212> RNA
   <213> Artificial Sequence
<220>
   <223> Description of Artificial Sequence: Synthetic oligonucleotide
<400> 182
   auguaaccaa gaguauucca u 21
<210> 183
   <211> 21
   <212> RNA
   <213> Artificial Sequence
<220>
   <223> Description of Artificial Sequence: Synthetic oligonucleotide
<400> 183
   auguaaccaa gaguauucca u 21
<210> 184
   <211> 21
   <212> RNA
   <213> Artificial Sequence
<220>
   <223> Description of Artificial Sequence: Synthetic oligonucleotide
<400> 184
   auguaaccaa gaguauucca u 21
<210> 185
   <211> 21
   <212> RNA
   <213> Artificial Sequence
<220>
   <223> Description of Artificial Sequence: Synthetic oligonucleotide
<400> 185
   auguaaccaa gaguauucca u 21
<210> 186
   <211> 21
   <212> RNA
   <213> Artificial Sequence
<220>
   <223> Description of Artificial Sequence: Synthetic oligonucleotide
<400> 186
   auguaaccaa gaguauucca u 21
<210> 187
   <211> 21
   <212> RNA
   <213> Artificial Sequence
<220>
   <223> Description of Artificial Sequence: Synthetic oligonucleotide
<400> 187
   auguaaccaa gaguauucca u 21
<210> 188
   <211> 21
   <212> RNA
   <213> Artificial Sequence
<220>
   <223> Description of Artificial Sequence: Synthetic oligonucleotide
<400> 188
   auguaaccaa gaguauucca u 21
<210> 189
   <211> 21
   <212> RNA
   <213> Artificial Sequence
<220>
   <223> Description of Artificial Sequence: Synthetic oligonucleotide
<400> 189
   auguaaccaa gaguauucca u 21
<210> 190
   <211> 21
   <212> RNA
   <213> Artificial Sequence
<220>
   <223> Description of Artificial Sequence: Synthetic oligonucleotide
<400> 190
   auguaaccaa gaguauucca u 21
<210> 191
   <211> 21
   <212> RNA
   <213> Artificial Sequence
<220>
   <223> Description of Artificial Sequence: Synthetic oligonucleotide
<400> 191
   auguaaccaa gaguauucca u 21
<210> 192
   <211> 21
   <212> RNA
   <213> Artificial Sequence
<220>
   <223> Description of Artificial Sequence: Synthetic oligonucleotide
<400> 192
   auguaaccaa gaguauucca u 21
<210> 193
   <211> 21
   <212> RNA
   <213> Artificial Sequence
<220>
   <223> Description of Artificial Sequence: Synthetic oligonucleotide
<400> 193
   auguaaccaa gaguauucca u 21
<210> 194
   <211> 21
   <212> RNA
   <213> Artificial Sequence
<220>
   <223> Description of Artificial Sequence: Synthetic oligonucleotide
<400> 194
   auguaaccaa gaguauucca u 21
<210> 195
   <211> 21
   <212> RNA
   <213> Artificial Sequence
<220>
   <223> Description of Artificial Sequence: Synthetic oligonucleotide
<400> 195
   auguaaccaa gaguauucca u 21
<210> 196
   <211> 21
   <212> RNA
   <213> Artificial Sequence
<220>
   <223> Description of Artificial Sequence: Synthetic oligonucleotide
<400> 196
   auguaaccaa gaguauucca u 21
<210> 197
   <211> 21
   <212> RNA
   <213> Artificial Sequence
<220>
   <223> Description of Artificial Sequence: Synthetic oligonucleotide
<400> 197
   auguaaccaa gaguauucca u 21
<210> 198
   <211> 21
   <212> RNA
   <213> Artificial Sequence
<220>
   <223> Description of Artificial Sequence: Synthetic oligonucleotide
<400> 198
   auguaaccaa gaguauucca u 21
<210> 199
   <211> 21
   <212> RNA
   <213> Artificial Sequence
<220>
   <223> Description of Artificial Sequence: Synthetic oligonucleotide
<400> 199
   auguaaccaa gaguauucca u 21
<210> 200
   <211> 21
   <212> RNA
   <213> Artificial Sequence
<220>
   <223> Description of Artificial Sequence: Synthetic oligonucleotide
<400> 200
   auguaaccaa gaguauucca u 21
<210> 201
   <211> 21
   <212> RNA
   <213> Artificial Sequence
<220>
   <223> Description of Artificial Sequence: Synthetic oligonucleotide
<400> 201
   auguaaccaa gaguauucca u 21
<210> 202
   <211> 21
   <212> RNA
   <213> Artificial Sequence
<220>
   <223> Description of Artificial Sequence: Synthetic oligonucleotide
<400> 202
   auguaaccaa gaguauucca u 21
<210> 203
   <211> 21
   <212> RNA
   <213> Artificial Sequence
<220>
   <223> Description of Artificial Sequence: Synthetic oligonucleotide
<400> 203
   auguaaccaa gaguauucca u 21
<210> 204
   <211> 21
   <212> RNA
   <213> Artificial Sequence
<220>
   <223> Description of Artificial Sequence: Synthetic oligonucleotide
<400> 204
   auguaaccaa gaguauucca u 21
<210> 205
   <211> 21
   <212> RNA
   <213> Artificial Sequence
<220>
   <223> Description of Artificial Sequence: Synthetic oligonucleotide
<400> 205
   auguaaccaa gaguauucca u 21
<210> 206
   <211> 21
   <212> RNA
   <213> Artificial Sequence
<220>
   <223> Description of Artificial Sequence: Synthetic oligonucleotide
<400> 206
   auguaaccaa gaguauucca u 21
<210> 207
   <211> 21
   <212> RNA
   <213> Artificial Sequence
<220>
   <223> Description of Artificial Sequence: Synthetic oligonucleotide
<400> 207
   auguaaccaa gaguauucca u 21
<210> 208
   <211> 21
   <212> RNA
   <213> Artificial Sequence
<220>
   <223> Description of Artificial Sequence: Synthetic oligonucleotide
<400> 208
   auguaaccaa gaguauucca u 21
<210> 209
   <211> 21
   <212> RNA
   <213> Artificial Sequence
<220>
   <223> Description of Artificial Sequence: Synthetic oligonucleotide
<400> 209
   auguaaccaa gaguauucca u 21
<210> 210
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 210
   auguaaccaa gagtattcca u 21
<210> 211
   <211> 21
   <212> RNA
   <213> Artificial Sequence
<220>
   <223> Description of Artificial Sequence: Synthetic oligonucleotide
<400> 211
   auguaaccaa gaguauucca u 21
<210> 212
   <211> 21
   <212> RNA
   <213> Artificial Sequence
<220>
   <223> Description of Artificial Sequence: Synthetic oligonucleotide
<400> 212
   auguaaccaa gaguauucca u 21
<210> 213
   <211> 21
   <212> RNA
   <213> Artificial Sequence
<220>
   <223> Description of Artificial Sequence: Synthetic oligonucleotide
<400> 213
   auguaaccaa gaguauucca u 21
<210> 214
   <211> 21
   <212> RNA
   <213> Artificial Sequence
<220>
   <223> Description of Artificial Sequence: Synthetic oligonucleotide
<400> 214
   auguaaccaa gaguauucca u 21
<210> 215
   <211> 21
   <212> RNA
   <213> Artificial Sequence
<220>
   <223> Description of Artificial Sequence: Synthetic oligonucleotide
<400> 215
   auguaaccaa gaguauucca u 21
<210> 216
   <211> 21
   <212> RNA
   <213> Artificial Sequence
<220>
   <223> Description of Artificial Sequence: Synthetic oligonucleotide
<400> 216
   auguaaccaa gaguauucca u 21
<210> 217
   <211> 21
   <212> RNA
   <213> Artificial Sequence
<220>
   <223> Description of Artificial Sequence: Synthetic oligonucleotide
<400> 217
   auguaaccaa gaguauucca u 21
<210> 218
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 218
   auguaaccaa gaguautcca u 21
<210> 219
   <211> 21
   <212> RNA
   <213> Artificial Sequence
<220>
   <223> Description of Artificial Sequence: Synthetic oligonucleotide
<400> 219
   auguaaccaa gaguauucca u 21
<210> 220
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 220
   auguaaccaa gaguattcca u 21
<210> 221
   <211> 21
   <212> RNA
   <213> Artificial Sequence
<220>
   <223> Description of Artificial Sequence: Synthetic oligonucleotide
<400> 221
   auguaaccaa gaguauucca u 21
<210> 222
   <211> 21
   <212> RNA
   <213> Artificial Sequence
<220>
   <223> Description of Artificial Sequence: Synthetic oligonucleotide
<400> 222
   auguaaccaa gaguauucca u 21
<210> 223
   <211> 21
   <212> RNA
   <213> Artificial Sequence
<220>
   <223> Description of Artificial Sequence: Synthetic oligonucleotide
<400> 223
   auguaaccaa gaguauucca u 21
<210> 224
   <211> 21
   <212> RNA
   <213> Artificial Sequence
<220>
   <223> Description of Artificial Sequence: Synthetic oligonucleotide
<400> 224
   auguaaccaa gaguauucca u 21
<210> 225
   <211> 21
   <212> RNA
   <213> Artificial Sequence
<220>
   <223> Description of Artificial Sequence: Synthetic oligonucleotide
<400> 225
   auguaaccaa gaguauucca u 21
<210> 226
   <211> 21
   <212> RNA
   <213> Artificial Sequence
<220>
   <223> Description of Artificial Sequence: Synthetic oligonucleotide
<400> 226
   auguaaccaa gaguauucca u 21
<210> 227
   <211> 21
   <212> RNA
   <213> Artificial Sequence
<220>
   <223> Description of Artificial Sequence: Synthetic oligonucleotide
<400> 227
   auguaaccaa gaguauucca u 21
<210> 228
   <211> 21
   <212> RNA
   <213> Artificial Sequence
<220>
   <223> Description of Artificial Sequence: Synthetic oligonucleotide
<400> 228
   auguaaccaa gaguauucca u 21
<210> 229
   <211> 21
   <212> RNA
   <213> Artificial Sequence
<220>
   <223> Description of Artificial Sequence: Synthetic oligonucleotide
<400> 229
   auguaaccaa gaguauucca u 21
<210> 230
   <211> 21
   <212> RNA
   <213> Artificial Sequence
<220>
   <223> Description of Artificial Sequence: Synthetic oligonucleotide
<400> 230
   auguaaccaa gaguauucca u 21
<210> 231
   <211> 21
   <212> RNA
   <213> Artificial Sequence
<220>
   <223> Description of Artificial Sequence: Synthetic oligonucleotide
<400> 231
   auguaaccaa gaguauucca u 21
<210> 232
   <211> 21
   <212> RNA
   <213> Artificial Sequence
<220>
   <223> Description of Artificial Sequence: Synthetic oligonucleotide
<400> 232
   auguaaccaa gaguauucca u 21
<210> 233
   <211> 21
   <212> RNA
   <213> Artificial Sequence
<220>
   <223> Description of Artificial Sequence: Synthetic oligonucleotide
<400> 233
   auguaaccaa gaguauucca u 21
<210> 234
   <211> 21
   <212> RNA
   <213> Artificial Sequence
<220>
   <223> Description of Artificial Sequence: Synthetic oligonucleotide
<400> 234
   auguaaccaa gaguauucca u 21
<210> 235
   <211> 21
   <212> RNA
   <213> Artificial Sequence
<220>
   <223> Description of Artificial Sequence: Synthetic oligonucleotide
<400> 235
   auguaaccaa gaguauucca u 21
<210> 236
   <211> 21
   <212> RNA
   <213> Artificial Sequence
<220>
   <223> Description of Artificial Sequence: Synthetic oligonucleotide
<400> 236
   auguaaccaa gaguauucca u 21
<210> 237
   <211> 21
   <212> RNA
   <213> Artificial Sequence
<220>
   <223> Description of Artificial Sequence: Synthetic oligonucleotide
<400> 237
   auguaaccaa gaguauucca u 21
<210> 238
   <211> 21
   <212> RNA
   <213> Artificial Sequence
<220>
   <223> Description of Artificial Sequence: Synthetic oligonucleotide
<400> 238
   auguaaccaa gaguauucca u 21
<210> 239
   <211> 21
   <212> RNA
   <213> Artificial Sequence
<220>
   <223> Description of Artificial Sequence: Synthetic oligonucleotide
<400> 239
   auguaaccaa gaguauucca u 21
<210> 240
   <211> 21
   <212> RNA
   <213> Artificial Sequence
<220>
   <223> Description of Artificial Sequence: Synthetic oligonucleotide
<400> 240
   auguaaccaa gaguauucca u 21
<210> 241
   <211> 21
   <212> RNA
   <213> Artificial Sequence
<220>
   <223> Description of Artificial Sequence: Synthetic oligonucleotide
<400> 241
   auguaaccaa gaguauucca u 21
<210> 242
   <211> 21
   <212> RNA
   <213> Artificial Sequence
<220>
   <223> Description of Artificial Sequence: Synthetic oligonucleotide
<400> 242
   auguaaccaa gaguauucca u 21
<210> 243
   <211> 21
   <212> RNA
   <213> Artificial Sequence
<220>
   <223> Description of Artificial Sequence: Synthetic oligonucleotide
<400> 243
   auguaaccaa gaguauucca u 21
<210> 244
   <211> 21
   <212> RNA
   <213> Artificial Sequence
<220>
   <223> Description of Artificial Sequence: Synthetic oligonucleotide
<400> 244
   auguaaccaa gaguauucca u 21
<210> 245
   <211> 21
   <212> RNA
   <213> Artificial Sequence
<220>
   <223> Description of Artificial Sequence: Synthetic oligonucleotide
<400> 245
   auguaaccaa gaguauucca u 21
<210> 246
   <211> 21
   <212> RNA
   <213> Artificial Sequence
<220>
   <223> Description of Artificial Sequence: Synthetic oligonucleotide
<400> 246
   auguaaccaa gaguauucca u 21
<210> 247
   <211> 21
   <212> RNA
   <213> Artificial Sequence
<220>
   <223> Description of Artificial Sequence: Synthetic oligonucleotide
<400> 247
   auguaaccaa gaguauucca u 21
<210> 248
   <211> 21
   <212> RNA
   <213> Artificial Sequence
<220>
   <223> Description of Artificial Sequence: Synthetic oligonucleotide
<400> 248
   auguaaccaa gaguauucca u 21
<210> 249
   <211> 21
   <212> RNA
   <213> Artificial Sequence
<220>
   <223> Description of Artificial Sequence: Synthetic oligonucleotide
<400> 249
   auguaaccaa gaguauucca u 21
<210> 250
   <211> 21
   <212> RNA
   <213> Artificial Sequence
<220>
   <223> Description of Artificial Sequence: Synthetic oligonucleotide
<400> 250
   auguaaccaa gaguauucca u 21
<210> 251
   <211> 21
   <212> RNA
   <213> Artificial Sequence
<220>
   <223> Description of Artificial Sequence: Synthetic oligonucleotide
<400> 251
   auguaaccaa gaguauucca u 21
<210> 252
   <211> 21
   <212> RNA
   <213> Artificial Sequence
<220>
   <223> Description of Artificial Sequence: Synthetic oligonucleotide
<400> 252
   auguaaccaa gaguauucca u 21
<210> 253
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 253
   aacaauguuc uugcucuaua a 21
<210> 254
   <211> 21
   <212> RNA
   <213> Artificial Sequence
<220>
   <223> Description of Artificial Sequence: Synthetic oligonucleotide
<400> 254
   aacaguguuc uugcucuaua a 21
<210> 255
   <211> 21
   <212> RNA
   <213> Artificial Sequence
<220>
   <223> Description of Artificial Sequence: Synthetic oligonucleotide
<400> 255
   aacaguguuc uugcucuaua a 21
<210> 256
   <211> 21
   <212> RNA
   <213> Artificial Sequence
<220>
   <223> Description of Artificial Sequence: Synthetic oligonucleotide
<400> 256
   aacaguguuc uugcucuaua a 21
<210> 257
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 257
   caguguucuu gcucuauaat t 21
<210> 258
   <211> 21
   <212> RNA
   <213> Artificial Sequence
<220>
   <223> Description of Artificial Sequence: Synthetic oligonucleotide
<400> 258
   aacaguguuc uugcucuaua a 21
<210> 259
   <211> 21
   <212> RNA
   <213> Artificial Sequence
<220>
   <223> Description of Artificial Sequence: Synthetic oligonucleotide
<400> 259
   aacaguguuc uugcucuaua a 21
<210> 260
   <211> 21
   <212> RNA
   <213> Artificial Sequence
<220>
   <223> Description of Artificial Sequence: Synthetic oligonucleotide
<400> 260
   aacaguguuc uugcucuaua a 21
<210> 261
   <211> 21
   <212> RNA
   <213> Artificial Sequence
<220>
   <223> Description of Artificial Sequence: Synthetic oligonucleotide
<400> 261
   aacaguguuc uugcucuaua a 21
<210> 262
   <211> 21
   <212> RNA
   <213> Artificial Sequence
<220>
   <223> Description of Artificial Sequence: Synthetic oligonucleotide
<400> 262
   aacaguguuc uugcucuaua a 21
<210> 263
   <211> 21
   <212> RNA
   <213> Artificial Sequence
<220>
   <223> Description of Artificial Sequence: Synthetic oligonucleotide
<400> 263
   aacaguguuc uugcucuaua a 21
<210> 264
   <211> 21
   <212> RNA
   <213> Artificial Sequence
<220>
   <223> Description of Artificial Sequence: Synthetic oligonucleotide
<400> 264
   aacaguguuc uugcucuaua a 21
<210> 265
   <211> 21
   <212> RNA
   <213> Artificial Sequence
<220>
   <223> Description of Artificial Sequence: Synthetic oligonucleotide
<400> 265
   aacaguguuc uugcucuaua a 21
<210> 266
   <211> 21
   <212> RNA
   <213> Artificial Sequence
<220>
   <223> Description of Artificial Sequence: Synthetic oligonucleotide
<400> 266
   aacaguguuc uugcucuaua a 21
<210> 267
   <211> 21
   <212> RNA
   <213> Artificial Sequence
<220>
   <223> Description of Artificial Sequence: Synthetic oligonucleotide
<400> 267
   aacaguguuc uugcucuaua a 21
<210> 268
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 268
   caguguucuu gcucuauaat t 21
<210> 269
   <211> 21
   <212> RNA
   <213> Artificial Sequence
<220>
   <223> Description of Artificial Sequence: Synthetic oligonucleotide
<400> 269
   aacaguguuc uugcucuaua a 21
<210> 270
   <211> 21
   <212> RNA
   <213> Artificial Sequence
<220>
   <223> Description of Artificial Sequence: Synthetic oligonucleotide
<400> 270
   aacaguguuc uugcucuaua a 21
<210> 271
   <211> 21
   <212> RNA
   <213> Artificial Sequence
<220>
   <223> Description of Artificial Sequence: Synthetic oligonucleotide
<400> 271
   aacaguguuc uugcucuaua a 21
<210> 272
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 272
   aacaauguuc uugcucuaua a 21
<210> 273
   <211> 21
   <212> RNA
   <213> Artificial Sequence
<220>
   <223> Description of Artificial Sequence: Synthetic oligonucleotide
<400> 273
   aacaguguuc uugcucuaua a 21
<210> 274
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 274
   caguguucuu gcucuauaat t 21
<210> 275
   <211> 21
   <212> RNA
   <213> Artificial Sequence
<220>
   <223> Description of Artificial Sequence: Synthetic oligonucleotide
<400> 275
   aacaguguuc uugcucuaua a 21
<210> 276
   <211> 21
   <212> RNA
   <213> Artificial Sequence
<220>
   <223> Description of Artificial Sequence: Synthetic oligonucleotide
<400> 276
   aacaguguuc uugcucuaua a 21
<210> 277
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 277
   caguguucuu gcucuauaat t 21
<210> 278
   <211> 21
   <212> RNA
   <213> Artificial Sequence
<220>
   <223> Description of Artificial Sequence: Synthetic oligonucleotide
<400> 278
   aacaguguuc uugcucuaua a 21
<210> 279
   <211> 21
   <212> RNA
   <213> Artificial Sequence
<220>
   <223> Description of Artificial Sequence: Synthetic oligonucleotide
<400> 279
   aacaguguuc uugcucuaua a 21
<210> 280
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 280
   caguguucuu gcucuauaat t 21
<210> 281
   <211> 21
   <212> RNA
   <213> Artificial Sequence
<220>
   <223> Description of Artificial Sequence: Synthetic oligonucleotide
<400> 281
   aacaguguuc uugcucuaua a 21
<210> 282
   <211> 21
   <212> RNA
   <213> Artificial Sequence
<220>
   <223> Description of Artificial Sequence: Synthetic oligonucleotide
<400> 282
   aacaguguuc uugcucuaua a 21
<210> 283
   <211> 21
   <212> RNA
   <213> Artificial Sequence
<220>
   <223> Description of Artificial Sequence: Synthetic oligonucleotide
<400> 283
   aacaguguuc uugcucuaua a 21
<210> 284
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 284
   caguguucuu gcucuauaat t 21
<210> 285
   <211> 21
   <212> RNA
   <213> Artificial Sequence
<220>
   <223> Description of Artificial Sequence: Synthetic oligonucleotide
<400> 285
   aacaguguuc uugcucuaua a 21
<210> 286
   <211> 21
   <212> RNA
   <213> Artificial Sequence
<220>
   <223> Description of Artificial Sequence: Synthetic oligonucleotide
<400> 286
   aacaguguuc uugcucuaua a 21
<210> 287
   <211> 21
   <212> RNA
   <213> Artificial Sequence
<220>
   <223> Description of Artificial Sequence: Synthetic oligonucleotide
<400> 287
   aacaguguuc uugcucuaua a 21
<210> 288
   <211> 21
   <212> RNA
   <213> Artificial Sequence
<220>
   <223> Description of Artificial Sequence: Synthetic oligonucleotide
<400> 288
   aacaguguuc uugcucuaua a 21
<210> 289
   <211> 21
   <212> RNA
   <213> Artificial Sequence
<220>
   <223> Description of Artificial Sequence: Synthetic oligonucleotide
<400> 289
   aacaguguuc uugcucuaua a 21
<210> 290
   <211> 21
   <212> RNA
   <213> Artificial Sequence
<220>
   <223> Description of Artificial Sequence: Synthetic oligonucleotide
<400> 290
   aacaguguuc uugcucuaua a 21
<210> 291
   <211> 21
   <212> RNA
   <213> Artificial Sequence
<220>
   <223> Description of Artificial Sequence: Synthetic oligonucleotide
<400> 291
   aacaguguuc uugcucuaua a 21
<210> 292
   <211> 21
   <212> RNA
   <213> Artificial Sequence
<220>
   <223> Description of Artificial Sequence: Synthetic oligonucleotide
<400> 292
   aacaguguuc uugcucuaua a 21
<210> 293
   <211> 21
   <212> RNA
   <213> Artificial Sequence
<220>
   <223> Description of Artificial Sequence: Synthetic oligonucleotide
<400> 293
   aacaguguuc uugcucuaua a 21
<210> 294
   <211> 21
   <212> RNA
   <213> Artificial Sequence
<220>
   <223> Description of Artificial Sequence: Synthetic oligonucleotide
<400> 294
   aacaguguuc uugcucuaua a 21
<210> 295
   <211> 21
   <212> RNA
   <213> Artificial Sequence
<220>
   <223> Description of Artificial Sequence: Synthetic oligonucleotide
<400> 295
   aacaguguuc uugcucuaua a 21
<210> 296
   <211> 21
   <212> RNA
   <213> Artificial Sequence
<220>
   <223> Description of Artificial Sequence: Synthetic oligonucleotide
<400> 296
   aacaguguuc uugcucuaua a 21
<210> 297
   <211> 21
   <212> RNA
   <213> Artificial Sequence
<220>
   <223> Description of Artificial Sequence: Synthetic oligonucleotide
<400> 297
   aacaguguuc uugcucuaua a 21
<210> 298
   <211> 21
   <212> RNA
   <213> Artificial Sequence
<220>
   <223> Description of Artificial Sequence: Synthetic oligonucleotide
<400> 298
   aacaguguuc uugcucuaua a 21
<210> 299
   <211> 21
   <212> RNA
   <213> Artificial Sequence
<220>
   <223> Description of Artificial Sequence: Synthetic oligonucleotide
<400> 299
   aacaguguuc uugcucuaua a 21
<210> 300
   <211> 21
   <212> RNA
   <213> Artificial Sequence
<220>
   <223> Description of Artificial Sequence: Synthetic oligonucleotide
<400> 300
   aacaguguuc uugcucuaua a 21
<210> 301
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 301
   aacagtguuc tugctcuata a 21
<210> 302
   <211> 21
   <212> RNA
   <213> Artificial Sequence
<220>
   <223> Description of Artificial Sequence: Synthetic oligonucleotide
<400> 302
   aacaguguuc uugcucuaua a 21
<210> 303
   <211> 21
   <212> RNA
   <213> Artificial Sequence
<220>
   <223> Description of Artificial Sequence: Synthetic oligonucleotide
<400> 303
   aacaguguuc uugcucuaua a 21
<210> 304
   <211> 21
   <212> RNA
   <213> Artificial Sequence
<220>
   <223> Description of Artificial Sequence: Synthetic oligonucleotide
<400> 304
   aacaguguuc uugcucuaua a 21
<210> 305
   <211> 21
   <212> RNA
   <213> Artificial Sequence
<220>
   <223> Description of Artificial Sequence: Synthetic oligonucleotide
<400> 305
   aacaguguuc uugcucuaua a 21
<210> 306
   <211> 21
   <212> RNA
   <213> Artificial Sequence
<220>
   <223> Description of Artificial Sequence: Synthetic oligonucleotide
<400> 306
   aacaguguuc uugcucuaua a 21
<210> 307
   <211> 21
   <212> RNA
   <213> Artificial Sequence
<220>
   <223> Description of Artificial Sequence: Synthetic oligonucleotide
<400> 307
   aacaguguuc uugcucuaua a 21
<210> 308
   <211> 21
   <212> RNA
   <213> Artificial Sequence
<220>
   <223> Description of Artificial Sequence: Synthetic oligonucleotide
<400> 308
   aacaguguuc uugcucuaua a 21
<210> 309
   <211> 21
   <212> RNA
   <213> Artificial Sequence
<220>
   <223> Description of Artificial Sequence: Synthetic oligonucleotide
<400> 309
   aacaguguuc uugcucuaua a 21
<210> 310
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 310
   aacagugtuc ttgctcuata a 21
<210> 311
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 311
   aacaguguuc uugcucuaua a 21
<210> 312
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 312
   aacaguguuc utgcucuaua a 21
<210> 313
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 313
   aacaguguuc uugcucuaua a 21
<210> 314
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 314
   aacaguguuc utgcucuaua a 21
<210> 315
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 315
   aacaauguuc utgcucuaua a 21
<210> 316
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 316
   aacaauguuc uugcucuata a 21
<210> 317
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 317
   aacaguguuc uugcucuaua a 21
<210> 318
   <211> 21
   <212> RNA
   <213> Artificial Sequence
<220>
   <223> Description of Artificial Sequence: Synthetic oligonucleotide
<400> 318
   aacaguguuc uugcucuaua a 21
<210> 319
   <211> 21
   <212> RNA
   <213> Artificial Sequence
<220>
   <223> Description of Artificial Sequence: Synthetic oligonucleotide
<400> 319
   aacaguguuc uugcucuaua a 21
<210> 320
   <211> 21
   <212> RNA
   <213> Artificial Sequence
<220>
   <223> Description of Artificial Sequence: Synthetic oligonucleotide
<400> 320
   aacaguguuc uugcucuaua a 21
<210> 321
   <211> 21
   <212> RNA
   <213> Artificial Sequence
<220>
   <223> Description of Artificial Sequence: Synthetic oligonucleotide
<400> 321
   aacaguguuc uugcucuaua a 21
<210> 322
   <211> 21
   <212> RNA
   <213> Artificial Sequence
<220>
   <223> Description of Artificial Sequence: Synthetic oligonucleotide
<400> 322
   aacaguguuc uugcucuaua a 21
<210> 323
   <211> 21
   <212> RNA
   <213> Artificial Sequence
<220>
   <223> Description of Artificial Sequence: Synthetic oligonucleotide
<400> 323
   aacaguguuc uugcucuaua a 21
<210> 324
   <211> 21
   <212> RNA
   <213> Artificial Sequence
<220>
   <223> Description of Artificial Sequence: Synthetic oligonucleotide
<400> 324
   aacaguguuc uugcucuaua a 21
<210> 325
   <211> 21
   <212> RNA
   <213> Artificial Sequence
<220>
   <223> Description of Artificial Sequence: Synthetic oligonucleotide
<400> 325
   aacaguguuc uugcucuaua a 21
<210> 326
   <211> 21
   <212> RNA
   <213> Artificial Sequence
<220>
   <223> Description of Artificial Sequence: Synthetic oligonucleotide
<400> 326
   aacaguguuc uugcucuaua a 21
<210> 327
   <211> 21
   <212> RNA
   <213> Artificial Sequence
<220>
   <223> Description of Artificial Sequence: Synthetic oligonucleotide
<400> 327
   aacaguguuc uugcucuaua a 21
<210> 328
   <211> 21
   <212> RNA
   <213> Artificial Sequence
<220>
   <223> Description of Artificial Sequence: Synthetic oligonucleotide
<400> 328
   aacaguguuc uugcucuaua a 21
<210> 329
   <211> 21
   <212> RNA
   <213> Artificial Sequence
<220>
   <223> Description of Artificial Sequence: Synthetic oligonucleotide
<400> 329
   aacaguguuc uugcucuaua a 21
<210> 330
   <211> 21
   <212> RNA
   <213> Artificial Sequence
<220>
   <223> Description of Artificial Sequence: Synthetic oligonucleotide
<400> 330
   aacaguguuc uugcucuaua a 21
<210> 331
   <211> 21
   <212> RNA
   <213> Artificial Sequence
<220>
   <223> Description of Artificial Sequence: Synthetic oligonucleotide
<400> 331
   aacaguguuc uugcucuaua a 21
<210> 332
   <211> 21
   <212> RNA
   <213> Artificial Sequence
<220>
   <223> Description of Artificial Sequence: Synthetic oligonucleotide
<400> 332
   aacaguguuc uugcucuaua a 21
<210> 333
   <211> 21
   <212> RNA
   <213> Artificial Sequence
<220>
   <223> Description of Artificial Sequence: Synthetic oligonucleotide
<400> 333
   aacaguguuc uugcucuaua a 21
<210> 334
   <211> 21
   <212> RNA
   <213> Artificial Sequence
<220>
   <223> Description of Artificial Sequence: Synthetic oligonucleotide
<400> 334
   aacaguguuc uugcucuaua a 21
<210> 335
   <211> 21
   <212> RNA
   <213> Artificial Sequence
<220>
   <223> Description of Artificial Sequence: Synthetic oligonucleotide
<400> 335
   aacaguguuc uugcucuaua a 21
<210> 336
   <211> 21
   <212> RNA
   <213> Artificial Sequence
<220>
   <223> Description of Artificial Sequence: Synthetic oligonucleotide
<400> 336
   aacaguguuc uugcucuaua a 21
<210> 337
   <211> 21
   <212> RNA
   <213> Artificial Sequence
<220>
   <223> Description of Artificial Sequence: Synthetic oligonucleotide
<400> 337
   aacaguguuc uugcucuaua a 21
<210> 338
   <211> 21
   <212> RNA
   <213> Artificial Sequence
<220>
   <223> Description of Artificial Sequence: Synthetic oligonucleotide
<400> 338
   aacaguguuc uugcucuaua a 21
<210> 339
   <211> 21
   <212> RNA
   <213> Artificial Sequence
<220>
   <223> Description of Artificial Sequence: Synthetic oligonucleotide
<400> 339
   aacaguguuc uugcucuaua a 21
<210> 340
   <211> 21
   <212> RNA
   <213> Artificial Sequence
<220>
   <223> Description of Artificial Sequence: Synthetic oligonucleotide
<400> 340
   aacaguguuc uugcucuaua a 21
<210> 341
   <211> 21
   <212> RNA
   <213> Artificial Sequence
<220>
   <223> Description of Artificial Sequence: Synthetic oligonucleotide
<400> 341
   aacaguguuc uugcucuaua a 21
<210> 342
   <211> 21
   <212> RNA
   <213> Artificial Sequence
<220>
   <223> Description of Artificial Sequence: Synthetic oligonucleotide
<400> 342
   aacaguguuc uugcucuaua a 21
<210> 343
   <211> 21
   <212> RNA
   <213> Artificial Sequence
<220>
   <223> Description of Artificial Sequence: Synthetic oligonucleotide
<400> 343
   aacaguguuc uugcucuaua a 21
<210> 344
   <211> 21
   <212> RNA
   <213> Artificial Sequence
<220>
   <223> Description of Artificial Sequence: Synthetic oligonucleotide
<400> 344
   aacaguguuc uugcucuaua a 21
<210> 345
   <211> 21
   <212> RNA
   <213> Artificial Sequence
<220>
   <223> Description of Artificial Sequence: Synthetic oligonucleotide
<400> 345
   aacaguguuc uugcucuaua a 21
<210> 346
   <211> 21
   <212> RNA
   <213> Artificial Sequence
<220>
   <223> Description of Artificial Sequence: Synthetic oligonucleotide
<400> 346
   aacaguguuc uugcucuaua a 21
<210> 347
   <211> 21
   <212> RNA
   <213> Artificial Sequence
<220>
   <223> Description of Artificial Sequence: Synthetic oligonucleotide
<400> 347
   aacaguguuc uugcucuaua a 21
<210> 348
   <211> 21
   <212> RNA
   <213> Artificial Sequence
<220>
   <223> Description of Artificial Sequence: Synthetic oligonucleotide
<400> 348
   aacaguguuc uugcucuaua a 21
<210> 349
   <211> 21
   <212> RNA
   <213> Artificial Sequence
<220>
   <223> Description of Artificial Sequence: Synthetic oligonucleotide
<400> 349
   aacaguguuc uugcucuaua a 21
<210> 350
   <211> 21
   <212> RNA
   <213> Artificial Sequence
<220>
   <223> Description of Artificial Sequence: Synthetic oligonucleotide
<400> 350
   aacaguguuc uugcucuaua a 21
<210> 351
   <211> 21
   <212> RNA
   <213> Artificial Sequence
<220>
   <223> Description of Artificial Sequence: Synthetic oligonucleotide
<400> 351
   aacaguguuc uugcucuaua a 21
<210> 352
   <211> 21
   <212> RNA
   <213> Artificial Sequence
<220>
   <223> Description of Artificial Sequence: Synthetic oligonucleotide
<400> 352
   aacaguguuc uugcucuaua a 21
<210> 353
   <211> 21
   <212> RNA
   <213> Artificial Sequence
<220>
   <223> Description of Artificial Sequence: Synthetic oligonucleotide
<400> 353
   aacaguguuc uugcucuaua a 21
<210> 354
   <211> 21
   <212> RNA
   <213> Artificial Sequence
<220>
   <223> Description of Artificial Sequence: Synthetic oligonucleotide
<400> 354
   aacaguguuc uugcucuaua a 21
<210> 355
   <211> 21
   <212> RNA
   <213> Artificial Sequence
<220>
   <223> Description of Artificial Sequence: Synthetic oligonucleotide
<400> 355
   aacaguguuc uugcucuaua a 21
<210> 356
   <211> 21
   <212> RNA
   <213> Artificial Sequence
<220>
   <223> Description of Artificial Sequence: Synthetic oligonucleotide
<400> 356
   aacaguguuc uugcucuaua a 21
<210> 357
   <211> 21
   <212> RNA
   <213> Artificial Sequence
<220>
   <223> Description of Artificial Sequence: Synthetic oligonucleotide
<400> 357
   aacaguguuc uugcucuaua a 21
<210> 358
   <211> 21
   <212> RNA
   <213> Artificial Sequence
<220>
   <223> Description of Artificial Sequence: Synthetic oligonucleotide
<400> 358
   aacaguguuc uugcucuaua a 21
<210> 359
   <211> 21
   <212> RNA
   <213> Artificial Sequence
<220>
   <223> Description of Artificial Sequence: Synthetic oligonucleotide
<400> 359
   aacaguguuc uugcucuaua a 21
<210> 360
   <211> 21
   <212> RNA
   <213> Artificial Sequence
<220>
   <223> Description of Artificial Sequence: Synthetic oligonucleotide
<400> 360
   aacaguguuc uugcucuaua a 21
<210> 361
   <211> 21
   <212> RNA
   <213> Artificial Sequence
<220>
   <223> Description of Artificial Sequence: Synthetic oligonucleotide
<400> 361
   aacaguguuc uugcucuaua a 21
<210> 362
   <211> 21
   <212> RNA
   <213> Artificial Sequence
<220>
   <223> Description of Artificial Sequence: Synthetic oligonucleotide
<400> 362
   aacaguguuc uugcucuaua a 21
<210> 363
   <211> 21
   <212> RNA
   <213> Artificial Sequence
<220>
   <223> Description of Artificial Sequence: Synthetic oligonucleotide
<400> 363
   aacaguguuc uugcucuaua a 21
<210> 364
   <211> 21
   <212> RNA
   <213> Artificial Sequence
<220>
   <223> Description of Artificial Sequence: Synthetic oligonucleotide
<400> 364
   aacaguguuc uugcucuaua a 21
<210> 365
   <211> 21
   <212> RNA
   <213> Artificial Sequence
<220>
   <223> Description of Artificial Sequence: Synthetic oligonucleotide
<400> 365
   aacaguguuc uugcucuaua a 21
<210> 366
   <211> 21
   <212> RNA
   <213> Artificial Sequence
<220>
   <223> Description of Artificial Sequence: Synthetic oligonucleotide
<400> 366
   aacaguguuc uugcucuaua a 21
<210> 367
   <211> 21
   <212> RNA
   <213> Artificial Sequence
<220>
   <223> Description of Artificial Sequence: Synthetic oligonucleotide
<400> 367
   aacaguguuc uugcucuaua a 21
<210> 368
   <211> 21
   <212> RNA
   <213> Artificial Sequence
<220>
   <223> Description of Artificial Sequence: Synthetic oligonucleotide
<400> 368
   aacaguguuc uugcucuaua a 21
<210> 369
   <211> 21
   <212> RNA
   <213> Artificial Sequence
<220>
   <223> Description of Artificial Sequence: Synthetic oligonucleotide
<400> 369
   aacaguguuc uugcucuaua a 21
<210> 370
   <211> 21
   <212> RNA
   <213> Artificial Sequence
<220>
   <223> Description of Artificial Sequence: Synthetic oligonucleotide
<400> 370
   aacaguguuc uugcucuaua a 21
<210> 371
   <211> 21
   <212> RNA
   <213> Artificial Sequence
<220>
   <223> Description of Artificial Sequence: Synthetic oligonucleotide
<400> 371
   aacaguguuc uugcucuaua a 21
<210> 372
   <211> 21
   <212> RNA
   <213> Artificial Sequence
<220>
   <223> Description of Artificial Sequence: Synthetic oligonucleotide
<400> 372
   aacaguguuc uugcucuaua a 21
<210> 373
   <211> 21
   <212> RNA
   <213> Artificial Sequence
<220>
   <223> Description of Artificial Sequence: Synthetic oligonucleotide
<400> 373
   aacaguguuc uugcucuaua a 21
<210> 374
   <211> 21
   <212> RNA
   <213> Artificial Sequence
<220>
   <223> Description of Artificial Sequence: Synthetic oligonucleotide
<400> 374
   aacaguguuc uugcucuaua a 21
<210> 375
   <211> 21
   <212> RNA
   <213> Artificial Sequence
<220>
   <223> Description of Artificial Sequence: Synthetic oligonucleotide
<400> 375
   aacaguguuc uugcucuaua a 21
<210> 376
   <211> 21
   <212> RNA
   <213> Artificial Sequence
<220>
   <223> Description of Artificial Sequence: Synthetic oligonucleotide
<400> 376
   aacaguguuc uugcucuaua a 21
<210> 377
   <211> 21
   <212> RNA
   <213> Artificial Sequence
<220>
   <223> Description of Artificial Sequence: Synthetic oligonucleotide
<400> 377
   aacaguguuc uugcucuaua a 21
<210> 378
   <211> 21
   <212> RNA
   <213> Artificial Sequence
<220>
   <223> Description of Artificial Sequence: Synthetic oligonucleotide
<400> 378
   aacaguguuc uugcucuaua a 21
<210> 379
   <211> 21
   <212> RNA
   <213> Artificial Sequence
<220>
   <223> Description of Artificial Sequence: Synthetic oligonucleotide
<400> 379
   aacaguguuc uugcucuaua a 21
<210> 380
   <211> 21
   <212> RNA
   <213> Artificial Sequence
<220>
   <223> Description of Artificial Sequence: Synthetic oligonucleotide
<400> 380
   aacaguguuc uugcucuaua a 21
<210> 381
   <211> 21
   <212> RNA
   <213> Artificial Sequence
<220>
   <223> Description of Artificial Sequence: Synthetic oligonucleotide
<400> 381
   aacaguguuc uugcucuaua a 21
<210> 382
   <211> 21
   <212> RNA
   <213> Artificial Sequence
<220>
   <223> Description of Artificial Sequence: Synthetic oligonucleotide
<400> 382
   aacaguguuc uugcucuaua a 21
<210> 383
   <211> 21
   <212> RNA
   <213> Artificial Sequence
<220>
   <223> Description of Artificial Sequence: Synthetic oligonucleotide
<400> 383
   aacaguguuc uugcucuaua a 21
<210> 384
   <211> 21
   <212> RNA
   <213> Artificial Sequence
<220>
   <223> Description of Artificial Sequence: Synthetic oligonucleotide
<400> 384
   aacaguguuc uugcucuaua a 21
<210> 385
   <211> 21
   <212> RNA
   <213> Artificial Sequence
<220>
   <223> Description of Artificial Sequence: Synthetic oligonucleotide
<400> 385
   aacaguguuc uugcucuaua a 21
<210> 386
   <211> 21
   <212> RNA
   <213> Artificial Sequence
<220>
   <223> Description of Artificial Sequence: Synthetic oligonucleotide
<400> 386
   aacaguguuc uugcucuaua a 21
<210> 387
   <211> 21
   <212> RNA
   <213> Artificial Sequence
<220>
   <223> Description of Artificial Sequence: Synthetic oligonucleotide
<400> 387
   aacaguguuc uugcucuaua a 21
<210> 388
   <211> 21
   <212> RNA
   <213> Artificial Sequence
<220>
   <223> Description of Artificial Sequence: Synthetic oligonucleotide
<400> 388
   aacaguguuc uugcucuaua a 21
<210> 389
   <211> 21
   <212> RNA
   <213> Artificial Sequence
<220>
   <223> Description of Artificial Sequence: Synthetic oligonucleotide
<400> 389
   aacaguguuc uugcucuaua a 21
<210> 390
   <211> 21
   <212> RNA
   <213> Artificial Sequence
<220>
   <223> Description of Artificial Sequence: Synthetic oligonucleotide
<400> 390
   aacaguguuc uugcucuaua a 21
<210> 391
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 391
   aacaguguuc uugcucuaua a 21
<210> 392
   <211> 21
   <212> RNA
   <213> Artificial Sequence
<220>
   <223> Description of Artificial Sequence: Synthetic oligonucleotide
<400> 392
   aacaguguuc uugcucuaua a 21
<210> 393
   <211> 21
   <212> RNA
   <213> Artificial Sequence
<220>
   <223> Description of Artificial Sequence: Synthetic oligonucleotide
<400> 393
   aacaguguuc uugcucuaua a 21
<210> 394
   <211> 21
   <212> RNA
   <213> Artificial Sequence
<220>
   <223> Description of Artificial Sequence: Synthetic oligonucleotide
<400> 394
   aacaguguuc uugcucuaua a 21
<210> 395
   <211> 21
   <212> RNA
   <213> Artificial Sequence
<220>
   <223> Description of Artificial Sequence: Synthetic oligonucleotide
<400> 395
   aacaguguuc uugcucuaua a 21
<210> 396
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 396
   aacaguguuc tugctctata a 21
<210> 397
   <211> 21
   <212> RNA
   <213> Artificial Sequence
<220>
   <223> Description of Artificial Sequence: Synthetic oligonucleotide
<400> 397
   aacaguguuc uugcucuaua a 21
<210> 398
   <211> 21
   <212> RNA
   <213> Artificial Sequence
<220>
   <223> Description of Artificial Sequence: Synthetic oligonucleotide
<400> 398
   aacaguguuc uugcucuaua a 21
<210> 399
   <211> 21
   <212> RNA
   <213> Artificial Sequence
<220>
   <223> Description of Artificial Sequence: Synthetic oligonucleotide
<400> 399
   aacaguguuc uugcucuaua a 21
<210> 400
   <211> 21
   <212> RNA
   <213> Artificial Sequence
<220>
   <223> Description of Artificial Sequence: Synthetic oligonucleotide
<400> 400
   aacaguguuc uugcucuaua a 21
<210> 401
   <211> 21
   <212> RNA
   <213> Artificial Sequence
<220>
   <223> Description of Artificial Sequence: Synthetic oligonucleotide
<400> 401
   aacaguguuc uugcucuaua a 21
<210> 402
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 402
   aacaauguuc uugcactaua a 21
<210> 403
   <211> 21
   <212> RNA
   <213> Artificial Sequence
<220>
   <223> Description of Artificial Sequence: Synthetic oligonucleotide
<400> 403
   aacaguguuc uugcucuaua a 21
<210> 404
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 404
   aacagugutc uugcuctaua a 21
<210> 405
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 405
   aacaauguuc uugcactaua a 21
<210> 406
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 406
   aacaauguuc uugcacuaua a 21
<210> 407
   <211> 21
   <212> RNA
   <213> Artificial Sequence
<220>
   <223> Description of Artificial Sequence: Synthetic oligonucleotide
<400> 407
   aacaguguuc uugcucuaua a 21
<210> 408
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 408
   aacaauguuc uugcacuata a 21
<210> 409
   <211> 21
   <212> RNA
   <213> Artificial Sequence
<220>
   <223> Description of Artificial Sequence: Synthetic oligonucleotide
<400> 409
   aacaguguuc uugcucuaua a 21
<210> 410
   <211> 21
   <212> RNA
   <213> Artificial Sequence
<220>
   <223> Description of Artificial Sequence: Synthetic oligonucleotide
<400> 410
   aacaguguuc uugcucuaua a 21
<210> 411
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 411
   aacaauguuc uugcacuata a 21
<210> 412
   <211> 21
   <212> RNA
   <213> Artificial Sequence
<220>
   <223> Description of Artificial Sequence: Synthetic oligonucleotide
<400> 412
   aacaguguuc uugcucuaua a 21
<210> 413
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 413
   aacaauguuc uugcactata a 21
<210> 414
   <211> 21
   <212> RNA
   <213> Artificial Sequence
<220>
   <223> Description of Artificial Sequence: Synthetic oligonucleotide
<400> 414
   aacaguguuc uugcucuaua a 21
<210> 415
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 415
   aacaauguuc uugcactata a 21
<210> 416
   <211> 21
   <212> RNA
   <213> Artificial Sequence
<220>
   <223> Description of Artificial Sequence: Synthetic oligonucleotide
<400> 416
   aacaguguuc uugcucuaua a 21
<210> 417
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 417
   aacagugtuc utgcucuaua a 21
<210> 418
   <211> 21
   <212> RNA
   <213> Artificial Sequence
<220>
   <223> Description of Artificial Sequence: Synthetic oligonucleotide
<400> 418
   aacaguguuc uugcucuaua a 21
<210> 419
   <211> 21
   <212> RNA
   <213> Artificial Sequence
<220>
   <223> Description of Artificial Sequence: Synthetic oligonucleotide
<400> 419
   aacaguguuc uugcucuaua a 21
<210> 420
   <211> 21
   <212> RNA
   <213> Artificial Sequence
<220>
   <223> Description of Artificial Sequence: Synthetic oligonucleotide
<400> 420
   aacaguguuc uugcucuaua a 21
<210> 421
   <211> 21
   <212> RNA
   <213> Artificial Sequence
<220>
   <223> Description of Artificial Sequence: Synthetic oligonucleotide
<400> 421
   aacaguguuc uugcucuaua a 21
<210> 422
   <211> 21
   <212> RNA
   <213> Artificial Sequence
<220>
   <223> Description of Artificial Sequence: Synthetic oligonucleotide
<400> 422
   aacaguguuc uugcucuaua a 21
<210> 423
   <211> 21
   <212> RNA
   <213> Artificial Sequence
<220>
   <223> Description of Artificial Sequence: Synthetic oligonucleotide
<400> 423
   aacaguguuc uugcucuaua a 21
<210> 424
   <211> 21
   <212> RNA
   <213> Artificial Sequence
<220>
   <223> Description of Artificial Sequence: Synthetic oligonucleotide
<400> 424
   aacaguguuc uugcucuaua a 21
<210> 425
   <211> 23
   <212> RNA
   <213> Artificial Sequence
<220>
   <223> Description of Artificial Sequence: Synthetic oligonucleotide
<400> 425
   auggaauacu cuugguuaca uga 23
<210> 426
   <211> 23
   <212> RNA
   <213> Artificial Sequence
<220>
   <223> Description of Artificial Sequence: Synthetic oligonucleotide
<400> 426
   auggaauacu cuugguuaca uga 23
<210> 427
   <211> 23
   <212> RNA
   <213> Artificial Sequence
<220>
   <223> Description of Artificial Sequence: Synthetic oligonucleotide
<400> 427
   auggaauacu cuugguuaca uga 23
<210> 428
   <211> 23
   <212> RNA
   <213> Artificial Sequence
<220>
   <223> Description of Artificial Sequence: Synthetic oligonucleotide
<400> 428
   auggaauacu cuugguuaca uga 23
<210> 429
   <211> 23
   <212> RNA
   <213> Artificial Sequence
<220>
   <223> Description of Artificial Sequence: Synthetic oligonucleotide
<400> 429
   auggaauacu cuugguuaca uga 23
<210> 430
   <211> 23
   <212> RNA
   <213> Artificial Sequence
<220>
   <223> Description of Artificial Sequence: Synthetic oligonucleotide
<400> 430
   auggaauacu cuugguuaca uga 23
<210> 431
   <211> 23
   <212> RNA
   <213> Artificial Sequence
<220>
   <223> Description of Artificial Sequence: Synthetic oligonucleotide
<400> 431
   auggaauacu cuugguuaca uga 23
<210> 432
   <211> 23
   <212> RNA
   <213> Artificial Sequence
<220>
   <223> Description of Artificial Sequence: Synthetic oligonucleotide
<400> 432
   auggaauacu cuugguuaca uga 23
<210> 433
   <211> 23
   <212> RNA
   <213> Artificial Sequence
<220>
   <223> Description of Artificial Sequence: Synthetic oligonucleotide
<400> 433
   auggaauacu cuugguuaca uga 23
<210> 434
   <211> 23
   <212> RNA
   <213> Artificial Sequence
<220>
   <223> Description of Artificial Sequence: Synthetic oligonucleotide
<400> 434
   ucuugguuac augaaauccc auc 23
<210> 435
   <211> 23
   <212> RNA
   <213> Artificial Sequence
<220>
   <223> Description of Artificial Sequence: Synthetic oligonucleotide
<400> 435
   ucuugguuac augaaauccc auc 23
<210> 436
   <211> 23
   <212> RNA
   <213> Artificial Sequence
<220>
   <223> Description of Artificial Sequence: Synthetic oligonucleotide
<400> 436
   auggaauacu cuugguuaca uga 23
<210> 437
   <211> 23
   <212> RNA
   <213> Artificial Sequence
<220>
   <223> Description of Artificial Sequence: Synthetic oligonucleotide
<400> 437
   ucuugguuac augaaauccc auc 23
<210> 438
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 438
   auggaauacu cuuggutaca tga 23
<210> 439
   <211> 23
   <212> RNA
   <213> Artificial Sequence
<220>
   <223> Description of Artificial Sequence: Synthetic oligonucleotide
<400> 439
   auggaauacu cuugguuaca uga 23
<210> 440
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 440
   auggaatacu cuugguuaca uga 23
<210> 441
   <211> 23
   <212> RNA
   <213> Artificial Sequence
<220>
   <223> Description of Artificial Sequence: Synthetic oligonucleotide
<400> 441
   auggaauacu cuugguuaca uga 23
<210> 442
   <211> 23
   <212> RNA
   <213> Artificial Sequence
<220>
   <223> Description of Artificial Sequence: Synthetic oligonucleotide
<400> 442
   ucuugguuac augaaauccc auc 23
<210> 443
   <211> 23
   <212> RNA
   <213> Artificial Sequence
<220>
   <223> Description of Artificial Sequence: Synthetic oligonucleotide
<400> 443
   auggaauacu cuugguuaca uga 23
<210> 444
   <211> 23
   <212> RNA
   <213> Artificial Sequence
<220>
   <223> Description of Artificial Sequence: Synthetic oligonucleotide
<400> 444
   auggaauacu cuugguuaca uga 23
<210> 445
   <211> 23
   <212> RNA
   <213> Artificial Sequence
<220>
   <223> Description of Artificial Sequence: Synthetic oligonucleotide
<400> 445
   auggaauacu cuugguuaca uga 23
<210> 446
   <211> 23
   <212> RNA
   <213> Artificial Sequence
<220>
   <223> Description of Artificial Sequence: Synthetic oligonucleotide
<400> 446
   auggaauacu cuugguuaca uga 23
<210> 447
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 447
   uctuggtuac augaaauccc atc 23
<210> 448
   <211> 23
   <212> RNA
   <213> Artificial Sequence
<220>
   <223> Description of Artificial Sequence: Synthetic oligonucleotide
<400> 448
   auggaauacu cuugguuaca uga 23
<210> 449
   <211> 23
   <212> RNA
   <213> Artificial Sequence
<220>
   <223> Description of Artificial Sequence: Synthetic oligonucleotide
<400> 449
   ucuugguuac augaaauccc auc 23
<210> 450
   <211> 23
   <212> RNA
   <213> Artificial Sequence
<220>
   <223> Description of Artificial Sequence: Synthetic oligonucleotide
<400> 450
   ucuugguuac augaaauccc auc 23
<210> 451
   <211> 23
   <212> RNA
   <213> Artificial Sequence
<220>
   <223> Description of Artificial Sequence: Synthetic oligonucleotide
<400> 451
   ucuugguuac augaaauccc auc 23
<210> 452
   <211> 23
   <212> RNA
   <213> Artificial Sequence
<220>
   <223> Description of Artificial Sequence: Synthetic oligonucleotide
<400> 452
   ucuugguuac augaaauccc auc 23
<210> 453
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 453
   atggaatact cuugguuaca uga 23
<210> 454
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 454
   auggaatacu cutggutaca tga 23
<210> 455
   <211> 23
   <212> RNA
   <213> Artificial Sequence
<220>
   <223> Description of Artificial Sequence: Synthetic oligonucleotide
<400> 455
   ucuugguuac augaaauccc auc 23
<210> 456
   <211> 23
   <212> RNA
   <213> Artificial Sequence
<220>
   <223> Description of Artificial Sequence: Synthetic oligonucleotide
<400> 456
   auggaauacu cuugguuaca uga 23
<210> 457
   <211> 23
   <212> RNA
   <213> Artificial Sequence
<220>
   <223> Description of Artificial Sequence: Synthetic oligonucleotide
<400> 457
   auggaauacu cuugguuaca uga 23
<210> 458
   <211> 23
   <212> RNA
   <213> Artificial Sequence
<220>
   <223> Description of Artificial Sequence: Synthetic oligonucleotide
<400> 458
   ucuugguuac augaaauccc auc 23
<210> 459
   <211> 23
   <212> RNA
   <213> Artificial Sequence
<220>
   <223> Description of Artificial Sequence: Synthetic oligonucleotide
<400> 459
   auggaauacu cuugguuaca uga 23
<210> 460
   <211> 23
   <212> RNA
   <213> Artificial Sequence
<220>
   <223> Description of Artificial Sequence: Synthetic oligonucleotide
<400> 460
   ucuugguuac augaaauccc auc 23
<210> 461
   <211> 23
   <212> RNA
   <213> Artificial Sequence
<220>
   <223> Description of Artificial Sequence: Synthetic oligonucleotide
<400> 461
   ucuugguuac augaaauccc auc 23
<210> 462
   <211> 23
   <212> RNA
   <213> Artificial Sequence
<220>
   <223> Description of Artificial Sequence: Synthetic oligonucleotide
<400> 462
   ucuugguuac augaaauccc auc 23
<210> 463
   <211> 23
   <212> RNA
   <213> Artificial Sequence
<220>
   <223> Description of Artificial Sequence: Synthetic oligonucleotide
<400> 463
   ucuugguuac augaaauccc auc 23
<210> 464
   <211> 23
   <212> RNA
   <213> Artificial Sequence
<220>
   <223> Description of Artificial Sequence: Synthetic oligonucleotide
<400> 464
   auggaauacu cuugguuaca uga 23
<210> 465
   <211> 23
   <212> RNA
   <213> Artificial Sequence
<220>
   <223> Description of Artificial Sequence: Synthetic oligonucleotide
<400> 465
   auggaauacu cuugguuaca uga 23
<210> 466
   <211> 23
   <212> RNA
   <213> Artificial Sequence
<220>
   <223> Description of Artificial Sequence: Synthetic oligonucleotide
<400> 466
   auggaauacu cuugguuaca uga 23
<210> 467
   <211> 23
   <212> RNA
   <213> Artificial Sequence
<220>
   <223> Description of Artificial Sequence: Synthetic oligonucleotide
<400> 467
   ucuugguuac augaaauccc auc 23
<210> 468
   <211> 23
   <212> RNA
   <213> Artificial Sequence
<220>
   <223> Description of Artificial Sequence: Synthetic oligonucleotide
<400> 468
   auggaauacu cuugguuaca uga 23
<210> 469
   <211> 23
   <212> RNA
   <213> Artificial Sequence
<220>
   <223> Description of Artificial Sequence: Synthetic oligonucleotide
<400> 469
   auggaauacu cuugguuaca uga 23
<210> 470
   <211> 23
   <212> RNA
   <213> Artificial Sequence
<220>
   <223> Description of Artificial Sequence: Synthetic oligonucleotide
<400> 470
   auggaauacu cuugguuaca uga 23
<210> 471
   <211> 23
   <212> RNA
   <213> Artificial Sequence
<220>
   <223> Description of Artificial Sequence: Synthetic oligonucleotide
<400> 471
   auggaauacu cuugguuaca uga 23
<210> 472
   <211> 23
   <212> RNA
   <213> Artificial Sequence
<220>
   <223> Description of Artificial Sequence: Synthetic oligonucleotide
<400> 472
   ucuugguuac augaaauccc auc 23
<210> 473
   <211> 23
   <212> RNA
   <213> Artificial Sequence
<220>
   <223> Description of Artificial Sequence: Synthetic oligonucleotide
<400> 473
   auggaauacu cuugguuaca uga 23
<210> 474
   <211> 23
   <212> RNA
   <213> Artificial Sequence
<220>
   <223> Description of Artificial Sequence: Synthetic oligonucleotide
<400> 474
   auggaauacu cuugguuaca uga 23
<210> 475
   <211> 23
   <212> RNA
   <213> Artificial Sequence
<220>
   <223> Description of Artificial Sequence: Synthetic oligonucleotide
<400> 475
   ucuugguuac augaaauccc auc 23
<210> 476
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 476
   auggaauact ctuggtuaca uga 23
<210> 477
   <211> 23
   <212> RNA
   <213> Artificial Sequence
<220>
   <223> Description of Artificial Sequence: Synthetic oligonucleotide
<400> 477
   auggaauacu cuugguuaca uga 23
<210> 478
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 478
   auggaauacu cuugguuaca uga 23
<210> 479
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 479
   atggaauacu cuugguuaca uga 23
<210> 480
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 480
   auggaatacu cuugguuaca uga 23
<210> 481
   <211> 23
   <212> RNA
   <213> Artificial Sequence
<220>
   <223> Description of Artificial Sequence: Synthetic oligonucleotide
<400> 481
   auggaauacu cuugguuaca uga 23
<210> 482
   <211> 23
   <212> RNA
   <213> Artificial Sequence
<220>
   <223> Description of Artificial Sequence: Synthetic oligonucleotide
<400> 482
   auggaauacu cuugguuaca uga 23
<210> 483
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 483
   auggaauact ctuggtuaca uga 23
<210> 484
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 484
   auggaatacu cuugguuaca uga 23
<210> 485
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 485
   atggaatact cuugguuaca tga 23
<210> 486
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 486
   atggaauact cuugguuaca uga 23
<210> 487
   <211> 23
   <212> RNA
   <213> Artificial Sequence
<220>
   <223> Description of Artificial Sequence: Synthetic oligonucleotide
<400> 487
   auggaauacu cuugguuaca uga 23
<210> 488
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 488
   atggaauact cutggutaca tga 23
<210> 489
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 489
   atggaatacu cuugguuaca uga 23
<210> 490
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 490
   auggaatacu cuugguuaca uga 23
<210> 491
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 491
   atggaatacu cuugguuaca uga 23
<210> 492
   <211> 23
   <212> RNA
   <213> Artificial Sequence
<220>
   <223> Description of Artificial Sequence: Synthetic oligonucleotide
<400> 492
   auggaauacu cuugguuaca uga 23
<210> 493
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 493
   auggaauacu cuugguuaca uga 23
<210> 494
   <211> 23
   <212> RNA
   <213> Artificial Sequence
<220>
   <223> Description of Artificial Sequence: Synthetic oligonucleotide
<400> 494
   auggaauacu cuugguuaca uga 23
<210> 495
   <211> 23
   <212> RNA
   <213> Artificial Sequence
<220>
   <223> Description of Artificial Sequence: Synthetic oligonucleotide
<400> 495
   auggaauacu cuugguuaca uga 23
<210> 496
   <211> 23
   <212> RNA
   <213> Artificial Sequence
<220>
   <223> Description of Artificial Sequence: Synthetic oligonucleotide
<400> 496
   ucuugguuac augaaauccc auc 23
<210> 497
   <211> 23
   <212> RNA
   <213> Artificial Sequence
<220>
   <223> Description of Artificial Sequence: Synthetic oligonucleotide
<400> 497
   auggaauacu cuugguuaca uga 23
<210> 498
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 498
   auggaatacu cuugguuaca uga 23
<210> 499
   <211> 23
   <212> RNA
   <213> Artificial Sequence
<220>
   <223> Description of Artificial Sequence: Synthetic oligonucleotide
<400> 499
   auggaauacu cuugguuaca uga 23
<210> 500
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 500
   auggaatacu cuugguuaca uga 23
<210> 501
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 501
   auggaatacu cuugguuaca uga 23
<210> 502
   <211> 23
   <212> RNA
   <213> Artificial Sequence
<220>
   <223> Description of Artificial Sequence: Synthetic oligonucleotide
<400> 502
   auggaauacu cuugguuaca uga 23
<210> 503
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 503
   auggaatacu cuugguuaca uga 23
<210> 504
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 504
   auggaatacu cuugguuaca uga 23
<210> 505
   <211> 23
   <212> RNA
   <213> Artificial Sequence
<220>
   <223> Description of Artificial Sequence: Synthetic oligonucleotide
<400> 505
   auggaauacu cuugguuaca uga 23
<210> 506
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 506
   atggaauacu cuugguuaca uga 23
<210> 507
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 507
   auggaauacu cuuggutaca uga 23
<210> 508
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 508
   atggaatacu cuugguuaca uga 23
<210> 509
   <211> 23
   <212> RNA
   <213> Artificial Sequence
<220>
   <223> Description of Artificial Sequence: Synthetic oligonucleotide
<400> 509
   auggaauacu cuugguuaca uga 23
<210> 510
   <211> 23
   <212> RNA
   <213> Artificial Sequence
<220>
   <223> Description of Artificial Sequence: Synthetic oligonucleotide
<400> 510
   auggaauacu cuugguuaca uga 23
<210> 511
   <211> 23
   <212> RNA
   <213> Artificial Sequence
<220>
   <223> Description of Artificial Sequence: Synthetic oligonucleotide
<400> 511
   auggaauacu cuugguuaca uga 23
<210> 512
   <211> 23
   <212> RNA
   <213> Artificial Sequence
<220>
   <223> Description of Artificial Sequence: Synthetic oligonucleotide
<400> 512
   auggaauacu cuugguuaca uga 23
<210> 513
   <211> 23
   <212> RNA
   <213> Artificial Sequence
<220>
   <223> Description of Artificial Sequence: Synthetic oligonucleotide
<400> 513
   auggaauacu cuugguuaca uga 23
<210> 514
   <211> 23
   <212> RNA
   <213> Artificial Sequence
<220>
   <223> Description of Artificial Sequence: Synthetic oligonucleotide
<400> 514
   auggaauacu cuugguuaca uga 23
<210> 515
   <211> 23
   <212> RNA
   <213> Artificial Sequence
<220>
   <223> Description of Artificial Sequence: Synthetic oligonucleotide
<400> 515
   auggaauacu cuugguuaca uga 23
<210> 516
   <211> 23
   <212> RNA
   <213> Artificial Sequence
<220>
   <223> Description of Artificial Sequence: Synthetic oligonucleotide
<400> 516
   auggaauacu cuugguuaca uga 23
<210> 517
   <211> 23
   <212> RNA
   <213> Artificial Sequence
<220>
   <223> Description of Artificial Sequence: Synthetic oligonucleotide
<400> 517
   auggaauacu cuugguuaca uga 23
<210> 518
   <211> 23
   <212> RNA
   <213> Artificial Sequence
<220>
   <223> Description of Artificial Sequence: Synthetic oligonucleotide
<400> 518
   auggaauacu cuugguuaca uga 23
<210> 519
   <211> 23
   <212> RNA
   <213> Artificial Sequence
<220>
   <223> Description of Artificial Sequence: Synthetic oligonucleotide
<400> 519
   auggaauacu cuugguuaca uga 23
<210> 520
   <211> 23
   <212> RNA
   <213> Artificial Sequence
<220>
   <223> Description of Artificial Sequence: Synthetic oligonucleotide
<400> 520
   auggaauacu cuugguuaca uga 23
<210> 521
   <211> 23
   <212> RNA
   <213> Artificial Sequence
<220>
   <223> Description of Artificial Sequence: Synthetic oligonucleotide
<400> 521
   auggaauacu cuugguuaca uga 23
<210> 522
   <211> 23
   <212> RNA
   <213> Artificial Sequence
<220>
   <223> Description of Artificial Sequence: Synthetic oligonucleotide
<400> 522
   auggaauacu cuugguuaca uga 23
<210> 523
   <211> 23
   <212> RNA
   <213> Artificial Sequence
<220>
   <223> Description of Artificial Sequence: Synthetic oligonucleotide
<400> 523
   auggaauacu cuugguuaca uga 23
<210> 524
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 524
   auggaatacu cuugguuaca uga 23
<210> 525
   <211> 23
   <212> RNA
   <213> Artificial Sequence
<220>
   <223> Description of Artificial Sequence: Synthetic oligonucleotide
<400> 525
   auggaauacu cuugguuaca uga 23
<210> 526
   <211> 23
   <212> RNA
   <213> Artificial Sequence
<220>
   <223> Description of Artificial Sequence: Synthetic oligonucleotide
<400> 526
   auggaauacu cuugguuaca uga 23
<210> 527
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 527
   auggaauacu ctugguuaca uga 23
<210> 528
   <211> 23
   <212> RNA
   <213> Artificial Sequence
<220>
   <223> Description of Artificial Sequence: Synthetic oligonucleotide
<400> 528
   auggaauacu cuugguuaca uga 23
<210> 529
   <211> 23
   <212> RNA
   <213> Artificial Sequence
<220>
   <223> Description of Artificial Sequence: Synthetic oligonucleotide
<400> 529
   auggaauacu cuugguuaca uga 23
<210> 530
   <211> 23
   <212> RNA
   <213> Artificial Sequence
<220>
   <223> Description of Artificial Sequence: Synthetic oligonucleotide
<400> 530
   auggaauacu cuugguuaca uga 23
<210> 531
   <211> 23
   <212> RNA
   <213> Artificial Sequence
<220>
   <223> Description of Artificial Sequence: Synthetic oligonucleotide
<400> 531
   auggaauacu cuugguuaca uga 23
<210> 532
   <211> 23
   <212> RNA
   <213> Artificial Sequence
<220>
   <223> Description of Artificial Sequence: Synthetic oligonucleotide
<400> 532
   auggaauacu cuugguuaca uga 23
<210> 533
   <211> 23
   <212> RNA
   <213> Artificial Sequence
<220>
   <223> Description of Artificial Sequence: Synthetic oligonucleotide
<400> 533
   auggaauacu cuugguuaca uga 23
<210> 534
   <211> 23
   <212> RNA
   <213> Artificial Sequence
<220>
   <223> Description of Artificial Sequence: Synthetic oligonucleotide
<400> 534
   auggaauacu cuugguuaca uga 23
<210> 535
   <211> 23
   <212> RNA
   <213> Artificial Sequence
<220>
   <223> Description of Artificial Sequence: Synthetic oligonucleotide
<400> 535
   auggaauacu cuugguuaca uga 23
<210> 536
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 536
   auggaauacu cuugguuaca uga 23
<210> 537
   <211> 23
   <212> RNA
   <213> Artificial Sequence
<220>
   <223> Description of Artificial Sequence: Synthetic oligonucleotide
<400> 537
   auggaauacu cuugguuaca uga 23
<210> 538
   <211> 23
   <212> RNA
   <213> Artificial Sequence
<220>
   <223> Description of Artificial Sequence: Synthetic oligonucleotide
<400> 538
   auggaauacu cuugguuaca uga 23
<210> 539
   <211> 23
   <212> RNA
   <213> Artificial Sequence
<220>
   <223> Description of Artificial Sequence: Synthetic oligonucleotide
<400> 539
   auggaauacu cuugguuaca uga 23
<210> 540
   <211> 23
   <212> RNA
   <213> Artificial Sequence
<220>
   <223> Description of Artificial Sequence: Synthetic oligonucleotide
<400> 540
   auggaauacu cuugguuaca uga 23
<210> 541
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 541
   atggaatacu cuugguuaca uga 23
<210> 542
   <211> 23
   <212> RNA
   <213> Artificial Sequence
<220>
   <223> Description of Artificial Sequence: Synthetic oligonucleotide
<400> 542
   auggaauacu cuugguuaca uga 23
<210> 543
   <211> 23
   <212> RNA
   <213> Artificial Sequence
<220>
   <223> Description of Artificial Sequence: Synthetic oligonucleotide
<400> 543
   auggaauacu cuugguuaca uga 23
<210> 544
   <211> 23
   <212> RNA
   <213> Artificial Sequence
<220>
   <223> Description of Artificial Sequence: Synthetic oligonucleotide
<400> 544
   auggaauacu cuugguuaca uga 23
<210> 545
   <211> 23
   <212> RNA
   <213> Artificial Sequence
<220>
   <223> Description of Artificial Sequence: Synthetic oligonucleotide
<400> 545
   auggaauacu cuugguuaca uga 23
<210> 546
   <211> 23
   <212> RNA
   <213> Artificial Sequence
<220>
   <223> Description of Artificial Sequence: Synthetic oligonucleotide
<400> 546
   auggaauacu cuugguuaca uga 23
<210> 547
   <211> 23
   <212> RNA
   <213> Artificial Sequence
<220>
   <223> Description of Artificial Sequence: Synthetic oligonucleotide
<400> 547
   auggaauacu cuugguuaca uga 23
<210> 548
   <211> 23
   <212> RNA
   <213> Artificial Sequence
<220>
   <223> Description of Artificial Sequence: Synthetic oligonucleotide
<400> 548
   auggaauacu cuugguuaca uga 23
<210> 549
   <211> 23
   <212> RNA
   <213> Artificial Sequence
<220>
   <223> Description of Artificial Sequence: Synthetic oligonucleotide
<400> 549
   auggaauacu cuugguuaca uga 23
<210> 550
   <211> 23
   <212> RNA
   <213> Artificial Sequence
<220>
   <223> Description of Artificial Sequence: Synthetic oligonucleotide
<400> 550
   auggaauacu cuugguuaca uga 23
<210> 551
   <211> 23
   <212> RNA
   <213> Artificial Sequence
<220>
   <223> Description of Artificial Sequence: Synthetic oligonucleotide
<400> 551
   auggaauacu cuugguuaca uga 23
<210> 552
   <211> 23
   <212> RNA
   <213> Artificial Sequence
<220>
   <223> Description of Artificial Sequence: Synthetic oligonucleotide
<400> 552
   auggaauacu cuugguuaca uga 23
<210> 553
   <211> 23
   <212> RNA
   <213> Artificial Sequence
<220>
   <223> Description of Artificial Sequence: Synthetic oligonucleotide
<400> 553
   auggaauacu cuugguuaca uga 23
<210> 554
   <211> 23
   <212> RNA
   <213> Artificial Sequence
<220>
   <223> Description of Artificial Sequence: Synthetic oligonucleotide
<400> 554
   auggaauacu cuugguuaca uga 23
<210> 555
   <211> 23
   <212> RNA
   <213> Artificial Sequence
<220>
   <223> Description of Artificial Sequence: Synthetic oligonucleotide
<400> 555
   auggaauacu cuugguuaca uga 23
<210> 556
   <211> 23
   <212> RNA
   <213> Artificial Sequence
<220>
   <223> Description of Artificial Sequence: Synthetic oligonucleotide
<400> 556
   auggaauacu cuugguuaca uga 23
<210> 557
   <211> 23
   <212> RNA
   <213> Artificial Sequence
<220>
   <223> Description of Artificial Sequence: Synthetic oligonucleotide
<400> 557
   auggaauacu cuugguuaca uga 23
<210> 558
   <211> 23
   <212> RNA
   <213> Artificial Sequence
<220>
   <223> Description of Artificial Sequence: Synthetic oligonucleotide
<400> 558
   auggaauacu cuugguuaca uga 23
<210> 559
   <211> 23
   <212> RNA
   <213> Artificial Sequence
<220>
   <223> Description of Artificial Sequence: Synthetic oligonucleotide
<400> 559
   auggaauacu cuugguuaca uga 23
<210> 560
   <211> 23
   <212> RNA
   <213> Artificial Sequence
<220>
   <223> Description of Artificial Sequence: Synthetic oligonucleotide
<400> 560
   auggaauacu cuugguuaca uga 23
<210> 561
   <211> 23
   <212> RNA
   <213> Artificial Sequence
<220>
   <223> Description of Artificial Sequence: Synthetic oligonucleotide
<400> 561
   auggaauacu cuugguuaca uga 23
<210> 562
   <211> 23
   <212> RNA
   <213> Artificial Sequence
<220>
   <223> Description of Artificial Sequence: Synthetic oligonucleotide
<400> 562
   auggaauacu cuugguuaca uga 23
<210> 563
   <211> 23
   <212> RNA
   <213> Artificial Sequence
<220>
   <223> Description of Artificial Sequence: Synthetic oligonucleotide
<400> 563
   auggaauacu cuugguuaca uga 23
<210> 564
   <211> 23
   <212> RNA
   <213> Artificial Sequence
<220>
   <223> Description of Artificial Sequence: Synthetic oligonucleotide
<400> 564
   auggaauacu cuugguuaca uga 23
<210> 565
   <211> 23
   <212> RNA
   <213> Artificial Sequence
<220>
   <223> Description of Artificial Sequence: Synthetic oligonucleotide
<400> 565
   auggaauacu cuugguuaca uga 23
<210> 566
   <211> 23
   <212> RNA
   <213> Artificial Sequence
<220>
   <223> Description of Artificial Sequence: Synthetic oligonucleotide
<400> 566
   auggaauacu cuugguuaca uga 23
<210> 567
   <211> 23
   <212> RNA
   <213> Artificial Sequence
<220>
   <223> Description of Artificial Sequence: Synthetic oligonucleotide
<400> 567
   auggaauacu cuugguuaca uga 23
<210> 568
   <211> 23
   <212> RNA
   <213> Artificial Sequence
<220>
   <223> Description of Artificial Sequence: Synthetic oligonucleotide
<400> 568
   auggaauacu cuugguuaca uga 23
<210> 569
   <211> 23
   <212> RNA
   <213> Artificial Sequence
<220>
   <223> Description of Artificial Sequence: Synthetic oligonucleotide
<400> 569
   auggaauacu cuugguuaca uga 23
<210> 570
   <211> 23
   <212> RNA
   <213> Artificial Sequence
<220>
   <223> Description of Artificial Sequence: Synthetic oligonucleotide
<400> 570
   auggaauacu cuugguuaca uga 23
<210> 571
   <211> 23
   <212> RNA
   <213> Artificial Sequence
<220>
   <223> Description of Artificial Sequence: Synthetic oligonucleotide
<400> 571
   auggaauacu cuugguuaca uga 23
<210> 572
   <211> 23
   <212> RNA
   <213> Artificial Sequence
<220>
   <223> Description of Artificial Sequence: Synthetic oligonucleotide
<400> 572
   auggaauacu cuugguuaca uga 23
<210> 573
   <211> 23
   <212> RNA
   <213> Artificial Sequence
<220>
   <223> Description of Artificial Sequence: Synthetic oligonucleotide
<400> 573
   auggaauacu cuugguuaca uga 23
<210> 574
   <211> 23
   <212> RNA
   <213> Artificial Sequence
<220>
   <223> Description of Artificial Sequence: Synthetic oligonucleotide
<400> 574
   auggaauacu cuugguuaca uga 23
<210> 575
   <211> 23
   <212> RNA
   <213> Artificial Sequence
<220>
   <223> Description of Artificial Sequence: Synthetic oligonucleotide
<400> 575
   auggaauacu cuugguuaca uga 23
<210> 576
   <211> 23
   <212> RNA
   <213> Artificial Sequence
<220>
   <223> Description of Artificial Sequence: Synthetic oligonucleotide
<400> 576
   auggaauacu cuugguuaca uga 23
<210> 577
   <211> 23
   <212> RNA
   <213> Artificial Sequence
<220>
   <223> Description of Artificial Sequence: Synthetic oligonucleotide
<400> 577
   auggaauacu cuugguuaca uga 23
<210> 578
   <211> 23
   <212> RNA
   <213> Artificial Sequence
<220>
   <223> Description of Artificial Sequence: Synthetic oligonucleotide
<400> 578
   auggaauacu cuugguuaca uga 23
<210> 579
   <211> 23
   <212> RNA
   <213> Artificial Sequence
<220>
   <223> Description of Artificial Sequence: Synthetic oligonucleotide
<400> 579
   ucuugguuac augaaauccc auc 23
<210> 580
   <211> 23
   <212> RNA
   <213> Artificial Sequence
<220>
   <223> Description of Artificial Sequence: Synthetic oligonucleotide
<400> 580
   ucuugguuac augaaauccc auc 23
<210> 581
   <211> 23
   <212> RNA
   <213> Artificial Sequence
<220>
   <223> Description of Artificial Sequence: Synthetic oligonucleotide
<400> 581
   ucuugguuac augaaauccc auc 23
<210> 582
   <211> 23
   <212> RNA
   <213> Artificial Sequence
<220>
   <223> Description of Artificial Sequence: Synthetic oligonucleotide
<400> 582
   ucuugguuac augaaauccc auc 23
<210> 583
   <211> 23
   <212> RNA
   <213> Artificial Sequence
<220>
   <223> Description of Artificial Sequence: Synthetic oligonucleotide
<400> 583
   ucuugguuac augaaauccc auc 23
<210> 584
   <211> 23
   <212> RNA
   <213> Artificial Sequence
<220>
   <223> Description of Artificial Sequence: Synthetic oligonucleotide
<400> 584
   ucuugguuac augaaauccc auc 23
<210> 585
   <211> 23
   <212> RNA
   <213> Artificial Sequence
<220>
   <223> Description of Artificial Sequence: Synthetic oligonucleotide
<400> 585
   ucuugguuac augaaauccc auc 23
<210> 586
   <211> 23
   <212> RNA
   <213> Artificial Sequence
<220>
   <223> Description of Artificial Sequence: Synthetic oligonucleotide
<400> 586
   ucuugguuac augaaauccc auc 23
<210> 587
   <211> 23
   <212> RNA
   <213> Artificial Sequence
<220>
   <223> Description of Artificial Sequence: Synthetic oligonucleotide
<400> 587
   auggaauacu cuugguuaca uga 23
<210> 588
   <211> 23
   <212> RNA
   <213> Artificial Sequence
<220>
   <223> Description of Artificial Sequence: Synthetic oligonucleotide
<400> 588
   auggaauacu cuugguuaca uga 23
<210> 589
   <211> 23
   <212> RNA
   <213> Artificial Sequence
<220>
   <223> Description of Artificial Sequence: Synthetic oligonucleotide
<400> 589
   auggaauacu cuugguuaca uga 23
<210> 590
   <211> 23
   <212> RNA
   <213> Artificial Sequence
<220>
   <223> Description of Artificial Sequence: Synthetic oligonucleotide
<400> 590
   auggaauacu cuugguuaca uga 23
<210> 591
   <211> 23
   <212> RNA
   <213> Artificial Sequence
<220>
   <223> Description of Artificial Sequence: Synthetic oligonucleotide
<400> 591
   auggaauacu cuugguuaca uga 23
<210> 592
   <211> 23
   <212> RNA
   <213> Artificial Sequence
<220>
   <223> Description of Artificial Sequence: Synthetic oligonucleotide
<400> 592
   auggaauacu cuugguuaca uga 23
<210> 593
   <211> 23
   <212> RNA
   <213> Artificial Sequence
<220>
   <223> Description of Artificial Sequence: Synthetic oligonucleotide
<400> 593
   auggaauacu cuugguuaca uga 23
<210> 594
   <211> 23
   <212> RNA
   <213> Artificial Sequence
<220>
   <223> Description of Artificial Sequence: Synthetic oligonucleotide
<400> 594
   auggaauacu cuugguuaca uga 23
<210> 595
   <211> 23
   <212> RNA
   <213> Artificial Sequence
<220>
   <223> Description of Artificial Sequence: Synthetic oligonucleotide
<400> 595
   auggaauacu cuugguuaca uga 23
<210> 596
   <211> 23
   <212> RNA
   <213> Artificial Sequence
<220>
   <223> Description of Artificial Sequence: Synthetic oligonucleotide
<400> 596
   auggaauacu cuugguuaca uga 23
<210> 597
   <211> 23
   <212> RNA
   <213> Artificial Sequence
<220>
   <223> Description of Artificial Sequence: Synthetic oligonucleotide
<400> 597
   auggaauacu cuugguuaca uga 23
<210> 598
   <211> 23
   <212> RNA
   <213> Artificial Sequence
<220>
   <223> Description of Artificial Sequence: Synthetic oligonucleotide
<400> 598
   auggaauacu cuugguuaca uga 23
<210> 599
   <211> 23
   <212> RNA
   <213> Artificial Sequence
<220>
   <223> Description of Artificial Sequence: Synthetic oligonucleotide
<400> 599
   auggaauacu cuugguuaca uga 23
<210> 600
   <211> 23
   <212> RNA
   <213> Artificial Sequence
<220>
   <223> Description of Artificial Sequence: Synthetic oligonucleotide
<400> 600
   auggaauacu cuugguuaca uga 23
<210> 601
   <211> 23
   <212> RNA
   <213> Artificial Sequence
<220>
   <223> Description of Artificial Sequence: Synthetic oligonucleotide
<400> 601
   auggaauacu cuugguuaca uga 23
<210> 602
   <211> 23
   <212> RNA
   <213> Artificial Sequence
<220>
   <223> Description of Artificial Sequence: Synthetic oligonucleotide
<400> 602
   auggaauacu cuugguuaca uga 23
<210> 603
   <211> 23
   <212> RNA
   <213> Artificial Sequence
<220>
   <223> Description of Artificial Sequence: Synthetic oligonucleotide
<400> 603
   auggaauacu cuugguuaca uga 23
<210> 604
   <211> 23
   <212> RNA
   <213> Artificial Sequence
<220>
   <223> Description of Artificial Sequence: Synthetic oligonucleotide
<400> 604
   auggaauacu cuugguuaca uga 23
<210> 605
   <211> 23
   <212> RNA
   <213> Artificial Sequence
<220>
   <223> Description of Artificial Sequence: Synthetic oligonucleotide
<400> 605
   auggaauacu cuugguuaca uga 23
<210> 606
   <211> 23
   <212> RNA
   <213> Artificial Sequence
<220>
   <223> Description of Artificial Sequence: Synthetic oligonucleotide
<400> 606
   auggaauacu cuugguuaca uga 23
<210> 607
   <211> 23
   <212> RNA
   <213> Artificial Sequence
<220>
   <223> Description of Artificial Sequence: Synthetic oligonucleotide
<400> 607
   auggaauacu cuugguuaca uga 23
<210> 608
   <211> 23
   <212> RNA
   <213> Artificial Sequence
<220>
   <223> Description of Artificial Sequence: Synthetic oligonucleotide
<400> 608
   auggaauacu cuugguuaca uga 23
<210> 609
   <211> 23
   <212> RNA
   <213> Artificial Sequence
<220>
   <223> Description of Artificial Sequence: Synthetic oligonucleotide
<400> 609
   auggaauacu cuugguuaca uga 23
<210> 610
   <211> 23
   <212> RNA
   <213> Artificial Sequence
<220>
   <223> Description of Artificial Sequence: Synthetic oligonucleotide
<400> 610
   auggaauacu cuugguuaca uga 23
<210> 611
   <211> 23
   <212> RNA
   <213> Artificial Sequence
<220>
   <223> Description of Artificial Sequence: Synthetic oligonucleotide
<400> 611
   auggaauacu cuugguuaca uga 23
<210> 612
   <211> 23
   <212> RNA
   <213> Artificial Sequence
<220>
   <223> Description of Artificial Sequence: Synthetic oligonucleotide
<400> 612
   auggaauacu cuugguuaca uga 23
<210> 613
   <211> 23
   <212> RNA
   <213> Artificial Sequence
<220>
   <223> Description of Artificial Sequence: Synthetic oligonucleotide
<400> 613
   auggaauacu cuugguuaca uga 23
<210> 614
   <211> 23
   <212> RNA
   <213> Artificial Sequence
<220>
   <223> Description of Artificial Sequence: Synthetic oligonucleotide
<400> 614
   auggaauacu cuugguuaca uga 23
<210> 615
   <211> 23
   <212> RNA
   <213> Artificial Sequence
<220>
   <223> Description of Artificial Sequence: Synthetic oligonucleotide
<400> 615
   auggaauacu cuugguuaca uga 23
<210> 616
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 616
   auggaauacu cuuggutaca tga 23
<210> 617
   <211> 23
   <212> RNA
   <213> Artificial Sequence
<220>
   <223> Description of Artificial Sequence: Synthetic oligonucleotide
<400> 617
   auggaauacu cuugguuaca uga 23
<210> 618
   <211> 23
   <212> RNA
   <213> Artificial Sequence
<220>
   <223> Description of Artificial Sequence: Synthetic oligonucleotide
<400> 618
   auggaauacu cuugguuaca uga 23
<210> 619
   <211> 23
   <212> RNA
   <213> Artificial Sequence
<220>
   <223> Description of Artificial Sequence: Synthetic oligonucleotide
<400> 619
   auggaauacu cuugguuaca uga 23
<210> 620
   <211> 23
   <212> RNA
   <213> Artificial Sequence
<220>
   <223> Description of Artificial Sequence: Synthetic oligonucleotide
<400> 620
   auggaauacu cuugguuaca uga 23
<210> 621
   <211> 23
   <212> RNA
   <213> Artificial Sequence
<220>
   <223> Description of Artificial Sequence: Synthetic oligonucleotide
<400> 621
   auggaauacu cuugguuaca uga 23
<210> 622
   <211> 23
   <212> RNA
   <213> Artificial Sequence
<220>
   <223> Description of Artificial Sequence: Synthetic oligonucleotide
<400> 622
   auggaauacu cuugguuaca uga 23
<210> 623
   <211> 23
   <212> RNA
   <213> Artificial Sequence
<220>
   <223> Description of Artificial Sequence: Synthetic oligonucleotide
<400> 623
   auggaauacu cuugguuaca uga 23
<210> 624
   <211> 23
   <212> RNA
   <213> Artificial Sequence
<220>
   <223> Description of Artificial Sequence: Synthetic oligonucleotide
<400> 624
   auggaauacu cuugguuaca uga 23
<210> 625
   <211> 23
   <212> RNA
   <213> Artificial Sequence
<220>
   <223> Description of Artificial Sequence: Synthetic oligonucleotide
<400> 625
   auggaauacu cuugguuaca uga 23
<210> 626
   <211> 23
   <212> RNA
   <213> Artificial Sequence
<220>
   <223> Description of Artificial Sequence: Synthetic oligonucleotide
<400> 626
   auggaauacu cuugguuaca uga 23
<210> 627
   <211> 23
   <212> RNA
   <213> Artificial Sequence
<220>
   <223> Description of Artificial Sequence: Synthetic oligonucleotide
<400> 627
   auggaauacu cuugguuaca uga 23
<210> 628
   <211> 23
   <212> RNA
   <213> Artificial Sequence
<220>
   <223> Description of Artificial Sequence: Synthetic oligonucleotide
<400> 628
   auggaauacu cuugguuaca uga 23
<210> 629
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 629
   atggaauacu cuugguuaca uga 23
<210> 630
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 630
   auggaatacu cuugguuaca uga 23
<210> 631
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 631
   atggaauacu cuugguuaca uga 23
<210> 632
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 632
   atggaauact cuugguuaca uga 23
<210> 633
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 633
   auggaauacu cuugguuaca uga 23
<210> 634
   <211> 23
   <212> RNA
   <213> Artificial Sequence
<220>
   <223> Description of Artificial Sequence: Synthetic oligonucleotide
<400> 634
   auggaauacu cuugguuaca uga 23
<210> 635
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 635
   auggaatacu cuugguuaca uga 23
<210> 636
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 636
   auggaatacu cuugguuaca uga 23
<210> 637
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 637
   auggaauacu cuuggutaca tga 23
<210> 638
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 638
   atggaauacu cuugguuaca uga 23
<210> 639
   <211> 23
   <212> RNA
   <213> Artificial Sequence
<220>
   <223> Description of Artificial Sequence: Synthetic oligonucleotide
<400> 639
   auggaauacu cuugguuaca uga 23
<210> 640
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 640
   auggaauacu cuugguuaca uga 23
<210> 641
   <211> 23
   <212> RNA
   <213> Artificial Sequence
<220>
   <223> Description of Artificial Sequence: Synthetic oligonucleotide
<400> 641
   auggaauacu cuugguuaca uga 23
<210> 642
   <211> 23
   <212> RNA
   <213> Artificial Sequence
<220>
   <223> Description of Artificial Sequence: Synthetic oligonucleotide
<400> 642
   auggaauacu cuugguuaca uga 23
<210> 643
   <211> 23
   <212> RNA
   <213> Artificial Sequence
<220>
   <223> Description of Artificial Sequence: Synthetic oligonucleotide
<400> 643
   auggaauacu cuugguuaca uga 23
<210> 644
   <211> 23
   <212> RNA
   <213> Artificial Sequence
<220>
   <223> Description of Artificial Sequence: Synthetic oligonucleotide
<400> 644
   auggaauacu cuugguuaca uga 23
<210> 645
   <211> 23
   <212> RNA
   <213> Artificial Sequence
<220>
   <223> Description of Artificial Sequence: Synthetic oligonucleotide
<400> 645
   auggaauacu cuugguuaca uga 23
<210> 646
   <211> 23
   <212> RNA
   <213> Artificial Sequence
<220>
   <223> Description of Artificial Sequence: Synthetic oligonucleotide
<400> 646
   auggaauacu cuugguuaca uga 23
<210> 647
   <211> 23
   <212> RNA
   <213> Artificial Sequence
<220>
   <223> Description of Artificial Sequence: Synthetic oligonucleotide
<400> 647
   auggaauacu cuugguuaca uga 23
<210> 648
   <211> 23
   <212> RNA
   <213> Artificial Sequence
<220>
   <223> Description of Artificial Sequence: Synthetic oligonucleotide
<400> 648
   auggaauacu cuugguuaca aga 23
<210> 649
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 649
   auggaauacu cutgguuaca uga 23
<210> 650
   <211> 23
   <212> RNA
   <213> Artificial Sequence
<220>
   <223> Description of Artificial Sequence: Synthetic oligonucleotide
<400> 650
   auggaauacu cuugguuaca uga 23
<210> 651
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 651
   auggaauacu ctugguuaca uga 23
<210> 652
   <211> 23
   <212> RNA
   <213> Artificial Sequence
<220>
   <223> Description of Artificial Sequence: Synthetic oligonucleotide
<400> 652
   auggaauacu cuugguuaca uga 23
<210> 653
   <211> 23
   <212> RNA
   <213> Artificial Sequence
<220>
   <223> Description of Artificial Sequence: Synthetic oligonucleotide
<400> 653
   auggaauacu cuugguuaca uga 23
<210> 654
   <211> 23
   <212> RNA
   <213> Artificial Sequence
<220>
   <223> Description of Artificial Sequence: Synthetic oligonucleotide
<400> 654
   auggaauacu cuugguuaca uga 23
<210> 655
   <211> 23
   <212> RNA
   <213> Artificial Sequence
<220>
   <223> Description of Artificial Sequence: Synthetic oligonucleotide
<400> 655
   auggaauacu cuugguuaca uga 23
<210> 656
   <211> 23
   <212> RNA
   <213> Artificial Sequence
<220>
   <223> Description of Artificial Sequence: Synthetic oligonucleotide
<400> 656
   auggaauacu cuugguuaca uga 23
<210> 657
   <211> 23
   <212> RNA
   <213> Artificial Sequence
<220>
   <223> Description of Artificial Sequence: Synthetic oligonucleotide
<400> 657
   auggaauacu cuugguuaca uga 23
<210> 658
   <211> 23
   <212> RNA
   <213> Artificial Sequence
<220>
   <223> Description of Artificial Sequence: Synthetic oligonucleotide
<400> 658
   auggaauacu cuugguuaca uga 23
<210> 659
   <211> 23
   <212> RNA
   <213> Artificial Sequence
<220>
   <223> Description of Artificial Sequence: Synthetic oligonucleotide
<400> 659
   auggaauacu cuugguuaca uga 23
<210> 660
   <211> 23
   <212> RNA
   <213> Artificial Sequence
<220>
   <223> Description of Artificial Sequence: Synthetic oligonucleotide
<400> 660
   auggaauacu cuugguuaca uga 23
<210> 661
   <211> 23
   <212> RNA
   <213> Artificial Sequence
<220>
   <223> Description of Artificial Sequence: Synthetic oligonucleotide
<400> 661
   auggaauacu cuugguuaca uga 23
<210> 662
   <211> 23
   <212> RNA
   <213> Artificial Sequence
<220>
   <223> Description of Artificial Sequence: Synthetic oligonucleotide
<400> 662
   auggaauacu cuugguuaca uga 23
<210> 663
   <211> 23
   <212> RNA
   <213> Artificial Sequence
<220>
   <223> Description of Artificial Sequence: Synthetic oligonucleotide
<400> 663
   auggaauacu cuugguuaca uga 23
<210> 664
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 664
   auggaauacu cuugguuaca uga 23
<210> 665
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 665
   auggaauacu cuugguuaca uga 23
<210> 666
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 666
   auggaauacu cuugguuaca uga 23
<210> 667
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 667
   auggaatacu cuugguuaca uga 23
<210> 668
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 668
   auggaauacu cuugguuaca uga 23
<210> 669
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 669
   auggaatacu cuugguuaca uga 23
<210> 670
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 670
   auggaauacu cuugguuaca uga 23
<210> 671
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 671
   auggaatacu cuugguuaca uga 23
<210> 672
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 672
   atggaauacu cuugguuaca uga 23
<210> 673
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 673
   tuauagagca agaacacugu uuu 23
<210> 674
   <211> 23
   <212> RNA
   <213> Artificial Sequence
<220>
   <223> Description of Artificial Sequence: Synthetic oligonucleotide
<400> 674
   uuauagagca agaacacugu uuu 23
<210> 675
   <211> 23
   <212> RNA
   <213> Artificial Sequence
<220>
   <223> Description of Artificial Sequence: Synthetic oligonucleotide
<400> 675
   uuauagagca agaacacugu uuu 23
<210> 676
   <211> 23
   <212> RNA
   <213> Artificial Sequence
<220>
   <223> Description of Artificial Sequence: Synthetic oligonucleotide
<400> 676
   uuauagagca agaacacugu uuu 23
<210> 677
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 677
   uuauagagca agaacacugt t 21
<210> 678
   <211> 23
   <212> RNA
   <213> Artificial Sequence
<220>
   <223> Description of Artificial Sequence: Synthetic oligonucleotide
<400> 678
   uuauagagca agaacacugu uuu 23
<210> 679
   <211> 23
   <212> RNA
   <213> Artificial Sequence
<220>
   <223> Description of Artificial Sequence: Synthetic oligonucleotide
<400> 679
   uuauagagca agaacacugu uuu 23
<210> 680
   <211> 23
   <212> RNA
   <213> Artificial Sequence
<220>
   <223> Description of Artificial Sequence: Synthetic oligonucleotide
<400> 680
   uuauagagca agaacacugu uuu 23
<210> 681
   <211> 23
   <212> RNA
   <213> Artificial Sequence
<220>
   <223> Description of Artificial Sequence: Synthetic oligonucleotide
<400> 681
   uuauagagca agaacacugu uuu 23
<210> 682
   <211> 23
   <212> RNA
   <213> Artificial Sequence
<220>
   <223> Description of Artificial Sequence: Synthetic oligonucleotide
<400> 682
   uuauagagca agaacacugu uuu 23
<210> 683
   <211> 23
   <212> RNA
   <213> Artificial Sequence
<220>
   <223> Description of Artificial Sequence: Synthetic oligonucleotide
<400> 683
   uuauagagca agaacacugu uuu 23
<210> 684
   <211> 23
   <212> RNA
   <213> Artificial Sequence
<220>
   <223> Description of Artificial Sequence: Synthetic oligonucleotide
<400> 684
   uuauagagca agaacacugu uuu 23
<210> 685
   <211> 23
   <212> RNA
   <213> Artificial Sequence
<220>
   <223> Description of Artificial Sequence: Synthetic oligonucleotide
<400> 685
   uuauagagca agaacacugu uuu 23
<210> 686
   <211> 23
   <212> RNA
   <213> Artificial Sequence
<220>
   <223> Description of Artificial Sequence: Synthetic oligonucleotide
<400> 686
   uuauagagca agaacacugu uuu 23
<210> 687
   <211> 23
   <212> RNA
   <213> Artificial Sequence
<220>
   <223> Description of Artificial Sequence: Synthetic oligonucleotide
<400> 687
   uuauagagca agaacacugu uuu 23
<210> 688
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 688
   uuauagagca agaacacugt t 21
<210> 689
   <211> 23
   <212> RNA
   <213> Artificial Sequence
<220>
   <223> Description of Artificial Sequence: Synthetic oligonucleotide
<400> 689
   uuauagagca agaacacugu uuu 23
<210> 690
   <211> 23
   <212> RNA
   <213> Artificial Sequence
<220>
   <223> Description of Artificial Sequence: Synthetic oligonucleotide
<400> 690
   uuauagagca agaacacugu uuu 23
<210> 691
   <211> 23
   <212> RNA
   <213> Artificial Sequence
<220>
   <223> Description of Artificial Sequence: Synthetic oligonucleotide
<400> 691
   uuauagagca agaacacugu uuu 23
<210> 692
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 692
   tuauagagca agaacacagu uuu 23
<210> 693
   <211> 23
   <212> RNA
   <213> Artificial Sequence
<220>
   <223> Description of Artificial Sequence: Synthetic oligonucleotide
<400> 693
   uuauagagca agaacacugu uuu 23
<210> 694
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 694
   uuauagagca agaacacugt t 21
<210> 695
   <211> 23
   <212> RNA
   <213> Artificial Sequence
<220>
   <223> Description of Artificial Sequence: Synthetic oligonucleotide
<400> 695
   uuauagagca agaacacugu uuu 23
<210> 696
   <211> 23
   <212> RNA
   <213> Artificial Sequence
<220>
   <223> Description of Artificial Sequence: Synthetic oligonucleotide
<400> 696
   uuauagagca agaacacugu uuu 23
<210> 697
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 697
   uuauagagca agaacacugt t 21
<210> 698
   <211> 23
   <212> RNA
   <213> Artificial Sequence
<220>
   <223> Description of Artificial Sequence: Synthetic oligonucleotide
<400> 698
   uuauagagca agaacacugu uuu 23
<210> 699
   <211> 23
   <212> RNA
   <213> Artificial Sequence
<220>
   <223> Description of Artificial Sequence: Synthetic oligonucleotide
<400> 699
   uuauagagca agaacacugu uuu 23
<210> 700
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 700
   uuauagagca agaacacugt t 21
<210> 701
   <211> 23
   <212> RNA
   <213> Artificial Sequence
<220>
   <223> Description of Artificial Sequence: Synthetic oligonucleotide
<400> 701
   uuauagagca agaacacugu uuu 23
<210> 702
   <211> 23
   <212> RNA
   <213> Artificial Sequence
<220>
   <223> Description of Artificial Sequence: Synthetic oligonucleotide
<400> 702
   uuauagagca agaacacugu uuu 23
<210> 703
   <211> 23
   <212> RNA
   <213> Artificial Sequence
<220>
   <223> Description of Artificial Sequence: Synthetic oligonucleotide
<400> 703
   uuauagagca agaacacugu uuu 23
<210> 704
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 704
   uuauagagca agaacacugt t 21
<210> 705
   <211> 23
   <212> RNA
   <213> Artificial Sequence
<220>
   <223> Description of Artificial Sequence: Synthetic oligonucleotide
<400> 705
   uuauagagca agaacacugu uuu 23
<210> 706
   <211> 23
   <212> RNA
   <213> Artificial Sequence
<220>
   <223> Description of Artificial Sequence: Synthetic oligonucleotide
<400> 706
   uuauagagca agaacacugu uuu 23
<210> 707
   <211> 23
   <212> RNA
   <213> Artificial Sequence
<220>
   <223> Description of Artificial Sequence: Synthetic oligonucleotide
<400> 707
   uuauagagca agaacacugu uuu 23
<210> 708
   <211> 23
   <212> RNA
   <213> Artificial Sequence
<220>
   <223> Description of Artificial Sequence: Synthetic oligonucleotide
<400> 708
   uuauagagca agaacacugu uuu 23
<210> 709
   <211> 23
   <212> RNA
   <213> Artificial Sequence
<220>
   <223> Description of Artificial Sequence: Synthetic oligonucleotide
<400> 709
   uuauagagca agaacacugu uuu 23
<210> 710
   <211> 23
   <212> RNA
   <213> Artificial Sequence
<220>
   <223> Description of Artificial Sequence: Synthetic oligonucleotide
<400> 710
   uuauagagca agaacacugu uuu 23
<210> 711
   <211> 23
   <212> RNA
   <213> Artificial Sequence
<220>
   <223> Description of Artificial Sequence: Synthetic oligonucleotide
<400> 711
   uuauagagca agaacacugu uuu 23
<210> 712
   <211> 23
   <212> RNA
   <213> Artificial Sequence
<220>
   <223> Description of Artificial Sequence: Synthetic oligonucleotide
<400> 712
   uuauagagca agaacacugu uuu 23
<210> 713
   <211> 23
   <212> RNA
   <213> Artificial Sequence
<220>
   <223> Description of Artificial Sequence: Synthetic oligonucleotide
<400> 713
   uuauagagca agaacacugu uuu 23
<210> 714
   <211> 23
   <212> RNA
   <213> Artificial Sequence
<220>
   <223> Description of Artificial Sequence: Synthetic oligonucleotide
<400> 714
   uuauagagca agaacacugu uuu 23
<210> 715
   <211> 23
   <212> RNA
   <213> Artificial Sequence
<220>
   <223> Description of Artificial Sequence: Synthetic oligonucleotide
<400> 715
   uuauagagca agaacacugu uuu 23
<210> 716
   <211> 23
   <212> RNA
   <213> Artificial Sequence
<220>
   <223> Description of Artificial Sequence: Synthetic oligonucleotide
<400> 716
   uuauagagca agaacacugu uuu 23
<210> 717
   <211> 23
   <212> RNA
   <213> Artificial Sequence
<220>
   <223> Description of Artificial Sequence: Synthetic oligonucleotide
<400> 717
   uuauagagca agaacacugu uuu 23
<210> 718
   <211> 23
   <212> RNA
   <213> Artificial Sequence
<220>
   <223> Description of Artificial Sequence: Synthetic oligonucleotide
<400> 718
   uuauagagca agaacacugu uuu 23
<210> 719
   <211> 23
   <212> RNA
   <213> Artificial Sequence
<220>
   <223> Description of Artificial Sequence: Synthetic oligonucleotide
<400> 719
   uuauagagca agaacacugu uuu 23
<210> 720
   <211> 23
   <212> RNA
   <213> Artificial Sequence
<220>
   <223> Description of Artificial Sequence: Synthetic oligonucleotide
<400> 720
   uuauagagca agaacacugu uuu 23
<210> 721
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 721
   ttauagagca agaacactgu ttu 23
<210> 722
   <211> 23
   <212> RNA
   <213> Artificial Sequence
<220>
   <223> Description of Artificial Sequence: Synthetic oligonucleotide
<400> 722
   uuauagagca agaacacugu uuu 23
<210> 723
   <211> 23
   <212> RNA
   <213> Artificial Sequence
<220>
   <223> Description of Artificial Sequence: Synthetic oligonucleotide
<400> 723
   uuauagagca agaacacugu uuu 23
<210> 724
   <211> 23
   <212> RNA
   <213> Artificial Sequence
<220>
   <223> Description of Artificial Sequence: Synthetic oligonucleotide
<400> 724
   uuauagagca agaacacugu uuu 23
<210> 725
   <211> 23
   <212> RNA
   <213> Artificial Sequence
<220>
   <223> Description of Artificial Sequence: Synthetic oligonucleotide
<400> 725
   uuauagagca agaacacugu uuu 23
<210> 726
   <211> 23
   <212> RNA
   <213> Artificial Sequence
<220>
   <223> Description of Artificial Sequence: Synthetic oligonucleotide
<400> 726
   uuauagagca agaacacugu uuu 23
<210> 727
   <211> 23
   <212> RNA
   <213> Artificial Sequence
<220>
   <223> Description of Artificial Sequence: Synthetic oligonucleotide
<400> 727
   uuauagagca agaacacugu uuu 23
<210> 728
   <211> 23
   <212> RNA
   <213> Artificial Sequence
<220>
   <223> Description of Artificial Sequence: Synthetic oligonucleotide
<400> 728
   uuauagagca agaacacugu uuu 23
<210> 729
   <211> 23
   <212> RNA
   <213> Artificial Sequence
<220>
   <223> Description of Artificial Sequence: Synthetic oligonucleotide
<400> 729
   uuauagagca agaacacugu uuu 23
<210> 730
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 730
   utatagagca agaacacugt tuu 23
<210> 731
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 731
   tuauagagca agaacacugu uuu 23
<210> 732
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 732
   tuauagagca agaacacugu uuu 23
<210> 733
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 733
   tuauagagca agaacacugu uuu 23
<210> 734
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 734
   tuauagagca agaacacugu uuu 23
<210> 735
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 735
   tuauagagca agaacacagu uuu 23
<210> 736
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 736
   ttatagagca agaacacagu uuu 23
<210> 737
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 737
   tuauagagca agaacacugu uuu 23
<210> 738
   <211> 23
   <212> RNA
   <213> Artificial Sequence
<220>
   <223> Description of Artificial Sequence: Synthetic oligonucleotide
<400> 738
   uuauagagca agaacacugu uuu 23
<210> 739
   <211> 23
   <212> RNA
   <213> Artificial Sequence
<220>
   <223> Description of Artificial Sequence: Synthetic oligonucleotide
<400> 739
   uuauagagca agaacacugu uuu 23
<210> 740
   <211> 23
   <212> RNA
   <213> Artificial Sequence
<220>
   <223> Description of Artificial Sequence: Synthetic oligonucleotide
<400> 740
   uuauagagca agaacacugu uuu 23
<210> 741
   <211> 23
   <212> RNA
   <213> Artificial Sequence
<220>
   <223> Description of Artificial Sequence: Synthetic oligonucleotide
<400> 741
   uuauagagca agaacacugu uuu 23
<210> 742
   <211> 23
   <212> RNA
   <213> Artificial Sequence
<220>
   <223> Description of Artificial Sequence: Synthetic oligonucleotide
<400> 742
   uuauagagca agaacacugu uuu 23
<210> 743
   <211> 23
   <212> RNA
   <213> Artificial Sequence
<220>
   <223> Description of Artificial Sequence: Synthetic oligonucleotide
<400> 743
   uuauagagca agaacacugu uuu 23
<210> 744
   <211> 23
   <212> RNA
   <213> Artificial Sequence
<220>
   <223> Description of Artificial Sequence: Synthetic oligonucleotide
<400> 744
   uuauagagca agaacacugu uuu 23
<210> 745
   <211> 23
   <212> RNA
   <213> Artificial Sequence
<220>
   <223> Description of Artificial Sequence: Synthetic oligonucleotide
<400> 745
   uuauagagca agaacacugu uuu 23
<210> 746
   <211> 23
   <212> RNA
   <213> Artificial Sequence
<220>
   <223> Description of Artificial Sequence: Synthetic oligonucleotide
<400> 746
   uuauagagca agaacacugu uuu 23
<210> 747
   <211> 23
   <212> RNA
   <213> Artificial Sequence
<220>
   <223> Description of Artificial Sequence: Synthetic oligonucleotide
<400> 747
   uuauagagca agaacacugu uuu 23
<210> 748
   <211> 23
   <212> RNA
   <213> Artificial Sequence
<220>
   <223> Description of Artificial Sequence: Synthetic oligonucleotide
<400> 748
   uuauagagca agaacacugu uuu 23
<210> 749
   <211> 23
   <212> RNA
   <213> Artificial Sequence
<220>
   <223> Description of Artificial Sequence: Synthetic oligonucleotide
<400> 749
   uuauagagca agaacacugu uuu 23
<210> 750
   <211> 23
   <212> RNA
   <213> Artificial Sequence
<220>
   <223> Description of Artificial Sequence: Synthetic oligonucleotide
<400> 750
   uuauagagca agaacacugu uuu 23
<210> 751
   <211> 23
   <212> RNA
   <213> Artificial Sequence
<220>
   <223> Description of Artificial Sequence: Synthetic oligonucleotide
<400> 751
   uuauagagca agaacacugu uuu 23
<210> 752
   <211> 23
   <212> RNA
   <213> Artificial Sequence
<220>
   <223> Description of Artificial Sequence: Synthetic oligonucleotide
<400> 752
   uuauagagca agaacacugu uuu 23
<210> 753
   <211> 23
   <212> RNA
   <213> Artificial Sequence
<220>
   <223> Description of Artificial Sequence: Synthetic oligonucleotide
<400> 753
   uuauagagca agaacacugu uuu 23
<210> 754
   <211> 23
   <212> RNA
   <213> Artificial Sequence
<220>
   <223> Description of Artificial Sequence: Synthetic oligonucleotide
<400> 754
   uuauagagca agaacacugu uuu 23
<210> 755
   <211> 23
   <212> RNA
   <213> Artificial Sequence
<220>
   <223> Description of Artificial Sequence: Synthetic oligonucleotide
<400> 755
   uuauagagca agaacacugu uuu 23
<210> 756
   <211> 23
   <212> RNA
   <213> Artificial Sequence
<220>
   <223> Description of Artificial Sequence: Synthetic oligonucleotide
<400> 756
   uuauagagca agaacacugu uuu 23
<210> 757
   <211> 23
   <212> RNA
   <213> Artificial Sequence
<220>
   <223> Description of Artificial Sequence: Synthetic oligonucleotide
<400> 757
   uuauagagca agaacacugu uuu 23
<210> 758
   <211> 23
   <212> RNA
   <213> Artificial Sequence
<220>
   <223> Description of Artificial Sequence: Synthetic oligonucleotide
<400> 758
   uuauagagca agaacacugu uuu 23
<210> 759
   <211> 23
   <212> RNA
   <213> Artificial Sequence
<220>
   <223> Description of Artificial Sequence: Synthetic oligonucleotide
<400> 759
   uuauagagca agaacacugu uuu 23
<210> 760
   <211> 23
   <212> RNA
   <213> Artificial Sequence
<220>
   <223> Description of Artificial Sequence: Synthetic oligonucleotide
<400> 760
   uuauagagca agaacacugu uuu 23
<210> 761
   <211> 23
   <212> RNA
   <213> Artificial Sequence
<220>
   <223> Description of Artificial Sequence: Synthetic oligonucleotide
<400> 761
   uuauagagca agaacacugu uuu 23
<210> 762
   <211> 23
   <212> RNA
   <213> Artificial Sequence
<220>
   <223> Description of Artificial Sequence: Synthetic oligonucleotide
<400> 762
   uuauagagca agaacacugu uuu 23
<210> 763
   <211> 23
   <212> RNA
   <213> Artificial Sequence
<220>
   <223> Description of Artificial Sequence: Synthetic oligonucleotide
<400> 763
   uuauagagca agaacacugu uuu 23
<210> 764
   <211> 23
   <212> RNA
   <213> Artificial Sequence
<220>
   <223> Description of Artificial Sequence: Synthetic oligonucleotide
<400> 764
   uuauagagca agaacacugu uuu 23
<210> 765
   <211> 23
   <212> RNA
   <213> Artificial Sequence
<220>
   <223> Description of Artificial Sequence: Synthetic oligonucleotide
<400> 765
   uuauagagca agaacacugu uuu 23
<210> 766
   <211> 23
   <212> RNA
   <213> Artificial Sequence
<220>
   <223> Description of Artificial Sequence: Synthetic oligonucleotide
<400> 766
   uuauagagca agaacacugu uuu 23
<210> 767
   <211> 23
   <212> RNA
   <213> Artificial Sequence
<220>
   <223> Description of Artificial Sequence: Synthetic oligonucleotide
<400> 767
   uuauagagca agaacacugu uuu 23
<210> 768
   <211> 23
   <212> RNA
   <213> Artificial Sequence
<220>
   <223> Description of Artificial Sequence: Synthetic oligonucleotide
<400> 768
   uuauagagca agaacacugu uuu 23
<210> 769
   <211> 23
   <212> RNA
   <213> Artificial Sequence
<220>
   <223> Description of Artificial Sequence: Synthetic oligonucleotide
<400> 769
   uuauagagca agaacacugu uuu 23
<210> 770
   <211> 23
   <212> RNA
   <213> Artificial Sequence
<220>
   <223> Description of Artificial Sequence: Synthetic oligonucleotide
<400> 770
   uuauagagca agaacacugu uuu 23
<210> 771
   <211> 23
   <212> RNA
   <213> Artificial Sequence
<220>
   <223> Description of Artificial Sequence: Synthetic oligonucleotide
<400> 771
   uuauagagca agaacacugu uuu 23
<210> 772
   <211> 23
   <212> RNA
   <213> Artificial Sequence
<220>
   <223> Description of Artificial Sequence: Synthetic oligonucleotide
<400> 772
   uuauagagca agaacacugu uuu 23
<210> 773
   <211> 23
   <212> RNA
   <213> Artificial Sequence
<220>
   <223> Description of Artificial Sequence: Synthetic oligonucleotide
<400> 773
   uuauagagca agaacacugu uuu 23
<210> 774
   <211> 23
   <212> RNA
   <213> Artificial Sequence
<220>
   <223> Description of Artificial Sequence: Synthetic oligonucleotide
<400> 774
   uuauagagca agaacacugu uuu 23
<210> 775
   <211> 23
   <212> RNA
   <213> Artificial Sequence
<220>
   <223> Description of Artificial Sequence: Synthetic oligonucleotide
<400> 775
   uuauagagca agaacacugu uuu 23
<210> 776
   <211> 23
   <212> RNA
   <213> Artificial Sequence
<220>
   <223> Description of Artificial Sequence: Synthetic oligonucleotide
<400> 776
   uuauagagca agaacacugu uuu 23
<210> 777
   <211> 23
   <212> RNA
   <213> Artificial Sequence
<220>
   <223> Description of Artificial Sequence: Synthetic oligonucleotide
<400> 777
   uuauagagca agaacacugu uuu 23
<210> 778
   <211> 23
   <212> RNA
   <213> Artificial Sequence
<220>
   <223> Description of Artificial Sequence: Synthetic oligonucleotide
<400> 778
   uuauagagca agaacacugu uuu 23
<210> 779
   <211> 23
   <212> RNA
   <213> Artificial Sequence
<220>
   <223> Description of Artificial Sequence: Synthetic oligonucleotide
<400> 779
   uuauagagca agaacacugu uuu 23
<210> 780
   <211> 23
   <212> RNA
   <213> Artificial Sequence
<220>
   <223> Description of Artificial Sequence: Synthetic oligonucleotide
<400> 780
   uuauagagca agaacacugu uuu 23
<210> 781
   <211> 23
   <212> RNA
   <213> Artificial Sequence
<220>
   <223> Description of Artificial Sequence: Synthetic oligonucleotide
<400> 781
   uuauagagca agaacacugu uuu 23
<210> 782
   <211> 23
   <212> RNA
   <213> Artificial Sequence
<220>
   <223> Description of Artificial Sequence: Synthetic oligonucleotide
<400> 782
   uuauagagca agaacacugu uuu 23
<210> 783
   <211> 23
   <212> RNA
   <213> Artificial Sequence
<220>
   <223> Description of Artificial Sequence: Synthetic oligonucleotide
<400> 783
   uuauagagca agaacacugu uuu 23
<210> 784
   <211> 23
   <212> RNA
   <213> Artificial Sequence
<220>
   <223> Description of Artificial Sequence: Synthetic oligonucleotide
<400> 784
   uuauagagca agaacacugu uuu 23
<210> 785
   <211> 23
   <212> RNA
   <213> Artificial Sequence
<220>
   <223> Description of Artificial Sequence: Synthetic oligonucleotide
<400> 785
   uuauagagca agaacacugu uuu 23
<210> 786
   <211> 23
   <212> RNA
   <213> Artificial Sequence
<220>
   <223> Description of Artificial Sequence: Synthetic oligonucleotide
<400> 786
   uuauagagca agaacacugu uuu 23
<210> 787
   <211> 23
   <212> RNA
   <213> Artificial Sequence
<220>
   <223> Description of Artificial Sequence: Synthetic oligonucleotide
<400> 787
   uuauagagca agaacacugu uuu 23
<210> 788
   <211> 23
   <212> RNA
   <213> Artificial Sequence
<220>
   <223> Description of Artificial Sequence: Synthetic oligonucleotide
<400> 788
   uuauagagca agaacacugu uuu 23
<210> 789
   <211> 23
   <212> RNA
   <213> Artificial Sequence
<220>
   <223> Description of Artificial Sequence: Synthetic oligonucleotide
<400> 789
   uuauagagca agaacacugu uuu 23
<210> 790
   <211> 23
   <212> RNA
   <213> Artificial Sequence
<220>
   <223> Description of Artificial Sequence: Synthetic oligonucleotide
<400> 790
   uuauagagca agaacacugu uuu 23
<210> 791
   <211> 23
   <212> RNA
   <213> Artificial Sequence
<220>
   <223> Description of Artificial Sequence: Synthetic oligonucleotide
<400> 791
   uuauagagca agaacacugu uuu 23
<210> 792
   <211> 23
   <212> RNA
   <213> Artificial Sequence
<220>
   <223> Description of Artificial Sequence: Synthetic oligonucleotide
<400> 792
   uuauagagca agaacacugu uuu 23
<210> 793
   <211> 23
   <212> RNA
   <213> Artificial Sequence
<220>
   <223> Description of Artificial Sequence: Synthetic oligonucleotide
<400> 793
   uuauagagca agaacacugu uuu 23
<210> 794
   <211> 23
   <212> RNA
   <213> Artificial Sequence
<220>
   <223> Description of Artificial Sequence: Synthetic oligonucleotide
<400> 794
   uuauagagca agaacacugu uuu 23
<210> 795
   <211> 23
   <212> RNA
   <213> Artificial Sequence
<220>
   <223> Description of Artificial Sequence: Synthetic oligonucleotide
<400> 795
   uuauagagca agaacacugu uuu 23
<210> 796
   <211> 23
   <212> RNA
   <213> Artificial Sequence
<220>
   <223> Description of Artificial Sequence: Synthetic oligonucleotide
<400> 796
   uuauagagca agaacacugu uuu 23
<210> 797
   <211> 23
   <212> RNA
   <213> Artificial Sequence
<220>
   <223> Description of Artificial Sequence: Synthetic oligonucleotide
<400> 797
   uuauagagca agaacacugu uuu 23
<210> 798
   <211> 23
   <212> RNA
   <213> Artificial Sequence
<220>
   <223> Description of Artificial Sequence: Synthetic oligonucleotide
<400> 798
   uuauagagca agaacacugu uuu 23
<210> 799
   <211> 23
   <212> RNA
   <213> Artificial Sequence
<220>
   <223> Description of Artificial Sequence: Synthetic oligonucleotide
<400> 799
   uuauagagca agaacacugu uuu 23
<210> 800
   <211> 23
   <212> RNA
   <213> Artificial Sequence
<220>
   <223> Description of Artificial Sequence: Synthetic oligonucleotide
<400> 800
   uuauagagca agaacacugu uuu 23
<210> 801
   <211> 23
   <212> RNA
   <213> Artificial Sequence
<220>
   <223> Description of Artificial Sequence: Synthetic oligonucleotide
<400> 801
   uuauagagca agaacacugu uuu 23
<210> 802
   <211> 23
   <212> RNA
   <213> Artificial Sequence
<220>
   <223> Description of Artificial Sequence: Synthetic oligonucleotide
<400> 802
   uuauagagca agaacacugu uuu 23
<210> 803
   <211> 23
   <212> RNA
   <213> Artificial Sequence
<220>
   <223> Description of Artificial Sequence: Synthetic oligonucleotide
<400> 803
   uuauagagca agaacacugu uuu 23
<210> 804
   <211> 23
   <212> RNA
   <213> Artificial Sequence
<220>
   <223> Description of Artificial Sequence: Synthetic oligonucleotide
<400> 804
   uuauagagca agaacacugu uuu 23
<210> 805
   <211> 23
   <212> RNA
   <213> Artificial Sequence
<220>
   <223> Description of Artificial Sequence: Synthetic oligonucleotide
<400> 805
   uuauagagca agaacacugu uuu 23
<210> 806
   <211> 23
   <212> RNA
   <213> Artificial Sequence
<220>
   <223> Description of Artificial Sequence: Synthetic oligonucleotide
<400> 806
   uuauagagca agaacacugu uuu 23
<210> 807
   <211> 23
   <212> RNA
   <213> Artificial Sequence
<220>
   <223> Description of Artificial Sequence: Synthetic oligonucleotide
<400> 807
   uuauagagca agaacacugu uuu 23
<210> 808
   <211> 23
   <212> RNA
   <213> Artificial Sequence
<220>
   <223> Description of Artificial Sequence: Synthetic oligonucleotide
<400> 808
   uuauagagca agaacacugu uuu 23
<210> 809
   <211> 23
   <212> RNA
   <213> Artificial Sequence
<220>
   <223> Description of Artificial Sequence: Synthetic oligonucleotide
<400> 809
   uuauagagca agaacacugu uuu 23
<210> 810
   <211> 23
   <212> RNA
   <213> Artificial Sequence
<220>
   <223> Description of Artificial Sequence: Synthetic oligonucleotide
<400> 810
   uuauagagca agaacacugu uuu 23
<210> 811
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 811
   uuauagagca agaacacugt tuu 23
<210> 812
   <211> 23
   <212> RNA
   <213> Artificial Sequence
<220>
   <223> Description of Artificial Sequence: Synthetic oligonucleotide
<400> 812
   uuauagagca agaacacugu uuu 23
<210> 813
   <211> 23
   <212> RNA
   <213> Artificial Sequence
<220>
   <223> Description of Artificial Sequence: Synthetic oligonucleotide
<400> 813
   uuauagagca agaacacugu uuu 23
<210> 814
   <211> 23
   <212> RNA
   <213> Artificial Sequence
<220>
   <223> Description of Artificial Sequence: Synthetic oligonucleotide
<400> 814
   uuauagagca agaacacugu uuu 23
<210> 815
   <211> 23
   <212> RNA
   <213> Artificial Sequence
<220>
   <223> Description of Artificial Sequence: Synthetic oligonucleotide
<400> 815
   uuauagagca agaacacugu uuu 23
<210> 816
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 816
   uuauagagca agaacacugu tuu 23
<210> 817
   <211> 23
   <212> RNA
   <213> Artificial Sequence
<220>
   <223> Description of Artificial Sequence: Synthetic oligonucleotide
<400> 817
   uuauagagca agaacacugu uuu 23
<210> 818
   <211> 23
   <212> RNA
   <213> Artificial Sequence
<220>
   <223> Description of Artificial Sequence: Synthetic oligonucleotide
<400> 818
   uuauagagca agaacacugu uuu 23
<210> 819
   <211> 23
   <212> RNA
   <213> Artificial Sequence
<220>
   <223> Description of Artificial Sequence: Synthetic oligonucleotide
<400> 819
   uuauagagca agaacacugu uuu 23
<210> 820
   <211> 23
   <212> RNA
   <213> Artificial Sequence
<220>
   <223> Description of Artificial Sequence: Synthetic oligonucleotide
<400> 820
   uuauagagca agaacacugu uuu 23
<210> 821
   <211> 23
   <212> RNA
   <213> Artificial Sequence
<220>
   <223> Description of Artificial Sequence: Synthetic oligonucleotide
<400> 821
   uuauagagca agaacacugu uuu 23
<210> 822
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 822
   uuatagagca agagcacagu uuu 23
<210> 823
   <211> 23
   <212> RNA
   <213> Artificial Sequence
<220>
   <223> Description of Artificial Sequence: Synthetic oligonucleotide
<400> 823
   uuauagagca agaacacugu uuu 23
<210> 824
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 824
   utatagagca agaacactgt utu 23
<210> 825
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 825
   uuauagagca agagcacagu uuu 23
<210> 826
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 826
   uuauagagca agagcacagu uuu 23
<210> 827
   <211> 23
   <212> RNA
   <213> Artificial Sequence
<220>
   <223> Description of Artificial Sequence: Synthetic oligonucleotide
<400> 827
   uuauagagca agaacacugu uuu 23
<210> 828
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 828
   utauagagca agagcacagu uuu 23
<210> 829
   <211> 23
   <212> RNA
   <213> Artificial Sequence
<220>
   <223> Description of Artificial Sequence: Synthetic oligonucleotide
<400> 829
   uuauagagca agaacacugu uuu 23
<210> 830
   <211> 23
   <212> RNA
   <213> Artificial Sequence
<220>
   <223> Description of Artificial Sequence: Synthetic oligonucleotide
<400> 830
   uuauagagca agaacacugu uuu 23
<210> 831
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 831
   utauagagca agagcacagu uuu 23
<210> 832
   <211> 23
   <212> RNA
   <213> Artificial Sequence
<220>
   <223> Description of Artificial Sequence: Synthetic oligonucleotide
<400> 832
   uuauagagca agaacacugu uuu 23
<210> 833
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 833
   utatagagca agaacacagu uuu 23
<210> 834
   <211> 23
   <212> RNA
   <213> Artificial Sequence
<220>
   <223> Description of Artificial Sequence: Synthetic oligonucleotide
<400> 834
   uuauagagca agaacacugu uuu 23
<210> 835
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 835
   utatagagca agagcacagu uuu 23
<210> 836
   <211> 23
   <212> RNA
   <213> Artificial Sequence
<220>
   <223> Description of Artificial Sequence: Synthetic oligonucleotide
<400> 836
   uuauagagca agaacacugu uuu 23
<210> 837
   <211> 23
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 837
   utatagagca agaacacugt tuu 23
<210> 838
   <211> 23
   <212> RNA
   <213> Artificial Sequence
<220>
   <223> Description of Artificial Sequence: Synthetic oligonucleotide
<400> 838
   uuauagagca agaacacugu uuu 23
<210> 839
   <211> 23
   <212> RNA
   <213> Artificial Sequence
<220>
   <223> Description of Artificial Sequence: Synthetic oligonucleotide
<400> 839
   uuauagagca agaacacugu uuu 23
<210> 840
   <211> 23
   <212> RNA
   <213> Artificial Sequence
<220>
   <223> Description of Artificial Sequence: Synthetic oligonucleotide
<400> 840
   uuauagagca agaacacugu uuu 23
<210> 841
   <211> 23
   <212> RNA
   <213> Artificial Sequence
<220>
   <223> Description of Artificial Sequence: Synthetic oligonucleotide
<400> 841
   uuauagagca agaacacugu uuu 23
<210> 842
   <211> 23
   <212> RNA
   <213> Artificial Sequence
<220>
   <223> Description of Artificial Sequence: Synthetic oligonucleotide
<400> 842
   uuauagagca agaacacugu uuu 23
<210> 843
   <211> 23
   <212> RNA
   <213> Artificial Sequence
<220>
   <223> Description of Artificial Sequence: Synthetic oligonucleotide
<400> 843
   uuauagagca agaacacugu uuu 23
<210> 844
   <211> 23
   <212> RNA
   <213> Artificial Sequence
<220>
   <223> Description of Artificial Sequence: Synthetic oligonucleotide
<400> 844
   uuauagagca agaacacugu uuu 23
<210> 845
   <211> 21
   <212> RNA
   <213> Artificial Sequence
<220>
   <223> Description of Artificial Sequence: Synthetic oligonucleotide
<400> 845
   ucacaauuaa gcuccuucuu u 21
<210> 846
   <211> 21
   <212> RNA
   <213> Artificial Sequence
<220>
   <223> Description of Artificial Sequence: Synthetic oligonucleotide
<400> 846
   uuauuguucc ucuaguuauu u 21
<210> 847
   <211> 21
   <212> RNA
   <213> Artificial Sequence
<220>
   <223> Description of Artificial Sequence: Synthetic oligonucleotide
<400> 847
   gcuauguuag acgauguaaa a 21
<210> 848
   <211> 21
   <212> RNA
   <213> Artificial Sequence
<220>
   <223> Description of Artificial Sequence: Synthetic oligonucleotide
<400> 848
   ggacaugguc uuaaagacuu u 21
<210> 849
   <211> 21
   <212> RNA
   <213> Artificial Sequence
<220>
   <223> Description of Artificial Sequence: Synthetic oligonucleotide
<400> 849
   caaaaacuca acauauuuga u 21
<210> 850
   <211> 21
   <212> RNA
   <213> Artificial Sequence
<220>
   <223> Description of Artificial Sequence: Synthetic oligonucleotide
<400> 850
   accagugaaa ucaaagaaga a 21
<210> 851
   <211> 21
   <212> RNA
   <213> Artificial Sequence
<220>
   <223> Description of Artificial Sequence: Synthetic oligonucleotide
<400> 851
   cacaauuaag cuccuucuuu u 21
<210> 852
   <211> 21
   <212> RNA
   <213> Artificial Sequence
<220>
   <223> Description of Artificial Sequence: Synthetic oligonucleotide
<400> 852
   cuauguuaga cgauguaaaa a 21
<210> 853
   <211> 21
   <212> RNA
   <213> Artificial Sequence
<220>
   <223> Description of Artificial Sequence: Synthetic oligonucleotide
<400> 853
   ucaacauauu ugaucagucu u 21
<210> 854
   <211> 21
   <212> RNA
   <213> Artificial Sequence
<220>
   <223> Description of Artificial Sequence: Synthetic oligonucleotide
<400> 854
   aacugagaag aacuacauau a 21
<210> 855
   <211> 21
   <212> RNA
   <213> Artificial Sequence
<220>
   <223> Description of Artificial Sequence: Synthetic oligonucleotide
<400> 855
   acaauuaagc uccuucuuuu u 21
<210> 856
   <211> 21
   <212> RNA
   <213> Artificial Sequence
<220>
   <223> Description of Artificial Sequence: Synthetic oligonucleotide
<400> 856
   cuccagagcc aaaaucaaga u 21
<210> 857
   <211> 21
   <212> RNA
   <213> Artificial Sequence
<220>
   <223> Description of Artificial Sequence: Synthetic oligonucleotide
<400> 857
   cgauguaaaa auuuuagcca a 21
<210> 858
   <211> 21
   <212> RNA
   <213> Artificial Sequence
<220>
   <223> Description of Artificial Sequence: Synthetic oligonucleotide
<400> 858
   gucuuaaaga cuuuguccau a 21
<210> 859
   <211> 21
   <212> RNA
   <213> Artificial Sequence
<220>
   <223> Description of Artificial Sequence: Synthetic oligonucleotide
<400> 859
   caacauauuu gaucagucuu u 21
<210> 860
   <211> 21
   <212> RNA
   <213> Artificial Sequence
<220>
   <223> Description of Artificial Sequence: Synthetic oligonucleotide
<400> 860
   acugagaaga acuacauaua a 21
<210> 861
   <211> 21
   <212> RNA
   <213> Artificial Sequence
<220>
   <223> Description of Artificial Sequence: Synthetic oligonucleotide
<400> 861
   ccagagccaa aaucaagauu u 21
<210> 862
   <211> 21
   <212> RNA
   <213> Artificial Sequence
<220>
   <223> Description of Artificial Sequence: Synthetic oligonucleotide
<400> 862
   gauguaaaaa uuuuagccaa u 21
<210> 863
   <211> 21
   <212> RNA
   <213> Artificial Sequence
<220>
   <223> Description of Artificial Sequence: Synthetic oligonucleotide
<400> 863
   ucuuaaagac uuuguccaua a 21
<210> 864
   <211> 21
   <212> RNA
   <213> Artificial Sequence
<220>
   <223> Description of Artificial Sequence: Synthetic oligonucleotide
<400> 864
   aacauauuug aucagucuuu u 21
<210> 865
   <211> 21
   <212> RNA
   <213> Artificial Sequence
<220>
   <223> Description of Artificial Sequence: Synthetic oligonucleotide
<400> 865
   cugagaagaa cuacauauaa a 21
<210> 866
   <211> 21
   <212> RNA
   <213> Artificial Sequence
<220>
   <223> Description of Artificial Sequence: Synthetic oligonucleotide
<400> 866
   aauuaagcuc cuucuuuuua u 21
<210> 867
   <211> 21
   <212> RNA
   <213> Artificial Sequence
<220>
   <223> Description of Artificial Sequence: Synthetic oligonucleotide
<400> 867
   aaaucaagau uugcuauguu a 21
<210> 868
   <211> 21
   <212> RNA
   <213> Artificial Sequence
<220>
   <223> Description of Artificial Sequence: Synthetic oligonucleotide
<400> 868
   uucaguuggg acauggucuu a 21
<210> 869
   <211> 21
   <212> RNA
   <213> Artificial Sequence
<220>
   <223> Description of Artificial Sequence: Synthetic oligonucleotide
<400> 869
   gggccaaauu aaugacauau u 21
<210> 870
   <211> 21
   <212> RNA
   <213> Artificial Sequence
<220>
   <223> Description of Artificial Sequence: Synthetic oligonucleotide
<400> 870
   acauauuuga ucagucuuuu u 21
<210> 871
   <211> 21
   <212> RNA
   <213> Artificial Sequence
<220>
   <223> Description of Artificial Sequence: Synthetic oligonucleotide
<400> 871
   agaacuacau auaaacuaca a 21
<210> 872
   <211> 21
   <212> RNA
   <213> Artificial Sequence
<220>
   <223> Description of Artificial Sequence: Synthetic oligonucleotide
<400> 872
   auuaagcucc uucuuuuuau u 21
<210> 873
   <211> 21
   <212> RNA
   <213> Artificial Sequence
<220>
   <223> Description of Artificial Sequence: Synthetic oligonucleotide
<400> 873
   agauuugcua uguuagacga u 21
<210> 874
   <211> 21
   <212> RNA
   <213> Artificial Sequence
<220>
   <223> Description of Artificial Sequence: Synthetic oligonucleotide
<400> 874
   ucaguuggga cauggucuua a 21
<210> 875
   <211> 21
   <212> RNA
   <213> Artificial Sequence
<220>
   <223> Description of Artificial Sequence: Synthetic oligonucleotide
<400> 875
   ggccaaauua augacauauu u 21
<210> 876
   <211> 21
   <212> RNA
   <213> Artificial Sequence
<220>
   <223> Description of Artificial Sequence: Synthetic oligonucleotide
<400> 876
   cauauuugau cagucuuuuu a 21
<210> 877
   <211> 21
   <212> RNA
   <213> Artificial Sequence
<220>
   <223> Description of Artificial Sequence: Synthetic oligonucleotide
<400> 877
   uacauauaaa cuacaaguca a 21
<210> 878
   <211> 21
   <212> RNA
   <213> Artificial Sequence
<220>
   <223> Description of Artificial Sequence: Synthetic oligonucleotide
<400> 878
   uuuuauuguu ccucuaguua u 21
<210> 879
   <211> 21
   <212> RNA
   <213> Artificial Sequence
<220>
   <223> Description of Artificial Sequence: Synthetic oligonucleotide
<400> 879
   uugcuauguu agacgaugua a 21
<210> 880
   <211> 21
   <212> RNA
   <213> Artificial Sequence
<220>
   <223> Description of Artificial Sequence: Synthetic oligonucleotide
<400> 880
   caguugggac auggucuuaa a 21
<210> 881
   <211> 21
   <212> RNA
   <213> Artificial Sequence
<220>
   <223> Description of Artificial Sequence: Synthetic oligonucleotide
<400> 881
   aaauuaauga cauauuucaa a 21
<210> 882
   <211> 21
   <212> RNA
   <213> Artificial Sequence
<220>
   <223> Description of Artificial Sequence: Synthetic oligonucleotide
<400> 882
   gaucagucuu uuuaugaucu a 21
<210> 883
   <211> 21
   <212> RNA
   <213> Artificial Sequence
<220>
   <223> Description of Artificial Sequence: Synthetic oligonucleotide
<400> 883
   acauauaaac uacaagucaa a 21
<210> 884
   <211> 21
   <212> RNA
   <213> Artificial Sequence
<220>
   <223> Description of Artificial Sequence: Synthetic oligonucleotide
<400> 884
   uuuauuguuc cucuaguuau u 21
<210> 885
   <211> 21
   <212> RNA
   <213> Artificial Sequence
<220>
   <223> Description of Artificial Sequence: Synthetic oligonucleotide
<400> 885
   ugcuauguua gacgauguaa a 21
<210> 886
   <211> 21
   <212> RNA
   <213> Artificial Sequence
<220>
   <223> Description of Artificial Sequence: Synthetic oligonucleotide
<400> 886
   gggacauggu cuuaaagacu u 21
<210> 887
   <211> 21
   <212> RNA
   <213> Artificial Sequence
<220>
   <223> Description of Artificial Sequence: Synthetic oligonucleotide
<400> 887
   ugacauauuu caaaaacuca a 21
<210> 888
   <211> 21
   <212> RNA
   <213> Artificial Sequence
<220>
   <223> Description of Artificial Sequence: Synthetic oligonucleotide
<400> 888
   aucagucuuu uuaugaucua u 21
<210> 889
   <211> 21
   <212> RNA
   <213> Artificial Sequence
<220>
   <223> Description of Artificial Sequence: Synthetic oligonucleotide
<400> 889
   cauauaaacu acaagucaaa a 21
<210> 890
   <211> 21
   <212> RNA
   <213> Artificial Sequence
<220>
   <223> Description of Artificial Sequence: Synthetic oligonucleotide
<400> 890
   cuugaacuca acucaaaacu u 21
<210> 891
   <211> 21
   <212> RNA
   <213> Artificial Sequence
<220>
   <223> Description of Artificial Sequence: Synthetic oligonucleotide
<400> 891
   cuacuucaac aaaaagugaa a 21
<210> 892
   <211> 21
   <212> RNA
   <213> Artificial Sequence
<220>
   <223> Description of Artificial Sequence: Synthetic oligonucleotide
<400> 892
   aagagcaacu aacuaacuua a 21
<210> 893
   <211> 21
   <212> RNA
   <213> Artificial Sequence
<220>
   <223> Description of Artificial Sequence: Synthetic oligonucleotide
<400> 893
   aaacaagaua auagcaucaa a 21
<210> 894
   <211> 21
   <212> RNA
   <213> Artificial Sequence
<220>
   <223> Description of Artificial Sequence: Synthetic oligonucleotide
<400> 894
   gcauagucaa auaaaagaaa u 21
<210> 895
   <211> 21
   <212> RNA
   <213> Artificial Sequence
<220>
   <223> Description of Artificial Sequence: Synthetic oligonucleotide
<400> 895
   auauaaacua caagucaaaa a 21
<210> 896
   <211> 21
   <212> RNA
   <213> Artificial Sequence
<220>
   <223> Description of Artificial Sequence: Synthetic oligonucleotide
<400> 896
   gaacucaacu caaaacuuga a 21
<210> 897
   <211> 21
   <212> RNA
   <213> Artificial Sequence
<220>
   <223> Description of Artificial Sequence: Synthetic oligonucleotide
<400> 897
   uacuucaaca aaaagugaaa u 21
<210> 898
   <211> 21
   <212> RNA
   <213> Artificial Sequence
<220>
   <223> Description of Artificial Sequence: Synthetic oligonucleotide
<400> 898
   agagcaacua acuaacuuaa u 21
<210> 899
   <211> 21
   <212> RNA
   <213> Artificial Sequence
<220>
   <223> Description of Artificial Sequence: Synthetic oligonucleotide
<400> 899
   gauaauagca ucaaagaccu u 21
<210> 900
   <211> 21
   <212> RNA
   <213> Artificial Sequence
<220>
   <223> Description of Artificial Sequence: Synthetic oligonucleotide
<400> 900
   cauagucaaa uaaaagaaau a 21
<210> 901
   <211> 21
   <212> RNA
   <213> Artificial Sequence
<220>
   <223> Description of Artificial Sequence: Synthetic oligonucleotide
<400> 901
   uauaaacuac aagucaaaaa u 21
<210> 902
   <211> 21
   <212> RNA
   <213> Artificial Sequence
<220>
   <223> Description of Artificial Sequence: Synthetic oligonucleotide
<400> 902
   aacucaacuc aaaacuugaa a 21
<210> 903
   <211> 21
   <212> RNA
   <213> Artificial Sequence
<220>
   <223> Description of Artificial Sequence: Synthetic oligonucleotide
<400> 903
   acuucaacaa aaagugaaau a 21
<210> 904
   <211> 21
   <212> RNA
   <213> Artificial Sequence
<220>
   <223> Description of Artificial Sequence: Synthetic oligonucleotide
<400> 904
   gagcaacuaa cuaacuuaau u 21
<210> 905
   <211> 21
   <212> RNA
   <213> Artificial Sequence
<220>
   <223> Description of Artificial Sequence: Synthetic oligonucleotide
<400> 905
   aaccaacagc auagucaaau a 21
<210> 906
   <211> 21
   <212> RNA
   <213> Artificial Sequence
<220>
   <223> Description of Artificial Sequence: Synthetic oligonucleotide
<400> 906
   agucaaauaa aagaaauaga a 21
<210> 907
   <211> 21
   <212> RNA
   <213> Artificial Sequence
<220>
   <223> Description of Artificial Sequence: Synthetic oligonucleotide
<400> 907
   agucaaaaau gaagagguaa a 21
<210> 908
   <211> 21
   <212> RNA
   <213> Artificial Sequence
<220>
   <223> Description of Artificial Sequence: Synthetic oligonucleotide
<400> 908
   cuugaaagcc uccuagaaga a 21
<210> 909
   <211> 21
   <212> RNA
   <213> Artificial Sequence
<220>
   <223> Description of Artificial Sequence: Synthetic oligonucleotide
<400> 909
   cuucaacaaa aagugaaaua u 21
<210> 910
   <211> 21
   <212> RNA
   <213> Artificial Sequence
<220>
   <223> Description of Artificial Sequence: Synthetic oligonucleotide
<400> 910
   caacuaacua acuuaauuca a 21
<210> 911
   <211> 21
   <212> RNA
   <213> Artificial Sequence
<220>
   <223> Description of Artificial Sequence: Synthetic oligonucleotide
<400> 911
   accaacagca uagucaaaua a 21
<210> 912
   <211> 21
   <212> RNA
   <213> Artificial Sequence
<220>
   <223> Description of Artificial Sequence: Synthetic oligonucleotide
<400> 912
   gaacccacag aaauuucucu a 21
<210> 913
   <211> 21
   <212> RNA
   <213> Artificial Sequence
<220>
   <223> Description of Artificial Sequence: Synthetic oligonucleotide
<400> 913
   gaauauguca cuugaacuca a 21
<210> 914
   <211> 21
   <212> RNA
   <213> Artificial Sequence
<220>
   <223> Description of Artificial Sequence: Synthetic oligonucleotide
<400> 914
   ugaaagccuc cuagaagaaa a 21
<210> 915
   <211> 21
   <212> RNA
   <213> Artificial Sequence
<220>
   <223> Description of Artificial Sequence: Synthetic oligonucleotide
<400> 915
   uucaacaaaa agugaaauau u 21
<210> 916
   <211> 21
   <212> RNA
   <213> Artificial Sequence
<220>
   <223> Description of Artificial Sequence: Synthetic oligonucleotide
<400> 916
   aacuaacuaa cuuaauucaa a 21
<210> 917
   <211> 21
   <212> RNA
   <213> Artificial Sequence
<220>
   <223> Description of Artificial Sequence: Synthetic oligonucleotide
<400> 917
   ccaacagcau agucaaauaa a 21
<210> 918
   <211> 21
   <212> RNA
   <213> Artificial Sequence
<220>
   <223> Description of Artificial Sequence: Synthetic oligonucleotide
<400> 918
   aacccacaga aauuucucua u 21
<210> 919
   <211> 21
   <212> RNA
   <213> Artificial Sequence
<220>
   <223> Description of Artificial Sequence: Synthetic oligonucleotide
<400> 919
   ugucacuuga acucaacuca a 21
<210> 920
   <211> 21
   <212> RNA
   <213> Artificial Sequence
<220>
   <223> Description of Artificial Sequence: Synthetic oligonucleotide
<400> 920
   gaaagccucc uagaagaaaa a 21
<210> 921
   <211> 21
   <212> RNA
   <213> Artificial Sequence
<220>
   <223> Description of Artificial Sequence: Synthetic oligonucleotide
<400> 921
   aauauuuaga agagcaacua a 21
<210> 922
   <211> 21
   <212> RNA
   <213> Artificial Sequence
<220>
   <223> Description of Artificial Sequence: Synthetic oligonucleotide
<400> 922
   acuaacuaac uuaauucaaa a 21
<210> 923
   <211> 21
   <212> RNA
   <213> Artificial Sequence
<220>
   <223> Description of Artificial Sequence: Synthetic oligonucleotide
<400> 923
   caacagcaua gucaaauaaa a 21
<210> 924
   <211> 21
   <212> RNA
   <213> Artificial Sequence
<220>
   <223> Description of Artificial Sequence: Synthetic oligonucleotide
<400> 924
   ccacagaaau uucucuaucu u 21
<210> 925
   <211> 21
   <212> RNA
   <213> Artificial Sequence
<220>
   <223> Description of Artificial Sequence: Synthetic oligonucleotide
<400> 925
   gucacuugaa cucaacucaa a 21
<210> 926
   <211> 21
   <212> RNA
   <213> Artificial Sequence
<220>
   <223> Description of Artificial Sequence: Synthetic oligonucleotide
<400> 926
   cuccuagaag aaaaaauucu a 21
<210> 927
   <211> 21
   <212> RNA
   <213> Artificial Sequence
<220>
   <223> Description of Artificial Sequence: Synthetic oligonucleotide
<400> 927
   auuuagaaga gcaacuaacu a 21
<210> 928
   <211> 21
   <212> RNA
   <213> Artificial Sequence
<220>
   <223> Description of Artificial Sequence: Synthetic oligonucleotide
<400> 928
   cuaacuaacu uaauucaaaa u 21
<210> 929
   <211> 21
   <212> RNA
   <213> Artificial Sequence
<220>
   <223> Description of Artificial Sequence: Synthetic oligonucleotide
<400> 929
   cagcauaguc aaauaaaaga a 21
<210> 930
   <211> 21
   <212> RNA
   <213> Artificial Sequence
<220>
   <223> Description of Artificial Sequence: Synthetic oligonucleotide
<400> 930
   gaaauaagaa auguaaaaca u 21
<210> 931
   <211> 21
   <212> RNA
   <213> Artificial Sequence
<220>
   <223> Description of Artificial Sequence: Synthetic oligonucleotide
<400> 931
   ucacuugaac ucaacucaaa a 21
<210> 932
   <211> 21
   <212> RNA
   <213> Artificial Sequence
<220>
   <223> Description of Artificial Sequence: Synthetic oligonucleotide
<400> 932
   ucuacuucaa caaaaaguga a 21
<210> 933
   <211> 21
   <212> RNA
   <213> Artificial Sequence
<220>
   <223> Description of Artificial Sequence: Synthetic oligonucleotide
<400> 933
   uuuagaagag caacuaacua a 21
<210> 934
   <211> 21
   <212> RNA
   <213> Artificial Sequence
<220>
   <223> Description of Artificial Sequence: Synthetic oligonucleotide
<400> 934
   aaaacaagau aauagcauca a 21
<210> 935
   <211> 21
   <212> RNA
   <213> Artificial Sequence
<220>
   <223> Description of Artificial Sequence: Synthetic oligonucleotide
<400> 935
   agcauaguca aauaaaagaa a 21
<210> 936
   <211> 21
   <212> RNA
   <213> Artificial Sequence
<220>
   <223> Description of Artificial Sequence: Synthetic oligonucleotide
<400> 936
   agacccagca acucucaagu u 21
<210> 937
   <211> 21
   <212> RNA
   <213> Artificial Sequence
<220>
   <223> Description of Artificial Sequence: Synthetic oligonucleotide
<400> 937
   aguccaugga cauuaauuca a 21
<210> 938
   <211> 21
   <212> RNA
   <213> Artificial Sequence
<220>
   <223> Description of Artificial Sequence: Synthetic oligonucleotide
<400> 938
   gauggaucac aaaacuucaa u 21
<210> 939
   <211> 21
   <212> RNA
   <213> Artificial Sequence
<220>
   <223> Description of Artificial Sequence: Synthetic oligonucleotide
<400> 939
   cuagagaaga uauacuccau a 21
<210> 940
   <211> 21
   <212> RNA
   <213> Artificial Sequence
<220>
   <223> Description of Artificial Sequence: Synthetic oligonucleotide
<400> 940
   aaagacaaca aacauuauau u 21
<210> 941
   <211> 21
   <212> RNA
   <213> Artificial Sequence
<220>
   <223> Description of Artificial Sequence: Synthetic oligonucleotide
<400> 941
   cauuauauug aauauucuuu u 21
<210> 942
   <211> 21
   <212> RNA
   <213> Artificial Sequence
<220>
   <223> Description of Artificial Sequence: Synthetic oligonucleotide
<400> 942
   gacccagcaa cucucaaguu u 21
<210> 943
   <211> 21
   <212> RNA
   <213> Artificial Sequence
<220>
   <223> Description of Artificial Sequence: Synthetic oligonucleotide
<400> 943
   ggaucacaaa acuucaauga a 21
<210> 944
   <211> 21
   <212> RNA
   <213> Artificial Sequence
<220>
   <223> Description of Artificial Sequence: Synthetic oligonucleotide
<400> 944
   gaagauauac uccauaguga a 21
<210> 945
   <211> 21
   <212> RNA
   <213> Artificial Sequence
<220>
   <223> Description of Artificial Sequence: Synthetic oligonucleotide
<400> 945
   gacaacaaac auuauauuga a 21
<210> 946
   <211> 21
   <212> RNA
   <213> Artificial Sequence
<220>
   <223> Description of Artificial Sequence: Synthetic oligonucleotide
<400> 946
   gggaaaucac gaaaccaacu a 21
<210> 947
   <211> 21
   <212> RNA
   <213> Artificial Sequence
<220>
   <223> Description of Artificial Sequence: Synthetic oligonucleotide
<400> 947
   acccagcaac ucucaaguuu u 21
<210> 948
   <211> 21
   <212> RNA
   <213> Artificial Sequence
<220>
   <223> Description of Artificial Sequence: Synthetic oligonucleotide
<400> 948
   ggacauuaau ucaacaucga a 21
<210> 949
   <211> 21
   <212> RNA
   <213> Artificial Sequence
<220>
   <223> Description of Artificial Sequence: Synthetic oligonucleotide
<400> 949
   gaucacaaaa cuucaaugaa a 21
<210> 950
   <211> 21
   <212> RNA
   <213> Artificial Sequence
<220>
   <223> Description of Artificial Sequence: Synthetic oligonucleotide
<400> 950
   acuccauagu gaagcaaucu a 21
<210> 951
   <211> 21
   <212> RNA
   <213> Artificial Sequence
<220>
   <223> Description of Artificial Sequence: Synthetic oligonucleotide
<400> 951
   acaacaaaca uuauauugaa u 21
<210> 952
   <211> 21
   <212> RNA
   <213> Artificial Sequence
<220>
   <223> Description of Artificial Sequence: Synthetic oligonucleotide
<400> 952
   ggaaaucacg aaaccaacua u 21
<210> 953
   <211> 21
   <212> RNA
   <213> Artificial Sequence
<220>
   <223> Description of Artificial Sequence: Synthetic oligonucleotide
<400> 953
   cccagcaacu cucaaguuuu u 21
<210> 954
   <211> 21
   <212> RNA
   <213> Artificial Sequence
<220>
   <223> Description of Artificial Sequence: Synthetic oligonucleotide
<400> 954
   gacauuaauu caacaucgaa u 21
<210> 955
   <211> 21
   <212> RNA
   <213> Artificial Sequence
<220>
   <223> Description of Artificial Sequence: Synthetic oligonucleotide
<400> 955
   aacgugggag aacuacaaau a 21
<210> 956
   <211> 21
   <212> RNA
   <213> Artificial Sequence
<220>
   <223> Description of Artificial Sequence: Synthetic oligonucleotide
<400> 956
   cuccauagug aagcaaucua a 21
<210> 957
   <211> 21
   <212> RNA
   <213> Artificial Sequence
<220>
   <223> Description of Artificial Sequence: Synthetic oligonucleotide
<400> 957
   caacaaacau uauauugaau a 21
<210> 958
   <211> 21
   <212> RNA
   <213> Artificial Sequence
<220>
   <223> Description of Artificial Sequence: Synthetic oligonucleotide
<400> 958
   gaaaucacga aaccaacuau a 21
<210> 959
   <211> 21
   <212> RNA
   <213> Artificial Sequence
<220>
   <223> Description of Artificial Sequence: Synthetic oligonucleotide
<400> 959
   cucucaaguu uuucaugucu a 21
<210> 960
   <211> 21
   <212> RNA
   <213> Artificial Sequence
<220>
   <223> Description of Artificial Sequence: Synthetic oligonucleotide
<400> 960
   acauuaauuc aacaucgaau a 21
<210> 961
   <211> 21
   <212> RNA
   <213> Artificial Sequence
<220>
   <223> Description of Artificial Sequence: Synthetic oligonucleotide
<400> 961
   gggagaacua caaauauggu u 21
<210> 962
   <211> 21
   <212> RNA
   <213> Artificial Sequence
<220>
   <223> Description of Artificial Sequence: Synthetic oligonucleotide
<400> 962
   uccauaguga agcaaucuaa u 21
<210> 963
   <211> 21
   <212> RNA
   <213> Artificial Sequence
<220>
   <223> Description of Artificial Sequence: Synthetic oligonucleotide
<400> 963
   aacaaacauu auauugaaua u 21
<210> 964
   <211> 21
   <212> RNA
   <213> Artificial Sequence
<220>
   <223> Description of Artificial Sequence: Synthetic oligonucleotide
<400> 964
   uggcaauguc cccaaugcaa u 21
<210> 965
   <211> 21
   <212> RNA
   <213> Artificial Sequence
<220>
   <223> Description of Artificial Sequence: Synthetic oligonucleotide
<400> 965
   ucagguaguc cauggacauu a 21
<210> 966
   <211> 21
   <212> RNA
   <213> Artificial Sequence
<220>
   <223> Description of Artificial Sequence: Synthetic oligonucleotide
<400> 966
   uuaauucaac aucgaauaga u 21
<210> 967
   <211> 21
   <212> RNA
   <213> Artificial Sequence
<220>
   <223> Description of Artificial Sequence: Synthetic oligonucleotide
<400> 967
   ggagaacuac aaauaugguu u 21
<210> 968
   <211> 21
   <212> RNA
   <213> Artificial Sequence
<220>
   <223> Description of Artificial Sequence: Synthetic oligonucleotide
<400> 968
   ccauagugaa gcaaucuaau u 21
<210> 969
   <211> 21
   <212> RNA
   <213> Artificial Sequence
<220>
   <223> Description of Artificial Sequence: Synthetic oligonucleotide
<400> 969
   acaaacauua uauugaauau u 21
<210> 970
   <211> 21
   <212> RNA
   <213> Artificial Sequence
<220>
   <223> Description of Artificial Sequence: Synthetic oligonucleotide
<400> 970
   aaugcaaucc cggaaaacaa a 21
<210> 971
   <211> 21
   <212> RNA
   <213> Artificial Sequence
<220>
   <223> Description of Artificial Sequence: Synthetic oligonucleotide
<400> 971
   cagguagucc auggacauua a 21
<210> 972
   <211> 21
   <212> RNA
   <213> Artificial Sequence
<220>
   <223> Description of Artificial Sequence: Synthetic oligonucleotide
<400> 972
   uucaacaucg aauagaugga u 21
<210> 973
   <211> 21
   <212> RNA
   <213> Artificial Sequence
<220>
   <223> Description of Artificial Sequence: Synthetic oligonucleotide
<400> 973
   guugggccua gagaagauau a 21
<210> 974
   <211> 21
   <212> RNA
   <213> Artificial Sequence
<220>
   <223> Description of Artificial Sequence: Synthetic oligonucleotide
<400> 974
   cauagugaag caaucuaauu a 21
<210> 975
   <211> 21
   <212> RNA
   <213> Artificial Sequence
<220>
   <223> Description of Artificial Sequence: Synthetic oligonucleotide
<400> 975
   aacauuauau ugaauauucu u 21
<210> 976
   <211> 21
   <212> RNA
   <213> Artificial Sequence
<220>
   <223> Description of Artificial Sequence: Synthetic oligonucleotide
<400> 976
   gcaaucccgg aaaacaaaga u 21
<210> 977
   <211> 21
   <212> RNA
   <213> Artificial Sequence
<220>
   <223> Description of Artificial Sequence: Synthetic oligonucleotide
<400> 977
   gguaguccau ggacauuaau u 21
<210> 978
   <211> 21
   <212> RNA
   <213> Artificial Sequence
<220>
   <223> Description of Artificial Sequence: Synthetic oligonucleotide
<400> 978
   aucgaauaga uggaucacaa a 21
<210> 979
   <211> 21
   <212> RNA
   <213> Artificial Sequence
<220>
   <223> Description of Artificial Sequence: Synthetic oligonucleotide
<400> 979
   ccuagagaag auauacucca u 21
<210> 980
   <211> 21
   <212> RNA
   <213> Artificial Sequence
<220>
   <223> Description of Artificial Sequence: Synthetic oligonucleotide
<400> 980
   guuggaagac uggaaagaca a 21
<210> 981
   <211> 21
   <212> RNA
   <213> Artificial Sequence
<220>
   <223> Description of Artificial Sequence: Synthetic oligonucleotide
<400> 981
   acauuauauu gaauauucuu u 21
<210> 982
   <211> 21
   <212> RNA
   <213> Artificial Sequence
<220>
   <223> Description of Artificial Sequence: Synthetic oligonucleotide
<400> 982
   caaucccgga aaacaaagau u 21
<210> 983
   <211> 21
   <212> RNA
   <213> Artificial Sequence
<220>
   <223> Description of Artificial Sequence: Synthetic oligonucleotide
<400> 983
   cuacuuggga ucacaaagca a 21
<210> 984
   <211> 21
   <212> RNA
   <213> Artificial Sequence
<220>
   <223> Description of Artificial Sequence: Synthetic oligonucleotide
<400> 984
   acaaccuaaa ugguaaauau a 21
<210> 985
   <211> 21
   <212> RNA
   <213> Artificial Sequence
<220>
   <223> Description of Artificial Sequence: Synthetic oligonucleotide
<400> 985
   auccauccaa cagauucaga a 21
<210> 986
   <211> 21
   <212> RNA
   <213> Artificial Sequence
<220>
   <223> Description of Artificial Sequence: Synthetic oligonucleotide
<400> 986
   aacugaggca aauuuaaaag a 21
<210> 987
   <211> 21
   <212> RNA
   <213> Artificial Sequence
<220>
   <223> Description of Artificial Sequence: Synthetic oligonucleotide
<400> 987
   agaguaugug uaaaaaucug u 21
<210> 988
   <211> 21
   <212> RNA
   <213> Artificial Sequence
<220>
   <223> Description of Artificial Sequence: Synthetic oligonucleotide
<400> 988
   aaucccggaa aacaaagauu u 21
<210> 989
   <211> 21
   <212> RNA
   <213> Artificial Sequence
<220>
   <223> Description of Artificial Sequence: Synthetic oligonucleotide
<400> 989
   uacuugggau cacaaagcaa a 21
<210> 990
   <211> 21
   <212> RNA
   <213> Artificial Sequence
<220>
   <223> Description of Artificial Sequence: Synthetic oligonucleotide
<400> 990
   caaccuaaau gguaaauaua a 21
<210> 991
   <211> 21
   <212> RNA
   <213> Artificial Sequence
<220>
   <223> Description of Artificial Sequence: Synthetic oligonucleotide
<400> 991
   uugaaugaac ugaggcaaau u 21
<210> 992
   <211> 21
   <212> RNA
   <213> Artificial Sequence
<220>
   <223> Description of Artificial Sequence: Synthetic oligonucleotide
<400> 992
   acugaggcaa auuuaaaagg a 21
<210> 993
   <211> 21
   <212> RNA
   <213> Artificial Sequence
<220>
   <223> Description of Artificial Sequence: Synthetic oligonucleotide
<400> 993
   gaguaugugu aaaaaucugu a 21
<210> 994
   <211> 21
   <212> RNA
   <213> Artificial Sequence
<220>
   <223> Description of Artificial Sequence: Synthetic oligonucleotide
<400> 994
   acuugggauc acaaagcaaa a 21
<210> 995
   <211> 21
   <212> RNA
   <213> Artificial Sequence
<220>
   <223> Description of Artificial Sequence: Synthetic oligonucleotide
<400> 995
   augguaaaua uaacaaacca a 21
<210> 996
   <211> 21
   <212> RNA
   <213> Artificial Sequence
<220>
   <223> Description of Artificial Sequence: Synthetic oligonucleotide
<400> 996
   ugaaugaacu gaggcaaauu u 21
<210> 997
   <211> 21
   <212> RNA
   <213> Artificial Sequence
<220>
   <223> Description of Artificial Sequence: Synthetic oligonucleotide
<400> 997
   cugaggcaaa uuuaaaaggc a 21
<210> 998
   <211> 21
   <212> RNA
   <213> Artificial Sequence
<220>
   <223> Description of Artificial Sequence: Synthetic oligonucleotide
<400> 998
   aguaugugua aaaaucugua a 21
<210> 999
   <211> 21
   <212> RNA
   <213> Artificial Sequence
<220>
   <223> Description of Artificial Sequence: Synthetic oligonucleotide
<400> 999
   gaaaacaaag auuugguguu u 21
<210> 1000
   <211> 21
   <212> RNA
   <213> Artificial Sequence
<220>
   <223> Description of Artificial Sequence: Synthetic oligonucleotide
<400> 1000
   aguguggaga aaacaaccua a 21
<210> 1001
   <211> 21
   <212> RNA
   <213> Artificial Sequence
<220>
   <223> Description of Artificial Sequence: Synthetic oligonucleotide
<400> 1001
   gucucaaaau ggaagguuau a 21
<210> 1002
   <211> 21
   <212> RNA
   <213> Artificial Sequence
<220>
   <223> Description of Artificial Sequence: Synthetic oligonucleotide
<400> 1002
   gaaugaacug aggcaaauuu a 21
<210> 1003
   <211> 21
   <212> RNA
   <213> Artificial Sequence
<220>
   <223> Description of Artificial Sequence: Synthetic oligonucleotide
<400> 1003
   ugaggcaaau uuaaaaggca a 21
<210> 1004
   <211> 21
   <212> RNA
   <213> Artificial Sequence
<220>
   <223> Description of Artificial Sequence: Synthetic oligonucleotide
<400> 1004
   guauguguaa aaaucuguaa u 21
<210> 1005
   <211> 21
   <212> RNA
   <213> Artificial Sequence
<220>
   <223> Description of Artificial Sequence: Synthetic oligonucleotide
<400> 1005
   aaaacaaaga uuugguguuu u 21
<210> 1006
   <211> 21
   <212> RNA
   <213> Artificial Sequence
<220>
   <223> Description of Artificial Sequence: Synthetic oligonucleotide
<400> 1006
   guguggagaa aacaaccuaa a 21
<210> 1007
   <211> 21
   <212> RNA
   <213> Artificial Sequence
<220>
   <223> Description of Artificial Sequence: Synthetic oligonucleotide
<400> 1007
   auggaagguu auacucuaua a 21
<210> 1008
   <211> 21
   <212> RNA
   <213> Artificial Sequence
<220>
   <223> Description of Artificial Sequence: Synthetic oligonucleotide
<400> 1008
   aaugaacuga ggcaaauuua a 21
<210> 1009
   <211> 21
   <212> RNA
   <213> Artificial Sequence
<220>
   <223> Description of Artificial Sequence: Synthetic oligonucleotide
<400> 1009
   gaggcaaauu uaaaaggcaa u 21
<210> 1010
   <211> 21
   <212> RNA
   <213> Artificial Sequence
<220>
   <223> Description of Artificial Sequence: Synthetic oligonucleotide
<400> 1010
   uauguguaaa aaucuguaau a 21
<210> 1011
   <211> 21
   <212> RNA
   <213> Artificial Sequence
<220>
   <223> Description of Artificial Sequence: Synthetic oligonucleotide
<400> 1011
   acaaagauuu gguguuuucu a 21
<210> 1012
   <211> 21
   <212> RNA
   <213> Artificial Sequence
<220>
   <223> Description of Artificial Sequence: Synthetic oligonucleotide
<400> 1012
   uguggagaaa acaaccuaaa u 21
<210> 1013
   <211> 21
   <212> RNA
   <213> Artificial Sequence
<220>
   <223> Description of Artificial Sequence: Synthetic oligonucleotide
<400> 1013
   uggaagguua uacucuauaa a 21
<210> 1014
   <211> 21
   <212> RNA
   <213> Artificial Sequence
<220>
   <223> Description of Artificial Sequence: Synthetic oligonucleotide
<400> 1014
   augaacugag gcaaauuuaa a 21
<210> 1015
   <211> 21
   <212> RNA
   <213> Artificial Sequence
<220>
   <223> Description of Artificial Sequence: Synthetic oligonucleotide
<400> 1015
   aggcaaauuu aaaaggcaau a 21
<210> 1016
   <211> 21
   <212> RNA
   <213> Artificial Sequence
<220>
   <223> Description of Artificial Sequence: Synthetic oligonucleotide
<400> 1016
   aagauuuggu guuuucuacu u 21
<210> 1017
   <211> 21
   <212> RNA
   <213> Artificial Sequence
<220>
   <223> Description of Artificial Sequence: Synthetic oligonucleotide
<400> 1017
   aaacaaccua aaugguaaau a 21
<210> 1018
   <211> 21
   <212> RNA
   <213> Artificial Sequence
<220>
   <223> Description of Artificial Sequence: Synthetic oligonucleotide
<400> 1018
   auacucuaua aaaucaacca a 21
<210> 1019
   <211> 21
   <212> RNA
   <213> Artificial Sequence
<220>
   <223> Description of Artificial Sequence: Synthetic oligonucleotide
<400> 1019
   ugaacugagg caaauuuaaa a 21
<210> 1020
   <211> 21
   <212> RNA
   <213> Artificial Sequence
<220>
   <223> Description of Artificial Sequence: Synthetic oligonucleotide
<400> 1020
   ggcaaauuua aaaggcaaua a 21
<210> 1021
   <211> 21
   <212> RNA
   <213> Artificial Sequence
<220>
   <223> Description of Artificial Sequence: Synthetic oligonucleotide
<400> 1021
   uuuucuacuu gggaucacaa a 21
<210> 1022
   <211> 21
   <212> RNA
   <213> Artificial Sequence
<220>
   <223> Description of Artificial Sequence: Synthetic oligonucleotide
<400> 1022
   aacaaccuaa augguaaaua u 21
<210> 1023
   <211> 21
   <212> RNA
   <213> Artificial Sequence
<220>
   <223> Description of Artificial Sequence: Synthetic oligonucleotide
<400> 1023
   uacucuauaa aaucaaccaa a 21
<210> 1024
   <211> 21
   <212> RNA
   <213> Artificial Sequence
<220>
   <223> Description of Artificial Sequence: Synthetic oligonucleotide
<400> 1024
   gaacugaggc aaauuuaaaa a 21
<210> 1025
   <211> 21
   <212> RNA
   <213> Artificial Sequence
<220>
   <223> Description of Artificial Sequence: Synthetic oligonucleotide
<400> 1025
   cagaguaugu guaaaaaucu u 21
<210> 1026
   <211> 23
   <212> RNA
   <213> Artificial Sequence
<220>
   <223> Description of Artificial Sequence: Synthetic oligonucleotide
<400> 1026
   aaagaaggag cuuaauugug aac 23
<210> 1027
   <211> 23
   <212> RNA
   <213> Artificial Sequence
<220>
   <223> Description of Artificial Sequence: Synthetic oligonucleotide
<400> 1027
   aaauaacuag aggaacaaua aaa 23
<210> 1028
   <211> 23
   <212> RNA
   <213> Artificial Sequence
<220>
   <223> Description of Artificial Sequence: Synthetic oligonucleotide
<400> 1028
   uuuuacaucg ucuaacauag caa 23
<210> 1029
   <211> 23
   <212> RNA
   <213> Artificial Sequence
<220>
   <223> Description of Artificial Sequence: Synthetic oligonucleotide
<400> 1029
   aaagucuuua agaccauguc cca 23
<210> 1030
   <211> 23
   <212> RNA
   <213> Artificial Sequence
<220>
   <223> Description of Artificial Sequence: Synthetic oligonucleotide
<400> 1030
   aucaaauaug uugaguuuuu gaa 23
<210> 1031
   <211> 23
   <212> RNA
   <213> Artificial Sequence
<220>
   <223> Description of Artificial Sequence: Synthetic oligonucleotide
<400> 1031
   uucuucuuug auuucacugg uuu 23
<210> 1032
   <211> 23
   <212> RNA
   <213> Artificial Sequence
<220>
   <223> Description of Artificial Sequence: Synthetic oligonucleotide
<400> 1032
   aaaagaagga gcuuaauugu gaa 23
<210> 1033
   <211> 23
   <212> RNA
   <213> Artificial Sequence
<220>
   <223> Description of Artificial Sequence: Synthetic oligonucleotide
<400> 1033
   uuuuuacauc gucuaacaua gca 23
<210> 1034
   <211> 23
   <212> RNA
   <213> Artificial Sequence
<220>
   <223> Description of Artificial Sequence: Synthetic oligonucleotide
<400> 1034
   aagacugauc aaauauguug agu 23
<210> 1035
   <211> 23
   <212> RNA
   <213> Artificial Sequence
<220>
   <223> Description of Artificial Sequence: Synthetic oligonucleotide
<400> 1035
   uauauguagu ucuucucagu ucc 23
<210> 1036
   <211> 23
   <212> RNA
   <213> Artificial Sequence
<220>
   <223> Description of Artificial Sequence: Synthetic oligonucleotide
<400> 1036
   aaaaagaagg agcuuaauug uga 23
<210> 1037
   <211> 23
   <212> RNA
   <213> Artificial Sequence
<220>
   <223> Description of Artificial Sequence: Synthetic oligonucleotide
<400> 1037
   aucuugauuu uggcucugga gau 23
<210> 1038
   <211> 23
   <212> RNA
   <213> Artificial Sequence
<220>
   <223> Description of Artificial Sequence: Synthetic oligonucleotide
<400> 1038
   uuggcuaaaa uuuuuacauc guc 23
<210> 1039
   <211> 23
   <212> RNA
   <213> Artificial Sequence
<220>
   <223> Description of Artificial Sequence: Synthetic oligonucleotide
<400> 1039
   uauggacaaa gucuuuaaga cca 23
<210> 1040
   <211> 23
   <212> RNA
   <213> Artificial Sequence
<220>
   <223> Description of Artificial Sequence: Synthetic oligonucleotide
<400> 1040
   aaagacugau caaauauguu gag 23
<210> 1041
   <211> 23
   <212> RNA
   <213> Artificial Sequence
<220>
   <223> Description of Artificial Sequence: Synthetic oligonucleotide
<400> 1041
   uuauauguag uucuucucag uuc 23
<210> 1042
   <211> 23
   <212> RNA
   <213> Artificial Sequence
<220>
   <223> Description of Artificial Sequence: Synthetic oligonucleotide
<400> 1042
   aaaucuugau uuuggcucug gag 23
<210> 1043
   <211> 23
   <212> RNA
   <213> Artificial Sequence
<220>
   <223> Description of Artificial Sequence: Synthetic oligonucleotide
<400> 1043
   auuggcuaaa auuuuuacau cgu 23
<210> 1044
   <211> 23
   <212> RNA
   <213> Artificial Sequence
<220>
   <223> Description of Artificial Sequence: Synthetic oligonucleotide
<400> 1044
   uuauggacaa agucuuuaag acc 23
<210> 1045
   <211> 23
   <212> RNA
   <213> Artificial Sequence
<220>
   <223> Description of Artificial Sequence: Synthetic oligonucleotide
<400> 1045
   aaaagacuga ucaaauaugu uga 23
<210> 1046
   <211> 23
   <212> RNA
   <213> Artificial Sequence
<220>
   <223> Description of Artificial Sequence: Synthetic oligonucleotide
<400> 1046
   uuuauaugua guucuucuca guu 23
<210> 1047
   <211> 23
   <212> RNA
   <213> Artificial Sequence
<220>
   <223> Description of Artificial Sequence: Synthetic oligonucleotide
<400> 1047
   auaaaaagaa ggagcuuaau ugu 23
<210> 1048
   <211> 23
   <212> RNA
   <213> Artificial Sequence
<220>
   <223> Description of Artificial Sequence: Synthetic oligonucleotide
<400> 1048
   uaacauagca aaucuugauu uug 23
<210> 1049
   <211> 23
   <212> RNA
   <213> Artificial Sequence
<220>
   <223> Description of Artificial Sequence: Synthetic oligonucleotide
<400> 1049
   uaagaccaug ucccaacuga agg 23
<210> 1050
   <211> 23
   <212> RNA
   <213> Artificial Sequence
<220>
   <223> Description of Artificial Sequence: Synthetic oligonucleotide
<400> 1050
   aauaugucau uaauuuggcc cuu 23
<210> 1051
   <211> 23
   <212> RNA
   <213> Artificial Sequence
<220>
   <223> Description of Artificial Sequence: Synthetic oligonucleotide
<400> 1051
   aaaaagacug aucaaauaug uug 23
<210> 1052
   <211> 23
   <212> RNA
   <213> Artificial Sequence
<220>
   <223> Description of Artificial Sequence: Synthetic oligonucleotide
<400> 1052
   uuguaguuua uauguaguuc uuc 23
<210> 1053
   <211> 23
   <212> RNA
   <213> Artificial Sequence
<220>
   <223> Description of Artificial Sequence: Synthetic oligonucleotide
<400> 1053
   aauaaaaaga aggagcuuaa uug 23
<210> 1054
   <211> 23
   <212> RNA
   <213> Artificial Sequence
<220>
   <223> Description of Artificial Sequence: Synthetic oligonucleotide
<400> 1054
   aucgucuaac auagcaaauc uug 23
<210> 1055
   <211> 23
   <212> RNA
   <213> Artificial Sequence
<220>
   <223> Description of Artificial Sequence: Synthetic oligonucleotide
<400> 1055
   uuaagaccau gucccaacug aag 23
<210> 1056
   <211> 23
   <212> RNA
   <213> Artificial Sequence
<220>
   <223> Description of Artificial Sequence: Synthetic oligonucleotide
<400> 1056
   aaauauguca uuaauuuggc ccu 23
<210> 1057
   <211> 23
   <212> RNA
   <213> Artificial Sequence
<220>
   <223> Description of Artificial Sequence: Synthetic oligonucleotide
<400> 1057
   uaaaaagacu gaucaaauau guu 23
<210> 1058
   <211> 23
   <212> RNA
   <213> Artificial Sequence
<220>
   <223> Description of Artificial Sequence: Synthetic oligonucleotide
<400> 1058
   uugacuugua guuuauaugu agu 23
<210> 1059
   <211> 23
   <212> RNA
   <213> Artificial Sequence
<220>
   <223> Description of Artificial Sequence: Synthetic oligonucleotide
<400> 1059
   auaacuagag gaacaauaaa aag 23
<210> 1060
   <211> 23
   <212> RNA
   <213> Artificial Sequence
<220>
   <223> Description of Artificial Sequence: Synthetic oligonucleotide
<400> 1060
   uuacaucguc uaacauagca aau 23
<210> 1061
   <211> 23
   <212> RNA
   <213> Artificial Sequence
<220>
   <223> Description of Artificial Sequence: Synthetic oligonucleotide
<400> 1061
   uuuaagacca ugucccaacu gaa 23
<210> 1062
   <211> 23
   <212> RNA
   <213> Artificial Sequence
<220>
   <223> Description of Artificial Sequence: Synthetic oligonucleotide
<400> 1062
   uuugaaauau gucauuaauu ugg 23
<210> 1063
   <211> 23
   <212> RNA
   <213> Artificial Sequence
<220>
   <223> Description of Artificial Sequence: Synthetic oligonucleotide
<400> 1063
   uagaucauaa aaagacugau caa 23
<210> 1064
   <211> 23
   <212> RNA
   <213> Artificial Sequence
<220>
   <223> Description of Artificial Sequence: Synthetic oligonucleotide
<400> 1064
   uuugacuugu aguuuauaug uag 23
<210> 1065
   <211> 23
   <212> RNA
   <213> Artificial Sequence
<220>
   <223> Description of Artificial Sequence: Synthetic oligonucleotide
<400> 1065
   aauaacuaga ggaacaauaa aaa 23
<210> 1066
   <211> 23
   <212> RNA
   <213> Artificial Sequence
<220>
   <223> Description of Artificial Sequence: Synthetic oligonucleotide
<400> 1066
   uuuacaucgu cuaacauagc aaa 23
<210> 1067
   <211> 23
   <212> RNA
   <213> Artificial Sequence
<220>
   <223> Description of Artificial Sequence: Synthetic oligonucleotide
<400> 1067
   aagucuuuaa gaccaugucc caa 23
<210> 1068
   <211> 23
   <212> RNA
   <213> Artificial Sequence
<220>
   <223> Description of Artificial Sequence: Synthetic oligonucleotide
<400> 1068
   uugaguuuuu gaaauauguc auu 23
<210> 1069
   <211> 23
   <212> RNA
   <213> Artificial Sequence
<220>
   <223> Description of Artificial Sequence: Synthetic oligonucleotide
<400> 1069
   auagaucaua aaaagacuga uca 23
<210> 1070
   <211> 23
   <212> RNA
   <213> Artificial Sequence
<220>
   <223> Description of Artificial Sequence: Synthetic oligonucleotide
<400> 1070
   uuuugacuug uaguuuauau gua 23
<210> 1071
   <211> 23
   <212> RNA
   <213> Artificial Sequence
<220>
   <223> Description of Artificial Sequence: Synthetic oligonucleotide
<400> 1071
   aaguuuugag uugaguucaa gug 23
<210> 1072
   <211> 23
   <212> RNA
   <213> Artificial Sequence
<220>
   <223> Description of Artificial Sequence: Synthetic oligonucleotide
<400> 1072
   uuucacuuuu uguugaagua gaa 23
<210> 1073
   <211> 23
   <212> RNA
   <213> Artificial Sequence
<220>
   <223> Description of Artificial Sequence: Synthetic oligonucleotide
<400> 1073
   uuaaguuagu uaguugcucu ucu 23
<210> 1074
   <211> 23
   <212> RNA
   <213> Artificial Sequence
<220>
   <223> Description of Artificial Sequence: Synthetic oligonucleotide
<400> 1074
   uuugaugcua uuaucuuguu uuu 23
<210> 1075
   <211> 23
   <212> RNA
   <213> Artificial Sequence
<220>
   <223> Description of Artificial Sequence: Synthetic oligonucleotide
<400> 1075
   auuucuuuua uuugacuaug cug 23
<210> 1076
   <211> 23
   <212> RNA
   <213> Artificial Sequence
<220>
   <223> Description of Artificial Sequence: Synthetic oligonucleotide
<400> 1076
   uuuuugacuu guaguuuaua ugu 23
<210> 1077
   <211> 23
   <212> RNA
   <213> Artificial Sequence
<220>
   <223> Description of Artificial Sequence: Synthetic oligonucleotide
<400> 1077
   uucaaguuuu gaguugaguu caa 23
<210> 1078
   <211> 23
   <212> RNA
   <213> Artificial Sequence
<220>
   <223> Description of Artificial Sequence: Synthetic oligonucleotide
<400> 1078
   auuucacuuu uuguugaagu aga 23
<210> 1079
   <211> 23
   <212> RNA
   <213> Artificial Sequence
<220>
   <223> Description of Artificial Sequence: Synthetic oligonucleotide
<400> 1079
   auuaaguuag uuaguugcuc uuc 23
<210> 1080
   <211> 23
   <212> RNA
   <213> Artificial Sequence
<220>
   <223> Description of Artificial Sequence: Synthetic oligonucleotide
<400> 1080
   aaggucuuug augcuauuau cuu 23
<210> 1081
   <211> 23
   <212> RNA
   <213> Artificial Sequence
<220>
   <223> Description of Artificial Sequence: Synthetic oligonucleotide
<400> 1081
   uauuucuuuu auuugacuau gcu 23
<210> 1082
   <211> 23
   <212> RNA
   <213> Artificial Sequence
<220>
   <223> Description of Artificial Sequence: Synthetic oligonucleotide
<400> 1082
   auuuuugacu uguaguuuau aug 23
<210> 1083
   <211> 23
   <212> RNA
   <213> Artificial Sequence
<220>
   <223> Description of Artificial Sequence: Synthetic oligonucleotide
<400> 1083
   uuucaaguuu ugaguugagu uca 23
<210> 1084
   <211> 23
   <212> RNA
   <213> Artificial Sequence
<220>
   <223> Description of Artificial Sequence: Synthetic oligonucleotide
<400> 1084
   uauuucacuu uuuguugaag uag 23
<210> 1085
   <211> 23
   <212> RNA
   <213> Artificial Sequence
<220>
   <223> Description of Artificial Sequence: Synthetic oligonucleotide
<400> 1085
   aauuaaguua guuaguugcu cuu 23
<210> 1086
   <211> 23
   <212> RNA
   <213> Artificial Sequence
<220>
   <223> Description of Artificial Sequence: Synthetic oligonucleotide
<400> 1086
   uauuugacua ugcuguuggu uua 23
<210> 1087
   <211> 23
   <212> RNA
   <213> Artificial Sequence
<220>
   <223> Description of Artificial Sequence: Synthetic oligonucleotide
<400> 1087
   uucuauuucu uuuauuugac uau 23
<210> 1088
   <211> 23
   <212> RNA
   <213> Artificial Sequence
<220>
   <223> Description of Artificial Sequence: Synthetic oligonucleotide
<400> 1088
   uuuaccucuu cauuuuugac uug 23
<210> 1089
   <211> 23
   <212> RNA
   <213> Artificial Sequence
<220>
   <223> Description of Artificial Sequence: Synthetic oligonucleotide
<400> 1089
   uucuucuagg aggcuuucaa guu 23
<210> 1090
   <211> 23
   <212> RNA
   <213> Artificial Sequence
<220>
   <223> Description of Artificial Sequence: Synthetic oligonucleotide
<400> 1090
   auauuucacu uuuuguugaa gua 23
<210> 1091
   <211> 23
   <212> RNA
   <213> Artificial Sequence
<220>
   <223> Description of Artificial Sequence: Synthetic oligonucleotide
<400> 1091
   uugaauuaag uuaguuaguu gcu 23
<210> 1092
   <211> 23
   <212> RNA
   <213> Artificial Sequence
<220>
   <223> Description of Artificial Sequence: Synthetic oligonucleotide
<400> 1092
   uuauuugacu augcuguugg uuu 23
<210> 1093
   <211> 23
   <212> RNA
   <213> Artificial Sequence
<220>
   <223> Description of Artificial Sequence: Synthetic oligonucleotide
<400> 1093
   uagagaaauu ucuguggguu cuu 23
<210> 1094
   <211> 23
   <212> RNA
   <213> Artificial Sequence
<220>
   <223> Description of Artificial Sequence: Synthetic oligonucleotide
<400> 1094
   uugaguucaa gugacauauu cuu 23
<210> 1095
   <211> 23
   <212> RNA
   <213> Artificial Sequence
<220>
   <223> Description of Artificial Sequence: Synthetic oligonucleotide
<400> 1095
   uuuucuucua ggaggcuuuc aag 23
<210> 1096
   <211> 23
   <212> RNA
   <213> Artificial Sequence
<220>
   <223> Description of Artificial Sequence: Synthetic oligonucleotide
<400> 1096
   aauauuucac uuuuuguuga agu 23
<210> 1097
   <211> 23
   <212> RNA
   <213> Artificial Sequence
<220>
   <223> Description of Artificial Sequence: Synthetic oligonucleotide
<400> 1097
   uuugaauuaa guuaguuagu ugc 23
<210> 1098
   <211> 23
   <212> RNA
   <213> Artificial Sequence
<220>
   <223> Description of Artificial Sequence: Synthetic oligonucleotide
<400> 1098
   uuuauuugac uaugcuguug guu 23
<210> 1099
   <211> 23
   <212> RNA
   <213> Artificial Sequence
<220>
   <223> Description of Artificial Sequence: Synthetic oligonucleotide
<400> 1099
   auagagaaau uucugugggu ucu 23
<210> 1100
   <211> 23
   <212> RNA
   <213> Artificial Sequence
<220>
   <223> Description of Artificial Sequence: Synthetic oligonucleotide
<400> 1100
   uugaguugag uucaagugac aua 23
<210> 1101
   <211> 23
   <212> RNA
   <213> Artificial Sequence
<220>
   <223> Description of Artificial Sequence: Synthetic oligonucleotide
<400> 1101
   uuuuucuucu aggaggcuuu caa 23
<210> 1102
   <211> 23
   <212> RNA
   <213> Artificial Sequence
<220>
   <223> Description of Artificial Sequence: Synthetic oligonucleotide
<400> 1102
   uuaguugcuc uucuaaauau uuc 23
<210> 1103
   <211> 23
   <212> RNA
   <213> Artificial Sequence
<220>
   <223> Description of Artificial Sequence: Synthetic oligonucleotide
<400> 1103
   uuuugaauua aguuaguuag uug 23
<210> 1104
   <211> 23
   <212> RNA
   <213> Artificial Sequence
<220>
   <223> Description of Artificial Sequence: Synthetic oligonucleotide
<400> 1104
   uuuuauuuga cuaugcuguu ggu 23
<210> 1105
   <211> 23
   <212> RNA
   <213> Artificial Sequence
<220>
   <223> Description of Artificial Sequence: Synthetic oligonucleotide
<400> 1105
   aagauagaga aauuucugug ggu 23
<210> 1106
   <211> 23
   <212> RNA
   <213> Artificial Sequence
<220>
   <223> Description of Artificial Sequence: Synthetic oligonucleotide
<400> 1106
   uuugaguuga guucaaguga cau 23
<210> 1107
   <211> 23
   <212> RNA
   <213> Artificial Sequence
<220>
   <223> Description of Artificial Sequence: Synthetic oligonucleotide
<400> 1107
   uagaauuuuu ucuucuagga ggc 23
<210> 1108
   <211> 23
   <212> RNA
   <213> Artificial Sequence
<220>
   <223> Description of Artificial Sequence: Synthetic oligonucleotide
<400> 1108
   uaguuaguug cucuucuaaa uau 23
<210> 1109
   <211> 23
   <212> RNA
   <213> Artificial Sequence
<220>
   <223> Description of Artificial Sequence: Synthetic oligonucleotide
<400> 1109
   auuuugaauu aaguuaguua guu 23
<210> 1110
   <211> 23
   <212> RNA
   <213> Artificial Sequence
<220>
   <223> Description of Artificial Sequence: Synthetic oligonucleotide
<400> 1110
   uucuuuuauu ugacuaugcu guu 23
<210> 1111
   <211> 23
   <212> RNA
   <213> Artificial Sequence
<220>
   <223> Description of Artificial Sequence: Synthetic oligonucleotide
<400> 1111
   auguuuuaca uuucuuauuu cau 23
<210> 1112
   <211> 23
   <212> RNA
   <213> Artificial Sequence
<220>
   <223> Description of Artificial Sequence: Synthetic oligonucleotide
<400> 1112
   uuuugaguug aguucaagug aca 23
<210> 1113
   <211> 23
   <212> RNA
   <213> Artificial Sequence
<220>
   <223> Description of Artificial Sequence: Synthetic oligonucleotide
<400> 1113
   uucacuuuuu guugaaguag aau 23
<210> 1114
   <211> 23
   <212> RNA
   <213> Artificial Sequence
<220>
   <223> Description of Artificial Sequence: Synthetic oligonucleotide
<400> 1114
   uuaguuaguu gcucuucuaa aua 23
<210> 1115
   <211> 23
   <212> RNA
   <213> Artificial Sequence
<220>
   <223> Description of Artificial Sequence: Synthetic oligonucleotide
<400> 1115
   uugaugcuau uaucuuguuu uuc 23
<210> 1116
   <211> 23
   <212> RNA
   <213> Artificial Sequence
<220>
   <223> Description of Artificial Sequence: Synthetic oligonucleotide
<400> 1116
   uuucuuuuau uugacuaugc ugu 23
<210> 1117
   <211> 23
   <212> RNA
   <213> Artificial Sequence
<220>
   <223> Description of Artificial Sequence: Synthetic oligonucleotide
<400> 1117
   aacuugagag uugcuggguc uga 23
<210> 1118
   <211> 23
   <212> RNA
   <213> Artificial Sequence
<220>
   <223> Description of Artificial Sequence: Synthetic oligonucleotide
<400> 1118
   uugaauuaau guccauggac uac 23
<210> 1119
   <211> 23
   <212> RNA
   <213> Artificial Sequence
<220>
   <223> Description of Artificial Sequence: Synthetic oligonucleotide
<400> 1119
   auugaaguuu ugugauccau cua 23
<210> 1120
   <211> 23
   <212> RNA
   <213> Artificial Sequence
<220>
   <223> Description of Artificial Sequence: Synthetic oligonucleotide
<400> 1120
   uauggaguau aucuucucua ggc 23
<210> 1121
   <211> 23
   <212> RNA
   <213> Artificial Sequence
<220>
   <223> Description of Artificial Sequence: Synthetic oligonucleotide
<400> 1121
   aauauaaugu uuguugucuu ucc 23
<210> 1122
   <211> 23
   <212> RNA
   <213> Artificial Sequence
<220>
   <223> Description of Artificial Sequence: Synthetic oligonucleotide
<400> 1122
   aaaagaauau ucaauauaau guu 23
<210> 1123
   <211> 23
   <212> RNA
   <213> Artificial Sequence
<220>
   <223> Description of Artificial Sequence: Synthetic oligonucleotide
<400> 1123
   aaacuugaga guugcugggu cug 23
<210> 1124
   <211> 23
   <212> RNA
   <213> Artificial Sequence
<220>
   <223> Description of Artificial Sequence: Synthetic oligonucleotide
<400> 1124
   uucauugaag uuuugugauc cau 23
<210> 1125
   <211> 23
   <212> RNA
   <213> Artificial Sequence
<220>
   <223> Description of Artificial Sequence: Synthetic oligonucleotide
<400> 1125
   uucacuaugg aguauaucuu cuc 23
<210> 1126
   <211> 23
   <212> RNA
   <213> Artificial Sequence
<220>
   <223> Description of Artificial Sequence: Synthetic oligonucleotide
<400> 1126
   uucaauauaa uguuuguugu cuu 23
<210> 1127
   <211> 23
   <212> RNA
   <213> Artificial Sequence
<220>
   <223> Description of Artificial Sequence: Synthetic oligonucleotide
<400> 1127
   uaguugguuu cgugauuucc caa 23
<210> 1128
   <211> 23
   <212> RNA
   <213> Artificial Sequence
<220>
   <223> Description of Artificial Sequence: Synthetic oligonucleotide
<400> 1128
   aaaacuugag aguugcuggg ucu 23
<210> 1129
   <211> 23
   <212> RNA
   <213> Artificial Sequence
<220>
   <223> Description of Artificial Sequence: Synthetic oligonucleotide
<400> 1129
   uucgauguug aauuaauguc cau 23
<210> 1130
   <211> 23
   <212> RNA
   <213> Artificial Sequence
<220>
   <223> Description of Artificial Sequence: Synthetic oligonucleotide
<400> 1130
   uuucauugaa guuuugugau cca 23
<210> 1131
   <211> 23
   <212> RNA
   <213> Artificial Sequence
<220>
   <223> Description of Artificial Sequence: Synthetic oligonucleotide
<400> 1131
   uagauugcuu cacuauggag uau 23
<210> 1132
   <211> 23
   <212> RNA
   <213> Artificial Sequence
<220>
   <223> Description of Artificial Sequence: Synthetic oligonucleotide
<400> 1132
   auucaauaua auguuuguug ucu 23
<210> 1133
   <211> 23
   <212> RNA
   <213> Artificial Sequence
<220>
   <223> Description of Artificial Sequence: Synthetic oligonucleotide
<400> 1133
   auaguugguu ucgugauuuc cca 23
<210> 1134
   <211> 23
   <212> RNA
   <213> Artificial Sequence
<220>
   <223> Description of Artificial Sequence: Synthetic oligonucleotide
<400> 1134
   aaaaacuuga gaguugcugg guc 23
<210> 1135
   <211> 23
   <212> RNA
   <213> Artificial Sequence
<220>
   <223> Description of Artificial Sequence: Synthetic oligonucleotide
<400> 1135
   auucgauguu gaauuaaugu cca 23
<210> 1136
   <211> 23
   <212> RNA
   <213> Artificial Sequence
<220>
   <223> Description of Artificial Sequence: Synthetic oligonucleotide
<400> 1136
   uauuuguagu ucucccacgu uuc 23
<210> 1137
   <211> 23
   <212> RNA
   <213> Artificial Sequence
<220>
   <223> Description of Artificial Sequence: Synthetic oligonucleotide
<400> 1137
   uuagauugcu ucacuaugga gua 23
<210> 1138
   <211> 23
   <212> RNA
   <213> Artificial Sequence
<220>
   <223> Description of Artificial Sequence: Synthetic oligonucleotide
<400> 1138
   uauucaauau aauguuuguu guc 23
<210> 1139
   <211> 23
   <212> RNA
   <213> Artificial Sequence
<220>
   <223> Description of Artificial Sequence: Synthetic oligonucleotide
<400> 1139
   uauaguuggu uucgugauuu ccc 23
<210> 1140
   <211> 23
   <212> RNA
   <213> Artificial Sequence
<220>
   <223> Description of Artificial Sequence: Synthetic oligonucleotide
<400> 1140
   uagacaugaa aaacuugaga guu 23
<210> 1141
   <211> 23
   <212> RNA
   <213> Artificial Sequence
<220>
   <223> Description of Artificial Sequence: Synthetic oligonucleotide
<400> 1141
   uauucgaugu ugaauuaaug ucc 23
<210> 1142
   <211> 23
   <212> RNA
   <213> Artificial Sequence
<220>
   <223> Description of Artificial Sequence: Synthetic oligonucleotide
<400> 1142
   aaccauauuu guaguucucc cac 23
<210> 1143
   <211> 23
   <212> RNA
   <213> Artificial Sequence
<220>
   <223> Description of Artificial Sequence: Synthetic oligonucleotide
<400> 1143
   auuagauugc uucacuaugg agu 23
<210> 1144
   <211> 23
   <212> RNA
   <213> Artificial Sequence
<220>
   <223> Description of Artificial Sequence: Synthetic oligonucleotide
<400> 1144
   auauucaaua uaauguuugu ugu 23
<210> 1145
   <211> 23
   <212> RNA
   <213> Artificial Sequence
<220>
   <223> Description of Artificial Sequence: Synthetic oligonucleotide
<400> 1145
   auugcauugg ggacauugcc agu 23
<210> 1146
   <211> 23
   <212> RNA
   <213> Artificial Sequence
<220>
   <223> Description of Artificial Sequence: Synthetic oligonucleotide
<400> 1146
   uaauguccau ggacuaccug aua 23
<210> 1147
   <211> 23
   <212> RNA
   <213> Artificial Sequence
<220>
   <223> Description of Artificial Sequence: Synthetic oligonucleotide
<400> 1147
   aucuauucga uguugaauua aug 23
<210> 1148
   <211> 23
   <212> RNA
   <213> Artificial Sequence
<220>
   <223> Description of Artificial Sequence: Synthetic oligonucleotide
<400> 1148
   aaaccauauu uguaguucuc cca 23
<210> 1149
   <211> 23
   <212> RNA
   <213> Artificial Sequence
<220>
   <223> Description of Artificial Sequence: Synthetic oligonucleotide
<400> 1149
   aauuagauug cuucacuaug gag 23
<210> 1150
   <211> 23
   <212> RNA
   <213> Artificial Sequence
<220>
   <223> Description of Artificial Sequence: Synthetic oligonucleotide
<400> 1150
   aauauucaau auaauguuug uug 23
<210> 1151
   <211> 23
   <212> RNA
   <213> Artificial Sequence
<220>
   <223> Description of Artificial Sequence: Synthetic oligonucleotide
<400> 1151
   uuuguuuucc gggauugcau ugg 23
<210> 1152
   <211> 23
   <212> RNA
   <213> Artificial Sequence
<220>
   <223> Description of Artificial Sequence: Synthetic oligonucleotide
<400> 1152
   uuaaugucca uggacuaccu gau 23
<210> 1153
   <211> 23
   <212> RNA
   <213> Artificial Sequence
<220>
   <223> Description of Artificial Sequence: Synthetic oligonucleotide
<400> 1153
   auccaucuau ucgauguuga auu 23
<210> 1154
   <211> 23
   <212> RNA
   <213> Artificial Sequence
<220>
   <223> Description of Artificial Sequence: Synthetic oligonucleotide
<400> 1154
   uauaucuucu cuaggcccaa cca 23
<210> 1155
   <211> 23
   <212> RNA
   <213> Artificial Sequence
<220>
   <223> Description of Artificial Sequence: Synthetic oligonucleotide
<400> 1155
   uaauuagauu gcuucacuau gga 23
<210> 1156
   <211> 23
   <212> RNA
   <213> Artificial Sequence
<220>
   <223> Description of Artificial Sequence: Synthetic oligonucleotide
<400> 1156
   aagaauauuc aauauaaugu uug 23
<210> 1157
   <211> 23
   <212> RNA
   <213> Artificial Sequence
<220>
   <223> Description of Artificial Sequence: Synthetic oligonucleotide
<400> 1157
   aucuuuguuu uccgggauug cau 23
<210> 1158
   <211> 23
   <212> RNA
   <213> Artificial Sequence
<220>
   <223> Description of Artificial Sequence: Synthetic oligonucleotide
<400> 1158
   aauuaauguc cauggacuac cug 23
<210> 1159
   <211> 23
   <212> RNA
   <213> Artificial Sequence
<220>
   <223> Description of Artificial Sequence: Synthetic oligonucleotide
<400> 1159
   uuugugaucc aucuauucga ugu 23
<210> 1160
   <211> 23
   <212> RNA
   <213> Artificial Sequence
<220>
   <223> Description of Artificial Sequence: Synthetic oligonucleotide
<400> 1160
   auggaguaua ucuucucuag gcc 23
<210> 1161
   <211> 23
   <212> RNA
   <213> Artificial Sequence
<220>
   <223> Description of Artificial Sequence: Synthetic oligonucleotide
<400> 1161
   uugucuuucc agucuuccaa cuc 23
<210> 1162
   <211> 23
   <212> RNA
   <213> Artificial Sequence
<220>
   <223> Description of Artificial Sequence: Synthetic oligonucleotide
<400> 1162
   aaagaauauu caauauaaug uuu 23
<210> 1163
   <211> 23
   <212> RNA
   <213> Artificial Sequence
<220>
   <223> Description of Artificial Sequence: Synthetic oligonucleotide
<400> 1163
   aaucuuuguu uuccgggauu gca 23
<210> 1164
   <211> 23
   <212> RNA
   <213> Artificial Sequence
<220>
   <223> Description of Artificial Sequence: Synthetic oligonucleotide
<400> 1164
   uugcuuugug aucccaagua gaa 23
<210> 1165
   <211> 23
   <212> RNA
   <213> Artificial Sequence
<220>
   <223> Description of Artificial Sequence: Synthetic oligonucleotide
<400> 1165
   uauauuuacc auuuagguug uuu 23
<210> 1166
   <211> 23
   <212> RNA
   <213> Artificial Sequence
<220>
   <223> Description of Artificial Sequence: Synthetic oligonucleotide
<400> 1166
   uucugaaucu guuggaugga uca 23
<210> 1167
   <211> 23
   <212> RNA
   <213> Artificial Sequence
<220>
   <223> Description of Artificial Sequence: Synthetic oligonucleotide
<400> 1167
   ucuuuuaaau uugccucagu uca 23
<210> 1168
   <211> 23
   <212> RNA
   <213> Artificial Sequence
<220>
   <223> Description of Artificial Sequence: Synthetic oligonucleotide
<400> 1168
   acagauuuuu acacauacuc ugu 23
<210> 1169
   <211> 23
   <212> RNA
   <213> Artificial Sequence
<220>
   <223> Description of Artificial Sequence: Synthetic oligonucleotide
<400> 1169
   aaaucuuugu uuuccgggau ugc 23
<210> 1170
   <211> 23
   <212> RNA
   <213> Artificial Sequence
<220>
   <223> Description of Artificial Sequence: Synthetic oligonucleotide
<400> 1170
   uuugcuuugu gaucccaagu aga 23
<210> 1171
   <211> 23
   <212> RNA
   <213> Artificial Sequence
<220>
   <223> Description of Artificial Sequence: Synthetic oligonucleotide
<400> 1171
   uuauauuuac cauuuagguu guu 23
<210> 1172
   <211> 23
   <212> RNA
   <213> Artificial Sequence
<220>
   <223> Description of Artificial Sequence: Synthetic oligonucleotide
<400> 1172
   aauuugccuc aguucauuca aag 23
<210> 1173
   <211> 23
   <212> RNA
   <213> Artificial Sequence
<220>
   <223> Description of Artificial Sequence: Synthetic oligonucleotide
<400> 1173
   uccuuuuaaa uuugccucag uuc 23
<210> 1174
   <211> 23
   <212> RNA
   <213> Artificial Sequence
<220>
   <223> Description of Artificial Sequence: Synthetic oligonucleotide
<400> 1174
   uacagauuuu uacacauacu cug 23
<210> 1175
   <211> 23
   <212> RNA
   <213> Artificial Sequence
<220>
   <223> Description of Artificial Sequence: Synthetic oligonucleotide
<400> 1175
   uuuugcuuug ugaucccaag uag 23
<210> 1176
   <211> 23
   <212> RNA
   <213> Artificial Sequence
<220>
   <223> Description of Artificial Sequence: Synthetic oligonucleotide
<400> 1176
   uugguuuguu auauuuacca uuu 23
<210> 1177
   <211> 23
   <212> RNA
   <213> Artificial Sequence
<220>
   <223> Description of Artificial Sequence: Synthetic oligonucleotide
<400> 1177
   aaauuugccu caguucauuc aaa 23
<210> 1178
   <211> 23
   <212> RNA
   <213> Artificial Sequence
<220>
   <223> Description of Artificial Sequence: Synthetic oligonucleotide
<400> 1178
   ugccuuuuaa auuugccuca guu 23
<210> 1179
   <211> 23
   <212> RNA
   <213> Artificial Sequence
<220>
   <223> Description of Artificial Sequence: Synthetic oligonucleotide
<400> 1179
   uuacagauuu uuacacauac ucu 23
<210> 1180
   <211> 23
   <212> RNA
   <213> Artificial Sequence
<220>
   <223> Description of Artificial Sequence: Synthetic oligonucleotide
<400> 1180
   aaacaccaaa ucuuuguuuu ccg 23
<210> 1181
   <211> 23
   <212> RNA
   <213> Artificial Sequence
<220>
   <223> Description of Artificial Sequence: Synthetic oligonucleotide
<400> 1181
   uuagguuguu uucuccacac uca 23
<210> 1182
   <211> 23
   <212> RNA
   <213> Artificial Sequence
<220>
   <223> Description of Artificial Sequence: Synthetic oligonucleotide
<400> 1182
   uauaaccuuc cauuuugaga cuu 23
<210> 1183
   <211> 23
   <212> RNA
   <213> Artificial Sequence
<220>
   <223> Description of Artificial Sequence: Synthetic oligonucleotide
<400> 1183
   uaaauuugcc ucaguucauu caa 23
<210> 1184
   <211> 23
   <212> RNA
   <213> Artificial Sequence
<220>
   <223> Description of Artificial Sequence: Synthetic oligonucleotide
<400> 1184
   uugccuuuua aauuugccuc agu 23
<210> 1185
   <211> 23
   <212> RNA
   <213> Artificial Sequence
<220>
   <223> Description of Artificial Sequence: Synthetic oligonucleotide
<400> 1185
   auuacagauu uuuacacaua cuc 23
<210> 1186
   <211> 23
   <212> RNA
   <213> Artificial Sequence
<220>
   <223> Description of Artificial Sequence: Synthetic oligonucleotide
<400> 1186
   aaaacaccaa aucuuuguuu ucc 23
<210> 1187
   <211> 23
   <212> RNA
   <213> Artificial Sequence
<220>
   <223> Description of Artificial Sequence: Synthetic oligonucleotide
<400> 1187
   uuuagguugu uuucuccaca cuc 23
<210> 1188
   <211> 23
   <212> RNA
   <213> Artificial Sequence
<220>
   <223> Description of Artificial Sequence: Synthetic oligonucleotide
<400> 1188
   uuauagagua uaaccuucca uuu 23
<210> 1189
   <211> 23
   <212> RNA
   <213> Artificial Sequence
<220>
   <223> Description of Artificial Sequence: Synthetic oligonucleotide
<400> 1189
   uuaaauuugc cucaguucau uca 23
<210> 1190
   <211> 23
   <212> RNA
   <213> Artificial Sequence
<220>
   <223> Description of Artificial Sequence: Synthetic oligonucleotide
<400> 1190
   auugccuuuu aaauuugccu cag 23
<210> 1191
   <211> 23
   <212> RNA
   <213> Artificial Sequence
<220>
   <223> Description of Artificial Sequence: Synthetic oligonucleotide
<400> 1191
   uauuacagau uuuuacacau acu 23
<210> 1192
   <211> 23
   <212> RNA
   <213> Artificial Sequence
<220>
   <223> Description of Artificial Sequence: Synthetic oligonucleotide
<400> 1192
   uagaaaacac caaaucuuug uuu 23
<210> 1193
   <211> 23
   <212> RNA
   <213> Artificial Sequence
<220>
   <223> Description of Artificial Sequence: Synthetic oligonucleotide
<400> 1193
   auuuagguug uuuucuccac acu 23
<210> 1194
   <211> 23
   <212> RNA
   <213> Artificial Sequence
<220>
   <223> Description of Artificial Sequence: Synthetic oligonucleotide
<400> 1194
   uuuauagagu auaaccuucc auu 23
<210> 1195
   <211> 23
   <212> RNA
   <213> Artificial Sequence
<220>
   <223> Description of Artificial Sequence: Synthetic oligonucleotide
<400> 1195
   uuuaaauuug ccucaguuca uuc 23
<210> 1196
   <211> 23
   <212> RNA
   <213> Artificial Sequence
<220>
   <223> Description of Artificial Sequence: Synthetic oligonucleotide
<400> 1196
   uauugccuuu uaaauuugcc uca 23
<210> 1197
   <211> 23
   <212> RNA
   <213> Artificial Sequence
<220>
   <223> Description of Artificial Sequence: Synthetic oligonucleotide
<400> 1197
   aaguagaaaa caccaaaucu uug 23
<210> 1198
   <211> 23
   <212> RNA
   <213> Artificial Sequence
<220>
   <223> Description of Artificial Sequence: Synthetic oligonucleotide
<400> 1198
   uauuuaccau uuagguuguu uuc 23
<210> 1199
   <211> 23
   <212> RNA
   <213> Artificial Sequence
<220>
   <223> Description of Artificial Sequence: Synthetic oligonucleotide
<400> 1199
   uugguugauu uuauagagua uaa 23
<210> 1200
   <211> 23
   <212> RNA
   <213> Artificial Sequence
<220>
   <223> Description of Artificial Sequence: Synthetic oligonucleotide
<400> 1200
   uuuuaaauuu gccucaguuc auu 23
<210> 1201
   <211> 23
   <212> RNA
   <213> Artificial Sequence
<220>
   <223> Description of Artificial Sequence: Synthetic oligonucleotide
<400> 1201
   uuauugccuu uuaaauuugc cuc 23
<210> 1202
   <211> 23
   <212> RNA
   <213> Artificial Sequence
<220>
   <223> Description of Artificial Sequence: Synthetic oligonucleotide
<400> 1202
   uuugugaucc caaguagaaa aca 23
<210> 1203
   <211> 23
   <212> RNA
   <213> Artificial Sequence
<220>
   <223> Description of Artificial Sequence: Synthetic oligonucleotide
<400> 1203
   auauuuacca uuuagguugu uuu 23
<210> 1204
   <211> 23
   <212> RNA
   <213> Artificial Sequence
<220>
   <223> Description of Artificial Sequence: Synthetic oligonucleotide
<400> 1204
   uuugguugau uuuauagagu aua 23
<210> 1205
   <211> 23
   <212> RNA
   <213> Artificial Sequence
<220>
   <223> Description of Artificial Sequence: Synthetic oligonucleotide
<400> 1205
   uuuuuaaauu ugccucaguu cau 23
<210> 1206
   <211> 23
   <212> RNA
   <213> Artificial Sequence
<220>
   <223> Description of Artificial Sequence: Synthetic oligonucleotide
<400> 1206
   aagauuuuua cacauacucu gug 23
<210> 1207
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 1207
   cuuacgcuga guacuucgat t 21
<210> 1208
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> see specification as filed for detailed description of substitutions and preferred embodiments
<400> 1208
   ucgaaguacu cagcguaagt t 21